# EUROPÄISCHE PATENTSCHRIFT

(11) **EP 1 102 775 B1**
(45) Veröffentlichungstag und Bekanntmachung des Hinweises auf die Patenterteilung: **08.06.2005**
(21) Anmeldenummer: 99942816.2
(22) Anmeldetag: 06.08.1999
(51) Int. Cl.: C07F 9/10, A61K 31/685, A61K 9/127, C07F 9/113

(54) **PHOSPHOLIPIDE MIT UNGESATTIGTEN ALKYL- UND ACYLKETTEN**
NOVEL PHOSPHOLIPIDS WITH UNSATURATED ALKYL AND ACYL CHAINS
PHOSPHOLIPIDES RENFERMANT DES CHAINES ALKYLE ET ACYLE NON SATUREES

(30) Priorität: 06.08.1998 DE 19835611
(43) Veröffentlichungstag der Anmeldung: 30.05.2001
(73) Patentinhaber: Max-Planck-Gesellschaft zur Förderung der Wissenschaften e.V., 80539 München (DE)
(72) Erfinder: EIBL, Hansjörg, D-37120 Bovenden-Eddigehausen (DE); HOTTKOWITZ, Thomas, D-67435 Neustadt an der Weinstrasse (DE)
(74) Vertreter: Huber, Bernhard, Dipl.-Chem.
(86) Internationale Anmeldenummer: PCT/EP1999/005710
(87) Internationale Veröffentlichungsnummer: WO 2000/008031

(56) Entgegenhaltungen:
- EP-A- 0 507 337
- EP-A- 0 534 445
- WO-A-97/30058
- WO-A-99/09037
- DE-A- 4 013 632

## Beschreibung

Die Erfindung betrifft phospholipidartige Verbindungen der Formel (I) mit definierten apolaren Bestandteilen, sowie ein Verfahren zu deren Herstellung. Die Erfindung betrifft außerdem die Verwendung der phospholipidartigen Verbindungen als Liposomen, Wirkstoffe und Lösungsvermittler.

Phospholipidartige Verbindungen besitzen vielfache Verwendungsmöglichkeiten, z.B. als Liposomenbestandteile zum Transport von Arzneimitteln oder als Gentransportvehikel, als Lösungsvermittler für im Wasser schlecht lösliche Arzneimittel und selbst als Wirkstoffe gegen Erkrankungen wie etwa Krebs oder Leishmaniose.

Phospholipidartige Verbindungen dieser Art bestehen aus einem polaren und einem apolaren Teil. Glycerophospholipide enthalten als wesentlichen Bestandteil das Glycerin, welches in sn-1- und sn-2-Position überwiegend mit Fettsäuren verestert ist (apolarer Teil). Ist mindestens eine der beiden OH-Gruppen am Glyceringerüst mit einem Alkohol verethert, spricht man von Etherphospholipiden. Die Polarität der erfindungsgemäßen Verbindungen rührt von der negativ geladenen Phosphatgruppe und der veresterten Alkoholkomponente, die einen quartären, positiv geladenen Stickstoff enthält. Diese Gruppe kann einfach oder mehrfach oder auch gar nicht vorhanden sein, wobei sich jeweils eine negative oder positive Überschußladung oder auch keine Ladung ergibt.

Der apolare Anteil wird durch Alkyl- bzw. Acylketten gebildet, die in gesättigter oder ungesättigter Form vorliegen können. Die Variationsmöglichkeiten bei der Synthese des apolaren Bereichs waren bisher auf in der Natur vorkommende Acylreste oder Alkylketten begrenzt. Durch gezielte Modifikationen des apolaren Bereiches lassen sich die physikalischen, biochemischen und biologischen Eigenschaften der Phospholipidverbindungen deutlich verändern und gezielt steuern.

Liposomen als Transportvehikel oder Arzneimittelträger sind bekannt. Häufig verwendete Phosphatidylcholine, wie 1,2-Dipalmitoyl-*sn*-glycero-3-phosphocholin (DPPC), 1,2-Distearoyl-*sn*-glycero-3-phosphocholin (DSPC) oder 1,2-Dioleyl-*sn*-glycero-3-phosphocholin (DOPC) bilden mit Cholesterin im Verhältnis 60:40 bei Beschallung Liposomen in der Größenordnung von 60 nm. Oft kann es jedoch von Vorteil sein, Liposomen mit einem größeren Innenvolumen herzustellen, da mit diesen größere Mengen an Wirkstoffen transportiert werden können. Hier besteht jedoch das Problem, daß man für die Herstellung von Liposomen mit einer Größe von über 100 nm Durchmesser Verfahrenstechniken wie etwa die Extrusion benötigt, die mit deutlichen Nachteilen behaftet ist, z.B. durch die Brüchigkeit der Polycarbonatmembranen oder das Verstopfen der Poren. Dies erschwert vor allem die Präparation größerer Ansätze für pharmazeutische Zwecke. Indem man die Alkyl- bzw. Acylketten des apolaren Teils verlängert, kann man bei der Vesikelbildung aufgrund sterischer Faktoren eine Anordnung der Moleküle mit einer niedrigeren Krümmung erreichen. Die Folge ist die Bildung von größeren Liposomen, die durch Ultraschallbehandlung ohne Extrusionverfahren erreicht werden kann. Um die Phasenumwandlungstemperatur von Phospholipiden mit extrem langen Fettsäuren (mit mehr als 22 C-Atomen) in einem für die Liposomenbildung günstigen Bereich zu halten, werden Fettsäuren mit möglichst mittig liegender Cis-Doppelbindung verwendet. Solche extrem langkettigen Fettsäuren kommen in der Natur nur in kleinen Mengen vor.

Phospholipidverbindungen können auch direkt als pharmazeutische Wirkstoffe eingesetzt werden. Die antineoplastische und immunmodulatorische Wirkung von Lysolecithinen (die am Glycerin nur eine statt zwei Fettsäuren aufweisen) und Etherlysolecithinen in Zellkulturexperimenten ist bereits seit über 30 Jahren bekannt. Grundvoraussetzung für die antineoplatische Aktivität von Lysophospholipiden und Analoga ist eine Anreicherung im erkrankten Gewebe. Lysophosphatidylcholine werdendurch Phospholipasen oder Acyltransferasen leicht metabolisiert und stehen dem Organismus nicht mehr zur Verfügung, während Etherlysolecithine durch oxidative Spaltung der Etherbindung oder Acylierung der *sn*-2-Position entgiftet werden können. Daher wurden Substanzen synthetisiert, die weniger gute Substrate für Phospholipid-metabolisierende Enzyme darstellen, aber trotzdem eine Lysolecithin ähnliche Struktur besitzen. Mit dem Etherlipid 1-O-Octadecyl-2-O-methyl-rac-giycero-3-phosphocholin (ET18-OCH₃, auch bekannt als Edelfosin) wurde zum erstenmal ein Phosphocholin mit antitumoraler Wirksamkeit gefunden. ET18-OCH₃ zeigt in Zellkulturexperimenten hervorragende antineoplastische Aktivität, stellte sich in komplexen Organismen aber als nahezu unwirksam heraus.

Durch den Verzicht auf den Glyceringrundkörper erhielt man die metabolisch stabileren Alkylphosphocholine (APC), Substanzen, die sich in Membranen anreichern und Zelleigenschaften merklich beeinflussen. Die nicht in der Natur vorkommenden Alkylphosphocholine sind Phosphocholinester langkettiger Alkohole, die aufgrund ihrer vereinfachten Struktur nur noch Substrateigenschaften für Phospholipase D besitzen. Der bisher bekannteste Vertreter dieser Substanzklasse ist Hexadecylphosphocholin (HePC), ein bereits 1992 als Medikament unter dem Namen Miltex® (Wirkstoff: Miltefosin) zugelassenes und daher auch intensiv untersuchtes Alkylphosphocholin. HePC wird zur topischen Behandlung von kutan metastasierenden Mammakarzinomen und Lymphomen eingesetzt. Neben der Tumorreduktion aktivieren Alkylphosphocholine cytotoxische Makrophagen und inhibieren die Invasion neoplastischer Zellen in gesundes Gewebe. Neueren Untersuchungen nach sind APCs (und vor allem HePC) potente Wirkstoffe im Kampf gegen Leishmaniose und Trypanosomiasis. Die direkte intravenöse Gabe einer HePC-Lösung verursacht in Ratten Thrombophlebitis. HePC zeigt in klinischen Studien bei oraler Gabe Toxizitäten im Gastrointestinaltrankt und kann daher nicht in wirksamen Konzentrationen verabreicht werden.

Eine Ausnahme ist HePC zur Bekämpfung der Leishmaniose: HePC wirkt in so geringen Dosen, daß die oben beschriebenen Nebenwirkungen nicht auftreten.

Mit Erucylphosphocholin (ErPC), einem Phosphocholin mit C₂₂-Alkylkette und Cis-Doppelbindung in ω-9-Position, wurde erstmals ein intravenös injizierbares Alkylphosphocholin gefunden. Es stellte sich heraus, daß Strukturvariationen im apolaren Bereich von ungesättigten und somit intravenös applizierbaren Alkylphosphocholinen zu einer im Verhältnis zum Erucylphosphocholin, der bisher wirksamsten Verbindung, verbesserten antitumoralen Wirksamkeitführen, z.B. bei Verschiebung der Doppelbindung in die *ω*-12- bzw. ω-6-Position (siehe Tabelle 2 in Beispiel 5).

Weiterhin finden Phospholipide Anwendung als Lösungsvermittler für in Wasser schlecht lösliche Arzneimittel. Auch hier können die Lösevermittlungseigenschaften durch die Modifizierung des apolaren Bereiches verbessert werden.

Bisher war es bei der Synthese von Phospholipiden der oben genannten Klassen nur möglich, den polaren Teil gezielt zu modifizieren. Für den apolaren Anteil konnten bisher nur gewerblich erhältliche Fettsäuren und in der Natur vorkommende Fettsäuren verwendet werden.

In der Natur und speziell in Säugetieren vorkommende Phospholipide tragen überwiegend unverzweigte Fettsäuren mit 8 bis 24 C-Atomen, die aufgrund ihrer Biosynthese fast ausschließlich eine gerade Anzahl an Kohlenstoffatomen aufweisen. Ungesättigte Fettsäuren tragen,meist 1 bis 4 Doppelbindungen, die vorwiegend in Cis-Konfiguration vorliegen. Natürlich vorkommende einfach ungesättigte Fettsäuren tragen die Doppelbindung meist mittig, d.h. sie liegt bei der Palmitoleinsäure an der ω-7-Position oder an der (Z)-9-Position der hierin in den Beispielen verwendeten und bevorzugten Schreibweise. Die höheren Fettsäuren Olein-, Eicosen-, Eruca- und Nervonsäure haben die Doppelbindung jeweils an der ω-9-Position, der Kohlenstoffkette bzw. entsprechend an der (Z)-9-, (Z)-11-, (Z)-13- und (Z)-15-Position in der hierin bevorzugten Schreibweise.

Bei mehrfach ungesättigten Fettsäuren sind die Positionen der Unsättigungen dergestalt, daß jeweils nur eine CH₂-Gruppe zwischen ihnen liegt. Dies ist wichtig, um die Autoxidation der Fettsäuren zu erlauben. Gerade bei der Verwendung von Phospholipiden als Arzneimittel oder Liposomen wäre es aber von Vorteil, die Autoxidation zu verhindern, um stabilere Verbindungen zu erhalten. Dies kann nur durch Verbindungen erreicht werden, bei denen die Unsättigungen in den Alkyl- bzw. Acylketten mehr als eine Methylengruppe auseinander liegen.

Die deutsche Patentanmeldung DE 197 35 776.8 offenbart phospholipidanaloge Verbindungen als Liposomenbestandteile, pharmazeutische Wirkstoffe oder Lösungsvermittler, die gesättigte oder einfach ungesättigte Acyloder Alkylreste enthalten, wobei die Summe der Kohlenstoffatome in Acyl und Alkyl zwischen 16 und 44 liegt.

DE 40 13 632 offenbart Liposomen mit positiver Überschußladung, die ebenfalls auf Phospholipid-analogen Verbindungen beruhen und die ebenfalls Alkylreste mit 14 bis 18 C-Atomen, insbesondere Oleyl oder Oleoyl aufweisen können. Auch WO 97/30058 offenbart Phosphatidyloligoglycerine zur Herstellung von Liposomen. Auch hier weisen die Verbindungen am Glycerinbestandteil Alkylreste auf, die gesättigte oder ungesättigte C₁-C₂₄-Alkyl- oder Acylreste sein können. EP 0 507 337 offenbart Phospholipide, die einen Erucyl-, Brasidyl- oder Nervonylrest aufweisen. EP 0 534 445 offenbart ein Arzneimittel zur Behandlung von Protozoenerkrankungen auf der Basis von Phospholipiden ohne Glyceringrundgerüst und mit einem einfach oder mehrfach ungesättigten Kohlenwasserstoffrest mit 12 bis 20 C-Atomen. Keines der vorgenannten Dokumente offenbart Verbindungen der vorliegenden Erfindung mit gesättigten oder ungesättigten Acyl- oder Alkylresten, die ihre Doppelbindung nicht an einer natürlichen Position tragen.

Aufgabe der vorliegenden Erfindung war daher, Verbindungen bereitzustellen, die durch Modifikationen im apolaren Bereich für die zuvor genannten Anwendungen verbesserte Eigenschaften aufweisen und zusätzlich großtechnisch herzustellen sind. Weiterhin war es eine Aufgabe der vorliegenden Erfindung, durch ein neues Verfahren die Möglichkeit zu eröffnen, ungesättigte Fettsäuren herzustellen, bei denen die Doppelbindungen an Positionen liegen, die bei natürlich vorkommenden einfach und zweifach ungesättigten Fettsäuren nicht vorkommen, oder ein Verfahren zur Verfügung zu stellen, das die Herstellung schwer zugänglicher monoungesätigter Fettsäuren, z. B. der Nervonsäure, in technischen Mengen erlaubt.

Gelöst wird diese Aufgabe erfindungsgemäß durch eine Verbindung der allgemeinen Formel (I)

(I) A - PO₃⁻ - B

worin B einen Rest der allgemeinen Formel (II) darstellt 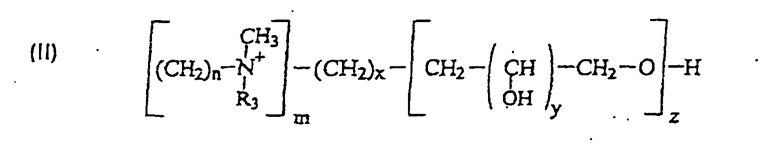 worin
- n: eine ganze Zahl von 2 bis 8 ist;
- m: 0, 1 oder 2 ist;
- x: eine ganze Zahl von 0 bis 8 ist;
- y: eine ganze Zahl von 1 bis 4 ist;
- z: eine ganze Zahl von 0 bis 5 ist;
- R₃: einen Alkylrest mit 1 bis 3 C-Atomen darstellt, der mit einer oder mehreren Hydroxylgruppen substituiert sein kann;
und worin A einen Rest, ausgewählt aus einer der Formeln (III) bis ( VII) darstellt: 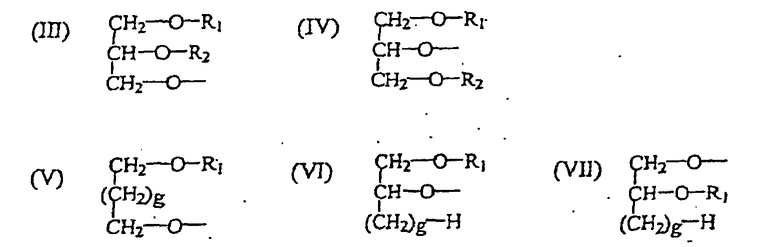 worin
- g: eine ganze Zahl von 0 bis 8 ist;
p, q, r, s ≥ 0;
12 ≤ p + q ≤ 30 und
8 ≤ s + t + r ≤ 26 ist;
wobei R₁ und R₂ jeweils unabhängig Wasserstoff, einen gesättigten oder ungesättigten Acyl- oder Alkylrest öder einen Rest, ausgewählt aus einer der Formeln (X), (XI), (XII) und (XIII), darstellen und mindestens einer von R₁ und R₂ einen Rest, ausgewählt aus einer der Formeln (X), (XI), (XII) und (XIII), darstellt: 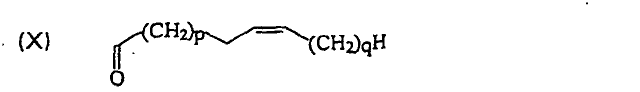 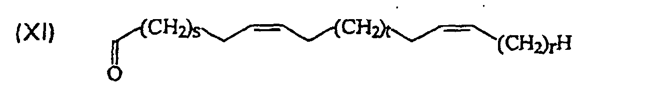 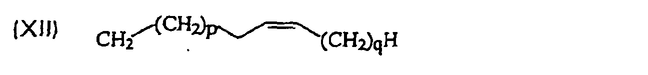 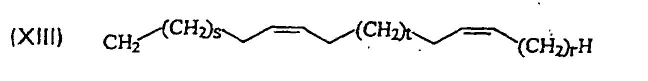 wobei q ≠ 8 für p + q = 14, 16, 18 oder 20 ist, wenn keiner der Reste R₁ und R₂ einen Rest der Formel (XI) oder (XIII) darstellt, t ≥ 0 ist und
p verschieden ist von der Bedeutung von p in den natürlich vorkommenden entsprechenden Resten.

Die in den hier beschriebenen Substanzen verwendeten Strukturelemente können beliebig variiert und maßgeschneidert der jeweiligen Verwendung angepaßt werden. Die Acyl- bzw. Alkylresten sind solche, die ihre Doppelbindung nicht an einer natürlichen Position tragen. Verbindungen, bei denen beide Reste R₁ und R₂ natürlich vorkommende einfach ungesättigte Acyl- oder Alkylketten darstellen, wie etwa diejenigen mit der C = C-Bindung in der ω-9-Position, sind also nicht Teil der Erfindung. Durch das erfindungsgemäße Verfahren kann die Position der Doppelbindung(en) frei gewählt werden, so daß bisher nicht zugängliche Alkyl-/Acylketten hergestellt werden können. Wie bereits oben erläutert, sind die Cis-Doppelbindungen von natürlichen doppelt ungesättigten Alkyl- und Acylketten jeweils durch nur eine Methylengruppe getrennt. Solche Verbindungen sind bei Raumtemperatur in Gegenwart von Sauerstoff nicht stabil und müssen daher bei tiefen Temperaturen unter Stickstoff aufbewahrt werden. Die Möglichkeit der Synthese von (Z)-Fettsäuren und (Z)-Alkenolen mit den Alkyl- oder Acylketten der Formeln (XI) und (XIII) mit 16 bis 34 C-Atomen erlaubt die Bereitstellung von Strukturelementen, bei denen mindestens 2 Meth'ylengruppen zwischen den Unsättigungen vorhanden sind. Dadurch erhält man eine erhebliche Stabilisierung der Fettsäuren und -alkohole und der daraus synthetisierten Verbindungs-klassen. Die Aufbewahrung erfindungsgemäßer Verbindungen bei Raumtem-peratur ohne Inertgas ist ohne weiteres möglich. Der Ausdruck (Z)-Fettsäuren oder -Alkenole, wie hier verwendet, umfaßt sowohl einfach als auch zweifach ungesättigte Ketten mit einer oder zwei cis-Döppelbindungen.

Der Vorteil der besonders bevorzugten Alkyl- bzw. Acylketten mit zwei Doppelbindungen liegt in den günstigen physiko-chemischen Eigenschaften. So ist beispielsweise die auf eine 28 Kohlenstoffkette aufbauende, zweifach ungesättigte Fettsäure (Z,Z)-1 O, 19-Octacosadiensäure bei Raumtemperatur flüssig, während einfach ungesättigte Fettsäuren dieser Kettenlänge unabhängig von der Position der Cis-Doppelbindung bei 20°C nur im festen Zustand vorkommen. Der Einbau der erfindungsgemäßen Strukturen in Phospholipide erlaubt die Übertragung dieser günstigen Eigenschaften auf die erfindungsgemäßen Verbindungen, was sich u.a. in niedrigen Phasenumwandlungstemperaturen widerspiegelt. Durch Verlängerung der Fettsäureketten wird es ebenfalls möglich, den Vesikeldurchmesser im Vergleich zu aus gebräuchlichen Lecithinen hergestellten Liposomen mehr als zu verdoppeln, was einer Verachtfachung des Innenvolumens von Ultraschallpräparierten Liposomen entspricht. Somit kann mehr als achtmal soviel Wirkstoff transportiert werden, wie es mit herkömmlichen Liposomen möglich ist. Zudem sind auch Präparationen von großen unilamellaren Vesikeln (LUVs) in hochviskosen Lösungen, z.B. Zuckerlösungen, möglich, in einem Medium also, in dem die Liposomenherstellung durch Extrusionsverfahren problematisch ist. Die Phasenumwandlungstemperaturen der Phospholipide mit erfindungsgemäßen, extrem langen Fettsäuren liegen aufgrund der Cis-Doppelbindung(en) in einem für Liposomenpräparationen günstigen Bereich.

Die Verbindung der allgemeinen Formel (I) weist zwei variable Komponenten A und B auf, die jeweils einzeln modifiziert werden können. Es handelt sich bei der erfindungsgemäßen Verbindung der Formel (I) nicht um ein Gemisch verschiedener Moleküle unbestimmter Zusammensetzung und Kettenlänge, sondern es kann gezielt eine gewünschte Struktur erhalten werden. Dies bedeutet, falls das gewünschte Produkt ein N, N-Dimethyl-N-(2)-hydroxypropyl-3,1-O,O-dihydroxypropyl)-ammoniumderivat ist, mit y = 1 und z = 2 in der Formel (I), daß die Verbindung chemisch definiert ist und kaum Anteile mit y = 1 und z = 1 oder y = 1 und z = 3 usw. enthält. Bevorzugt werden Hydroxypropylderivate einer ganz bestimmten Kettenlänge verwendet, die im wesentlichen frei von anderen Kettenlängen sind.

Erfindungsgemäß stellt die Verbindung der Formel (I) eine einheitliche Verbindung definierter Struktur dar. Bevorzugt ist die Verbindung hinsichtlich des Wertes von z größer als 99 % einheitlich. Es ist jedoch auch möglich, die Verbindung mit einer Einheitlichkeit von mehr als 99,9 % hinsichtlich des Wertes von z bereitzustellen.

Bevorzugt ist für B in der Verbindung der Formel (I) m = 1 mit n = 2 bis 8. Besonders bevorzugt ist n = 2 bis 6, noch stärker bevorzugt 2 bis 4. Bei z = 0 ist x bevorzugt eine ganze Zahl von 1 bis 3 und noch stärker bevorzugt 1.

Wenn z = 1 ist, weist y bevorzugt einen Wert von 1 bis 4 auf, und wenn z = 1 bis 5 ist, ist y bevorzugt 1. Im Falle y > 1 stammt der Rest -CH₂(-CHOH)_{y}-CH₂-OH bevorzugt von Zuckeralkoholen, die vier Hydroxylgruppen für y = 2, fünf Hydroxylgruppen für y = 3 und sechs Hydroxylgruppen für y = 4 aufweisen. Beispiele solcher Reste sind Mannitderivate für y = 4, Lyxitderivate für y = 3 und Threitderivate für y = 2.

x kann bevorzugt auch 0 sein. In diesem Fall ist y = 2 bis 4 für z = 1. Oder in einer anderen bevorzugten Ausführungsform ist z = 1 bis 5 für y = 1.

m kann auch bevorzugt 0 sein, wobei dann die Verbindung der Formel (I) aufgrund der negativ geladenen PO⁻₃-Gruppe eine negative Überschußladung aufweist. Für m = 0 ist x bevorzugt 0, und y = 1 für z = 1 bis 5, oder in einer ebenfalls bevorzugten Ausführungsform ist y = 2 bis 4 für z = 1.

Der Rest R₃ ist bevorzugt CH₃, C₂H₅ oder 1,2-Dihydroxypropyl.

Die Gruppen der Formeln (III) bis (VII) liegen bevorzugt in enantiomerenreiner Form vor. Sie können jedoch auch Racemate darstellen.

Erfindungsgemäß stellt die Verbindung der Formel (I) eine Verbindung definierter Struktur dar. Einfach ungesättigte Alkylketten sind bevorzugt mehr als 97 % einheitlich, können aber auch mit einer Einheitlichkeit von mehr als 99 % bereitgestellt werden. Zweifach ungesättigte Alkylketten sind bevorzugt mehr als 90 % einheitlich, können partiell aber auch in Reinheiten > 97 % bereitgestellt werden.

Bevorzugt handelt es sich bei der Verbindung um Phospholipide mit einfach bzw. zweifach ungesättigten Alkyl- bzw. Acylketten mit 16 - 34 Kettenkohlenstoffatomen.

Die durch die allgemeine Formel (I) erfaßten Verbindungen besitzen hervorragende biologische Eigenschaften und finden Verwendung als
1. Liposomenbestandteile zur Herstellung von Liposomen zur gezielten Anreicherung von Wirkstoffen oder Nukleinsäuren in Zielzellen (Alkyl-/Acylkettenlänge bevorzugt 16-32 C-Atome)
2. Wirkstoffe gegen Tumorerkrankungen und Protozoenerkrankungen (Alkyl-/Acylkettenlänge bevorzugt 16-26 C-Atome) und
3. Lösungsvermittler für schwer intravenös applizierbare Substanzen, wie z.B. Taxol (Alkyl-/Acylkettenlänge bevorzugt 16-30 C-Atome).

Herkömmliche Liposomen weisen im Serum eine Verweilzeit von bis zu 5 Stunden auf, insbesondere bei der Verwendung von Liposomen als Träger für pharmazeutische Wirkstoffe ist jedoch eine möglichst lange Verweilzeit von Liposomen im Blutkreislauf wünschenswert, insbesondere aber in Verbindung mit einer Aufnahme in ausgewählte Zielzellen.

Bei Ultraschall-Präparationen von Liposomen stellte sich heraus, daß symmetrische Lecithine mit (Z)-Fettsäuren mit bis zu 24 Kohlenstoffatomen im Gemisch mit Cholesterin Liposomen bilden, wobei die Homogenität der Vesikelpopulation entscheidend von der Position der Doppelbindung bestimmt wird. Eine enge Standardabweichung der Vesikelgröße setzt einen bestimmten Abstand der Doppelbindung zur Carboxylfunktion voraus. Zu erkennen ist eine im Vergleich zur herkömmlichen Lecithinen signifikante Vergrößerung des Vesikeldurchmessers, welcher bei (Z)-1 5-Tetracosensäure (Nervonsäure) 125 nm beträgt. Gemischtkettige Phosphatidylcholine mit einer gesättigten Acylkette in der *sn*-1-Position bilden auch mit sehr langkettigen (Z)-Fettsäuren Vesikel, wobei ein Interdigitieren der Fettsäureketten anzunehmen ist. Der mittlere hydrodynamische Liposomendurchmesser liegt bei Veresterung mit (Z)-15-Triacontensäure (30:1 Δ¹⁵) bei 111 nm (Stearinsäure in *sn*-1-Position). Eine deutliche Vesikelvergrößerung erhält man auch unter Verwendung extrem langer Fettsäuren bei Phospholipiden, die einen modifizierten polaren Bereich tragen, wie z.B. bei Phosphatidyloligoglycerinen oder bei Phospholipiden, die über Stickstoffatome verbundene Oligoglycerine enthalten.

Wenn die erfindungsgemäße Verbindung der allgemeinen Formel (I) als Liposomenbestandteil verwendet wird, ist der Bestandteil A bevorzugt ein zweikettiger, vom Glycerin abgeleiteter Rest der Formeln (III) oder (IV). Im Bestandteil B weisen diese Verbindungen bevorzugt eine Alkylammonium-Gruppe auf, d.h. m ist bevorzugt gleich 1. Die bevorzugten Parameter für als Liposomenbestandteile verwendete Verbindungen der Formel (I) sind:
m = 1, n = 2 - 6, x = 0, y = 1, z = 1 - 5 oder
m = 1, n = 2 - 6, x = 0, y = 2 - 4, z = 1 oder
m = 1, n = 2 - 6, x = 1, z = 0 oder
m = 0, x = 0, y = 1, z = 1 - 5, bevorzugt 2 - 4 oder
m = 0, x = 0, y = 2 - 4, z = 1.
R₃ ist in diesem Fall bevorzugt 1,2-Dihydroxypropyl, C₂H₅ oder noch stärker bevorzugt CH₃. Bevorzugt handelt es sich bei der Verbindung um Hydroxypropylderivate mit 1 bis 3 Hydroxypropyleinheiten, d.h. x = 0 und z = 1 bis 3. Da y bevorzugt 1 ist, handelt es sich hierbei um 1,3-verknüpfte lineare Oligoglycerinreste, die über einen 2-Hydroxypropylrest mit dem Stickstoffatom verknüpft sind.

Bevorzugt liegen bei diesen Verbindungen, die als Liposomenbestandteile geeignet sind, 2 Reste, also R₁ und R₂ vor. Diese können jeweils unabhängig einen Rest einer der Formeln (X) bis (XIII) darstellen. Wenn R₁ und R₂ identisch sind, weisen sie bevorzugt eine maximale Kettenlänge von jeweils 16 bis 26 C-Atomen auf. In einer weiteren bevorzugten Ausführungsform ist einer der Reste länger als 26 C-Atome und kann bevorzugt bis zu 32 C-Atome aufweisen. In diesem Fall liegt bevorzugt ein Methylrest am Stickstoff vor, d.h. daß bei z = 0 x bevorzugt 1 ist. Ebenfalls bevorzugt ist mindestens einer von R₁ und R₂ ein 2-fach ungesättigter erfindungsgemäßer Rest, noch stärker bevorzugt sind sowohl R₁ als auch R₂ ein 2-fach ungesättigter erfindungsgemäßer Rest.

Einer der Reste R₁ und R₂ kann auch einen gesättigten Acyl- bzw. Alkylrest darstellen. In diesem Fall stellt der andere Rest eine Verbindung einer der Formeln (X) bis (XIII) dar, und bevorzugt stellt er eine 2-fach ungesättigte Alkyl- bzw. Acylkette der Formel (XI) oder (XIII) dar.

In einer anderen bevorzugten Ausführungsform kann die Verbindung der allgemeinen Formel (I) als Liposomenbestandteil auch eine negative Überschußladung tragen. Dies ist der Fall, wenn m = 0 ist. Bevorzugt handelt es sich hierbei um Glycero-Glycerine sowie Phosphatidyl-glycero-glycero-glycerine und Phosphatidyl-glycero-glycero-glycero-glycerine (hierbei ist x = 0, y = 1 und z = 2 bis 4). Außerdem bevorzugt sind hierbei die bereits erwähnten Verbindungen mit y > 1, d.h. der Rest CH₂-(-CHOH)_{y}-CH₂-OH stammt bevorzugt von Zuckeralkoholen, die 4 Hydroxylgruppen für y = 2, 5 Hydroxylgruppen für y = 3 und 6 Hydroxylgruppen für y = 4 aufweisen. Ebenfalls bevorzugt sind hierbei Phospho-*sn*-G₁-Verbindungen.

Erfindungsgemäße Wirkstoffe stellen bevorzugt Verbindungen der allgemeinen Formel (I) dar, in denen der Strukturparameter A einen Rest einer der Formeln (VIII) oder (IX) darstellt. Es handelt sich also hierbei um ungesättigte Alkylphosphocholine.

Der Vorteil von ungesättigten Ketten im apolaren Bereich liegt darin, daß derartige Verbindungen intravenös applizierbar sind. Erfindungsgemäße Wirkstoffe weisen eine im Verhältnis zum Erucylphosphocholin, der bisher wirksamsten Verbindung, verbesserte antitumorale Wirksamkeit auf. Eine erhöhte zytostatische Wirkung erhält man beispielsweise durch Verschiebung der cis-Doppelbindung zur Phosphocholingruppe. So zeigt sich bereits bei der niedrigsten Dosis (Z)-10-Docosenyl-1-phosphocholin (42µmol/kg/Woche) eine Tumorreduktion auf 9 % (T/C), während Erucylphosphocholin bei einer mehr als doppelt so hohen Dosierung (90 µmol/kg/Woche) erst eine Reduktion auf 31 % (T/C) aufweist (siehe Beispiel 5, Tabelle 1).

Die bevorzugten Parameter für als Wirkstoffe geeignete Verbindungen der Formel (I) sind:
m = 1, n = 2 - 6, stärker bevorzugt n = 2 - 4, x = 1, z = 0.

Verbindungen der allgemeinen Formel (I) sind besonders geeignet als pharmakologische Wirkstoffe, wenn sie einen Alkylammoniumrest aufweisen (d. h. m = 1), bei dem ein Abstand zwischen Ammonium und Phosphat von größer oder gleich 2 vorliegt, d.h. n ist bevorzugt 2, 3 oder 4. In diesem Fall stellt R₃ bevorzugt eine CH₃- oder C₂H₅-Gruppe dar. Ebenfalls bevorzugt ist R₃ = 1,2-Diyhdroxypropyl. Diese Verbindungen sind besonders wirksam als Antitumormittel.

Am meisten bevorzugt sind Verbindungen mit einer N,N,N-Trimethylalkylammonium-Gruppe, so daß bevorzugt z = 0 und x = 1 ist.

Bei Wirkstoffen wird bevorzugt auf ein Glyceringrundgerüst oder einähnliches Grundgerüst nach einer der Formeln (III) bis (VII) verzichtet

Wenn ein einfach ungesättigter Alkylrest vorliegt, weist dieser bevorzugt 16 bis 23 Kohlenstoffatome auf. Es hat sich nämlich gezeigt, daß Verbindungen mit Ketten, die 24 C-Atome oder mehr aufweisen, schon deutlich ungeeigneter sind. Bei einem zweifach ungesättigten Alkylrest kommen längere Ketten in Frage, mit bevorzugt ca. 19 bis 26 C-Atomen. Es zeigte sich, daß bei den zweifach ungesättigten Ketten solche mit 16 bis 18 Kohlenstoffatomen nicht wirksam sind. Besonders hervorzuheben sind dabei die Alkadienylphosphocholine mit terminaler Doppelbindung (d.h. r = 0) in der Formel (IX), die bereits bei sehr niedriger Dosierung einen deutlichen antitumoralen Effekt aufweisen.

Verbindungen mit einem Glycerin-artigen Bestandteil zeigen auch antitumorale Wirksamkeit, d.h. es kann auch am Phosphatrest eine Verbindung nach einer der Formeln (III) bis (VII) vorliegen. Wenn dabei 2 Reste R₁ oder R₂ vorliegen, ist es jedoch wichtig, daß ein R eine kurze Kette darstellt. Bevorzugt ist diese kurze Kette ein Alkylrest mit 1 bis 4 C-Atomen. Der andere Rest R₁ oder R₂ stellt dann bevorzugt einen Rest der Formel XII oder XIII dar. Insbesondere stellt er einen Rest der Formel XIII dar.

Außerdem sind Verbindungen bevorzugt, bei denen beide Reste R₁ und R₂ jeweils durch eine Etherbindung mit dem Glycerinrest verknüpft sind, d.h. sie stellen jeweils unabhängig eine Gruppe der Formel (XII) oder (XIII) dar. Besonders bevorzugt ist auch eine Verbindung, wo R₁ und R₂ den gleichen einfach oder doppelt ungesättigten erfindungsgemäßen Rest darstellen.

Als eine weitere bevorzugte Ausführungsform der Verbindung der allgemeinen Formel (I) sind Verbindungen zu nennen, die sich durch eine gute Eigenschaft zur Lösungsvermittlung auszeichnen. Die bevorzugten Strukturparameter für als Lösungsvermittler geeignete Verbindungen der Formel (I) sind:
m = 1, n = 2 - 6, x = 0, y = 1, z = 1 - 3, stärker bevorzugt z = 1,
m = 1, n = 2 - 6, x = 0, y = 2 - 4; z = 1 oder
m = 1, n = 2 - 6, x = 1, z = 0.
R₃ ist bevorzugt CH₃, C₂H₅ oder 1,2-Dihydroxypropyl.
Bekannte Verbindungen dieser Art umfassen beispielsweise die Erucyl-(C₂₂)-Verbindungen. Bei den erfindungsgemäßen Verbindungen sind deshalb solche Verbindungen bevorzugt, welche als Strukturparameter A eine Gruppe nach einer der Formeln (III) bis (VII) besitzen, wobei einer der Reste R₁ und R₂ bevorzugt eine Verbindung der Formeln (X) oder (XI) darstellt, d.h. bevorzugt ist einer der Reste R₁ oder R₂ eine doppelt ungesättigte Kette gemäß der Erfindung. Bevorzugt sind bei den Lösungsvermittlern einkettige Verbindungen, d.h. wenn A eine Gruppe der Formeln (III) oder (IV) darstellt und einer von R₁ und R₂ -OH oder ein Alkyl mit 1 bis 4 C-Atomen ist.

Wenn A einen Rest nach einer der Formeln (V) bis (VII) darstellt, d.h. wenn nur ein R₁ vorhanden ist, ist R₁ ebenfalls bevorzugt eine doppelt ungesättigte Kette. Erfindungsgemäße Lösungsvermittler liegen vorzugsweise als Ester vor, d.h. es sind Ketten der Formel (X) oder (XI) bevorzugt. Ganz besonders bevorzugt sind hier wiederum Verbindungen mit einem oder zwei doppelt ungesättigten Alkadienylresten. Außerdem sind auch hier einige Verbindungen der bereits zuvor genannten Klassen geeignet. Ein Beispiel sind die einkettigen Glycero-phospho-Verbindungen, die nicht am Stickstoff hydroxyliert sind, d.h. im Strukturparameter B ist m = 1, x = 1 und z = 0.

Insbesondere sind als Lösungsvermittler Verbindungen bevorzugt, die nur einen langkettigen Rest aufweisen, wie etwa solche Verbindungen auf der Basis von Lysolecithin, welche an einem C-Atom des Glycerinrestes eine OH-Gruppe aufweisen. Bevorzugt sind daher besonders Verbindungen, in denen der Strukturparameter A ein Rest nach einer der Formeln (III) bis (VII) ist.

Manche Verbindungen mit 2 Resten R₁ und R₂ weisen allerdings auch besonders gute Lösungsmitteleigenschaften auf. Beispiele sind solche Verbindungen, in denen R₁ und R₂ zwei doppelt ungesättigte Reste mit 16 bis 24 C-Atomen darstellen.

Ein weiterer Gegenstand der vorliegenden Erfindung ist ein Verfahren zur Herstellung von ungesättigten (Z)-Fettsäuren bzw. (Z,Z)-Fettsäuren oder (Z)-Alkenolen bzw. (Z,Z)-Alkenolen mit 16 bis 34 Kohlenstoffatomen wobei durch das erfindungsgemäße Verfahren doppelt ungesättigte (Z,Z)-Fettsäuren bzw. Alkenole zugänglich werden, die zwischen den cis-Doppelbindungen mehr als eine CH₂-Gruppe aufweisen. Für dieses Verfahren wird als Ausgangsprodukt ein Lacton verwendet, welches 13 bis 19 C-Atome umfassen kann.

Das Verfahren umfaßt die folgenden Schritte:
1) Spalten des Lactonringes mit einem Trimethylsilylhalogenid zu dem entsprechenden Halogen-Carbonsäuretrimethylsilylester,
2) gleichzeitige oder anschließende Alkoholyse des Halogen-Carbonsäuretrimethylsilylesters zu dem entsprechenden Halogen-Carbonsäureester,
3) Umsetzung des Halogen-Carbonsäureesters mit Triphenylphosphan zu dem entsprechenden Phosphoniumsalz,
4) Umsetzung des Phosphoniumsalzes mit einem Aldehyd unter Verwendung einer Base und anschließender Verseifung zu einem entsprechenden (Z)-Fettsäuresatz,
5) Freisetzung der (Z)-Fettsäure aus dem (Z)-Fettsäuresalz, und
6) gegebenenfalls Umsetzung der (Z)-Fettsäure in das entsprechende (Z)-Alkenol mittels Lithiumaluminiumhydrid.

In Schritt 1) werden bevorzugt Lactone der Formel (XIV) verwendet 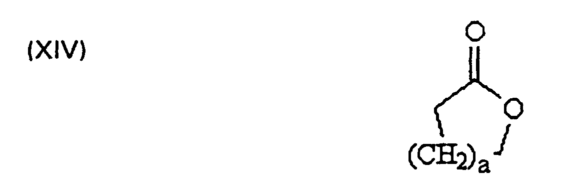 wobei a = 10 bis 16 ist. Die zur Spaltung des Lactonringes verwendeten Trimethylsilylhalogenide sind bevorzugt Trimethylsilyljodid oder Trimethylsilylchlorid. Der in Schritt 2) zur Alkoholyse verwendete Alkohol ist bevorzugt Ethanol. Die Umsetzung des Phosphoniumsalzes mit einem Aldehyd beruht auf dem Verfahren einer Wittig-Reaktion in Abwesenheit von Lithiumsalzen, was auch als salzfreie Wittig-Reaktion bezeichnet wird. Die Stereoselektivität solcher Reaktionen wird im allgemeinen durch Natrium- oder Kaliumhaltige Basen hervorgerufen, daher sind bevorzugte Basen z.B. NaNH₂, Kalium-tert.-Butylat, NaHMDS oder KHMDS. Besonders bevorzugt ist NaHMDS. Die Verseifung und anschließende Freisetzung sowie gegebenenfalls die Umsetzung der Fettsäuren in ein Alkenol geschieht nach bekannten Verfahren.

Eine besonders bevorzugte Ausführungsform des Verfahrens der vorliegenden Erfindung ist das Verfahren zur Herstellung der Nervonsäure ((Z)-15-Tetracosensäure). Hierbei wird als Ausgangslacton Cyclopentadecanolid und als Aldehyd in Schritt 4 Pelargonaldehyd verwendet. Durch dieses Verfahren kann Nervonsäure, die in der Natur nur in geringen Mengen vorkommt, auch großtechnisch synthetisiert werden.

Ein weiterer Gegenstand der vorliegenden Erfindung sind Liposomen, die als Liposomenhüllbestandteile phospholipidartige Verbindungen der Formel (I) umfassen. Außerdem enthalten diese Liposomen Phospholipide und/oder Alkylphospholipide und gegebenenfalls Cholesterin, wobei die Liposomen 1 bis 50 Mol-% einer erfindungsgemäßen Verbindung der Formel (1) oder deren Salz enthalten und zusammen mit den Phospholipiden, den Alkylphospholipiden und dem Cholesterin 100 Mol-% der Liposomenhülle ergeben.

Die erfindungsgemäßen Liposomen besitzen ein deutlich vergrößertes Innenvolumen. Sie können somit eine größere Menge an Wirkstoff und/oder Nukleinsäuren transportieren. Bevorzugte Liposomen gemäß der Erfindung umfassen zusätzlich einen Wirkstoff und gegebenenfalls pharmazeutisch annehmbare Verdünnungs-, Hilfs-, Träger- und Füllstoffe. Die Liposomen können zusätzlich zu dem Wirkstoff oder anstelle des Wirkstoffes eine Nukleinsäure enthalten. Erfindungsgemäß können als Wirkstoffe auch Wirkstoffe nach der Erfindung verwendet werden.

Ein weiterer Gegenstand der vorliegenden Erfindung ist eine pharmazeutische Zusammensetzung, die als wirksamen Bestandteil eine Verbindung der Formel (I) enthält, die als Wirkstoff geeignet ist. Außerdem kann die pharmazeutische Zusammensetzung zusätzlich pharmazeutisch annehmbare Verdünnungs-, Hilfs-, Träger- und Füllstoffe enthalten.

Ein weiterer Gegenstand der vorliegenden Erfindung ist die Verwendung der erfindungsgemäßen Verbindungen als Liposomenbestandteile, als pharmakologische Wirkstoffe oder als Lösungsvermittler. Es hat sich gezeigt, daß einige der erfindungsgemäßen Verbindungen eine besonders gute antitumorale Wirkung zeigen. Außer als Antitumorwirkstoff sind erfindungsgemäße Verbindungen auch gegen Protozoenerkrankungen, wie etwa Leishmaniose oder Trypanosomiasis, einsetzbar. Sie sind ebenfalls verwendbar, um die Löslichkeit von in Wasser schwer löslichen Stoffen zu fördern, beispielsweise Taxol, so daß diese Stoffe in Verbindung mit den erfindungsgemäßen Lösungsvermittlern auch intravenös verabreicht werden können.

Als Wirkstoffe können in der Regel alle Wirkstoffe verwendet werden, die sich mittels Liposomen überhaupt ins Plasma einbringen lassen. Bevorzugte Wirkstoffgruppen sind einerseits Cytostatika, insbesondere Anthracyclin-Antibiotika, wie etwa Doxorubicin, Epirubicin oder Daunomycin, wobei Doxorubicin besonders bevorzugt ist. Weitere bevorzugte Cytostatika sind Idarubicin, Alkylphosphocholine in den von uns beschriebenen Strukturvariationen, 1-Octadecyl-2-methyl-rac-glycero-3-phosphocholin und davon abgeleitete Strukturanaloga, 5-Fluoruracil, cis-Platinkomplexe wie Carboplatin und Novantron sowie Mitomycine.

Weitere bevorzugte Wirkstoffgruppen sind immunmodulierende Substanzen, wie etwa Cytokine, wobei unter diesen wiederum die Interferone und insbesondere das α-Interferon besonders bevorzugt sind, antimykotisch wirksame Substanzen (z.B. Amphotericin B) und Wirkstoffe gegen Protozoenerkrankungen (Malaria, Trypanosomen- und Leishmanien-Infektionen). Ebenfalls bevorzugt ist Taxol als Wirkstoff.

Eine weitere bevorzugte Wirkstoffgruppe sind lytische Wirkstoffe, wie sie in der DE 41 32 345 A1 beschrieben sind. Bevorzugt sind Miltefosin, Edelfosin, Ilmofosin sowie SRI62-834. Insbesondere bevorzugt sind Alkylphosphocholine auch mit erweiterten Alkylketten, z.B. Erucylphosphocholin und Erucylphosphocholine mit erweitertem Phospho-Stickstoffabstand.

Ein weiterer Gegenstand der vorliegenden Erfindung ist die Verwendung von erfindungsgemäßen Liposomen zur Herstellung eines Antitumormittels, wobei der Wirkstoff besonders bevorzugt Doxorubicin ist.

Noch ein weiterer Gegenstand der vorliegenden Erfindung ist die Verwendung der erfindungsgemäßen Liposomen zur Herstellung eines Mittels zur Beeinflussung der Zellproliferation, wobei der Wirkstoff ein Cytokin, besonders bevorzugt α-Interferon ist.

Die Liposomen der vorliegenden Erfindung können somit auch als Transportvehikel und speziell als Gentransportvehikel verwendet werden.

Das Verfahren sowie die Verbindungen der allgemeinen Formel (I) werden in den nachstehenden Beispielen genauer erläutert.

### Beispiele

### Beispiel 1: Synthese ω-substituierter Phosphoniumsalze

### 1a) Synthese über die Monobromierung von α,ω-Diolen

Als Ausgangsmaterialien zur Synthese olefinischer Alkohole dienen Alkandiole, die mit 48 %-iger Bromwasserstoffsäure zu w-Brom-alkan-1-olen monobromiert werden. Nach Acetylierung der verbleibenden Hydroxylgruppe werden die Verbindungen mit Triphenylphosphan zu den in ω-Position substituierten Triphenylphosphoniumbromiden verschmolzen. Diese werden nach Deprotonierung mit NaHMDS mit unsubstituierten Aldehyden olefiniert und anschließend zu (Z)-Fettalkoholen verseift.

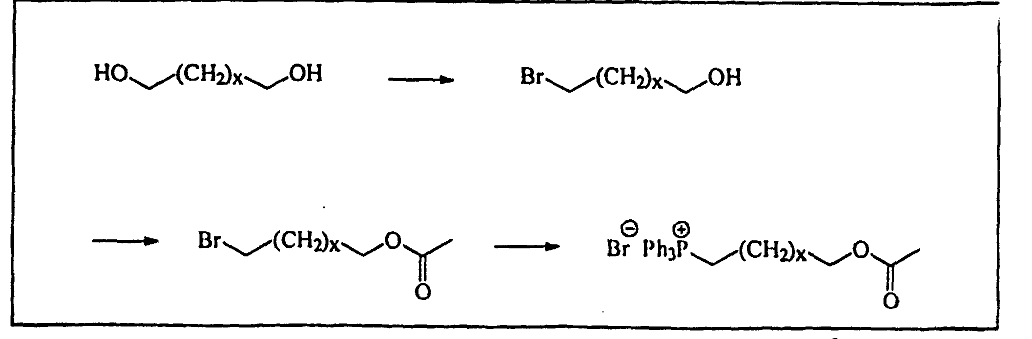

### Synthese von [ω-(Acetoxy)-alkyl]triphenylphosphoniumbromiden über die Monobromierung von α,ω-Diolen

### Monobromierung

### 6-Brom-1-hexanol

200,8 g (1,70 mol) 1,6-Hexandiol, 600 ml 48 %-ige Bromwasserstoffsäure und 2 I Toluol wurden unter intensivem Rühren 2 Stunden unter Rückfluß erhitzt. Nach Abkühlung auf Raumtemperatur wurden die Phasen getrennt. Die organische Phase wurde mit 2 x 500 ml ges. NaHCO₃-Lösung und 700 ml Wasser gewaschen. Nach Entfernung des Lösungsmittels erhielt man 301,2 g (1,66 mol, 98 %) 6-Brom-1-hexanol.
MG = 181,07 g/mol (C₆H₁₃BrO)
R_{f}(Edukt) = 0, 19 (Diethylether)
R_{f} = 0,59 (Diethylether)

### 10-Brom-1-decanol

87,8 g (0,50 mol) 1,10-Decandiol, 165,1 g 48 %-ige Bromwasserstoffsäure und 2,5 I hochsiedender Petrolether (Sdp. 100-140 °C) wurden unter intensivem Rühren 4 Stunden unter Rückfluß erhitzt. Man gab weitere 80,0 g 48 %-ige Bromwasserstoffsäure hinzu und ließ 5 Stunden sieden. Nach Abkühlung auf 30 °C wurden die Phasen getrennt. Die organische Phase wurde zuerst mit einer Lösung aus 100 g Na₂CO₃ in 500 ml Wasser, dann mit 2 x 500 ml Wasser gewaschen. Nach Entfernung des Lösungsmittels wurde an 700 g Kieselgel chromatographiert. Dabei wurde das als Nebenprodukt entstandene 1,10-Dibromdecan mit Cyclohexan/Diethylether (20: 1 ) eluiert. Chromatographie mit Cyclohexan/Diethylether (2:1) lieferte 103,9 g (0,44 mol, 87 %) 10-Brom-1-decanol.
MG = 237,18 g/mol (C₁₀H₂₁BrO)
R_{f} = 0,38 (Diisopropylether)
¹H-NMR (300 MHu, CDCl₃): δ = 1,30-1,43 (m, 12H, (CH₂)₆), 1,57 (m, 2H, CH₂CH₂OH), 1,85 (mc, 2H, CH₂CH₂Br), 2,22 (s, D₂O-austauschbar, 1H, OH), 3,41 (t, ³J = 6,9 Hz, 2H, CH₂Br), 3,64 (t, ³J = 6,7 Hz, 2H, CH₂OH)

### Acetylierung zu ω-Brom-alkylacetaten

Die Acetylierung der ω-Brom-alkan-1-ole wird mit Acetanhydrid unter DMAP-Katalyse in THF durchgeführt. Die Veresterungen verlaufen unabhängig von der Kettenlänge der Verbindung bei 30 °C zügig und sind bereits wenige Minuten nach Zugabe des reaktiven Säureanhydrids abgeschlossen.

### 6-Brom-hexylacetat

297,4 g (1,64 mol) 6-Brom-1-hexanol in 1500 ml THF wurden mit 20,1 g (0,16 mol) DMAP versetzt. Eine Lösung aus 184,4 g (1,81 mol) Acetanhydrid in 300 ml THF wurde so zugetropft, daß die Reaktionstemperatur 30 °C nicht überstieg. Nach beendeter Zugabe ließ man weitere 30 Minuten rühren. Das Reaktionsgemisch wurde mit 500 ml Diisopropylether versetzt und nacheinander gegen je 700 ml Wasser, 2 x ges. NaHCO₃-Lösung und Wasser extrahiert. Nach Trocknung über Natriumsulfat wurde das Lösungsmittel im Vakuum entfernt. Man erhielt 352,8 g (1,58 mol, 96 %) 6-Brom-hexylacetat.
MG = 223,11 g/mol (C₈H₁₅BrO₂)
R_{f} = 0,81 (Diethylether)
¹H-NMR (300 MHz, CDCl₃): δ = 1,33-1,53 (m, 4H, (CH₂)₂), 165 (mc, 2H, CH₂CH₂O), 1,87 (mc, 2H, CH₂CH₂Br), 2,04 (s, 3H, OOCCH₃), 3,41 (t, ³J = 6,8 Hz, 2H, CH₂Br), 4,06 (t, ³J = 6,7 Hz, 2H, CH₂O)
IR (Film): *v*[cm⁻¹] = 2937 (s), 2859 (s), 1736 (s), 1460 (m), 1365 (m), 1240 (s), 1044 (m), 731 (w), 641 (w), 561 (w)

### Quaternisierung zu Phosphoniumbromiden

### [10-(Acetoxy)-decyl]triphenylphosphoniumbromid

117,3 g (0,42 mol) des entsprechenden ω-substituierten Alkylbromids/iodids und 110,2 g (0,4 mol) Triphenylphosphan wurden unter Rühren (KPG-Rührer) 12 Stunden auf 130 °C erhitzt. Man entfernte die Heizung und ließ auf 90 °C abkühlen. Das Reaktionsgemisch wurde durch den Rückflußkühler langsam mit 400 ml THF versetzt und bis zur Bildung einer homogenen Phase gerührt. Man ließ auf Raumtemperatur abkühlen.

Nach Zugabe von 2 I Diethylether wurde 30 Minuten intensiv gerührt. Man ließ mehrere Tage bei -20 °C stehen, bevor man das überstehende Lösungsmittel vom festen Phosphoniumsalz abdekantierte. Das Produkt wurde mit 800 ml Toluol versetzt und mehrere Stunden bei 60 °C gerührt. Nach Trennung der Phasen nahm man das Phosphoniumsalz in 300 ml Dichlormethan auf. Es wurde 3I Diethylether zugegeben und mehrere Tage bei -20°C belassen. Nach erneutem Abdekantieren wurde das Produkt in Dichlormethan gelöst und in einen Kolben überführt. Das Phosphoniumsalz wurde 6 Stunden bei 80 °C im Vakuum getrocknet. Man erhielt 181,6 g (335 mmol, 80 %) [10-(Acetoxy)-decyl]triphenylphosphoniumbromid als gelbes, hochviskoses Öl.
MG = 541,51 g/mol (C₃₀H₃₈BrO₂P)
R_{f} = 0,23 (Chloroform/Methanol, 9: 1)

| *Analyse:* | C | H | P |
|---|---|---|---|
| ber. | 66,54 | 7,07 | 5,72 |
| gef. | 66,67 | 7,06 | 5,55 |

### 1b) Synthese über ω-Halogencarbonsäuren

### 11-Brom-undecansäureethylester

1000 g 90 %-ige 11-Brom-undecansäure (entspricht 3,39 mol), 304,0 g (6,60 mol) Ethanol und 20,0 g p-Toluolsulfonsäure wurden in einer Versuchsapparatur mit Wasserabscheider (für spezifisch schwerere Schlepper als Wasser) in 400 ml Chloroform vorgelegt. Das Gemisch wurde so lange unter Rückfluß erhitzt, bis sich kein Wasser mehr abschied (ca. 6 Stunden). Nachdem man die Lösung auf Raumtemperatur abgekühlt hatte, wurde nacheinander mit 1 I Wasser, 500 ml ges. NaHCO₃-Lösung und 1 I Wasser gewaschen. Das Lösungsmittel wurde im Vakuum entfernt. Durch Vakuumdestillation (Sdp. 131-133 °C/1 mbar) erhielt man 716,3 g (2,44 mol, 72 %) 11-Brom-undecansäureethylester.
MG = 293,24 g/mol (C₁₃H₂₅BrO₂)
R_{f} = 0,66 (Cyclohexan/Diisopropylether, 1:1)

| *Analyse:* | C | H |
|---|---|---|
| ber. | 53,25 | 8,59 |
| gef. | 53,22 | 8,57 |

¹H-NMR (300 MHz, CDCl₃): δ = 1,23-1,42 (m, 15H, COOCH₂CH₃, 6 x CH₂), 1,62 (mc, 2H, CH₂CH₂COO), 1,85 (mc, 2H, CH₂CH₂Br), 2,29 (t, ³J = 7,5 Hz, 2H, CH₂COO), 3,41 (t, ³J = 6,9 Hz, 2H, CH₂Br), 4,12 (quart, ³J = 7,1 Hz, 2H, COOCH₂CH₃)
IR (Film): ν[cm⁻¹] = 2930 (s), 2854 (s), 1737 (s), 1464 (m), 1372 (m), 1179 (s), 1118 (m), 723 (w), 645 (w), 563 (w)

### ω-lodcarbonsäureester

Zentrale Zwischenprodukte der Synthese von (Z)-15- bzw. (Z)-16-Olefinen: Durch Lactonspaltung von Cyclopentadecanolid und Cyclohexadecanolid mit Trimethylsilyliodid und anschließender Alkoholyse erhält man die ω-lodcarbonsäureethylester.

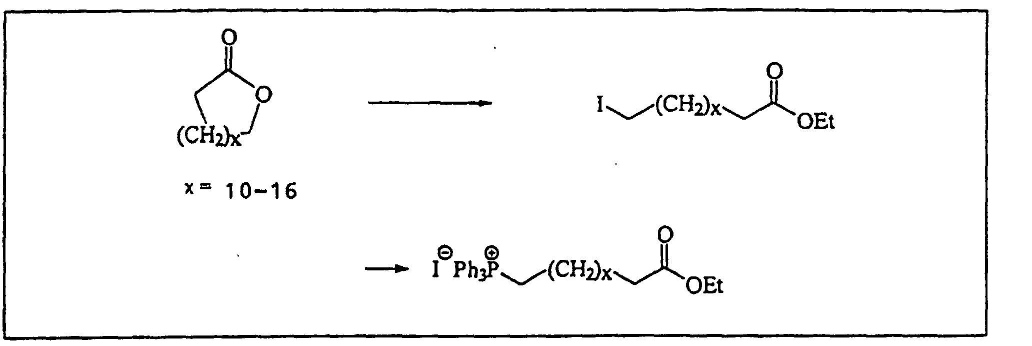

### Lactonspaltung

### 15-lod-pentadecansäureethylester

In einer Stickstoffatmosphäre wurden 150,3 g (0,63 mol) Cyclopentadecanolid in 500 ml Acetonitril gelöst und mit 229,0 g (1,53 mol) Natriumiodid versetzt. Durch ein Septum wurden 170 ml (1,34 mol) Trimethylsilylchlorid zugetropft. Man erhitzte 18 Stunden unter Rückfluß. Zum siedenden Reaktionsgemisch gab man vorsichtig 158,5 g (3,44 mol) Ethanol, erhitzte weitere 2 Stunden unter Rückfluß und ließ dann auf Raumtemperatur abkühlen. Es wurde mit 500 ml Diethylether versetzt und dreimal gegen je 500 ml 1 N Natriumhydroxid-Lösung extrahiert. Die wäßrigen Phasen wurden mit 300 ml Diethylether nachextrahiert und das Lösungsmittel der vereinigten organischen Phasen im Vakuum entfernt. Der Rückstand wurde zweimal bei -20°C aus Methanol kristallisiert. Nach mehrtägiger Trocknung im Vakuum erhielt man 202,3 g (0,51 mol, 81 %) 15-lod-pentadecansäureethylester. Obwohl das Produkt in guter Reinheit erhalten wurde, roch es aufgrund kleinster Mengen Lacton (Duftstoff!) intensiv nach Edukt.
MG = 396,35 g/mol (C₁₇H₃₃IO₂)
R_{f}(Zwischenprodukt) = 0,15 (Dichlormethan/Diisopropylether, 50:1)
R_{f} = 0,73 (Dichlormethan/Diisopropylether, 50:1)

| *Analyse:* | C | H |
|---|---|---|
| ber. | 51,52 | 8,39 |
| gef. | 51,40 | 8,24 |

Schmelzpunkt: 31,4 °C
¹H-NMR (300 MHz, CDCl₃): δ = 1,19-1,38 (m, 23H, COOCH₂CH₃, 10 x CH₂), 1,61 (mc, 2H, CH₂CH₂COO), 1,82 (mc, 2H, CH₂CH₂I), 2,29 (t, ³J = 7,6 Hz, 2H, CH₂COO), 3,19 (t, ³J = 7,0 Hz, 2H, CH₂I), 4,12 (quart, ³J = 7,1 Hz, 2H, COOCH₂CH₃)
IR (KBr): *v*[cm⁻¹] = 2916 (s), 2848 (s), 1735 (s), 1474 (w), 1464 (w), 1294 (w), 1248 (w), 1200 (m), 1166 (m), 720 (w)

### Umsetzung zu Phosphoniumsalzen

### [14-(Ethoxycarbonyl)-tetradecyl]triphenylphosphoniumiodid

119,0 g (0,30 mol) des entsprechenden ω-substitutierten Alkylbromids/iodids und 78,8 g (0,30 mol) Triphenylphosphan wurden unter Rühren (KPG-Rührer) 12 Stunden auf 130 °C erhitzt. Man entfernte die Heizung und ließ auf 90 °C abkühlen. Das Reaktionsgemisch wurde durch den Rückflußkühler langsam mit 400 ml THF versetzt und bis zur Bildung einer homogenen Phase gerührt. Man ließ auf Raumtemperatur abkühlen.

Das Produkt wurde durch Zugabe von 2 I Diethylether bei 0°C gefällt und das resultierende Gemisch einen Tag bei 4 °C gerührt. Danach wurde möglichst schnell über einen großen Glasfaserfilter abgesaugt, der Rückstand in Dichlormethan gelöst und in einen Kolben überführt. Nachdem man das Lösungsmittel im Vakuum abgetrennt hatte, wurde das Phosphoniumsalz 7 Stunden bei 70 °C im Vakuum getrocknet (am Rotationsverdampfer). Man erhielt 197,5 g (0,30 mol, 100 %) [14-(Ethoxycarbonyl)-tetradecyl]triphenylphosphoniumiodid.
MG = 658,64 g/mol (C₃₅H₄₈IO₂P)
R_{f} = 0,53 (Chloroform/Methanol, 9:1)

| *Analyse:* | **C** | **H** | **P** |
|---|---|---|---|
| ber. | 63,83 | 7,35 | 4,70 |
| gef. | 64,00 | 7,42 | 4,61 |

¹H-NMR (300 MHz, CDCl₃): δ = 1,19-1,28 (m, 25H, COOCH₂CH₃, 11 x CH₂), 1,63 (m, 2H, CH₂CH₂COO), 2,28 (t, ³J = 7,5 Hz, 2H, CH₂COO), 3,66 (m, 2H, CH₂P⁺Ph₃I⁻), 4,12 (quart, ³J = 7,1 Hz, 2H, COOCH₂CH₃), 7,69-7,86 (m, 15H, Aromaten-H)

### Beispiel 2: Synthese ω-substituierter Aldehyde

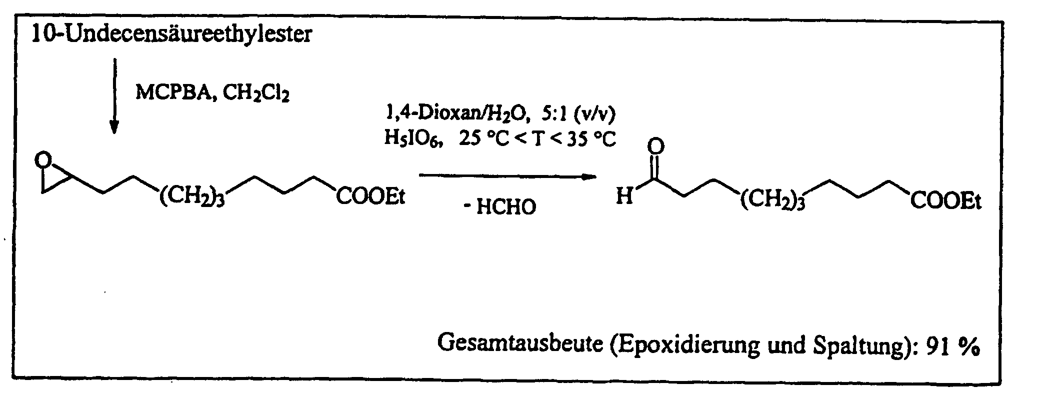

### Direkte Epoxidspaltung mit Periodsäure in wäßrigem 1,4-Dioxan

### 10, 11-Epoxy-undecansäureethylester

Zu 212,4 g (1,0 mol) 10-Undecensäureethylester in 2 l Dichlormethan gab man innerhalb von 1 1/2 Stunden 283,7 g (1,2 mol) 73 %-ige m-Chlorperoxybenzoesäure, wobei man die Temperatur unter 20 °C hielt. Nach 5-stündigem Rühren bei Raumtempertur (KPG-Rührer) wurde das Reaktionsgemisch über Nacht auf -20°C gestellt. Die ausgefallene m-Chlorbenzoesäure wurde abgesaugt und mit 500 ml kaltem Pentan (-20°C) gewaschen. Man entfernte das Lösungsmittel des Filtrats im Vakuum und nahm den Rückstand in 1 I Pentan auf. Diese Lösung wurde vorsichtig gegen 2 x 500 ml ges. NaHCO₃-Lösung und 500 ml Wasser extrahiert. Nach Trocknung über Natriumsulfat wurde das Lösungsmittel im Vakuum entfernt. Das so synthetisierte Epoxid enthielt noch m-Chlorbenzoesäure.
Rohausbeute: 259,5 g
MG = 228,33 g/mol (C₁₃H₂₄O₃)
R_{f} = 0,44 (Dichlormethan/Diisopropylether, 50:1)

### Oxidation von ω-Halogenverbindungen mittels Pvridin-N-oxid

### 6-Acetoxy-hexanal

In einer Inertgasatmosphäre wurden 29,0 g (130 mmol = 6-Bromhexylacetat, 31,6 g (332 mmol) Pyridin-N-oxid, 26,8 g (319 mmol) NaHCO₃ und 200 ml Toluol 18 Stunden unter Rückfluß erhitzt. Die Reaktionslösung wurde mit 400 ml Wasser gewaschen und die wäßrige Phase mit 300 ml Toluol nachextrahiert. Nachdem man das Lösungsmittel der vereinigten organischen Phasen im Vakuum abdestilliert hatte, wurde das Rohprodukt an 300 g Kieselgel (Diisopropylether/Cyclohexan, 1:1 ) säulenfiltriert.
Ausbeute: 12,5 g (79 mmol, 61 %)
MG = 158,20 g/mol (C₈H₁₄O₃)
R_{f} = 0,44 Diisopropylether)

| *Analyse:* | **C** | **H** |
|---|---|---|
| ber. | 60,74 | 8,92 |
| gef. | 60,66 | 8,92 |

¹H-NMR (300 MHz, CDCl₃): δ = 1,30-1,41 (m, 2H, 4-CH₂), 1,57-1,68 (m, 4H, CH₂CH₂CHO, CH₂CH₂O), 2,00 (s, 3H, OOCCH₃), 2,42 (dt, ³J_{2,1} = 1,6 Hz, ³J_{2,3} = 7,3 Hz, 2H, CH₂CHO), 4,02 (t ³J = 6,6 Hz, 2H, CH₂O), 9,73 (t, ³J = 1,6 Hz, 1H, CHO)
IR (Film): *v*[cm⁻¹] = 2941 (s), 2865 (s), 2724 (m), 1736 (s), 1462 (m), 1389 (m), 1367 (s), 1241 (s), 1048 (s), 634 (m), 607 (m)

### Beispiel 3

Die Synthese der (Z)-Alkenole bzw. der einfach ungesättigter (Z)-Fettsäuren erfolgt durch steroselektive Wittig-Reaktion eines ω-substituierten Aldehyds mit einem unsubstituierten Phosphoniumsalz bzw. durch Umsetzung eines ω-substituierten Phosphoniumsalzes mit einem unsubstituierten Aldehyd.

Unsubstituierte Aldehyde mit einer Reinheit von über 97 % sind bis zu einer Kettenlänge 12 Kohlenstoffatomen (Dodecanal) im Chemikalienhandel erhältlich und können direkt in die Wittig-Reaktion eingesetzt werden. Längerkettige Aldehyde können aus den käuflichen Fettalkoholen durch Swern- oder Kornblum-Oxidation erhalten werden. Unsubstituierte Alkylhalogenide (vowiegend Bromide sowie Chloride) dienen zur Herstellung einfacher Phosphoniumbromide, wobei Alkylhalogenide mit bis zu in über 97 %-iger Reinheit käuflich erworben werden können. Auf die Synthese ω-substituierter Wittig-Edukte wird im Beispiel 1 und 2 hingewiesen. Die Generierung der Ylid-Lösungen von Phosphoniumiodiden gestaltet sich einfacher, weil die Deprotonierung schon bei tieferen Temperaturen einsetzt und das Reaktionsgemisch somit nicht erhitzt werden muß. Die Fettsäuren lassen sich teilweise ohne chromatographische Reinigung durch Fällung ihrer Kaliumsalze in guter Reinheit gewinnen.

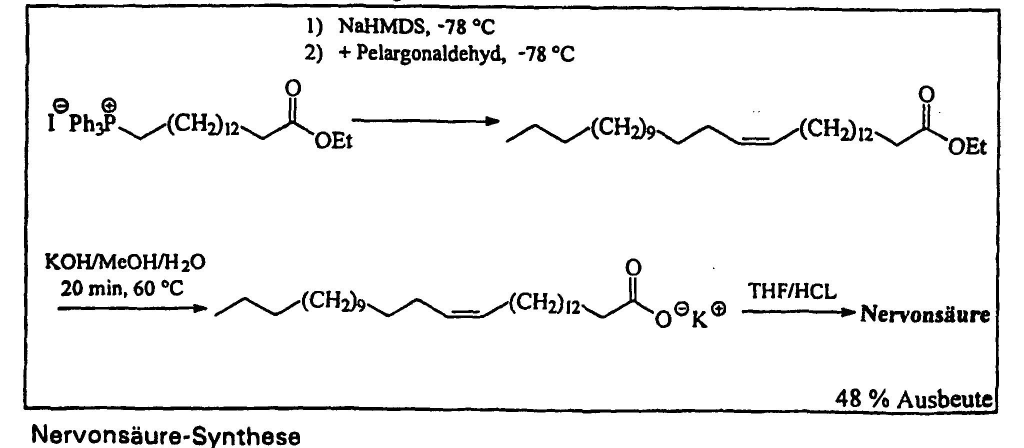

Ungesättigte Fettsäuren können durch in der Literatur beschriebene Verfahren mittels Lithiumaluminiumhydrid in die entsprechenden Fettalkohole überführt werden.

### (Z)-Steroselektive Wittig-Reaktion eines ω-substituierten Phosphoniumbromids

### (Z)-10-Docosen-1-ol

86,7 g (160 mmol) [10-(Acetoxy)-decyl]triphenylphosphoniumbromid wurden in 400 ml trockenem THF vorgelegt. In einer Argon-Atmosphäre wurden langsam 200 ml Natrium-bis(trimethylsilyl)amid (1M in THF) in die Reaktionslösung gespritzt. Man ließ 30 Minuten bei Raumtemperatur rühren (KPG-Rührer), bevor man eine Stunde unter Rückfluß erhitzte. Danach wurde die Ylid-Lösung erst auf 10 °C, dann auf -78 °C abgekühlt. nach 30 Minuten Rühren bei dieser Temperatur ließ man langsam 30,0 g (163 mmol) Laurinaldehyd in 50 ml THF zutropfen. Es wurde weitere 30 Minuten gerührt, dann ließ man über Nacht auf Raumtemperatur erwärmen.

### Aufarbeitung

Das Reaktionsgemisch wurde mit 600 ml Wasser und 200 ml Diethylether versetzt, die Phasen getrennt und das Lösungsmittel der organischen Phase im Vakuum entfernt. Zur Verseifung wurde eine Lösung aus 25 g Kaliumhydroxid in 10 ml Wasser/200 ml Methanol zugefügt und 20 Minuten bei 60 °C gerührt. Die Reaktionslösung wurde mit 600 ml Wasser versetzt und mit 300 ml Diethylether extrahiert. Nachdem man die organische Phase mit 500 ml ges. NaHCO₃-Lösung und 500 ml Wasser gewaschen hatte, wurde das Lösungsmittel im Vakuum abdestilliert. Das Rohprodukt wurde durch Säulenchromatographie (Cyclohexan/Diisopropylether; sukzessive Erhöhung der Polarität von 19:1 auf 1: 1) an 550 g Kieselgel gereinigt. Die Verbindung wurde bei -20 °C aus Aceton gefällt. Nach mehrtägiger Trocknung im Exsikkator erhielt man 26,8 g (82,6 mmol, 52 %) des langkettigen Fettalkohols.
¹H-NMR (300 MHz, CDCl₃): δ = 0,88 (t, ³J = 6,6 Hz, 3H, Alkyl-CH₃), 123-1,30 (m, 30H, -CH₂-), 1,56 (mc, 2H, CH₂CH₂OH), 2,00 (m, 4H, Allyl-H), 3,64 (t, ³J = 6,2 Hz, 2H, CH₂OH), 5,35 (t, ³J_{cis} = 3,8 Hz, 2H, -CH=CHcis)
IR (KBr): *v*[cm⁻¹] = 3366 (m), 2998 (m), 2918 (s), 2848 (s), 1459 (m), 1366 (w), 1067 (m), 724 (m), 688 (w), 580 (w)
MG (C₂₂H₄₄O) = 324,59 g/mol

| *Analyse:* | **C** | **H** |
|---|---|---|
| ber. | 81,41 | 13,66 |
| gef. | 81,56 | 13,72 |

### Stereoselektive Wittig-Reaktion eines ω-substituierten Phosphoniumiodids

### (Z)-15-Tetracosensäure (Nervonsäure)

In einer Inertgasatmosphäre wurden 197,4 g (300 mmol) des entsprechenden Phosphoniumsalzes in 1100 ml trockenem THF vorgelegt. Man kühlte auf -78 °C ab und tropfte unter Rühren (KPG-Rührer) langam 360 ml Natrium-bis(trimethylsilyl)amid (1M in THF) in die Reaktionslösung. Es wurde 30 Minuten bei dieser Temperatur gerührt, dann ließ man über einen Zeitraum von 40 Minuten eine Lösung aus 47,0 g (330 mmol) Pelargonaldehyd in 50 ml THF zutropfen. Nach 30 Minuten intensiven Rühren ließ man über Nacht auf Raumtemperatur erwärmen.

### Aufarbeitung

Zum Reaktionsgemisch wurden 50 ml Wasser gegeben, dann entfernte man das Lösungsmittel im Vakuum. Eine Lösung aus 25 g Kaliumhydroxid in 10 ml Wasser/200 ml Methanol wurde hinzugefügt und die Reaktionslösung 20 Minuten bei 60 °C gerührt. Danach wurde unter Zusatz von Toluol und Destillation im Vakuum azeotrop getrocknet. Der Rückstand wurde mit 1,5 I Aceton unter starkem Rühren 10 Minuten auf 60 °C erhitzt. Das dabei ausfallende Kaliumsalz wurde abgesaugt und mehrmals mit Aceton gewaschen. Mit Hilfe einer Lösung aus 600 ml THF/150 ml konz. Salzsäure wurde das Produkt vom Filter gelöst. Das resultierende zweiphasige Gemisch wurde mit 500 ml Diisopropylether versetzt und die Phasen getrennt. Die organische Phase wurde dreimal mit je 500 ml Wasser gewaschen, über Natriumsulfat getrocknet und das Lösungsmittel im Vakuum abdestilliert.

Das Rohprodukt wurde durch Säulenchromatographie an 1100 g Kieselgel gereinigt. Dabei wurde zuerst die apolare Verunreinigung mit Cyclohexan/Diisopropylether ( 19:1 ) eluiert. Chromatographie mit Cyclohexan/Diisopropylether (1:1) lieferte das Produkt.

Die Säure wurde in der Wärme in Aceton gelöst und bei -20 °C kristallisiert. Im trockenen Zustand erhielt man 52,5 g (142 mmol, 48 %) der Fettsäure als weißes, kristallines Pulver.
MG = 366,63 g/mol (C₂₄H₄₆O₂)

| *Analyse:* | **C** | **H** |
|---|---|---|
| ber. | 78,63 | 12,65 |
| gef. | 78,77 | 12,52 |

Schmelzpunkt: 41,1 °C (Lit. 42-43 °C)

Die Herstellung einfach ungesättigter (Z)-Alkenole und (Z)-Fettsäuren kann zudem durch Umsetzung ω-substituierter Aldehyde mit gesättigten Phosphoniumsalzen nach den oben beschriebenen Verfahren erfolgen.

Terminal ungesättigte Alkadiencarbonsäuren werden durch (Z)-selektive Wittig-Reaktion eines terminal ungesättigten Aldehyds mit einem ω-substituierten Phosphoniumsalz (z.B. 10-Undecenal) gewonnen.

### Beispiel 4

Durch beidseitige Umsetzung von α,ω-Dibromalkanen mit Triphenylphosphan erhält man [α,ω-Bis(triphenylphosphonium)alkan]dibromide. Nach Überführung in das Bis-phosphoran wird unter salzfreien Bedingungen mit einer Lösung aus einem substituierten und einem unsubstituierten Aldehyd stereospezifisch olefiniert. Die alkalische Verseifung des resultierenden Esters liefert je nach verwendetem Aldehyd (Z,Z)-Alkadioenole oder (Z,Z)-Fettsäuren.

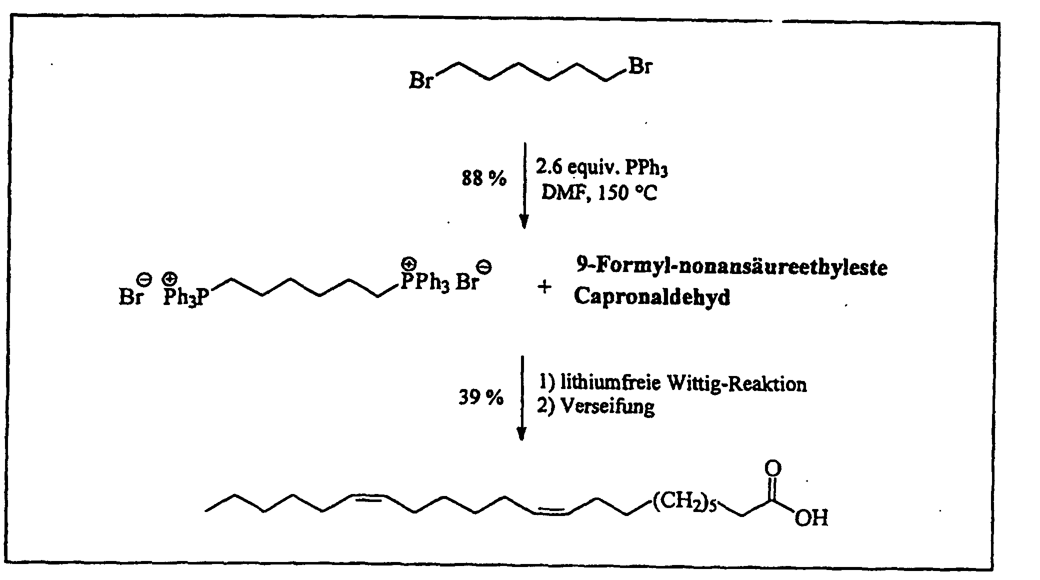

### Lithiumsalzfreie gekreuzte Wittig-Reaktion eines Bisphosphoniumsalzes mit einem unsubstituierten sowie einem ω-substituierten Aldehyd: Synthese von (Z,Z)-10,16-Docosadien-1-ol

### Synthese eines [α,ω-Bis(triphenylphosphonium)alkan]dibromids

### [1,6-Bis(triphenylphosphonium)hexan]dibromid (62)

122,2 g (0,50 mol) 1,6-Dibromhexan wurden zusammen mit 341,7 g (1,30 mol) Triphenylphosphan in 1500 ml DMF gelöst. Das Reaktionsgemisch wurde unter Rühren (KPG-Rührer) 4 Stunden unter Rückfluß erhitzt. Man ließ auf Raumtemperatur abkühlen. Das Produkt wurde abgesaugt und mit 2 x 250 ml Aceton und 200 ml Diethylether gewaschen. Man erhielt nach mehrtägigem Trocknen im Vakuum 336,5 g (0,44 mol, 88 %) des kristallinen Bis-phosphoniumsalzes.
MG = 768,55 g/mol (C₄₂H₄₂Br₂P₂)
R_{f} = 0,26 (Chloroform/Methanol, 9:1)

| *Analyse:* | **C** | **H** | **P** |
|---|---|---|---|
| ber. | 66,64 | 5,51 | 8,06 |
| gef. | 65,77 | 5,59 | 7,98 |

### Gekreuzte Wittig-Reaktion

### (Z,Z)-10,16-Docosadiensäure

76,9 g (100 mmol [1,6-Bis(triphenylphosphonium)hexan]dibromid wurden in 500 ml THF aufgeschlämmt. In einer Inertgasatmosphäre wurden 240 ml (240 mmol) Natrium-bis(trimethylsilyl)amid (1 M in THF) durch ein Septum zugespritzt. Die Ylid-Lösung wurde 30 Minuten bei Raumtemperatur, dann 1 Stunde unter Rückfluß gerührt. Nachdem man auf -78 °C abgekühlt hatte, wurde innerhalb von 30 Minuten eine Lösung aus 21,5 g (100 mmol) 9-Formyl-nonansäureethylester und 10,1 g (101 mmol) Capronaldehyd in 50 ml THF zugetropft. Man ließ weitere 30 Minuten rühren, dann ließ man über Nacht auf Raumtemperatur erwärmen.

Zum Reaktionsgemisch wurden 50 ml Wasser gegeben, dann entfernte man das Lösungsmittel im Vakuum. Eine Lösung aus 25 g Kaliumhydroxid in 10 ml Wasser/200 ml Methanol wurde hinzugefügt und die Reaktionslösung 20 Minuten bei 60 °C gerührt. Danach wurde unter Zusatz von Toluol und Destillation im Vakuum azeotrop getrocknet. Der Rückstand wurde mit 1,5 I Aceton unter starkem Rühren 10 Minuten auf 60 °C erhitzt. Das dabei ausfallende Kaliumsalz wurde abgesaugt und mehrmals mit Aceton gewaschen. Mit Hilfe einer Lösung aus 600 ml THF/150 ml konz. Salzsäure wurde das Profukt vom Filter gelöst. Das resultierende zweiphasige Gemisch wurde mit 500 ml Diisopropylether versetzt und die Phasen getrennt. Die organische Phase wurde dreimal mit je 500 ml Wasser gewaschen, über Natriumsulfat getrocknet und das Lösungsmittel im Vakuum abdestilliert.

Das Rohprodukt wurde durch Säulenchromatographie (Cyclohexan/Diisopropylether; sukzessive Erhöhung der Polarität von 4:1 auf 1:1) an 400 g Kieselgel gereinigt. Man erhielt 13,0 g (38,6 mmol, 39 %) der zweifach ungesättigten Fettsäure.
MG = 336,56 g/mol (C₂₂H₄₀O₂)
R_{f} = 0,35 (Cyclohexan/Diisopropylether, 1:1)

| *Analyse:* | **C** | **H** |
|---|---|---|
| ber. | 78,51 | 11,98 |
| gef. | 78,30 | 11,92 |

¹H-NMR (300 MHz, CDCl₃): δ = 0,89 (t, ³J = 6,8 Hz, 3H, -CH₃), 1.30-1,43 (m, 20H, 10 x CH₂), 1,63 (mc, 2H, CH₂CH₂COOH), 2,03 (bs, 8H, Allyl-H), 2,35 (t, ³J = 7,5 Hz,2H, CH₂COOH), 5,34 (mc, 4H, -CH =CH-cis)

### Beispiel 5

### Vergleich des bekannten antitumoralen Wirkstoffes Erucylphosphocholin mit erfindungsgemäßen Wirkstoffen

Der Vergleich einer nicht erfindungsgemäßen Verbindung (Erucylphosphocholin) mit zwei erfindungsgemäßen Wirkstoffen ist in Tabelle 1 dargestellt.

**Tabelle 1**

| Alkylphosphocholin | Wöchentliche Dosis [µmol/kg] | T/C [%]* |
|---|---|---|
| Erucylphosphocholin (Daten übernommen aus Kaufmann-Kolle et al. 1996) | 90 | 31 |
| | 180 | 6 |
| | 360 | < 0,1 |
| (Z)-10-Docosenyl-1-PC | 42 | 9 |
| | 170 | 0,5 |
| | 256 | 0,2 |
| (Z)-11,21-Docosadienyl-1-PC | 42 | 8 |
| | 170 | 2 |

| | | |
|---|---|---|
| * Quotient des medianen Tumorvolumens der behandelten und der Kontrollgruppe x 100. Auswertung nach 5-wöchiger Therapie. | | |

Nachdem die Unwirksamkeit eines (Z,Z)-Alkadienylphosphocholins mit Methylen unterbrochenen Doppelbindungen auf der Basis der C₁₈-Kette bereits nachgewiesen wurde, konnte die Wirksamkeit der Substanzklasse durch Verlängerung der Alkadienylkette und einer deutlicheren Isolierung der Doppelbindungen voneinander wiederhergestellt werden (Tabelle 2).

**Tabelle 2**

| Ungesättigtes Alkylphosphocholin | Dosis [µmol/kg] | Medianes Tumorvolumen [cm³] | |
|---|---|---|---|
| | | Therapieende | 2 Wochen später |
| (Z)-12-Heneicosenyl-1-phosphocholin | 42 | 3,4 | 4,5 |
| | 84 | 0,3 | 1,2 |
| | 170 | 0,1 | 0,1 |
| | 256 | 0,2 | 0,8 |
| (Z)-10-Docosenyl-1-phosphocholin (Doppelbindung in ω-12-Position) | 42 | 4,0 | 4,5 |
| | 84 | 1,2 | 3,4 |
| | 170 | 0,2 | 0,2 |
| | 256 | 0,1 | 0,2 |
| (Z)-16-Docosenyl-1-phosphocholin (Doppelbindung in ω-6-Position) | 42 | 26,9 | -- |
| | 84 | 2,5 | 7,6 |
| | 170 | 0,2 | 0,4 |
| (Z,Z)-6,12-Eicosadienyl-1-PC | 42 | 10 | 13,9 |
| | 84 | 3,2 | 13,9 |
| | 170 | 0,4 | 1,9 |
| | 256 | 0 | 0 |
| (Z)-11,21-Docosandienyl-1-PC | 42 | 1,5 | 2,5 |
| | 84 | 0,9 | 2,9 |
| | 170 | 0,4 | 0,5 |
| (Z,Z)-10,16-Docosadienyl-1-PC | 42 | 7,5 | 11,4 |
| | 84 | 0,6 | 0,6 |
| | 170 | 0,5 | 0,7 |

### Beispiel 6: Beispielsverbindungen

Die Rf-Werte der Beispielsverbindungen wurden im System CHCl₃/CH₃OH/Eisessig/H₂O: 100/60/20/5 (Volumenanteile) bestimmt. Sie liegen gruppenweise sehr dicht beisammen und zwar wie folgt:

| **Rf** | **Verbindungen Nr.** |
|---|---|
| 0,10-0,15 | 1454-1496 |
| 0,15-0,20 | 1399 - 1453; 1543 - 1555 |
| 0,20-0,25 | 1320 - 1398; 1523 - 1542; 1752-1812 |
| 0,25-0,30 | 1497 - 1522; 1691 - 1751 |
| 0,30-0,35 | 1083 - 1319; 1556 - 1568; 1630 - 1690 |
| 0,35-0,40 | 1569 - 1629 |
| 0,40-0,45 | 1813 - 1839 |
| 0,30-0,40 | 1 - 1082 |

### 1. Beispiele für (Z)-Alkenylphosphocholine (Vergleichsbeispiel)

### (A = VIII; n = 2; R₃, CH₃; m= 1, x = 1; z = 0)

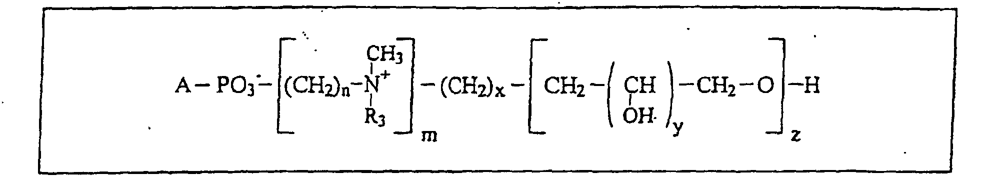

wobei A für eine einfach ungesättigte Alkylkette folgender Struktur steht (p,q ≥ 0; 12 ≤ p+q ≤ 30):

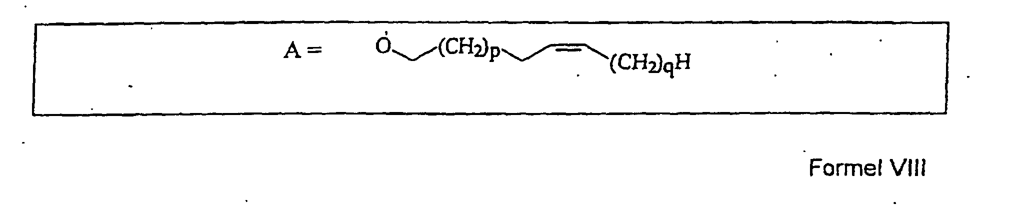

### 16 Kettenkohlenstoffatome

### C₂₁H₄₄NO₄P (405.56)

- 1.: (Z)-3-Hexadecenyl-1-phosphocholin
- 2.: (Z)-4-Hexadecenyl-1-phosphocholin
- 3.: (Z)-5-Hexadecenyl-1-phosphocholin
- 4.: (Z)-6-Hexadecenyl-1-phosphocholin
- 5.: (Z)-8-Hexadecenyl-1-phosphocholin
- 6.: (Z)-9-Hexadecenyl-1-phosphocholin
- 7.: (Z)-10-Hexadecenyl-1-phosphocholin
- 8.: (Z)-11-Hexadecenyl-1-phosphocholin
- 9.: (Z)-12-Hexadecenyl-1-phosphocholin
- 10.: (Z)-13-Hexadecenyl-1-phosphocholin
- 11.: (Z)-14-Hexadecenyl-1-phosphocholin
- 12.: 15-Hexadecenyl-1-phosphocholin

### 17 Kettenkohlenstoffatome

### C₂₂H₄₆NO₄P (419.59)

- 13.: (Z)-3-Heptadecenyl-1-phosphocholin
- 14.: (Z)-4-Heptadecenyl-1-phosphocholin
- 15.: (Z)-5-Heptadecenyl-1-phosphocholin
- 16.: (Z)-6-Heptadecenyl-1-phosphocholin
- 17.: (Z)-7-Heptadecenyl-1-phosphocholin
- 18.: (Z)-8-Heptadecenyl-1-phosphocholin
- 19.: (Z)-9-Heptadecenyl-1-phosphocholin
- 20.: (Z)-10-Heptadecenyl-1-phosphocholin
- 21.: (Z)-11-Heptadecenyl-1-phosphocholin
- 22.: (Z)-12-Heptadecenyl-1-phosphocholin
- 23.: (Z)-13-Heptadecenyl-1-phosphocholin
- 24.: (Z)-14-Heptadecenyl-1-phosphocholin
- 25.: (Z)-15-Heptadecenyl-1-phosphocholin
- 26.: 16-Heptadecenyl-1-phosphocholin

### 18 Kettenkohlenstoffatome

### C₂₃H₄₈NO₄P (433.61)

- 27.: (Z)-3-Octadecenyl-1-phosphocholin
- 28.: (Z)-4-Octadecenyl-1-phosphocholin
- 29.: (Z)-5-Octadecenyl-1-phosphocholin
- 30.: (Z)-6-Octadecenyl-1-phosphocholin
- 31.: (Z)-7-Octadecenyl-1-phosphocholin
- 32.: (Z)-8-Octadecenyl-1-phosphocholin
- 33.: (Z)-10-Octadecenyl-1-phosphocholin
- 34.: (Z)-11-Octadecenyl-1-phosphocholin
- 35.: (Z)-12-Octadecenyl-1-phosphocholin
- 36.: (Z)-13-Octadecenyl-1-phosphocholin
- 37.: (Z)-14-Octadecenyl-1-phosphocholin
- 38.: (Z)-15-Octadecenyl-1-phosphocholin
- 39.: (Z)-16-Octadecenyl-1-phosphocholin
- 40.: 17-Octadecenyl-1-phosphocholin

### 19 Kettenkohlenstoffatome

### C₂₄H₅₀NO₄P (447.64)

- 41.: (Z)-3-Nonadecenyl-1-phosphocholin
- 42.: (Z)-4-Nonadecenyl-1-phosphocholin
- 43.: (Z)-5-Nonadecenyl-1-phosphocholin
- 44.: (Z)-6-Nonadecenyl-1-phosphocholin
- 45.: (Z)-7-Nonadecenyl-1-phosphocholin
- 46.: (Z)-8-Nonadecenyl-1-phosphocholin
- 47.: (Z)-9-Nonadecenyl-1-phosphocholin
- 48.: (Z)-10-Nonadecenyl-1-phosphocholin
- 49.: (Z)-11-Nonadecenyl-1-phosphocholin
- 50.: (Z)-12-Nonadecenyl-1-phosphocholin
- 51.: (Z)-13-Nonadecenyl-1-phosphocholin
- 52.: (Z)-14-Nonadecenyl-1-phosphocholin
- 53.: (Z)-15-Nonadecenyl-1-phosphocholin
- 54.: (Z)-16-Nonadecenyl-1-phosphocholin
- 55.: (Z)-17-Nonadecenyl-1-phosphocholin
- 56.: 18-Nonadecenyl-1-phosphocholin

### 20 Kettenkohlenstoffatome

### C₂₅H₅₂NO₄P (461.67)

- 57.: (Z)-3-Eicosenyl-1-phosphocholin
- 58.: (Z)-4-Eicosenyl-1-phosphocholin
- 59.: (Z)-5-Eicosenyl-1-phosphocholin
- 60.: (Z)-6-Eicosenyl-1-phosphocholin
- 61.: (Z)-7-Eicosenyl-1-phosphocholin
- 62.: (Z)-8-Eicosenyl-1-phosphocholin
- 63.: (Z)-9-Eicosenyl-1-phosphocholin
- 64.: (Z)-10-Eicosenyl-1-phosphocholin
- 65.: (Z)-12-Eicosenyl-1-phosphocholin
- 66.: (Z)-13-Eicosenyl-1-phosphocholin
- 67.: (Z)-14-Eicosenyl-1-phosphocholin
- 68.: (Z)-15-Eicosenyl-1-phosphocholin
- 69.: (Z)-16-Eicosenyl-1-phosphocholin
- 70.: (Z)-17-Eicosenyl-1-phosphocholin
- 71.: (Z)-18-Eicosenyl-1-phosphocholin
- 72.: 19-Eicosenyl-1-phosphocholin

### 21 Kettenkohlenstoffatome

### C₂₆H₅₄NO₄P (475.69)

- 73.: (Z)-3-Heneicosenyl-1-phosphocholin
- 74.: (Z)-4-Heneicosenyl-1-phosphocholin
- 75.: (Z)-5-Heneicosenyl-1-phosphocholin
- 76.: (Z)-6-Heneicosenyl-1-phosphocholin
- 77.: (Z)-7-Heneicosenyl-1-phosphocholin
- 78.: (Z)-8-Heneicosenyl-1-phosphocholin
- 79.: (Z)-9-Heneicosenyl-1-phosphocholin
- 80.: (Z)-10-Heneicosenyl-1-phosphocholin
- 81.: (Z)-11-Heneicosenyl-1-phosphocholin
- 82.: (Z)-12-Heneicosenyl-1-phosphocholin
- 83.: (Z)-13-Heneicosenyl-1-phosphocholin
- 84.: (Z)-14-Heneicosenyl-1-phosphocholin
- 85.: (Z)-15-Heneicosenyl-1-phosphocholin
- 86.: (Z)-16-Heneicosenyl-1-phosphocholin
- 87.: (Z)-17-Heneicosenyl-1-phosphocholin
- 88.: (Z)-18-Heneicosenyl-1-phosphocholin
- 89.: (Z)-19-Heneicosenyl-1-phosphocholin
- 90.: 20-Heneicosenyl-1-phosphocholin

### 22 Kettenkohlenstoffatome

### C₂₇H₅₆NO₄P (489.72)

- 91.: (Z)-3-Docosenyl-1-phosphocholin
- 92.: (Z)-4-Docosenyl-1-phosphocholin
- 93.: (Z)-5-Docosenyl-1-phosphocholin
- 94.: (Z)-6-Docosenyl-1-phosphocholin
- 95.: (Z)-7-Docosenyl-1-phosphocholin
- 96.: (Z)-8-Docosenyl-1-phosphocholin
- 97.: (Z)-9-Docosenyl-1-phosphocholin
- 98.: (Z)-10-Docosenyl-1-phosphocholin
- 99.: (Z)-11-Docosenyl-1-phosphocholin
- 100.: (Z)-12-Docosenyl-1-phosphocholin
- 101.: (Z)-14-Docosenyl-1-phosphocholin
- 102.: (Z)-15-Docosenyl-1-phosphocholin
- 103.: (Z)-16-Docosenyl-1-phosphocholin
- 104.: (Z)-17-Docosenyl-1-phosphocholin
- 105.: (Z)-18-Oocosenyl-1-phosphocholin
- 106.: (Z)-19-Docosenyl-1-phosphocholin
- 107.: (Z)-20-Docosenyl-1-phosphocholin
- 108.: 21-Docosenyl-1-phosphocholin

### 23 Kettenkohlenstoffatome

### C₂₈H₅₈NO₄P (503.75)

- 109.: (Z)-3-Tricosenyl-1-phosphocholin
- 110.: (Z)-4-Tricosenyl-1-phosphocholin
- 111.: (Z)-5-Tricosenyl-1-phosphocholin
- 112.: (2)-6-Tricosenyl-1-phosphocholin
- 113.: (Z)-7-Tricosenyl-1-phosphocholin
- 114.: (Z)-8-Tricosenyl-1-phosphocholin
- 115.: (Z)-9-Tricosenyl-1-phosphocholin
- 116.: (Z)-10-Tricosenyl-1-phosphocholin
- 117.: (Z)-11-Tricosenyl-1-phosphocholin
- 118.: (Z)-12-Tricosenyl-1-phosphocholin
- 119.: (Z)-13-Tricosenyl-1-phosphocholin
- 120.: (Z)-14-Tricosenyl-1-phosphocholin
- 121.: (Z)-15-Tricosenyl-1-phosphocholin
- 122.: (Z)-16-Tricosenyl-1-phosphocholin
- 123.: (Z)-17-Tricosenyl-1-phosphocholin
- 124.: (Z)-18-Tricosenyl-1-phosphocholin
- 125.: (Z)-19-Tricosenyl-1-phosphocholin
- 126.: (Z)-20-Tricosenyl-1-phosphocholin
- 127.: (Z)-21-Tricosenyl-1-phosphocholin
- 128.: 22-Tricosenyl-1-phosphocholin

### 24 Kettenkohlenstoffatome

### C₂₉H₆₀NO₄P (517.77)

- 129.: (Z)-3-Tetracosenyl-1-phosphocholin
- 130.: (Z)-4-Tetracosenyl-1-phosphocholin
- 131.: (Z)-5-Tetracosenyl-1-phosphocholin
- 132.: (Z)-6-Tetracosenyl-1-phosphocholin
- 133.: (Z)-7-Tetracosenyl-1-phosphocholin
- 134.: (Z)-8-Tetracosenyl-1-phosphocholin
- 135.: (Z)-9-Tetracosenyl-1-phosphocholin
- 136.: (Z)-10-Tetracosenyl-1-phosphocholin
- 137.: (Z)-11-Tetracosenyl-1-phosphocholin
- 138.: (Z)-12-Tetracosenyl-1-phosphocholin
- 139.: (Z)-13-Tetracosenyl-1-phosphocholin
- 140.: (Z)-14-Tetracosenyl-1-phosphocholin
- 141.: (Z)-16-Tetracosenyl-1-phosphocholin
- 142.: (Z)-17-Tetracosenyl-1-phosphocholin
- 143.: (Z)-18-Tetracosenyl-1-phosphocholin

### 2. Beispiele für (Z)-Alkenyl-1-phospho-N,N,N-trimethyl-propylammonium-Verbindungen (Vergleichsbeispiel)

### (A = VIII; n = 3; R₃, CH₃; m= 1, x = 1; z = 0)

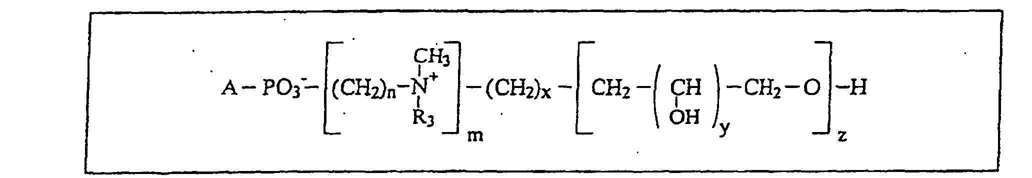

wobei A für eine einfach ungesättigte Alkylkette folgender Struktur steht (p,q ≥ 0; 12 ≤ p+q ≤ 30):

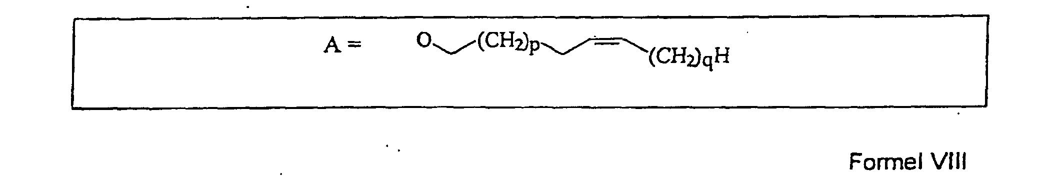

### 16 Kettenkohlenstoffatome

### C₂₂H₄₆NO₄P (419.59)

- 144.: (Z)-3-Hexadecenyl-1-phospho-N,N,N-trimethyl-propylammonium
- 145.: (Z)-4-Hexadecenyl-1-phospho-N,N,N-trimethyl-propylammonium
- 146.: (Z)-5-Hexadecenyl-1-phospho-N,N,N-trimethyl-propylammonium
- 147.: (Z)-6-Hexadecenyl-1-phospho-N,N,N-trimethyl-propylammonium
- 148.: (Z)-7-Hexadecenyl-1-phospho-N,N,N-trimethyl-propylammonium
- 149.: (Z)-8-Hexadecenyl-1-phospho-N,N,N-thmethyl-propylammonium
- 150.: (Z)-9-Hexadecenyl-1-phospho-N,N,N-trimethyl-propylammonium
- 151.: (Z)-10-Hexadecenyl-1-phospha-N,N,N-trimethyl-propylammonium
- 152.: (Z)-11-Hexadecenyl-1-phospho-N,N,N-trimethyl-propylammonium
- 153.: (Z)-12-Hexadecenyl-1-phospho-N,N,N-trimethyl-propylammonium
- 154.: (Z)-13-Hexadecenyl-1-phospho-N,N,N-trimethyl-propylammonium
- 155.: (Z)-14-Hexadecenyl-1-phospho-N,N,N-trimethyl-propylammonium
- 156.: 15-Hexadecenyl-1-phospho-N,N,N-trimethyl-propylammonium

### 17 Kettenkohlenstoffatome

### C₂₃H₄₈NO₄P (433.61)

- 157.: (Z)-3-Heptadecenyl-1-phospho-N,N,N-trimethyl-propylammonium
- 158.: (Z)-4-Heptadecenyl-1-phospho-N,N,N-trimethyl-propylammonium
- 159.: (Z)-5-Heptadecenyl-1-phospho-N,N,N-trimethyl-propylammonium
- 160.: (Z)-6-Heptadecenyl-1-phospho-N,N,N-trimethyl-propylammonium
- 161.: (Z)-7-Heptadecenyl-1-phospho-N,N,N-trimethyl-propylammonium
- 162.: (Z)-8-Heptadecenyl-1-phospho-N,N,N-trimethyl-propylammonium
- 163.: (Z)-9-Heptadecenyl-1-phospho-N,N,N-trimethyl-propylammonium
- 164.: (Z)-10-Heptadecenyl-1-phospho-N,N,N-trimethyl-propylammonium
- 165.: (Z)-11-Heptadecenyl-1-phospho-N,N,N-trimethyl-propylammonium
- 166.: (Z)-12-Heptadecenyl-1-phospho-N,N,N-trimethyl-propylammonium
- 167.: (Z)-13-Heptadecenyl-1-phospho-N,N,N-trimethyl-propylammonium
- 168.: (Z)-14-Heptadecenyl-1-phospho-N,N,N-trimethyl-propylammonium
- 169.: (Z)-15-Heptadecenyl-1-phospho-N,N,N-trimethyl-propylammonium
- 170.: 16-Heptadecenyl-1-phospho-N,N,N-trimethyl-propylammonium

### 18 Kettenkohlenstoffatome

### C₂₄H₅₀NO₄P (447.64)

- 171.: (Z)-3-Octadecenyl-1-phospho-N,N,N-trimethyl-propylammonium
- 172.: (Z)-4-Octadecenyl-1-phospho-N,N,N-trimethyl-propylammonium
- 173.: (Z)-5-Octadecenyl-1-phospho-N,N,N-trimethyl-propylammonium
- 174.: (Z)-6-Octadecenyl-1-phospho-N,N,N-trimethyl-propylammonium
- 175.: (Z)-7-Octadecenyl-1-phospho-N,N,N-trimethyl-propylammonium
- 176.: (Z)-8-Octadecenyl-1-phospho-N,N,N-trimethyl-propylammanium
- 177.: (Z)-10-Octadecenyl-1-phospho-N,N,N-trimethyl-propylammonium
- 178.: (Z)-11-Octadecenyl-1-phospho-N,N,N-trimethyl-propylammonium
- 179.: (Z)-12-Octadecenyl-1-phospho-N,N,N-trimethyl-propylammonium
- 180.: (Z)-13-Octadecenyl-1-phospho-N,N,N-trimethyl-propylammonium
- 181.: (Z)-14-Octadecenyl-1-phospho-N,N,N-trimethyl-propylammonium
- 182.: (Z)-15-Octadecenyl-1-phospho-N,N,N-trimethyl-propylammonium
- 183.: (Z)-16-Octadecenyl-1-phospho-N,N,N-trimethyl-propylammonium
- 184.: 17-Octadecenyl-1-phospho-N,N,N-trimethyl-propylammonium

### 19 Kettenkohlenstoffatome

### C₂₅H₅₂NO₄P (461.67)

- 185.: (Z)-3-Nonadecenyl-1-phospho-N,N,N-trimethyl-propylammonium
- 186.: (Z)-4-Nonadecenyl-1-phospho-N,N,N-trimethyl-propylammonium
- 187.: (Z)-5-Nonadecenyl-1-phospho-N,N,N-trimethyl-propylammonium
- 188.: (Z)-6-Nonadecenyl-1-phospho-N,N,N-trimethyl-propylammonium
- 189.: (Z)-7-Nonadecenyl-1-phospho-N,N,N-trimethyl-propylammonium
- 190.: (Z)-8-Nonadecenyl-1-phospho-N,N,N-trimethyl-propylammonium
- 191.: (Z)-9-Nonadecenyl-1-phospho-N,N,N-trimethyl-propylammonium
- 192.: (Z)-10-Nonadecenyl-1-phospho-N,N,N-trimethyl-propylammonium
- 193.: (Z)-11-Nonadecenyl-1-phospho-N,N,N-trimethyl-propylammonium
- 194.: (Z)-12-Nonadecenyl-1-phospho-N,N,N-trimethyl-propylammonium
- 195.: (Z)-13-Nonadecenyl-1-phospho-N,N,N-trimethyl-propylammonium
- 196.: (Z)-14-Nonadecenyl-1-phospho-N,N,N-trimethyl-propylammonium
- 197.: (Z)-15-Nonadecenyl-1-phospho-N,N,N-trimethyl-propylammonium
- 198.: (Z)-16-Nonadecenyl-1-phospho-N,N,N-trimethyl-propylammonium
- 199.: (Z)-17-Nonadecenyl-1-phospho-N,N,N-trimethyl-propylammonium
- 200.: 18-Nonadecenyl-1-phospho-N,N,N-trimethyl-propylammonium

### 20 Kettenkohlenstoffatome

### C₂₆H₅₄NO₄P (475.69)

- 201.: (Z)-3-Eicosenyl-1-phospho-N,N,N-trimethyl-propylammonium
- 202.: (Z)-4-Eicosenyl-1-phospho-N,N,N-trimethyl-propylammonium
- 203.: (Z)-5-Eicosenyl-1-phospho-N,N,N-trimethyl-propylammonium
- 204.: (Z)-6-Eicosenyl-1-phospho-N,N,N-trimethyl-propylammonium
- 205.: (Z)-7-Eicosenyl-1-phospho-N,N,N-trimethyl-propylammonium
- 206.: (Z)-8-Eicosenyl-1-phospho-N,N,N-trimethyl-propylammonium
- 207.: (Z)-9-Eicosenyl-1-phospho-N,N,N-trimethyl-propylammonium
- 208.: (Z)-10-Eicosenyl-1-phospho-N,N,N-trimethyl-propylammonium
- 209.: (Z)-12-Eicosenyl-1-phospho-N,N,N-trimethyl-propylammonium
- 210.: (Z)-13-Eicosenyl-1-phospho-N,N,N-trimethyl-propylammonium
- 211.: (Z)-14-Eicosenyl-1-phospho-N,N,N-trimethyl-propylammonium
- 212.: (Z)-15-Eicosenyl-1-phospho-N,N,N-trimethyl-propylammonium
- 213.: (Z)-16-Eicosenyl-1-phospho-N,N,N-trimethyl-propylammonium
- 214.: (Z)-17-Eicosenyl-1-phospho-N,N,N-trimethyl-propylammonium
- 215.: (Z)-18-Eicosenyl-1-phospho-N,N,N-trimethyl-propylammonium
- 216.: 19-Eicosenyl-1-phospho-N,N,N-trimethyl-propylammonium

### 21 Kettenkohlenstoffatome

### C₂₇H₅₆NO₄P (489.72)

- 217.: (Z)-3-Heneicosenyl-1-phospho-N,N,N-trimethyl-propylammonium
- 218.: (Z)-4-Heneicosenyl-1-phospho-N,N,N-trimethyl-propylammonium
- 219.: (Z)-5-Heneicosenyl-1-phospho-N,N,N-trimethyl-propylammonium
- 220.: (Z)-6-Heneicosenyl-1-phospho-N,N,N-trimethyl-propylammonium
- 221.: (Z)-7-Heneicosenyl-1-phospho-N,N,N-trimethyl-propylammonium
- 222.: (Z)-8-Heneicosenyl-1-phospho-N,N,N-trimethyl-propylammonium
- 223.: (Z)-9-Heneicosenyl-1-phospho-N,N,N-trimethyl-propylammonium
- 224.: (Z)-10-Heneicosenyl-1-phospho-N,N,N-trimethyl-propylammonium
- 225.: (Z)-11-Heneicosenyl-1-phospho-N,N,N-trimethyl-propylammonium
- 226.: (Z)-12-Heneicosenyl-1-phospho-N,N,N-trimethyl-propylammonium
- 227.: (Z)-13-Heneicosenyl-1-phospho-N,N,N-trimethyl-propylammonium
- 228.: (Z)-14-Heneicosenyl-1-phospho-N,N,N-trimethyl-propylammonium
- 229.: (Z)-15-Heneicosenyl-1-phospho-N,N,N-trimethyl-propylammonium
- 230.: (Z)-16-Heneicosenyl-1-phospho-N,N,N-trimethyl-propylammonium
- 231.: (Z)-17-Heneicosenyl-1-phospho-N,N,N-trimethyl-propylammonium
- 232.: (Z)-18-Heneicosenyl-1-phospho-N,N,N-trimethyl-propylammonium
- 233.: (Z)-19-Heneicosenyl-1-phospho-N,N,N-trimethyl-propylammonium
- 234.: 20-Heneicosenyl-1-phospho-N,N,N-trimethyl-propylammonium

### 22 Kettenkohlenstoffatome

### C₂₈H₅₈NO₄P (503.75)

- 235.: (Z)-3-Docosenyl-1-phospho-N,N,N-trimethyl-propylammonium
- 236.: (Z)-4-Docosenyl-1-phospho-N,N,N-trimethyl-propylammonium
- 237.: (Z)-5-Docosenyl-1-phospho-N,N,N-trimethyl-propylammonium
- 238.: (Z)-6-Docosenyl-1-phospho-N,N,N-trimethyl-propylammonium
- 239.: (Z)-7-Docosenyl-1-phospho-N,N,N-trimethyl-propylammonium
- 240.: (Z)-8-Docosenyl-1-phospho-N,N,N-trimethyl-propylammonium
- 241.: (Z)-9-Docosenyl-1-phospho-N,N,N-trimethyl-propylammonium
- 242.: (Z)-10-Docosenyl-1-phospho-N,N,N-trimethyl-propylammonium
- 243.: (Z)-11-Docosenyl-1-phospho-N,N,N-trimethyl-propylammonium
- 244.: (Z)-12-Docosenyl-1-phospho-N,N,N-trimethyl-propylammonium
- 245.: (Z)-14-Docosenyl-1-phospho-N,N,N-trimethyl-propylammonium
- 246.: (Z)-15-Docosenyl-1-phospho-N,N,N-trimethyl-propylammonium
- 247.: (Z)-16-Docosenyl-1-phospho-N,N,N-trimethyl-propylammonium
- 248.: (Z)-17-Docosenyl-1-phospho-N,N,N-trimethyl-propylammonium
- 249.: (Z)-18-Docosenyl-1-phospho-N,N,N-trimethyl-propylammonium
- 250.: (Z)-19-Docosenyl-1-phospho-N,N,N-trimethyl-propylammonium
- 251.: (Z)-20-Docosenyl-1-phospho-N,N,N-trimethyl-propylammonium
- 252.: 21-Docosenyl-1-phospho-N,N,N-trimethyl-propylammonium

### 23 Kettenkohlenstoffatome

### C₂₉H₆₀NO₄P (517.77)

- 253.: (Z)-3-Tricosenyl-1-phospho-N,N,N-trimethyl-propylammonium
- 254.: (Z)-4-Tricosenyl-1-phospho-N,N,N-trimethyl-propylammonium
- 255.: (Z)-5-Tricosenyl-1-phospho-N,N,N-trimethyl-propylammonium
- 256.: (Z)-6-Tricosenyl-1-phospho-N,N,N-trimethyl-propylammonium
- 257.: (Z)-7-Tricosenyl-1-phospho-N,N,N-trimethyl-propylammonium
- 258.: (Z)-8-Tricosenyl-1-phospho-N,N,N-trimethyl-propylammonium
- 259.: (Z)-9-Tricosenyl-1-phospho-N,N,N-trimethyl-propylammonium
- 260.: (Z)-10-Tricosenyl-1-phospho-N,N,N-trimethyl-propylammonium
- 261.: (Z)-11-Tricosenyl-1-phospho-N,N,N-trimethyl-propylammonium
- 262.: (Z)-12-Tricosenyl-1-phospho-N,N,N-trimethyl-propylammonium
- 263.: (Z)-13-Tricosenyl-1-phospho-N,N,N-trimethyl-propylammonium
- 264.: (Z)-14-Tricosenyl-1-phospho-N,N,N-trimethyl-propylammonium
- 265.: (Z)-15-Tricosenyl-1-phospho-N,N,N-trimethyl-propylammonium
- 266.: (Z)-16-Tricosenyl-1-phospho-N,N,N-trimethyl-propylammonium
- 267.: (Z)-17-Tricosenyl-1-phospho-N,N,N-trimethyl-propylammonium
- 268.: (Z)-18-Tricosenyl-1-phospho-N,N,N-trimethyl-propylammonium
- 269.: (Z)-19-Tricosenyl-1-phospho-N,N,N-trimethyl-propylammonium
- 270.: (Z)-20-Tricosenyl-1-phospho-N,N,N-trimethyl-propylammonium
- 271.: (Z)-21 -Tricosenyl-1-phospho-N,N,N-trimethyl-propylammonium
- 272.: 22-Tricosenyl-1-phospho-N,N,N-trimethyl-propylammonium

### 24 Kettenkohlenstoffatome

### C₃₀H₆₂NO₄P (531.80)

- 273.: (Z)-3-Tetracosenyl-1-phospho-N,N,N-trimethyl-propylammonium
- 274.: (Z)-4-Tetracosenyl-1-phospho-N,N,N-trimethyl-propylammonium
- 275.: (Z)-5-Tetracosenyl-1-phospho-N,N,N-trimethyl-propylammonium
- 276.: (Z)-6-Tetracosenyl-1-phospho-N,N,N-trimethyl-propylammonium
- 277.: (Z)-7-Tetracosenyl-1-phospho-N,N,N-trimethyl-propylammonium
- 278.: (Z)-8-Tetracosenyl-1-phospho-N,N,N-trimethyl-propylammonium
- 279.: (Z)-9-Tetracosenyl-1-phospha-N,N,N-trimethyl-propylammonium
- 280.: (Z)-10-Tetracosenyl-1-phospho-N,N,N-trimethyl-propylammonium
- 281.: (Z)-11-Tetracosenyl-1-phospho-N,N,N-trimethyl-propylammonium
- 282.: (Z)-12-Tetracosenyl-1-phospho-N,N,N-trimethyl-propylammonium
- 283.: (Z)-13-Tetracosenyl-1-phospho-N,N,N-trimethyl-propylammonium
- 284.: (Z)-14-Tetracosenyl-1-phospho-N,N,N-trimethyl-propylammonium
- 285.: (Z)-15-Tetracosenyl-1-phospho-N,N,N-trimethyl-propylammonium
- 286.: (Z)-16-Tetracosenyl-1-phospho-N,N,N-trimethyl-propylammonium
- 287.: (Z)-17-Tetracosenyl-1-phospho-N,N,N-trimethyl-propylammonium
- 288.: (Z)-18-Tetracosenyl-1-phospho-N,N,N-trimethyl-propylammonium

### 3. Beispiele für (Z)-Alkenyl-1-phospho-N,N,N-trimethyl-butylammonium-Verbindungen (Vergleichsbeispiel)

### (A = VIII; n = 4; R₃, CH₃; m= 1, x = 1; z = 0)

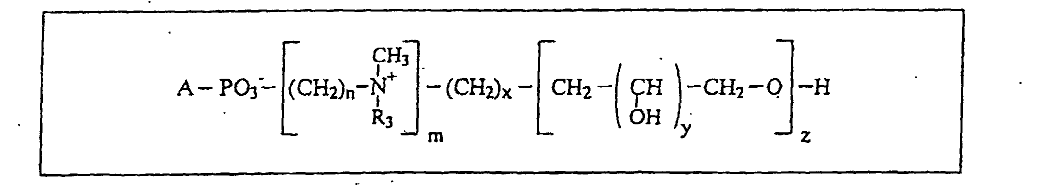

wobei A für eine einfach ungesättigte Alkylkette folgender Struktur steht (p,q ≥ 0; 12 ≤ p+q ≤ 30):

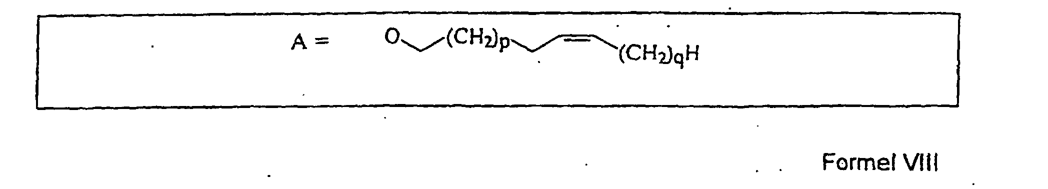

### 16 Kettenkohlenstoffatome

### C₂₃H₄₈NO₄P (433.61)

- 289.: (Z)-3-Hexadecenyl-1-phospho-N,N,N-trimethyl-butylammonium
- 290.: (Z)-4-Hexadecenyl-1-phospho-N,N,N-trimethyl-butylammonium
- 291.: (Z)-5-Hexadecenyl-1-phospho-N,N,N-trimethyl-butylammonium
- 292.: (Z)-6-Hexadecenyl-1-phospho-N,N,N-trimethyl-butylammonium
- 293.: (Z)-7-Hexadecenyl-1-phospho-N,N,N-trimethyl-butylammonium
- 294.: (Z)-8-Hexadecenyl-1-phospho-N,N,N-trimethyl-butylammonium
- 295.: (Z)-9-Hexadecenyl-1-phospho-N,N,N-trimethyl-butylammonium
- 296.: (Z)-10-Hexadecenyl-1-phospho-N,N,N-trimethyl-butylammonium
- 297.: (Z)-11-Hexadecenyl-1-phospho-N,N,N-trimethyl-butylammonium
- 298.: (Z)-12-Hexadecenyl-1-phospho-N,N,N-trimethyl-butylammonium
- 299.: (Z)-13-Hexadecenyl-1-phospho-N,N,N-trimethyl-butylammonium
- 300.: (Z)-14-Hexadecenyl-1-phospho-N,N,N-trimethyl-butylammonium
- 301.: 15-Hexadecenyl-1-phospho-N,N,N-trimethyl-butylammonium

### 17 Kettenkohlenstoffatome

### C₂₄H₅₀NO₄P (447.64)

- 302.: (Z)-3-Heptadecenyl-1-phospho-N,N,N-trimethyl-butylammonium
- 303.: (Z)-4-Heptadecenyl-1-phospho-N,N,N-trimethyl-butylammonium
- 304.: (Z)-5-Heptadecenyl-1-phospho-N,N,N-trimethyl-butylammonium
- 305.: (Z)-6-Heptadecenyl-1-phospho-N,N,N-trimethyl-butylammonium
- 306.: (Z)-7-Heptadecenyl-1-phospho-N,N,N-trimethyl-butylammonium
- 307.: (Z)-8-Heptadecenyl-1-phospho-N,N,N-trimethyl-butylammonium
- 308.: (Z)-9-Heptadecenyl-1-phospho-N,N,N-trimethyl-butylammonium
- 309.: (Z)-10-Heptadecenyl-1-phospho-N,N,N-trimethyl-butylammonium
- 310.: (Z)-11-Heptadecenyl-1-phospho-N,N,N-trimethyl-butylammonium
- 311.: (Z)-12-Heptadecenyl-1-phospho-N,N,N-trimethyl-butylammonium
- 312.: (Z)-13-Heptadecenyl-1-phospho-N,N,N-trimethyl-butylammonium
- 313.: (Z)-14-Heptadecenyl-1-phospho-N,N,N-trimethyl-butylammonium
- 314.: (Z)-15-Heptadecenyl-1-phospho-N,N,N-trimethyl-butylammonium
- 315.: 16-Heptadecenyl-1-phospho-N,N,N-trimethyl-butylammonium

### 18 Kettenkohlenstoffatome

### C₂₅H₅₂NO₄P (461.67)

- 316.: (Z)-3-Octadecenyl-1-phospho-N,N,N-trimethyl-butylammonium
- 317.: (Z)-4-Octadecenyl-1-phospho-N,N,N-trimethyl-butylammonium
- 318.: (Z)-5-Octadecenyl-1-phospho-N,N,N-trimethyl-butylammonium
- 319.: (Z)-6-Octadecenyl-1-phospho-N,N,N-trimethyl-butylammonium
- 320.: (Z)-7-Octadecenyl-1-phospho-N,N,N-trimethyl-butylammonium
- 321.: (Z)-8-Octadecenyl-1-phospho-N,N,N-trimethyl-butylammonium
- 322.: (Z)-10-Octadecenyl-1-phospho-N,N,N-trimethyl-butylammonium
- 323.: (Z)-11-Octadecenyl-1-phospho-N,N,N-trimethyl-butylammonium
- 324.: (Z)-12-Octadecenyl-1-phospho-N,N,N-trimethyl-butylammonium
- 325.: (Z)-13-Octadecenyl-1-phospho-N,N,N-trimethyl-butylammonium
- 326.: (Z)-14-Octadecenyl-1-phospho-N,N,N-trimethyl-butylammonium
- 327.: (Z)-15-Octadecenyl-1-phospho-N,N,N-trimethyl-butylammonium
- 328.: (Z)-16-Octadecenyl-1-phospho-N,N,N-trimethyl-butylammonium
- 329.: 17-Octadecenyl-1-phospho-N,N,N-trimethyl-butylammonium

### 19 Kettenkohlenstoffatome

### C₂₆H₅₄NO₄P (475.69)

- 330.: (Z)-3-Nonadecenyl-1-phospho-N,N,N-trimethyl-butylammonium
- 331.: (Z)-4-Nonadecenyl-1-phospho-N,N,N-trimethyl-butylammonium
- 332.: (Z)-5-Nonadecenyl-1-phospho-N,N,N-trimethyl-butylammonium
- 333.: (Z)-6-Nonadecenyl-1-phospho-N,N,N-trimethyl-butylammonium
- 334.: (Z)-7-Nonadecenyl-1-phospho-N,N,N-trimethyl-butylammonium
- 335.: (Z)-8-Nonadecenyl-1-phospho-N,N,N-trimethyl-butylammonium
- 336.: (Z)-9-Nonadecenyl-1-phospho-N,N,N-trimethyl-butylammonium
- 337.: (Z)-10-Nonadecenyl-1-phospho-N,N,N-trimethyl-butylammonium
- 338.: (Z)-11-Nonadecenyl-1-phospho-N,N,N-trimethyl-butylammonium
- 339.: (Z)-12-Nonadecenyl-1-phospho-N,N,N-trimethyl-butylammonium
- 340.: (Z)-13-Nonadecenyl-1-phospho-N,N,N-trimethyl-butylammonium
- 341.: (Z)-14-Nonadecenyl-1-phospho-N,N,N-trimethyl-butylammonium
- 342.: (Z)-15-Nonadecenyl-1-phospho-N,N,N-trimethyl-butylammonium
- 343.: (Z)-16-Nonadecenyl-1-phospho-N,N,N-trimethyl-butylammonium
- 344.: (Z)-17-Nonadecenyl-1-phospho-N,N,N-trimethyl-butylammonium
- 345.: 18-Nonadecenyl-1-phospho-N,N,N-trimethyl-butylammonium

### 20 Kettenkohlenstoffatome

### C₂₇H₅₆NO₄P (489.72)

- 346.: (Z)-3-Eicosenyl-1-phospho-N,N,N-trimethyl-butylammonium
- 347.: (Z)-4-Eicosenyl-1-phospho-N,N,N-trimethyl-butylammonium
- 348.: (Z)-5-Eicosenyl-1-phospho-N,N,N-trimethyl-butylammonium
- 349.: (Z)-6-Eicosenyl-1-phospho-N,N,N-trimethyl-butylammonium
- 350.: (Z)-7-Eicosenyl-1-phospho-N,N,N-trimethyl-butylammonium
- 351.: (Z)-8-Eicosenyl-1-phospho-N,N,N-trimethyl-butylammonium
- 352.: (Z)-9-Eicosenyl-1-phospho-N,N,N-trimethyl-butylammonium
- 353.: (Z)-10-Eicosenyl-1-phospho-N,N,N-trimethyl-butylammonium
- 354.: (Z)-11-Eicosenyl-1-phospho-N,N,N-trimethyl-butylammonium
- 355.: (Z)-12-Eicosenyl-1-phospho-N,N,N-trimethyl-butylammonium
- 356.: (Z)-13-Eicosenyl-1-phospho-N,N,N-trimethyl-butylammonium
- 357.: (Z)-14-Eicosenyl-1-phospho-N,N,N-trimethyl-butylammonium
- 358.: (Z)-15-Eicosenyl-1-phospho-N,N,N-trimethyl-butylammonium
- 359.: (Z)-16-Eicosenyl-1-phospho-N,N,N-trimethyl-butylammonium
- 360.: (Z)-17-Eicosenyl-1-phospho-N,N,N-trimethyl-butylammonium
- 361.: (Z)-18-Eicosenyl-1-phospho-N,N,N-trimethyl-butylammonium
- 362.: 19-Eicosenyl-1-phospho-N,N,N-trimethyl-butylammonium

### 21 Kettenkohlenstoffatome

### C₂₈H₅₈NO₄P (503.75)

- 363.: (Z)-3-Heneicosenyl-1-phospho-N,N,N-trimethyl-butylammonium
- 364.: (Z)-4-Heneicosenyl-1-phospho-N,N,N-trimethyl-butylammonium
- 365.: (Z)-5-Heneicosenyl-1-phospho-N,N,N-trimethyl-butylammonium
- 366.: (Z)-6-Heneicosenyl-1-phospho-N,N,N-trimethyl-butylammonium
- 367.: (Z)-7-Heneicosenyl-1-phospho-N,N,N-trimethyl-butylammonium
- 368.: (Z)-8-Heneicosenyl-1-phospho-N,N,N-trimethyl-butylammonium
- 369.: (Z)-9-Heneicosenyl-1-phospho-N,N,N-trimethyl-butylammonium
- 370.: (Z)-10-Heneicosenyl-1-phospho-N,N,N-trimethyl-butylammonium
- 371.: (Z)-11-Heneicosenyl-1-phospho-N,N,N-trimethyl-butylammonium
- 372.: (Z)-12-Heneicosenyl-1-phospho-N,N,N-trimethyl-butylammonium
- 373.: (Z)-13-Heneicosenyl-1-phospho-N,N,N-trimethyl-butylammonium
- 374.: (Z)-14-Heneicosenyl-1-phospho-N,N,N-trimethyl-butylammonium
- 375.: (Z)-15-Heneicosenyl-1-phospho-N,N,N-trimethyl-butylammonium
- 376.: (Z)-16-Heneicosenyl-1-phospho-N,N,N-trimethyl-butylammonium
- 377.: (Z)-17-Heneicosenyl-1-phospho-N,N,N-trimethyl-butylammonium
- 378.: (Z)-18-Heneicosenyl-1-phospho-N,N,N-trimethyl-butylammonium
- 379.: (Z)-19-Heneicosenyl-1-phospho-N,N,N-trimethyl-butylammonium
- 380.: 20-Heneicosenyl-1-phospho-N,N,N-trimethyl-butylammonium

### 22 Kettenkohlenstoffatome

### C₂₉H₆₀NO₄P (517.77)

- 381.: (Z)-3-Docosenyl-1-phospho-N,N,N-trimethyl-butylammonium
- 382.: (Z)-4-Docosenyl-1-phospho-N,N,N-trimethyl-butylammonium
- 383.: (Z)-5-Docosenyl-1-phospho-N,N,N-trimethyl-butylammonium
- 384.: (Z)-6-Docosenyl-1-phospho-N,N,N-trimethyl-butylammonium
- 385.: (Z)-7-Docosenyl-1-phospho-N,N,N-trimethyl-butylammonium
- 386.: (Z)-8-Docosenyl-1-phospho-N,N,N-trimethyl-butylammonium
- 387.: (Z)-9-Docosenyl-1-phospho-N,N,N-trimethyl-butylammonium
- 388.: (Z)-10-Docosenyl-1-phospho-N,N,N-trimethyl-butylammonium
- 389.: (Z)-11-Docosenyl-1-phospho-N,N,N-trimethyl-butylammonium
- 390.: (Z)-12-Docosenyl-1-phospho-N,N,N-trimethyl-butylammonium
- 391.: (Z)-14-Docosenyl-1-phospho-N,N,N-trimethyl-butylammonium
- 392.: (Z)-15-Docosenyl-1-phospho-N,N,N-trimethyl-butylammonium
- 393.: (Z)-16-Docosenyl-1-phospho-N,N,N-trimethyl-butylammonium
- 394.: (Z)-17-Docosenyl-1-phospho-N,N,N-trimethyl-butylammonium
- 395.: (Z)-18-Docosenyl-1-phospho-N,N,N-trimethyl-butylammonium
- 396.: (Z)-19-Docosenyl-1-phospho-N,N,N-trimethyl-butylammonium
- 397.: (Z)-20-Docosenyl-1-phospho-N,N,N-trimethyl-butylammonium
- 398.: 21-Docosenyl-1-phospho-N,N,N-trimethyl-butylammonium

### 23 Kettenkohlenstoffatome

### C₃₀H₆₂NO₄P (531.80)

- 399.: (Z)-3-Tricosenyl-1-phospho-N,N,N-trimethyl-butylammonium
- 400.: (Z)-4-Tricosenyl-1-phospho-N,N,N-trimethyl-butylammonium
- 401.: (Z)-5-Tricosenyl-1-phospho-N,N,N-trimethyl-butylammonium
- 402.: (Z)-6-Tricosenyl-1-phospho-N,N,N-trimethyl-butylammonium
- 403.: (Z)-7-Tricosenyl-1-phospho-N,N,N-trimethyl-butylammonium
- 404.: (Z)-8-Tricosenyl-1-phospho-N,N,N-trimethyl-butylammonium
- 405.: (Z)-9-Tricosenyl-1-phospho-N,N,N-trimethyl-butylammonium
- 406.: (Z)-10-Tricosenyl-1-phospho-N,N,N-trimethyl-butylammonium
- 407.: (Z)-11-Tricosenyl-1-phospho-N,N,N-trimethyl-butylammonium
- 408.: (Z)-12-Tricosenyl-1-phospho-N,N,N-trimethyl-butylammonium
- 409.: (Z)-13-Tricosenyl-1-phospho-N,N,N-trimethyl-butylammonium
- 410.: (Z)-14-Tricosenyl-1-phospho-N,N,N-trimethyl-butylammonium
- 411.: (Z)-15-Tricosenyl-1-phospho-N,N,N-trimethyl-butylammonium
- 412.: (Z)-16-Tricosenyl-1-phospho-N,N,N-trimethyl-butylammonium
- 413.: (Z)-17-Tricosenyl-1-phospho-N,N,N-trimethyl-butylammonium
- 414.: (Z)-18-Tricosenyl-1-phospho-N,N,N-trimethyl-butylammonium
- 415.: (Z)-19-Tricosenyl-1-phospho-N,N,N-trimethyl-butylammonium
- 416.: (Z)-20-Tricosenyl-1-phospho-N,N,N-trimethyl-butylammonium
- 417.: (Z)-21-Tricosenyl-1-phospho-N,N,N-trimethyl-butylammonium
- 418.: 22-Tricosenyl-1-phospho-N,N,N-trimethyl-butylammonium

### 24 Kettenkohlenstoffatome

### C₃₁H₆₄NO₄P (545.83)

- 419.: (Z)-3-Tetracosenyl-1-phospho-N,N,N-trimethyl-butylammonium
- 420.: (Z)-4-Tetracosenyl-1-phospho-N,N,N-trimethyl-butylammonium
- 421.: (Z)-5-Tetracosenyl-1-phospho-N,N,N-trimethyl-butylammonium
- 422.: (Z)-6-Tetracosenyl-1-phospho-N,N,N-trimethyl-butylammonium
- 423.: (Z)-7-Tetracosenyl-1-phospho-N,N,N-trimethyl-butylammonium
- 424.: (Z)-8-Tetracosenyl-1-phospho-N,N,N-trimethyl-butylammonium
- 425.: (Z)-9-Tetracosenyl-1-phospho-N,N,N-trimethyl-butylammonium
- 426.: (Z)-10-Tetracosenyl-1-phospho-N,N,N-trimethyl-butylammonium
- 427.: (Z)-11-Tetracosenyl-1-phaspho-N,N,N-trimethyl-butylammonium
- 428.: (Z)-12-Tetracosenyl-1-phospho-N,N,N-trimethyl-butylammonium
- 429.: (Z)-13-Tetracosenyl-1-phospho-N,N,N-trimethyl-butylammonium
- 430.: (Z)-14-Tetracosenyl-1-phospho-N,N,N-trimethyl-butylammonium
- 431.: (Z)-15-Tetracosenyl-1-phospho-N,N,N-trimethyl-butylammonium
- 432.: (Z)-16-Tetracosenyl-1-phospho-N,N,N-trimethyl-butylammonium
- 433.: (Z)-17-Tetracosenyl-1-phospho-N,N,N-trimethyl-butylammonium
- 434.: (Z)-18-Tetracosenyl-1-phospho-N,N,N-trimethyl-butylammonium

### 4. Beispiele für (Z,Z)-Alkadienylphosphohocholine (Vergleichsbeispiel)

### (A = IX; n = 2; R₃, CH₃; m= 1, x = 1; Z = 0)

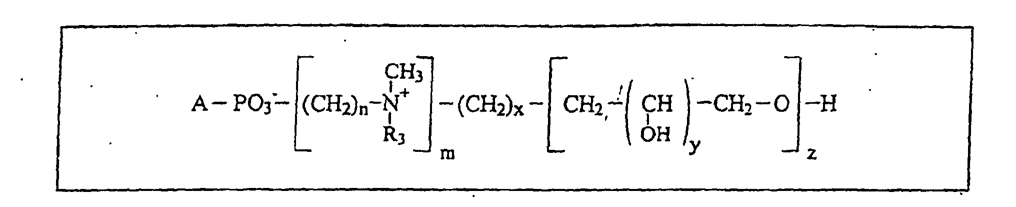

wobei A für eine zweifach ungesättigte Alkylkette folgender Struktur steht (s,t,r ≥ 0; 8 ≤ s+t+r ≤ 26):

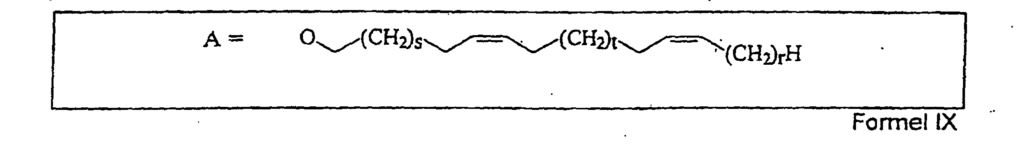

### 16 Kettenkohlenstoffatome

### C₂₁H₄₂NO₄P (403.54)

- 435.: (Z,Z)-3,7-Hexadecadienyl-1-phosphocholin
- 436.: (Z,Z)-4,8-Hexadecadienyl-1-phosphocholin
- 437.: (Z,Z)-5,9-Hexadecadienyl-1-phosphocholin
- 438.: (Z,Z)-6,10-Hexadecadienyl-1-phosphocholin
- 439.: (Z,Z)-7,11-Hexadecadienyl-1-phosphocholin
- 440.: (Z,Z)-8,12-Hexadecadienyl-1-phosphocholin
- 441.: (Z,Z)-9,13-Hexadecadienyl-1-phasphocholin
- 442.: (Z,Z)-3,8-Hexadecadienyl-1-phosphocholin
- 443.: (Z,Z)-4,9-Hexadecadienyl-1-phosphocholin
- 444.: (Z,Z)-5,10-Hexadecadienyl-1-phosphocholin
- 445.: (Z,Z)-6,11-Hexadecadienyl-1-phosphocholin
- 446.: (Z,Z)-7,12-Hexadecadienyl-1-phosphocholin
- 447.: (Z,Z)-8,13-Hexadecadienyl-1-phosphocholin
- 448.: (Z,Z)-3,9-Hexadecadienyl-1-phosphocholin
- 449.: (Z,Z)-4,10-Hexadecadienyl-1-phosphocholin
- 450.: (Z,Z)-5,11-Hexadecadienyl-1-phosphocholin
- 451.: (Z,Z)-6,12-Hexadecadienyl-1-phosphocholin
- 452.: (Z,Z)-7,13-Hexadecadienyl-1-phosphocholin
- 453.: (Z,Z)-3,10-Hexadecadienyl-1-phosphocholin
- 454.: (Z,Z)-4,11-Hexadecadienyl-1-phosphocholin
- 455.: (Z,Z)-5,12-Hexadecadienyl-1-phosphocholin
- 456.: (Z,Z)-6,13-Hexadecadienyl-1-phosphocholin
- 457.: (Z,Z)-3,11-Hexadecadienyl-1-phosphocholin
- 458.: (Z,Z)-4,12-Hexadecadienyl-1-phosphocholin
- 459.: (Z,Z)-5,13-Hexadecadienyl-1-phosphocholin
- 460.: (Z,Z)-3,12-Hexadecadienyl-1-phosphocholin
- 461.: (Z,Z)-4,13-Hexadecadienyl-1-phosphocholin
- 462.: (Z,Z)-3,13-Hexadecadienyl-1-phosphocholin

### 17 Kettenkohlenstoffatome

### C₂₂H₄₄NO₄P (417.57)

- 463.: (Z,Z)-3,7-Heptadecadienyl-1-phosphocholin
- 464.: (Z,Z)-4,8-Heptadecadienyl-1-phosphocholin
- 465.: (Z,Z)-5,9-Heptadecadienyl-1-phosphocholin
- 466.: (Z,Z)-6,10-Heptadecadienyl-1-phosphocholin
- 467.: (Z,Z)-7,11-Heptadecadienyl-1-phosphocholin
- 468.: (Z,Z)-8,12-Heptadecadienyl-1-phosphocholin
- 469.: (Z,Z)-9,13-Heptadecadienyl-1-phosphocholin
- 470.: (Z,Z)-10,14-Heptadecadienyl-1-phosphocholin
- 471.: (Z,Z)-3,8-Heptadecadienyl-1-phosphocholin
- 472.: (Z,Z)-4,9-Heptadecadienyl-1-phosphocholin
- 473.: (Z,Z)-5,10-Heptadecadienyl-1-phosphocholin
- 474.: (Z,Z)-6,11-Heptadecadienyl-1-phosphocholin
- 475.: (Z,Z)-7,12-Heptadecadienyl-1-phosphocholin
- 476.: (Z,Z)-8,13-Heptadecadienyl-1-phosphocholin
- 477.: (Z,Z)-9,14-Heptadecadienyl-1-phosphocholin
- 478.: (Z,Z)-3,9-Heptadecadienyl-1-phosphocholin
- 479.: (Z,Z)-4,10-Heptadecadienyl-1-phosphocholin,
- 480.: (Z,Z)-5,11-Heptadecadienyl-1-phosphocholin
- 481.: (Z,Z)-6,12-Heptadecadienyl-1-phosphocholin
- 482.: (Z,Z)-7,13-Heptadecadienyl-1-phosphocholin
- 483.: (Z,Z)-8,14-Heptadecadienyl-1-phosphocholin
- 484.: (Z,Z)-3,10-Heptadecadienyl-1-phosphocholin
- 485.: (Z,Z)-4,11-Heptadecadienyl-1-phosphocholin
- 486.: (Z,Z)-5,12-Heptadecadienyl-1-phosphocholin
- 487.: (Z,Z)-6,13-Heptadecadienyl-1-phosphocholin
- 488.: (Z,Z)-7,14-Heptadecadienyl-1-phosphocholin
- 489.: (Z,Z)-3,11-Heptadecadienyl-1-phosphocholin
- 490.: (Z,Z)-4,12-Heptadecadienyl-1-phosphocholin
- 491.: (Z,Z)-5,13-Heptadecadienyl-1-phosphocholin
- 492.: (Z,Z)-6,14-Heptadecadienyl-1-phosphocholin
- 493.: (Z,Z)-3,12-Heptadecadienyl-1-phosphocholin
- 494.: (Z,Z)-4,13-Heptadecadienyl-1-phosphocholin
- 495.: (Z,Z)-5,14-Heptadecadienyl-1-phosphocholin
- 496.: (Z,Z)-3,13-Heptadecadienyl-1-phosphocholin
- 497.: (Z,Z)-4,14-Heptadecadienyl-1-phosphocholin
- 498.: (Z,Z)-3,14-Heptadecadienyl-1-phosphocholin

### 18 Kettenkohlenstoffatome

### C₂₃H₄₆NO₄P (431.60)

- 499.: (Z,Z)-3,7-Octadecadienyl-1-phosphocholin
- 500.: (Z,Z)-4,8-Octadecadienyl-1-phosphocholin
- 501.: (Z,Z)-5,9-Octadecadienyl-1-phosphocholin
- 502.: (Z,Z)-6,10-Octadecadienyl-1-phosphocholin
- 503.: (Z,Z)-7,11-Octadecadienyl-1-phosphocholin
- 504.: (Z,Z)-8,12-Octadecadienyl-1-phosphocholin
- 505.: (Z,Z)-9,13-Octadecadienyl-1-phosphocholin
- 506.: (Z,Z)-10,14-Octadecadienyl-1-phosphocholin
- 507.: (Z,Z)-11,15-Octadecadienyl-1-phosphocholin
- 508.: (Z,Z)-3,8-Octadecadienyl-1-phosphocholin
- 509.: (Z,Z)-4,9-Octadecadienyl-1-phosphocholin
- 510.: (Z,Z)-5,10-Octadecadienyl-1-phosphocholin
- 511.: (Z,Z)-6,11-Octadecadienyl-1-phosphocholin
- 512.: (Z,Z)-7,12-Octadecadienyl-1-phosphocholin
- 513.: (Z,Z)-8,13-Octadecadienyl-1-phosphocholin
- 514.: (Z,Z)-9,14-Octadecadienyl-1-phosphocholin
- 515.: (Z,Z)-10,15-Octadecadienyl-1-phosphocholin
- 516.: (Z,Z)-3,9-Octadecadienyl-1-phosphocholin
- 517.: (Z,Z)-4,10-Octadecadienyl-1-phosphocholin
- 518.: (Z,Z)-5,11-Octadecadienyl-1-phosphocholin
- 519.: (Z,Z)-6,12-Octadecadienyl-1-phosphocholin
- 520.: (Z,Z)-7,13-Octadecadienyl-1-phosphocholin
- 521.: (Z,Z)-8,14-Octadecadienyl-1-phosphocholin
- 522.: (Z,Z)-9,15-Octadecadienyl-1-phosphocholin
- 523.: (Z,Z)-3,10-Octadecadienyl-1-phosphocholin
- 524.: (Z,Z)-4,11-Octadecadienyl-1-phosphocholin
- 525.: (Z,Z)-5,12-Octadecadienyl-1-phosphocholin
- 526.: (Z,Z)-6,13-Octadecadienyl-1-phosphocholin
- 527.: (Z,Z)-7,14-Octadecadienyl-1-phosphocholin
- 528.: (Z,Z)-8,15-Octadecadienyl-1-phosphocholin
- 529.: (Z,Z)-3,11-Octadecadienyl-1-phosphocholin
- 530.: (Z,Z)-4,12-Octadecadienyl-1-phosphocholin
- 531.: (Z,Z)-5,13-Octadecadienyl-1-phosphocholin
- 532.: (Z,Z)-6,14-Octadecadienyl-1-phosphocholin
- 533.: (Z,Z)-7,15-Octadecadienyl-1-phosphocholin
- 534.: (Z,Z)-3,12-Octadecadienyl-1-phosphocholin
- 535.: (Z,Z)-4,13-Octadecadienyl-1-phosphocholin
- 536.: (Z,Z)-5,14-Octadecadienyl-1-phosphocholin
- 537.: (Z,Z)-6,15-Octadecadienyl-1-phosphocholin
- 538.: (Z,Z)-3,13-Octadecadienyl-1-phosphocholin
- 539.: (Z,Z)-4,14-Octadecadienyl-1-phosphocholin
- 540.: (Z,Z)-5,15-Octadecadienyl-1-phosphocholin
- 541.: (Z,Z)-3,14-Octadecadienyl-1-phosphocholin
- 542.: (Z,Z)-4,15-Octadecadienyl-1-phosphocholin
- 543.: (Z,Z)-3,15-Octadecadienyl-1-phosphocholin

### 19 Kettenkohlenstoffatome

### C₂₄H₄₈NO₄P (445.62)

- 544.: (Z,Z)-3,7-Nonadecadienyl-1-phosphocholin
- 545.: (Z,Z)-4,8-Nonadecadienyl-1-phosphocholin
- 546.: (Z,Z)-5,9-Nonadecadienyl-1-phosphocholin
- 547.: (Z,Z)-6,10-Nonadecadienyl-1-phosphocholin
- 548.: (Z,Z)-7,11-Nonadecadienyl-1-phosphocholin
- 549.: (Z,Z)-8,12-Nonadecadienyl-1-phosphocholin
- 550.: (Z,Z)-9,13-Nonadecadienyl-1-phosphocholin
- 551.: (Z,Z)-10,14-Nonadecadienyl-1-phosphocholin
- 552.: (Z,Z)-11,15-Nonadecadienyl-1-phosphocholin
- 553.: (Z,Z)-12,16-Nonadecadienyl-1-phosphocholin
- 554.: (Z,Z)-3,8-Nonadecadienyl-1-phosphocholin
- 555.: (Z,Z)-4,9-Nonadecadienyl-1-phosphocholin
- 556.: (Z,Z)-5,10-Nonadecadienyl-1-phosphocholin
- 557.: (Z,Z)-6,11-Nonadecadienyl-1-phosphocholin
- 558.: (Z,Z)-7,12-Nonadecadienyl-1-phosphocholin
- 559.: (Z,Z)-8,13-Nonadecadienyl-1-phosphocholin
- 560.: (Z,Z)-9,14-Nonadecadienyl-1-phosphocholin
- 561.: (Z,Z)-10,15-Nonadecadienyl-1-phosphocholin
- 562.: (Z,Z)-11,16-Nonadecadienyl-1-phosphocholin
- 563.: (Z,Z)-3,9-Nonadecadienyl-1-phosphocholin
- 564.: (Z,Z)-4,10-Nonadecadienyl-1-phosphocholin
- 565.: (Z,Z)-5,11-Nonadecadienyl-1-phosphocholin
- 566.: (Z,Z)-6,12-Nonadecadienyl-1-phosphocholin
- 567.: (Z,Z)-7,13-Nonadecadienyl-1-phosphocholin
- 568.: (Z,Z)-8,14-Nonadecadienyl-1-phosphocholin
- 569.: (Z,Z)-9,15-Nonadecadienyl-1-phosphocholin
- 570.: (Z,Z)-10,16-Nonadecadienyl-1-phosphocholin
- 571.: (Z,Z)-3,10-Nonadecadienyl-1-phosphocholin
- 572.: (Z,Z)-4,11-Nonadecadienyl-1-phosphocholin
- 573.: (Z,Z)-5,12-Nonadecadienyl-1-phosphocholin
- 574.: (Z,Z)-6,13-Nonadecadienyl-1-phosphocholin
- 575.: (Z,Z)-7,14-Nonadecadienyl-1-phosphocholin
- 576.: (Z,Z)-8,15-Nonadecadienyl-1-phosphocholin
- 577.: (Z,Z)-9,16-Nonadecadienyl-1-phosphocholin
- 578.: (Z,Z)-3,11-Nonadecadienyl-1-phosphocholin
- 579.: (Z,Z)-4,12-Nonadecadienyl-1-phosphocholin
- 580.: (Z,Z)-5,13-Nonadecadienyl-1-phosphocholin
- 581.: (Z,Z)-6,14-Nonadecadienyl-1-phosphocholin
- 582.: (Z,Z)-7,15-Nonadecadienyl-1-phosphocholin
- 583.: (Z,Z)-8,16-Nonadecadienyl-1-phosphocholin
- 584.: (Z,Z)-3,12-Nonadecadienyl-1-phosphocholin
- 585.: (Z,Z)-4,13-Nonadecadienyl-1-phosphocholin
- 586.: (Z,Z)-5,14-Nonadecadienyl-1-phosphocholin
- 587.: (Z,Z)-6,15-Nonadecadienyl-1-phosphocholin
- 588.: (Z,Z)-7,16-Nonadecadienyl-1-phosphocholin
- 589.: (Z,Z)-3,13-Nonadecadienyl-1-phosphocholin
- 590.: (Z,Z)-4,14-Nonadecadienyl-1-phosphocholin
- 591.: (Z,Z)-5,15-Nonadecadienyl-1-phosphocholin
- 592.: (Z,Z)-6,16-Nonadecadienyl-1-phosphocholin
- 593.: (Z,Z)-3,14-Nonadecadienyl-1-phosphocholin
- 594.: (Z,Z)-4,15-Nonadecadienyl-1-phosphocholin
- 595.: (Z,Z)-5,16-Nonadecadienyl-1-phosphocholin
- 596.: (Z,Z)-3,15-Nonadecadienyl-1-phosphocholin
- 597.: (Z,Z)-4,16-Nonadecadienyl-1-phosphocholin

### 20 Kettenkohlenstoffatome

### C₂₅H₅₀NO₄P (459.65)

- 598.: (Z,Z)-3,7-Eicosadienyl-1-phosphocholin
- 599.: (Z,Z)-4,8-Eicosadienyl-1-phosphocholin
- 600.: (Z,Z)-5,9-Eicosadienyl-1-phosphocholin
- 601.: (Z,Z)-6,10-Eicosadienyl-1-phosphocholin
- 602.: (Z,Z)-7,11-Eicosadienyl-1-phosphocholin
- 603.: (Z,Z)-8,12-Eicosadienyl-1-phosphocholin
- 604.: (Z,Z)-9,13-Eicosadienyl-1-phosphocholin
- 605.: (Z,Z)-10,14-Eicosadienyl-1-phosphocholin
- 606.: (Z,Z)-11,15-Eicosadienyl-1-phosphocholin
- 607.: (Z,Z)-12,16-Eicosadienyl-1-phosphocholin
- 608.: (Z,Z)-13,17-Eicosadienyl-1-phosphocholin
- 609.: (Z,Z)-3,8-Eicosadienyl-1-phosphocholin
- 610.: (Z,Z)-4,9-Eicosadienyl-1-phosphocholin
- 611.: (Z,Z)-5,10-Eicosadienyl-1-phosphocholin
- 612.: (Z,Z)-6,11-Eicosadienyl-1-phosphocholin
- 613.: (Z,Z)-7,12-Eicosadienyl-1-phosphocholin
- 614.: (Z,Z)-8,13-Eicosadienyl-1-phosphocholin
- 615.: (Z,Z)-9,14-Eicosadienyl-1-phosphocholin
- 616.: (Z,Z)-10,15-Eicosadienyl-1-phosphocholin
- 617.: (Z,Z)-11,16-Eicosadienyl-1-phosphocholin
- 618.: (Z,Z)-12,17-Eicosadienyl-1-phosphocholin
- 619.: (Z,Z)-3,9-Eicosadienyl-1-phosphocholin
- 620.: (Z,Z)-4,10-Eicosadienyl-1-phosphocholin
- 621.: (Z,Z)-5,11-Eicosadienyl-1-phosphocholin
- 622.: (Z,Z)-6,12-Eicosadienyl-1-phosphocholin
- 623.: (Z,Z)-7,13-Eicosadienyl-1-phosphocholin
- 624.: (Z,Z)-8,14-Eicosadienyl-1-phosphocholin
- 625.: (Z,Z)-9,15-Eicosadienyl-1-phosphocholin
- 626.: (Z,Z)-10,16-Eicosadienyl-1-phosphocholin
- 627.: (Z,Z)-11,17-Eicosadienyl-1-phosphocholin
- 628.: (Z,Z)-3,10-Eicosadienyl-1-phosphocholin
- 629.: (Z,Z)-4,11-Eicosadienyl-1-phosphocholin
- 630.: (Z,Z)-5,12-Eicosadienyl-1-phosphocholin
- 631.: (Z,Z)-6,13-Eicosadienyl-1-phosphocholin
- 632.: (Z,Z)-7,14-Eicosadienyl-1-phosphocholin
- 633.: (Z,Z)-8,15-Eicosadienyl-1-phosphocholin
- 634.: (Z,Z)-9,16-Eicosadienyl-1-phosphocholin
- 635.: (Z,Z)-10,17-Eicosadienyl-1-phosphocholin
- 636.: (Z,Z)-3,11-Eicosadienyl-1-phosphocholin
- 637.: (Z,Z)-4,12-Eicosadienyl-1-phosphocholin
- 638.: (Z,Z)-5,13-Eicosadienyl-1-phosphocholin
- 639.: (Z,Z)-6,14-Eicosadienyl-1-phosphocholin
- 640.: (Z,Z)-7,15-Eicosadienyl-1-phosphocholin
- 641.: (Z,Z)-8,16-Eicosadienyl-1-phosphocholin
- 642.: (Z,Z)-9,17-Eicosadienyl-1-phosphocholin
- 643.: (Z,Z)-3,12-Eicosadienyl-1-phosphocholin
- 644.: (Z,Z)-4,13-Eicosadienyl-1-phosphocholin
- 645.: (Z,Z)-5,14-Eicosadienyl-1-phosphocholin
- 646.: (Z,Z)-6,15-Eicosadienyl-1-phosphocholin
- 647.: (Z,Z)-7,16-Eicosadienyl-1-phosphocholin
- 648.: (Z,Z)-8,17-Eicosadienyl-1-phosphocholin
- 649.: (Z,Z)-3,13-Eicosadienyl-1-phosphocholin
- 650.: (Z,Z)-4,14-Eicosadienyl-1-phosphocholin
- 651.: (Z,Z)-5,15-Eicosadienyl-1-phosphocholin
- 652.: (Z,Z)-6,16-Eicosadienyl-1-phosphocholin
- 653.: (Z,Z)-7,17-Eicosadienyl-1-phosphocholin
- 654.: (Z,Z)-3,14-Eicosadienyl-1-phosphocholin
- 655.: (Z,Z)-4,15-Eicosadienyl-1-phosphocholin
- 656.: (Z,Z)-5,16-Eicosadienyl-1-phosphocholin
- 657.: (Z,Z)-6,17-Eicosadienyl-1-phosphocholin
- 658.: (Z,Z)-3,15-Eicosadienyl-1-phosphocholin
- 659.: (Z,Z)-4,16-Eicosadienyl-1-phosphocholin
- 660.: (Z,Z)-5,17-Eicosadienyl-1-phosphocholin
- 661.: (Z,Z)-3,17-Eicosadienyl-1-phosphocholin

### 21 Kettenkohlenstoffatome

### C₂₆H₅₂NO₄P (473.68)

- 662.: (Z,Z)-3,7-Heneicosadienyl-1-phosphocholin
- 663.: (Z,Z)-4,8-Heneicosadienyl-1-phosphocholin
- 664.: (Z,Z)-5,9-Heneicosadienyl-1-phosphocholin
- 665.: (Z,Z)-6,10-Heneicosadienyl-1-phosphocholin
- 666.: (Z,Z)-7,11-Heneicosadienyl-1-phosphocholin
- 667.: (Z,Z)-8,12-Heneicosadienyl-1-phosphocholin
- 668.: (Z,Z)-9,13-Heneicosadienyl-1-phosphocholin
- 669.: (Z,Z)-10,14-Heneicosadienyl-1-phosphocholin
- 670.: (Z,Z)-11,15-Heneicosadienyl-1-phosphocholin
- 671.: (Z,Z)-12,16-Heneicosadienyl-1-phosphocholin
- 672.: (Z,Z)-13,17-Heneicosadienyl-1-phosphocholin
- 673.: (Z,Z)-14,18-Heneicosadienyl-1-phosphocholin
- 674.: (Z,Z)-3,8-Heneicosadienyl-1-phosphocholin
- 675.: (Z,Z)-4,9-Heneicosadienyl-1-phosphocholin
- 676.: (Z,Z)-5,10-Heneicosadienyl-1-phosphocholin
- 677.: (Z,Z)-6,11-Heneicosadienyl-1-phosphocholin
- 678.: (Z,Z)-7,12-Heneicosadienyl-1-phosphocholin
- 679.: (Z,Z)-8,13-Heneicosadienyl-1-phosphocholin
- 680.: (Z,Z)-9,14-Heneicosadienyl-1-phosphocholin
- 681.: (Z,Z)-10,15-Heneicosadienyl-1-phosphocholin
- 682.: (Z,Z)-11,16-Heneicosadienyl-1-phosphocholin
- 683.: (Z,Z)-12,17-Heneicosadienyl-1-phosphocholin
- 684.: (Z,Z)-13,18-Heneicosadienyl-1-phosphocholin
- 685.: (Z,Z)-3,9-Heneicosadienyl-1-phosphocholin
- 686.: (Z,Z)-4,10-Heneicosadienyl-1-phosphocholin
- 687.: (Z,Z)-5,11-Heneicosadienyl-1-phosphocholin
- 688.: (Z,Z)-6,12-Heneicosadienyl-1-phosphocholin
- 689.: (Z,Z)-7,13-Heneicosadienyl-1-phosphocholin
- 690.: (Z,Z)-8,14-Heneicosadienyl-1-phosphocholin
- 691.: (Z,Z)-9,15-Heneicosadienyl-1-phosphocholin
- 692.: (Z,Z)-10,16-Heneicosadienyl-1-phosphocholin
- 693.: (Z,Z)-11,17-Heneicosadienyl-1-phosphocholin
- 694.: (Z,Z)-12,18-Heneicosadienyl-1-phosphocholin
- 695.: (Z,Z)-3,10-Heneicosadienyl-1-phosphocholin
- 696.: (Z,Z)-4,11-Heneicosadienyl-1-phosphocholin
- 697.: (Z,Z)-5,12-Heneicosadienyl-1-phosphocholin
- 698.: (Z,Z)-6,13-Heneicosadienyl-1-phosphocholin
- 699.: (Z,Z)-7,14-Heneicosadienyl-1-phosphocholin
- 700.: (Z,Z)-8,15-Heneicosadienyl-1-phosphocholin
- 701.: (Z,Z)-9,16-Heneicosadienyl-1-phosphocholin
- 702.: (Z,Z)-10,17-Heneicosadienyl-1-phosphocholin
- 703.: (Z,Z)-11,18-Heneicosadienyl-1-phosphocholin
- 704.: (Z,Z)-3,11-Heneicosadienyl-1-phosphocholin
- 705.: (Z,Z)-4,12-Heneicosadienyl-1-phosphocholin
- 706.: (Z,Z)-5,13-Heneicosadienyl-1-phosphocholin
- 707.: (Z,Z)-6,14-Heneicosadienyl-1-phosphocholin
- 708.: (Z,Z)-7,15-Heneicosadienyl-1-phosphocholin
- 709.: (Z,Z)-8,16-Heneicosadienyl-1-phosphocholin
- 710.: (Z,Z)-9,17-Heneicosadienyl-1-phosphocholin
- 711.: (Z,Z)-10,18-Heneicosadienyl-1-phosphocholin
- 712.: (Z,Z)-3,12-Heneicosadienyl-1-phosphocholin
- 713.: (Z,Z)-4,13-Heneicosadienyl-1-phosphocholin
- 714.: (Z,Z)-5,14-Heneicosadienyl-1-phosphocholin
- 715.: (Z,Z)-6,15-Heneicosadienyl-1-phosphocholin
- 716.: (Z,Z)-7,16-Heneicosadienyl-1-phosphocholin
- 717.: (Z,Z)-8,17-Heneicosadienyl-1-phosphocholin
- 718.: (Z,Z)-9,18-Heneicosadienyl-1-phosphocholin
- 719.: (Z,Z)-3,13-Heneicosadienyl-1-phosphocholin
- 720.: (Z,Z)-4,14-Heneicosadienyl-1-phosphocholin
- 721.: (Z,Z)-5,15-Heneicosadienyl-1-phosphocholin
- 722.: (Z,Z)-6,16-Heneicosadienyl-1-phosphocholin
- 723.: (Z,Z)-7,17-Heneicosadienyl-1-phosphocholin
- 724.: (Z,Z)-8,18-Heneicosadienyl-1-phosphocholin
- 725.: (Z,Z)-3,14-Heneicosadienyl-1-phosphocholin
- 726.: (Z,Z)-4,15-Heneicosadienyl-1-phosphocholin
- 727.: (Z,Z)-5,16-Heneicosadienyl-1-phosphocholin
- 728.: (Z,Z)-6,17-Heneicosadienyl-1-phosphocholin
- 729.: (Z,Z)-7,18-Heneicosadienyl-1-phosphocholin
- 730.: (Z,Z)-3,15-Heneicosadienyl-1-phosphocholin
- 731.: (Z,Z)-4,16-Heneicosadienyl-1-phosphocholin
- 732.: (Z,Z)-5,17-Heneicosadienyl-1-phosphocholin
- 733.: (Z,Z)-6,18-Heneicosadienyl-1-phosphocholin
- 734.: (Z,Z)-3,17-Heneicosadienyl-1-phosphocholin
- 735.: (Z,Z)-4,18-Heneicosadienyl-1-phosphocholin

### 22 Kettenkohlenstoffatome

### C₂₇H₅₄NO₄P (487.70)

- 736.: (Z,Z)-3,7-Docosadienyl-1-phosphocholin
- 737.: (Z,Z)-4,8-Docosadienyl-1-phosphocholin
- 738.: (Z,Z)-5,9-Docosadienyl-1-phosphocholin
- 739.: (Z,Z)-6,10-Docosadienyl-1-phosphocholin
- 740.: (Z,Z)-7,11-Docosadienyl-1-phosphocholin
- 741.: (Z,Z)-8,12-Docosadienyl-1-phosphocholin
- 742.: (Z,Z)-9,13-Docosadienyl-1-phosphocholin
- 743.: (Z,Z)-10,14-Docosadienyl-1-phosphocholin
- 744.: (Z,Z)-11,15-Docosadienyl-1-phosphocholin
- 745.: (Z,Z)-12,16-Docosadienyl-1-phosphocholin
- 746.: (Z,Z)-13,17-Docosadienyl-1-phosphocholin
- 747.: (Z,Z)-14,18-Docosadienyl-1-phosphocholin
- 748.: (Z,Z)-15,19-Docosadienyl-1-phosphocholin
- 749.: (Z,Z)-3,8-Docosadienyl-1-phosphocholin
- 750.: (Z,Z)-4,9-Docosadienyl-1-phosphocholin
- 751.: (Z,Z)-5,10-Docosadienyl-1-phosphocholin
- 752.: (Z,Z)-6,11-Docosadienyl-1-phosphocholin
- 753.: (Z,Z)-7,12-Docosadienyl-1-phosphocholin
- 754.: (Z,Z)-8,13-Docosadienyl-1-phosphocholin
- 755.: (Z,Z)-9,14-Docosadienyl-1-phosphocholin
- 756.: (Z,Z)-10,15-Docosadienyl-1-phosphocholin
- 757.: (Z,Z)-11,16-Docosadienyl-1-phosphocholin
- 758.: (Z,Z)-12,17-Docosadienyl-1-phosphocholin
- 759.: (Z,Z)-13,18-Docosadienyl-1-phosphocholin
- 760.: (Z,Z)-14,19-Docosadienyl-1-phosphocholin
- 761.: (Z,Z)-3,9-Docosadienyl-1-phosphocholin
- 762.: (Z,Z)-4,10-Docosadienyl-1-phosphocholin
- 763.: (Z,Z)-5,11-Docosadienyl-1-phosphocholin
- 764.: (Z,Z)-6,12-Docosadienyl-1-phosphocholin
- 765.: (Z,Z)-7,13-Docosadienyl-1-phosphocholin
- 766.: (Z,Z)-8,14-Docosadienyl-1-phosphocholin
- 767.: (Z,Z)-9,15-Docosadienyl-1-phosphocholin
- 768.: (Z,Z)-10,16-Docosadienyl-1-phosphocholin
- 769.: (Z,Z)-11,17-Docosadienyl-1-phosphocholin
- 770.: (Z,Z)-12,18-Docosadienyl-1-phosphocholin
- 771.: (Z,Z)-13,19-Docosadienyl-1-phosphocholin
- 772.: (Z,Z)-3,10-Docosadienyl-1-phosphocholin
- 773.: (Z,Z)-4,11-Docosadienyl-1-phosphocholin
- 774.: (Z,Z)-5,12-Docosadienyl-1-phosphocholin
- 775.: (Z,Z)-6,13-Docosadienyl-1-phosphocholin
- 776.: (Z,Z)-7,14-Docosadienyl-1-phosphocholin
- 777.: (Z,Z)-8,15-Docosadienyl-1-phosphocholin
- 778.: (Z,Z)-9,16-Docosadienyl-1-phosphocholin
- 779.: (Z,Z)-10,17-Docosadienyl-1-phosphocholin
- 780.: (Z,Z)-11,18-Docosadienyl-1-phosphocholin
- 781.: (Z,Z)-12,19-Docosadienyl-1-phosphocholin
- 782.: (Z,Z)-3,11-Docosadienyl-1-phosphocholin
- 783.: (Z,Z)-4,12-Docosadienyl-1-phosphocholin
- 784.: (Z,Z)-5,13-Docosadienyl-1-phosphocholin
- 785.: (Z,Z)-6,14-Docosadienyl-1-phosphocholin
- 786.: (Z,Z)-7,15-Docosadienyl-1-phosphocholin
- 787.: (Z,Z)-8,16-Docosadienyl-1-phosphocholin
- 788.: (Z,Z)-9,17-Docosadienyl-1-phosphocholin
- 789.: (Z,Z)-10,18-Docosadienyl-1-phosphocholin
- 790.: (Z,Z)-11,19-Docosadienyl-1-phosphocholin
- 791.: (Z,Z)-3,12-Docosadienyl-1-phosphocholin
- 792.: (Z,Z)-4,13-Docosadienyl-1-phosphocholin
- 793.: (Z,Z)-5,14-Docosadienyl-1-phosphocholin
- 794.: (Z,Z)-6,15-Docosadienyl-1-phosphocholin
- 795.: (Z,Z)-7,16-Docosadienyl-1-phosphocholin
- 796.: (Z,Z)-8,17-Docosadienyl-1-phosphocholin
- 797.: (Z,Z)-9,18-Docosadienyl-1-phosphocholin
- 798.: (Z,Z)-10,19-Docosadienyl-1-phosphocholin
- 799.: (Z,Z)-3,13-Docosadienyl-1-phosphocholin
- 800.: (Z,Z)-4,14-Docosadienyl-1-phosphocholin
- 801.: (Z,Z)-5,15-Docosadienyl-1-phosphocholin
- 802.: (Z,Z)-6,16-Docosadienyl-1-phosphocholin
- 803.: (Z,Z)-7,17-Docosadienyl-1-phosphocholin
- 804.: (Z,Z)-8,18-Docosadienyl-1-phosphocholin
- 805.: (Z,Z)-9,19-Docosadienyl-1-phosphocholin
- 806.: (Z,Z)-3,14-Docosadienyl-1-phosphocholin
- 807.: (Z,Z)-4,15-Docosadienyl-1-phosphocholin
- 808.: (Z,Z)-5,16-Docosadienyl-1-phosphocholin
- 809.: (Z,Z)-6,17-Docosadienyl-1-phosphocholin
- 810.: (Z,Z)-7,18-Docosadienyl-1-phosphocholin
- 811.: (Z,Z)-8,19-Docosadienyl-1-phosphocholin
- 812.: (Z,Z)-3,15-Docosadienyl-1-phosphocholin
- 813.: (Z,Z)-4,16-Docosadienyl-1-phosphocholin
- 814.: (Z,Z)-5,17-Docosadienyl-1-phosphocholin
- 815.: (Z,Z)-6,18-Docosadienyl-1-phosphocholin
- 816.: (Z,Z)-7,19-Docosadienyl-1-phosphocholin
- 817.: (Z,Z)-3,17-Docosadienyl-1-phosphocholin
- 818.: (Z,Z)-4,18-Docosadienyl-1-phosphocholin
- 819.: (Z,Z)-5,19-Docosadienyl-1-phosphocholin
- 820.: (Z,Z)-3,19-Docosadienyl-1-phosphocholin

### 23 Kettenkohlenstoffatome

### C₂₈H₅₆NO₄P (501.73)

- 821.: (Z,Z)-3,7-Tricosadienyl-1-phosphocholin
- 822.: (Z,Z)-4,8-Tricosadienyl-1-phosphocholin
- 823.: (Z,Z)-5,9-Tricosadienyl-1-phosphocholin
- 824.: (Z,Z)-6,10-Tricosadienyl-1-phosphocholin
- 825.: (Z,Z)-7,11-Tricosadienyl-1-phosphocholin
- 826.: (Z,Z)-8,12-Tricosadienyl-1-phosphocholin
- 827.: (Z,Z)-9,13-Tricosadienyl-1-phosphocholin
- 828.: (Z,Z)-10,14-Tricosadienyl-1-phosphocholin
- 829.: (Z,Z)-11,15-Tricosadienyl-1-phosphocholin
- 830.: (Z,Z)-12,16-Tricosadienyl-1-phosphocholin
- 831.: 831. (Z,Z)-13,17-Tricosadienyl-1-phosphocholin
- 832.: (Z,Z)-14,18-Tricosadienyl-1-phosphocholin
- 833.: (Z,Z)-15,19-Tricosadienyl-1-phosphocholin
- 834.: (Z,Z)-16,20-Tricosadienyl-1-phosphocholin
- 835.: (Z,Z)-3,8-Tricosadienyl-1-phosphocholin
- 836.: (Z,Z)-4,9-Tricosadienyl-1-phosphocholin
- 837.: (Z,Z)-5,10-Tricosadienyl-1-phosphocholin
- 838.: (Z,Z)-6,11-Tricosadienyl-1-phosphocholin
- 839.: (Z,Z)-7,12-Tricosadienyl-1-phosphocholin
- 840.: (Z,Z)-8,13-Tricosadienyl-1-phosphocholin
- 841.: (Z,Z)-9,14-Tricosadienyl-1-phosphocholin
- 842.: (Z,Z)-10,15-Tricosadienyl-1-phosphocholin
- 843.: (Z,Z)-11,16-Tricosadienyl-1-phosphocholin
- 844.: (Z,Z)-12,17-Tricosadienyl-1-phosphocholin
- 845.: (Z,Z)-13,18-Tricosadienyl-1-phosphocholin
- 846.: (Z,Z)-14,19-Tricosadienyl-1-phosphocholin
- 847.: (Z,Z)-15,20-Tricosadienyl-1-phosphocholin
- 848.: (Z,Z)-3,9-Tricosadienyl-1-phosphocholin
- 849.: (Z,Z)-4,10-Tricosadienyl-1-phosphocholin
- 850.: (Z,Z)-5,11-Tricosadienyl-1-phosphocholin
- 851.: (Z,Z)-6,12-Tricosadienyl-1-phosphocholin
- 852.: (Z,Z)-7,13-Tricosadienyl-1-phosphocholin
- 853.: (Z,Z)-8,14-Tricosadienyl-1-phosphocholin
- 854.: (Z,Z)-9,15-Tricosadienyl-1-phosphocholin
- 855.: (Z,Z)-10,16-Tricosadienyl-1-phosphocholin
- 856.: (Z,Z)-11,17-Tricosadienyl-1-phosphocholin
- 857.: (Z,Z)-12,18-Tricosadienyl-1-phosphocholin
- 858.: (Z,Z)-13,19-Tricosadienyl-1-phosphocholin
- 859.: (Z,Z)-14,20-Tricosadienyl-1-phosphocholin
- 860.: (Z,Z)-3,10-Tricosadienyl-1-phosphocholin
- 861.: (Z,Z)-4,11-Tricosadienyl-1-phosphocholin
- 862.: (Z,Z)-5,12-Tricosadienyl-1-phosphocholin
- 863.: (Z,Z)-6,13-Tricosadienyl-1-phosphocholin
- 864.: (Z,Z)-7,14-Tricosadienyl-1-phosphocholin
- 865.: (Z,Z)-8,15-Tricosadienyl-1-phosphocholin
- 866.: (Z,Z)-9,16-Tricosadienyl-1-phosphocholin
- 867.: (Z,Z)-10,17-Tricosadienyl-1-phosphocholin
- 868.: (Z,Z)-11,18-Tricosadienyl-1-phosphocholin
- 869.: (Z,Z)-12,19-Tricosadienyl-1-phosphocholin
- 870.: (Z,Z)-13,20-Tricosadienyl-1-phosphocholin
- 871.: (Z,Z)-3,11-Tricosadienyl-1-phosphocholin
- 872.: (Z,Z)-4,12-Tricosadienyl-1-phosphocholin
- 873.: (Z,Z)-5,13-Tricosadienyl-1-phosphocholin
- 874.: (Z,Z)-6,14-Tricosadienyl-1-phosphocholin
- 875.: (Z,Z)-7,15-Tricosadienyl-1-phosphocholin
- 876.: (Z,Z)-8,16-Tricosadienyl-1-phosphocholin
- 877.: (Z,Z)-9,17-Tricosadienyl-1-phosphocholin
- 878.: (Z,Z)-10,18-Tricosadienyl-1-phosphocholin
- 879.: (Z,Z)-11,19-Tricosadienyl-1-phosphocholin
- 880.: (Z,Z)-12,20-Tricosadienyl-1-phosphocholin
- 881.: (Z,Z)-3,12-Tricosadienyl-1-phosphocholin
- 882.: (Z,Z)-4,13-Tricosadienyl-1-phosphocholin
- 883.: (Z,Z)-5,14-Tricosadienyl-1-phosphocholin
- 884.: (Z,Z)-6,15-Tricosadienyl-1-phosphocholin
- 885.: (Z,Z)-7,16-Tricosadienyl-1-phosphocholin
- 886.: (Z,Z)-8,17-Tricosadienyl-1-phosphocholin
- 887.: (Z,Z)-9,18-Tricosadienyl-1-phosphocholin
- 888.: (Z,Z)-10,19-Tricosadienyl-1-phosphocholin
- 889.: (Z,Z)-11,20-Tricosadienyl-1-phosphocholin
- 890.: (Z,Z)-3,13-Tricosadienyl-1-phosphocholin
- 891.: (Z,Z)-4,14-Tricosadienyl-1-phosphocholin
- 892.: (Z,Z)-5,15-Tricosadienyl-1-phosphocholin
- 893.: (Z,Z)-6,16-Tricosadienyl-1-phosphocholin
- 894.: (Z,Z)-7,17-Tricosadienyl-1-phosphocholin
- 895.: (Z,Z)-8,18-Tricosadienyl-1-phosphocholin
- 896.: (Z,Z)-9,19-Tricosadienyl-1-phosphocholin
- 897.: (Z,Z)-10,20-Tricosadienyl-1-phosphocholin
- 898.: (Z,Z)-3,14-Tricosadienyl-1-phosphocholin
- 899.: (Z,Z)-4,15-Tricosadienyl-1-phosphocholin
- 900.: (Z,Z)-5,16-Tricosadienyl-1-phosphocholin
- 901.: (Z,Z)-6,17-Tricosadienyl-1-phosphocholin
- 902.: (Z,Z)-7,18-Tricosadienyl-1-phosphocholin
- 903.: (Z,Z)-8,19-Tricosadienyl-1-phosphocholin
- 904.: (Z,Z)-9,20-Tricosadienyl-1-phosphocholin
- 905.: (Z,Z)-3,15-Tricosadienyl-1-phosphocholin
- 906.: (Z,Z)-4,16-Tricosadienyl-1-phosphocholin
- 907.: (Z,Z)-5,17-Tricosadienyl-1-phosphocholin
- 908.: (Z,Z)-6,18-Tricosadienyl-1-phosphocholin
- 909.: (Z,Z)-7,19-Tricosadienyl-1-phosphocholin
- 910.: (Z,Z)-8,20-Tricosadienyl-1-phosphocholin
- 911.: (Z,Z)-3,17-Tricosadienyl-1-phosphocholin
- 912.: (Z,Z)-4,18-Tricosadienyl-1-phosphocholin
- 913.: (Z,Z)-5,19-Tricosadienyl-1-phosphocholin
- 914.: (Z,Z)-6,20-Tricosadienyl-1-phosphocholin
- 915.: (Z,Z)-3,19-Tricosadienyl-1-phosphocholin
- 916.: (Z,Z)-4,20-Tricosadienyl-1-phosphocholin

### 24 Kettenkohlenstoffatome

### C₂₉H₅₈NO₄P (515.76)

- 917.: (Z,Z)-3,7-Tetracosadienyl-1-phosphocholin
- 918.: (Z,Z)-4,8-Tetracosadienyl-1-phosphocholin
- 919.: (Z,Z)-5,9-Tetracosadienyl-1-phosphocholin
- 920.: (Z,Z)-6,10-Tetracosadienyl-1-phosphocholin
- 921.: (Z,Z)-7,11-Tetracosadienyl-1-phosphocholin
- 922.: (Z,Z)-8,12-Tetracosadienyl-1-phosphocholin
- 923.: (Z,Z)-9,13-Tetracosadienyl-1-phosphocholin
- 924.: (Z,Z)-10,14-Tetracosadienyl-1-phosphocholin
- 925.: (Z,Z)-11,15-Tetracosadienyl-1-phosphocholin
- 926.: (Z,Z)-12,16-Tetracosadienyl-1-phosphocholin
- 927.: (Z,Z)-13,17-Tetracosadienyl-1-phosphocholin
- 928.: (Z,Z)-14,18-Tetracosadienyl-1-phosphocholin
- 929.: (Z,Z)-15,19-Tetracosadienyl-1-phosphocholin
- 930.: (Z,Z)-16,20-Tetracosadienyl-1-phosphocholin
- 931.: (Z,Z)-17,21-Tetracosadienyl-1-phosphocholin
- 932.: (Z,Z)-3,8-Tetracosadienyl-1-phosphocholin
- 933.: (Z,Z)-4,9-Tetracosadienyl-1-phosphocholin
- 934.: (Z,Z)-5,10-Tetracosadienyl-1-phosphocholin
- 935.: (Z,Z)-6,11-Tetracosadienyl-1-phosphocholin
- 936.: (Z,Z)-7,12-Tetracosadienyl-1-phosphocholin
- 937.: (Z,Z)-8,13-Tetracosadienyl-1-phosphocholin
- 938.: (Z,Z)-9,14-Tetracosadienyl-1-phosphocholin
- 939.: (Z,Z)-10,15-Tetracosadienyl-1-phosphocholin
- 940.: (Z,Z)-11,16-Tetracosadienyl-1-phosphocholin
- 941.: (Z,Z)-12,17-Tetracosadienyl-1-phosphocholin
- 942.: (Z,Z)-13,18-Tetracosadienyl-1-phosphocholin
- 943.: (Z,Z)-14,19-Tetracosadienyl-1-phosphocholin
- 944.: (Z,Z)-15,20-Tetracosadienyl-1-phosphocholin
- 945.: (Z,Z)-16,21-Tetracosadienyl-1-phosphocholin
- 946.: (Z,Z)-3,9-Tetracosadienyl-1-phosphocholin
- 947.: (Z,Z)-4,10-Tetracosadienyl-1-phosphocholin
- 948.: (Z,Z)-5,11-Tetracosadienyl-1-phosphocholin
- 949.: (Z,Z)-6,12-Tetracosadienyl-1-phosphocholin
- 950.: (Z,Z)-7,13-Tetracosadienyl-1-phosphocholin
- 951.: (Z,Z)-8,14-Tetracosadienyl-1-phosphocholin
- 952.: (Z,Z)-9,15-Tetracosadienyl-1-phosphocholin
- 953.: (Z,Z)-10,16-Tetracosadienyl-1-phosphocholin
- 954.: (Z,Z)-11,17-Tetracosadienyl-1-phosphocholin
- 955.: (Z,Z)-12,18-Tetracosadienyl-1-phosphocholin
- 956.: (Z,Z)-13,19-Tetracosadienyl-1-phosphocholin
- 957.: (Z,Z)-14,20-Tetracosadienyl-1-phosphocholin
- 958.: (Z,Z)-15,21-Tetracosadienyl-1-phosphocholin
- 959.: (Z,Z)-3,10-Tetracosadienyl-1-phosphocholin
- 960.: (Z,Z)-4,11-Tetracosadienyl-1-phosphocholin
- 961.: (Z,Z)-5,12-Tetracosadienyl-1-phosphocholin
- 962.: (Z,Z)-6,13-Tetracosadienyl-1-phosphocholin
- 963.: (Z,Z)-7,14-Tetracosadienyl-1-phosphocholin
- 964.: (Z,Z)-8,15-Tetracosadienyl-1-phosphocholin
- 965.: (Z,Z)-9,16-Tetracosadienyl-1-phosphocholin
- 966.: (Z,Z)-10,17-Tetracosadienyl-1-phosphocholin
- 967.: (Z,Z)-11,18-Tetracosadienyl-1-phosphocholin
- 968.: (Z,Z)-12,19-Tetracosadienyl-1-phosphocholin
- 969.: (Z,Z)-13,20-Tetracosadienyl-1-phosphocholin
- 970.: (Z,Z)-14,21-Tetracosadienyl-1-phosphocholin
- 971.: (Z,Z)-3,11-Tetracosadienyl-1-phosphocholin
- 972.: (Z,Z)-4,12-Tetracosadienyl-1-phosphocholin
- 973.: (Z,Z)-5,13-Tetracosadienyl-1-phosphocholin
- 974.: (Z,Z)-6,14-Tetracosadienyl-1-phosphocholin
- 975.: (Z,Z)-7,15-Tetracosadienyl-1-phosphocholin
- 976.: (Z,Z)-8,16-Tetracosadienyl-1-phosphocholin
- 977.: (Z,Z)-9,17-Tetracosadienyl-1-phosphocholin
- 978.: (Z,Z)-10,18-Tetracosadienyl-1-phosphocholin
- 979.: (Z,Z)-11,19-Tetracosadienyl-1-phosphocholin
- 980.: (Z,Z)-12,20-Tetracosadienyl-1-phosphocholin
- 981.: (Z,Z)-13,21-Tetracosadienyl-1-phosphocholin
- 982.: (Z,Z)-3,12-Tetracosadienyl-1-phosphocholin
- 983.: (Z,Z)-4,13-Tetracosadienyl-1-phosphocholin
- 984.: (Z,Z)-5,14-Tetracosadienyl-1-phosphocholin
- 985.: (Z,Z)-6,15-Tetracosadienyl-1-phosphocholin
- 986.: (Z,Z)-7,16-Tetracosadienyl-1-phosphocholin
- 987.: (Z,Z)-8,17-Tetracosadienyl-1-phosphocholin
- 988.: (Z,Z)-9,18-Tetracosadienyl-1-phosphocholin
- 989.: (Z,Z)-10,19-Tetracosadienyl-1-phosphocholin
- 990.: (Z,Z)-11,20-Tetracosadienyl-1-phosphocholin
- 991.: (Z,Z)-12,21-Tetracosadienyl-1-phosphocholin
- 992.: (Z,Z)-3,13-Tetracosadienyl-1-phosphocholin
- 993.: (Z,Z)-4,14-Tetracosadienyl-1-phosphocholin
- 994.: (Z,Z)-5,15-Tetracosadienyl-1-phosphocholin
- 995.: (Z,Z)-6,16-Tetracosadienyl-1-phosphocholin
- 996.: (Z,Z)-7,17-Tetracosadienyl-1-phosphocholin
- 997.: (Z,Z)-8,18-Tetracosadienyl-1-phosphocholin
- 998.: (Z,Z)-9,19-Tetracosadienyl-1-phosphocholin
- 999.: (Z,Z)-10,20-Tetracosadienyl-1-phosphocholin
- 1000.: (Z,Z)-11,21-Tetracosadienyl-1-phosphocholin
- 1001.: (Z,Z)-3,14-Tetracosadienyl-1-phosphocholin
- 1002.: (Z,Z)-4,15-Tetracosadienyl-1-phosphocholin
- 1003.: (Z,Z)-5,16-Tetracosadienyl-1-phosphocholin
- 1004.: (Z,Z)-6,17-Tetracosadienyl-1-phosphocholin
- 1005.: (Z,Z)-7,18-Tetracosadienyl-1-phosphocholin
- 1006.: (Z,Z)-8,19-Tetracosadienyl-1-phosphocholin
- 1007.: (Z,Z)-9,20-Tetracosadienyl-1-phosphocholin
- 1008.: (Z,Z)-10,21-Tetracosadienyl-1-phosphocholin
- 1009.: (Z,Z)-3,15-Tetracosadienyl-1-phosphocholin
- 1010.: (Z,Z)-4,16-Tetracosadienyl-1-phosphocholin
- 1011.: (Z,Z)-5,17-Tetracosadienyl-1-phosphocholin
- 1012.: (Z,Z)-6,18-Tetracosadienyl-1-phosphocholin
- 1013.: (Z,Z)-7,19-Tetracosadienyl-1-phosphocholin
- 1014.: (Z,Z)-8,20-Tetracosadienyl-1-phosphocholin
- 1015.: (Z,Z)-9,21-Tetracosadienyl-1-phosphocholin
- 1016.: (Z,Z)-3,17-Tetracosadienyl-1-phosphocholin
- 1017.: (Z,Z)-4,18-Tetracosadienyl-1-phosphocholin
- 1018.: (Z,Z)-5,19-Tetracosadienyl-1-phosphocholin
- 1019.: (Z,Z)-6,20-Tetracosadienyl-1-phosphocholin
- 1020.: (Z,Z)-7,21-Tetracosadienyl-1-phosphocholin
- 1021.: (Z,Z)-3,19-Tetracosadienyl-1-phosphocholin
- 1022.: (Z,Z)-4,20-Tetracosadienyl-1-phosphocholin
- 1023.: (Z,Z)-5,21-Tetracosadienyl-1-phosphocholin

### 25 Kettenkohlenstoffatome

### C₃₀H₆₀NO₄P (529.78)

- 1024.: (Z,Z)-6,12-Pentacosadienyl-1-phosphocholin
- 1025.: (Z,Z)-9,15-Pentacosadienyl-1-phosphocholin
- 1026.: (Z,Z)-6,16-Pentacosadienyl-1-phosphocholin
- 1027.: (Z,Z)-9,18-Pentacosadienyl-1-phosphocholin
- 1028.: (Z,Z)-10,20-Pentacosadienyl-1-phosphocholin
- 1029.: (Z,Z)-13,20-Pentacosadienyl-1-phosphocholin

### 26 Kettenkohlenstoffatome

### C₃₁H₆₂NO₄P (543.81)

- 1030.: (Z,Z)-6,12-Hexacosadienyl-1-phosphocholin
- 1031.: (Z,Z)-9,15-Hexacosadienyl-1-phosphocholin
- 1032.: (Z,Z)-6,16-Hexacosadienyl-1-phosphocholin
- 1033.: (Z,Z)-9,18-Hexacosadienyl-1-phosphocholin
- 1034.: (Z,Z)-6,20-Hexacosadienyl-1-phosphocholin

### 5. Beispiele für (Z,Z)-Alkadienyl-1-phospho-N,N,N-trimethylpropylammonium-Verbindungen (Vergleichsbeispiel)

### (A = IX; n = 3; R₃, CH₃; m= 1, x = 1; z = 0)

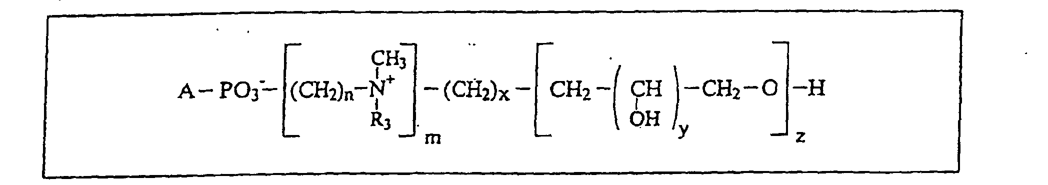

wobei A für eine zweifach ungesättigte Alkylkette folgender Struktur steht (s,t,r ≥ 0; 8 ≤ s+t+r ≤ 26): 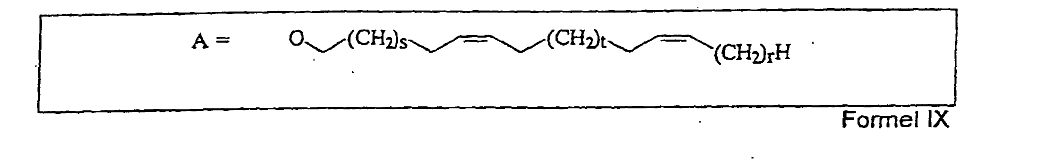
- 1035.): (Z,Z)-5,11-Hexadecadienyl-1-phospho-N,N,N-trimethyl-propylammonium
C₂₂H₄₄NO₄P (417.57)
- 1036.) (Z,Z)-5,11-Heptadecadienyl-1-phospho-N,N,N-trimethyl-propylammonium
C₂₃H₄₆NO₄P (431.60)
- 1037.): (Z,Z)-5,11-Octadecadienyl-1-phospho-N,N,N-trimethyl-propylammonium
C₂₄H₄₈NO₄P (445.62)
- 1038.): (Z,Z)-6,12-Nonadecadienyl-1-phospho-N,N,N-trimethyl-propylammonium
C₂₅H₅₀NO₄P (459.65)
- 1039.): (Z,Z)-10,16-Eicosadienyl-1-phospho-N,N,N-trimethyl-propylammonium
C₂₆H₅₂NO₄P (473.68)
- 1040.): (Z,Z)-10,16-Heneicosadienyl-1-phospho-N,N,N-trimethyl-propylammonium
C₂₇H₅₄NO₄P (487.70)
- 1041.): (Z,Z)-10,16-Docosadienyl-1-phospho-N,N,N-trimethyl-propylammonium
C₂₈H₅₆NO₄P (501.73)
- 1042.): (Z,Z)-10,16-Tricosadienyl-1-phospho-N,N,N-trimethyl-propylammonium
C₂₉H₉NO₄P (515.76)
- 1043.): (Z,Z)- 6,18-Tetracosadienyl-1-phospho-N,N,N-trimethyl-propylammonium
C₃₀H₆₀NO₄P (529.78)

### 6. Beispiele für (Z,Z)-Alkadienyl-1-phospho-N,N,N-trimethyl-butylammonium-Verbindungen (Vergleichsbeispiel)

### (A = IX; n = 4; R₃, CH₃; m = 1, x = 7; z = 0)

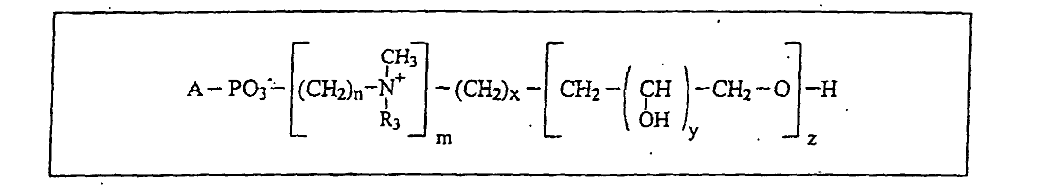

wobei A für eine zweifach ungesättigte Alkylkette folgender Struktur steht (s,t,r ≥ 0; 8 ≤ s+t+r ≤ 26): 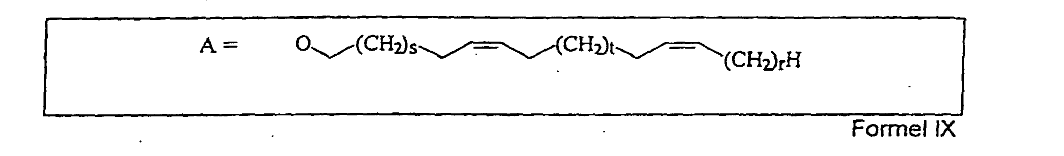
- 1044.): (Z,Z)-5,11-Hexadecadienyl-1-phospho-N,N,N-trimethyl-butylammonium
C₂₃H₄₆NO₄P (431.60)
- 1045.): (Z,Z)-5,11-Heptadecadienyl-1-phospho-N,N,N-trimethyl-butylammonium
C₂₄H₄₈NO₄P (445.62)
- 1046.): (Z,Z)-5,11-Octadecadienyl-1-phospho-N,N,N-trimethyl-butylammonium
C₂₅H_{5O}NO₄P (459.65)
- 1047.): (Z,Z)-6,12-Nonadecadienyl-1-phospho-N,N,N-trimethyl-butylammonium
C₂₆H₅₂NO₄P (473.68)
- 1048.): (Z,Z)-10,16-Eicosadienyl-1-phospho-N,N,N-trimethyl-butylammonium
C₂₇H₅₄NO₄P (487.70)
- 1049.): (Z,Z)-10,16-Heneicosadienyl-1-phospho-N,N,N-trimethyl-butylammonium
C₂₈H₅₆NO₄P (501.73)
- 1050.): (Z,Z)-10,16-Docosadienyl-1-phospho-N,N,N-trimethyl-butylammonium
C₂₉H₅₈N₄P (515.76)
- 1051.): (Z,Z)-10,16-Tricosadienyl-1-phospho-N,N,N-trimethyl-butylammonium
C₃₀H₆₀NO₄P (529.78)
- 1052.): (Z,Z)- 6,18-Tetracosadienyl-1-phospho-N,N,N-trimethyl-butylammonium
C₃₁H₆₂NO₄P (543.81)

### 7. Beispiele für terminal ungesättigte Alkadienylphosphocholine (Vergleichsbeispiel) (A = IX; n = 2; R₃, CH₃; m= 1, x = 1; z = 0)

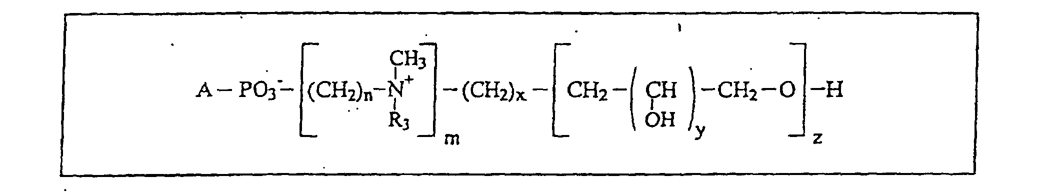

wobei A für eine zweifach ungesättigte Alkylkette folgender Struktur steht (s,t ≥ 0; r = 0; 8 ≤ s+t+r ≤ 26): 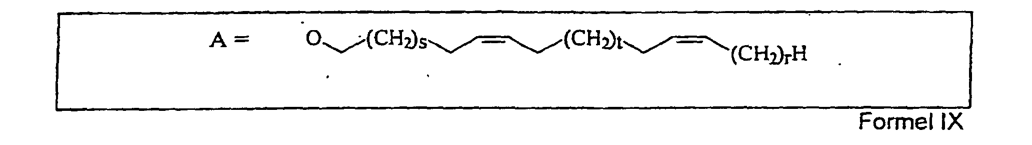
- 1053.): (Z)-11,15-Hexadecadienyl-1-phosphocholin
C₂₁H₄₂NO₄P (403.54)
- 1054.): (Z)-11,16-Heptadecadienyl-1-phosphocholin
C₂₂H₄₄NO₄P (417.57)
- 1055.): (Z)-11,17-Octadecadienyl-1-phosphocholin
C₂₃H₄₆NO₄P (431.60)
- 1056.): (Z)-11,18-Nonadecadienyl-1-phosphocholin
C₂₄H₄₈NO₄P (445.62)
- 1057.): (Z)-11,19-Eicosadienyl-1-phosphocholin
C₂₅H₅₀NO₄P (459.65)
- 1058.): (Z)-11,20-Heneicosadienyl-1-phosphocholin
C₂₆H₅₂NO₄P (473.68)
- 1059.): (Z)-11,21-Docasadienyl-1-phosphocholin
C₂₇H₅₄NO₄P (487.70)
- 1060.): (Z)-1 1,22-Tricosadienyl-1 -phosphocholin
C₂₈H₅₆NO₄P (501.73)
- 1061.): (Z)-11,23-Tetracosadienyl-1-phosphocholin
C₂₉H₅₈NO₄P (515.76)
- 1062.): (2)-11,24-Pentacosadienyl-1-phosphocholin
C₃₀H₅₀NO₄P (529.78)

### 8. Beispiele für terminal ungesättigte Alkadienyl-1-phospho-N,N,N-trimethylpropylammonium-Verbindungen (Vergleichsbeispiel)

### (A = IX; n = 3; R₃, CH₃; m = 1, x = 1; z = 0)

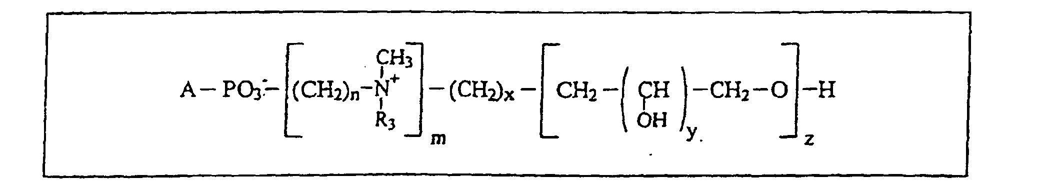

wobei A für eine zweifach ungesättigte Alkylkette folgender Struktur steht (s,t ≥ 0; r = 0; 8 ≤ s+t+r ≤ 26): 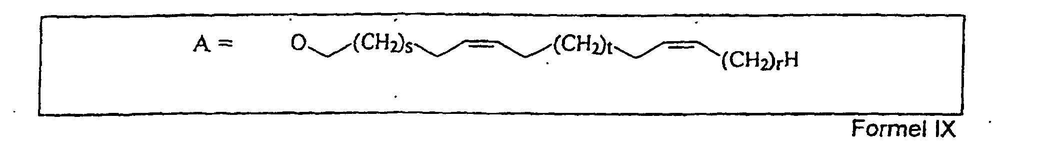
- 1063.): (Z)-11,15-Hexadecadienyl-1-phospho-N,N,N-trimethyl-prapylammonium
C₂₂H₄₄NO₄P (417.57)
- 1064.): (Z)-11,16-Heptadecadienyl-1-phospho-N,N,N-trimethyl-propylammonium
C₂₃H₄₆NO₄P (431.60)
- 1065.): (Z)-11,17-Octadecadienyl-1-phospho-N,N,N-trimethyl-propylammonium
C₂₄H₄₈NO₄P (445.62)
- 1066.): (Z)-11,18-Nonadecadienyl-1-phospho-N,N,N-trimethyl-propylammonium
C₂₅H₅₀NO₄P (459.65)
- 1067.): (Z)-11,19-Eicosadienyl-1-phospho-N,N,N-trimethyl-propylammonium
C₂₅H₅₂NO₄P (473.68)
- 1068.): (Z)-11,20-Heneicosadienyl-1-phospho-N,N,N-trimethyl-propylammonium
C₂₇H₅₄NO₄P (487.70)
- 1069.): (Z)-11,21-Docosadienyl-1-phospho-N,N,N-trimethyl-propylammonium
C₂₈H₅₆NO₄P (501.73)
- 1070.): (Z)-11,22-Tricosadienyl-1-phospho-N,N,N-trimethyl-propylammonium
C₂₉H₅₈NO₄P (515.76)
- 1071.): (Z)-11,23-Tetracosadienyl-1-phospho-N,N,N-trimethyl-propylammonium
C₃₀H₆₀NO₄P (529.78)
- 1072.): (Z)-11,24-Pentacosadienyl-1-phospho-N,N,N-trimethyl-propylammonium
C₃₁H₆₂NO₄P (543.81)

### 9. Beispiele für terminal ungesättigte Alkadienyl-1-phospho-N,N,N-trimethylbutylammonium-Verbindungen (Vergleichsbeispiel)

### (A = IX; n = 4; R₃, CH₃; m= 1, x = 1; z = 0)

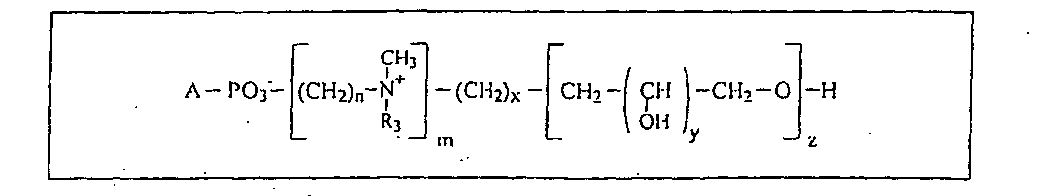

wobei A für eine zweifach ungesättigte Alkylkette folgender Struktur steht (s,t ≥ 0; r = 0; 8 ≤ s+t+r ≤ 26): 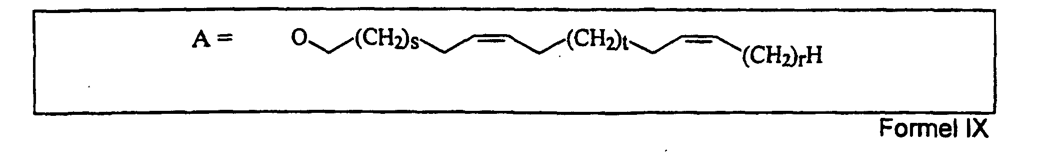
- 1073.): (Z)-11,15-Hexadecadienyl-1-phospho-N,N,N-trimethyl-butylammonium
C₂₃H₄₆NO₄P (431.60)
- 1074.): (Z)-11,16-Heptadecadienyl-1-phospho-N,N,N-trimethyl-butylammonium
C₂₄H₄₈NO₄P (445.62)
- 1075.): (Z)-11,17-Octadecadienyl-1-phospho-N,N,N-trimethyl-butylammonium
C₂₅H₅₀NO₄P (459.65)
- 1076.): (Z)-11,18-Nonadecadienyl-1-phospho-N,N,N-trimethyl-butylammonium
C₂₆H₅₂NO₄P (473.68)
- 1077.): (Z)-11,19-Eicosadienyl-1-phospho-N,N,N-trimethyl-butylammonium
C₂₇H₅₄NO₄P (487.70)
- 1078.): (Z)-11,20-Heneicosadienyl-1-phospho-N,N,N-trimethyl-butylammonium
C₂₈H₅₆NO₄P (501.73)
- 1079.): (Z)-11,21-Docosadienyl-1-phospho-N,N,N-trimethyl-butylammonium
C₂₉H₅₈NO₄P (515.76)
- 1080.): (Z)-11,22-Tricosadienyl-1-phospho-N,N,N-trimethyl-butylammonium
C₃₀H₆₀NO₄P (529.78)
- 1081.): (Z)-11,23-Tetracosadienyl-1-phospho-N,N,N-trimethyl-butylammonium
C₃₁H₆₂NO₄P (543.81)
- 1082.): (Z)-11,24-Pentacosadienyl-1-phospho-N,N,N-trimethyl-butylammonium
C₃₂H₆₄NO₄P (557.84)

### 10. Wirkstoffe, die auf alkylierten (Ether)-Lysolecithinen aufgebaut sind - einfach ungesättigte Verbindungen

### (A = III bzw. A = IV; n = 2-6; R₃, CH₃; m= 1, x = 1; z = 0)

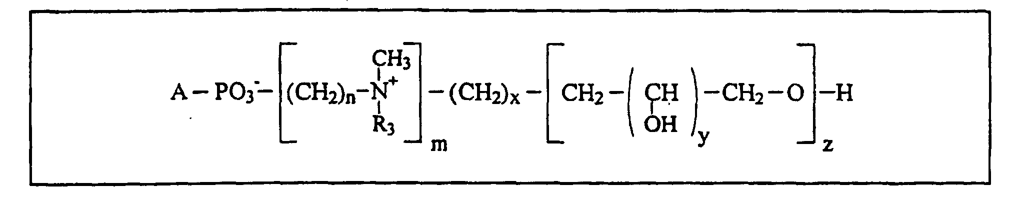

- 1083.): 1-O-(Z)-6-Octadecenyl-2-O-methyl-*sn*-glycero-3-phosphocholin (n = 2)
C₂₇H₅₆NO₆P (521.72)
- 1084.): 1-O-(Z)-10-Octadecenyl-2-O-methyl-*sn*-glycero-3-phosphocholin (n = 2)
C₂₇H₅₆NO₆P (521.72)
- 1085.): 1-O-(Z)-12-Octadecenyl-2-O-methyl-*sn*-glycero-3-phosphocholin (n = 2)
C₂₇H₅₆NO₆P (521.72)
- 1086.): 1-O-(Z)-6-Nonadecenyl-2-O-methyl-*sn*-glycero-3-phosphocholin (n = 2)
C₂₈H₅₈NO₆P (535.75)
- 1087.): 1-O-(Z)-10-Nonadecenyl-2-O-methyl-*sn*-glycero-3-phosphocholin (n = 2)
C₂₈H₅₈NO₆P (535.75)
- 1088.): 1-O-(Z)-12-Nonadecenyl-2-O-methyl-*sn*-glycero-3-phosphocholin (n = 2)
C₂₈H₅₈NO₆P (535.75)
- 1089.): 1-O-(Z)-6-Eicosenyl-2-O-methyl-*sn*-glycero-3-phosphocholin (n = 2)
C₂₉H₆₀NO₆P (549.77)
- 1090.): 1-O-(Z)-10-Eicosenyl-2-O-methyl-*sn*-glycero-3-phosphocholin (n = 2)
C₂₉H₆₀NO₆P (549.77)
- 1091.): 1-O-(Z)-12-Eicosenyl-2-O-methyl-*sn*-glycero-3-phosphocholin (n = 2)
C₂₉H₆₀NO₆P (549.77)
- 1092.): 1-O-(Z)-6-Heneicosenyl-2-O-methyl-*sn*-glycero-3-phosphocholin (n = 2)
C₃₀H₆₂NO₆P (563.80)
- 1093.): 1-O-(Z)-10-Heneicosenyl-2-O-methyl-*sn*-glycero-3-phosphocholin (n = 2)
C₃₀H₆₂NO₆P (563.80)
- 1094.): 1-O-(Z)-12-Heneicosenyl-2-O-methyl-*sn*-glycero-3-phosphocholin (n = 2)
C₃₀H₆₂NO₆P (563.80)
- 1095.): 1-O-(Z)-6-Docosenyl-2-O-methyl-*sn*-glycero-3-phosphocholin (n = 2)
C₃₁H₆₄NO₆P (577.83)
- 1096.): 1-O-(Z)-10-Docosenyl-2-O-methyl-*sn*-glycero-3-phosphocholin (n = 2)
C₃₁H₆₄NO₆P (577.83)
- 1097.): 1-O-(Z)-12-Docosenyl-2-O-methyl-*sn*-glycero-3-phosphocholin (n = 2)
C₃₁H₆₄NO₆P (577.83)
- 1098.): 1-O-(Z)-6-Tricosenyl-2-O-methyl-*sn*-glycero-3-phosphocholin (n = 2)
C₃₂H₆₆NO₆P (591.86)
- 1099.): 1-O-(Z)-10-Tricosenyl-2-O-methyl-*sn*-glycero-3-phosphocholin (n = 2)
C₃₂H₆₆NO₆P (591.86)
- 1100.): 1-O-(Z)-12-Tricosenyl-2-O-methyl-*sn*-glycero-3-phosphocholin (n = 2)
C₃₂H₆₆NO₆P (591.86)
- 1101.): 1-O-(Z)-6-Tetracosenyl-2-O-methyl-*sn*-glycero-3-phosphocholin (n = 2)
C₃₃H₆₈NO₆P (605.89)
- 1102.): 1-O-(Z)-10-Tetracosenyl-2-O-methyl-*sn*-glycero-3-phosphocholin (n = 2)
C₃₃H₆₈NO₆P (605.89)
- 1103.): 1-O-(Z)-12-Tetracosenyl-2-O-methyl-*sn*-glycero-3-phosphocholin (n = 2)
C₃₃H₆₈NO₆P (605.89)
- 1104.): 1-O-(Z)-6-Octadecenyl-2-O-methyl-*sn*-glycero-3-phospho-N,N,N-trimethylpropylammonium (n = 3)
C₂₈H₅₈NO₆P (535.75)
- 1105.): 1-O-(Z)-10-Octadecenyl-2-O-methyl-*sn*-glycero-3-phospho-N,N,N-trimethylpropylammonium (n = 3)
C₂₈H₅₈NO₆P (535.75)
- 1106.): 1-O-(Z)-12-Octadecenyl-2-O-methyl-*sn*-glycero-3-phospho-N,N,N-trimethylpropylammonium (n = 3)
C₂₈H₅₈NO₆P (535.75)
- 1107.): 1-O-(Z)-6-Nonadecenyl-2-O-methyl-*sn*-glycero-3-phospho-N,N,N-trimethyl-propylammonium (n = 3)
C₂₉H₆₀NO₆P (549.77)
- 1108.): 1-O-(Z)-10-Nonadecenyl-2-O-methyl-*sn*-glycero-3-phospho-N,N,N-trimethyl-propylammonium (n = 3)
C₂₉H₆₀NO₆P (549.77)
- 1109.): 1-O-(Z)-12-Nonadecenyl-2-O-methyl-*sn*-glycero-3-phospho-N,N,N-trimethyl-propylammonium (n = 3)
C₂₉H₆₀NO₆P (549.77)
- 1110.): 1-O-(Z)-6-Eicosenyl-2-O-methyl-*sn*-glycero-3-phospho-N,N,N-trimethyl-propylammonium (n = 3)
C₃₀H₆₂NO₆P (563.80)
- 1111.): 1-O-(Z)-10-Eicosenyl-2-O-methyl-*sn*-glycero-3-phospho-N,N,N-trimethyl-propylammonium (n = 3)
C₃₀H₆₂NO₆P (563.80)
- 1112.): 1-O-(Z)-12-Eicosenyl-2-O-methyl-*sn*-glycero-3-phospho-N,N,N-trimethyl-propylammonium (n = 3)
C₃₀H₆₂NO₆P (563.80)
- 1113.): 1-O-(Z)-6-Heneicosenyl-2-O-methyl-*sn*-glycero-3-phospho-N,N,N-trimethyl-propylammonium (n = 3)
C₃₁H₆₄NO₆P (577.83)
- 1114.): 1-O-(Z)-10-Heneicosenyl-2-O-methyl-*sn*-glycero-3-phospho-N,N,N-trimethyl-propylammonium (n = 3)
C₃₁H₆₄NO₆P (577.83)
- 1115.): 1-O-(Z)-12-Heneicosenyl-2-O-methyl-*sn*-glycero-3-phospho-N,N,N-trimethyl-propylammonium (n = 3)
C₃₁H₆₄NO₆P (577.83)
- 1116.): 1-O-(Z)-6-Docosenyl-2-O-methyl-*sn*-glycero-3-phospho-N,N,N-trimethyl-propylammonium (n = 3)
C₃₂H₆₆NO₆P (591.86)
- 1117.): 1-O-(Z)-10-Docosenyl-2-O-methyl-*sn*-glycero-3-phospho-N,N,N-trimethyl-propylammonium (n = 3)
C₃₂H₆₆NO₆P (591.86)
- 1118.): 1-O-(Z)-12-Docosenyl-2-O-methyl-*sn*-glycero-3-phospho-N,N,N-trimethyl-propylammonium (n = 3)
C₃₂H₆₆NO₆P (591.86)
- 1119.): 1-O-(Z)-6-Tricosenyl-2-O-methyl-*sn*-glycero-3-phospho-N,N,N-trimethyl-propylammonium (n = 3)
C₃₃H₆₈NO₆P (605.89)
- 1120.): 1-O-(Z)-10-Tricosenyl-2-O-methyl-*sn*-glycero-3-phospho-N,N,N-trimethyl-propylammonium (n = 3)
C₃₃H₆₈NO₆P (605.89)
- 1121.): 1-O-(Z)-12-Tricosenyl-2-O-methyl-*sn*-glycero-3-phospho-N,N,N-trimethyl-propylammonium (n = 3)
C₃₃H₆₈NO₆P (605.89)
- 1122.): 1-O-(Z)-6-Tetracosenyl-2-O-methyl-*sn*-glycero-3-phospho-N,N,N-trimethyl-propylammonium (n = 3)
C₃₄H₇₀NO₆P (619.91)
- 1123.): 1-O-(Z)-10-Tetracosenyl-2-O-methyl-*sn*-glycero-3-phospho-N,N,N-trimethyl-propylammonium (n = 3)
C₃₄H₇₀NO₆P (619.91)
- 1124.): 1-O-(Z)-12-Tetracosenyl-2-O-methyl-*sn*-glycero-3-phospho-N,N,N-trimethyl-propylammonium (n = 3)
C₃₄H₇₀NO₆P (619.91)
- 1125.): 1-O-(Z)-6-Octadecenyl-2-O-methyl-*sn*-glycero-3-phospho-N,N,N-trimethyl-butylammonium (n = 4)
C₂₉H₆₀NO₆P (549.77)
- 1126.): 1-O-(Z)-10-Octadecenyl-2-O-methyl-*sn*-glycero-3-phospho-N,N,N-trimethyl-butylammonium (n = 4)
C₂₉H₆₀NO₆P (549.77)
- 1127.): 1-O-(Z)-12-Octadecenyl-2-O-methyl-*sn*-glycero-3-phospho-N,N,N-trimethyl-butylammonium (n = 4)
C₂₉H₆₀NO₆P (549.77)
- 1128.): 1-O-(Z)-6-Nonadecenyl-2-O-methyl-*sn*-glycero-3-phospho-N,N,N-trimethyl-butylammonium (n = 4)
C₃₀H₆₂NO₆P (563.80)
- 1129.): 1-O-(Z)-10-Nonadecenyl-2-O-methyl-*sn*-glycero-3-phospho-N,N,N-trimethyl-butylammonium (n = 4)
C₃₀H₆₂NO₆P (563.80)
- 1130.): 1-O-(Z)-12-Nonadecenyl-2-O-methyl-*sn*-glycero-3-phospho-N,N,N-trimethyl-butylammonium (n = 4)
C₃₀H₆₂NO₆P (563.80)
- 1131.): 1-O-(Z)-6-Eicosenyl-2-O-methyl-*sn*-glycero-3-phospho-N,N,N-trimethyl-butylammonium (n = 4)
C₃₁H₆₄NO₆P (577.83)
- 1132.): 1-O-(Z)-10-Eicosenyl-2-O-methyl-*sn*-glycero-3-phospho-N,N,N-trimethyl-butylammonium (n = 4)
C₃₁H₆₄NO₆P (577.83)
- 1133.): 1-O-(Z)-12-Eicosenyl-2-O-methyl-*sn*-glycero-3-phospho-N,N,N-trimethyl-butylammonium (n = 4)
C₃₁H₆₄NO₆P (577.83)
- 1134.): 1-O-(Z)-6-Heneicosenyl-2-O-methyl-*sn*-glycero-3-phospho-N,N,N-trimethyl-butylammonium (n = 4)
C₃₂H₆₆NO₆P (591.86)
- 1135.): 1-O-(Z)-10-Heneicosenyl-2-O-methyl-*sn*-glycero-3-phospho-N,N,N-trimethyl-butylammonium (n = 4)
C₃₂H₆₆NO₆P (591.86)
- 1136.): 1-O-(Z)-12-Heneicosenyl-2-O-methyl-*sn*-glycero-3-phospho-N,N,N-trimethyl-butylammonium (n = 4)
C₃₂H₆₆NO₆P (591.86)
- 1137.): 1-O-(Z)-6-Docosenyl-2-O-methyl-*sn*-glycero-3-phospho-N,N,N-trimethyl-butylammonium (n = 4)
C₃₃H₆₈NO₆P (605.89)
- 1138.): 1-O-(Z)-10-Docosenyl-2-O-methyl-*sn*-glycero-3-phospho-N,N,N-trimethyl-butylammonium (n = 4)
C₃₃H₆₈NO₆P (605.89)
- 1139.): 1-O-(Z)-12-Docosenyl-2-O-methyl-*sn*-glycero-3-phospho-N,N,N-trimethyl-butylammonium (n = 4)
C₃₃H₆₈NO₆P (605.89)
- 1140.): 1-O-(Z)-6-Tricosenyl-2-O-methyl-*sn*-glycero-3-phospho-N,N,N-trimethyl-butylammonium (n = 4)
C₃₄H₇₀NO₆P (619.91)
- 1141.): 1-O-(Z)-10-Tricosenyl-2-O-methyl-*sn*-glycero-3-phospho-N,N,N-trimethyl-butylammonium (n = 4)
C₃₄H₇₀NO₆P (619.91)
- 1142.): 1-O-(Z)-12-Tricosenyl-2-O-methyl-*sn*-glycero-3-phospho-N,N,N-trimethyl-butylammonium (n = 4)
C₃₄H₇₀NO₆P (619.91)
- 1143.): 1-O-(Z)-6-Tetracosenyl-2-O-methyl-*sn*-glycero-3-phospho-N,N,N-trimethyl-butylammonium (n = 4)
C₃₅H₇₂NO₆P (633.93)
- 1144.): 1-O-(Z)-10-Tetracosenyl-2-O-methyl-*sn*-glycero-3-phospho-N,N,N-trimethyl-butylammonium (n = 4)
C₃₅H₇₂NO₆P (633.93)
- 1145.): 1-O-(Z)-12-Tetracosenyl-2-O-methyl-*sn*-glycero-3-phospho-N,N,N-trimethyl-butylammonium (n = 4)
C₃₅H₇₂NO₆P (633.93)
- 1146.): 1-O-(Z)-10-Octadecenyl-3-O-methyl-*sn*-glycero-2-phosphocholin (n = 2)
C₂₇H₅₆NO₆P (521.72)
- 1147.): 1-O-(Z)-6-Nonadecenyl-3-O-methyl-*sn*-glycero-2-phosphocholin (n = 2)
C₂₈H₅₈NO₆P (535.75)
- 1148.): 1-O-(Z)-12-Eicosenyl-3-O-methyl-*sn*-glycero-2-phosphocholin (n = 2)
C₂₉H₆₀NO₆P (549.77)
- 1149.): 1-O-(Z)-10-Heneicosenyl-3-O-methyl-*sn*-glycero-2-phosphocholin (n = 2)
C₃₀H₆₂NO₆P (563.80)
- 1150.): 1-O-(Z)-10-Docosenyl-3-O-methyl-*sn*-glycero-2-phosphocholin (n = 2)
C₃₁H₆₄NO₆P (577.83)
- 1151.): 1-O-(Z)-12-Docosenyl-3-O-methyl-*sn*-glycero-2-phosphocholin (n = 2)
C₃₁H₆₄NO₆P (577.83)
- 1152.): 1-O-(Z)-10-Tricosenyl-3-O-methyl-*sn*-glycero-2-phosphocholin (n = 2)
C₃₂H₆₆NO₆P (591.86)
- 1153.): 1-O-(Z)-10-Tetracosenyl-3-O-methyl-*sn*-glycero-2-phosphocholin (n = 2)
C₃₃H₆₈NO₆P (605.89)
- 1154.): 1-O-(Z)-10-Octadecenyl-3-O-methyl-*sn*-glycero-2-phospho-N,N,N-trimethyl-propylammonium (n = 3)
C₂₈H₅₈NO₆P (535.75)
- 1155.): 1-O-(Z)-6-Nonadecenyl-3-O-methyl-*sn*-glycero-2-phospho-N,N,N-trimethyl-propylammonium (n = 3)
C₂₉H₆₀NO₆P (549.77)
- 1156.): 1-O-(Z)-12-Eicosenyl-3-O-methyl-*sn*-glycero-2-phospho-N,N,N-trimethyl-propylammonium (n = 3)
C₃₀H₆₂NO₆P (563.80)
- 1157.): 1-O-(Z)-10-Heneicosenyl-3-O-methyl-*sn*-glycero-2-phospho-N,N,N-trimethyl-propylammonium (n = 3)
C₃₁H₆₄NO₆P (577.83)
- 1158.): 1-O-(Z)-10-Docosenyl-3-O-methyl-*sn*-glycero-2-phospho-N,N,N-trimethyl-propylammonium (n = 3)
C₃₂H₆₆NO₆P (591.86)
- 1159.): 1-O-(Z)-12-Docosenyl-3-O-methyl-*sn*-glycero-2-phospho-N,N,N-trimethyl-propylammonium (n = 3)
C₃₂H₆₆NO₆P (591.86)
- 1160.): 1-O-(Z)-10-Tricosenyl-3-O-methyl-*sn*-glycero-2-phospho-N,N,N-trimethyl-propylammonium (n = 3)
C₃₃H₆₈NO₆P (605.89)
- 1161.): 1-O-(Z)-10-Tetracosenyl-3-O-methyl-*sn*-glycero-2-phospho-N,N,N-trimethyl-propylammonium (n = 3)
C₃₄H₇₀NO₆P (619.91)
- 1162.): 1-O-(Z)-10-Octadecenyl-2-O-tert.butyl-*sn*-glycero-3-phosphocholin (n = 2)
C₃₀H₆₂NO₆P (563.80)
- 1163.): 1-O-(Z)-6-Nonadecenyl-2-O-tert.butyl-*sn*-glycero-3-phosphocholin (n = 2)
C₃₁H₆₄NO₆P (577.82)
- 1164.): 1-O-(Z)-12-Eicosenyl-2-O-tert.butyl-*sn*-glycero-3-phosphocholin (n = 2)
C₃₂H₆₆NO₆P (591.85)
- 1165.): 1-O-(Z)-10-Heneicosenyl-2-O-tert.butyl-*sn*-glycero-3-phosphocholin (n = 2)
C₃₃H₆₈NO₆P (605.88)
- 1166.): 1-O-(Z)-10-Docosenyl-2-O-tert.butyl-*sn*-glycero-3-phosphocholin (n = 2)
C₃₄H₇₀NO₆P (619.91)
- 1167.): 1-O-(Z)-12-Docosenyl-2-O-tert.butyl-*sn*-glycero-3-phosphocholin (n = 2)
C₃₄H₇₀NO₆P (619.91)
- 1168.): 1-O-(Z)-10-Tricosenyl-2-O-tert.butyl-*sn*-glycero-3-phosphocholin (n = 2)
C₃₅H₇₂NO₆P (633.94)
- 1169.): 1-O-(Z)-10-Tetracosenyl-2-O-tert.butyl-*sn*-glycero-3-phosphocholin (n = 2)
C₃₆H₇₄NO₆P (647.97)
- 1170.): 1-O-(Z)-10-Octadecenyl-2-O-tert.butyl-*sn*-glycero-3-phospho-N,N,N-trimethyl-propylammonium (n = 3)
C₃₁H₆₄NO₆P (577.82)
- 1171.): 1-O-(Z)-6-Nonadecenyl-2-O-tert.butyl-*sn*-glycero-3-phospho-N,N,N-trimethyl-propylammonium (n = 3)
C₃₂H₆₆NO₆P (591.85)
- 1172.): 1-O-(Z)-12-Eicosenyl-2-O-tert.butyl-*sn*-glycero-3-phospho-N,N,N-trimethyl-propylammonium (n = 3)
C₃₃H₆₈NO₆P (605.88)
- 1173.): 1-O-(Z)-10-Heneicosenyl-2-O-tert.butyl-*sn*-glycero-3-phospho-N,N,N-trimethyl-propylammonium (n = 3)
C₃₄H₇₀NO₆P (619.91)
- 1174.): 1-O-(Z)-10-Docosenyl-2-O-tert.butyl-*sn*-glycero-3-phospho-N,N,N-trimethyl-propylammonium (n = 3)
C₃₅H₇₂NO₆P (633.94)
- 1175.): 1-O-(Z)-12-Docosenyl-2-O-tert.butyl-*sn*-glycero-3-phospho-N,N,N-trimethyl-propylammonium (n = 3)
C₃₅H₇₂NO₆P (633.94)
- 1176.): 1-O-(Z)-10-Tricosenyl-2-O-tert.butyl-*sn*-glycero-3-phospho-N,N,N-trimethyl-propylammonium (n = 3)
C₃₆H₇₄NO₆P (647.97)
- 1177.): 1-O-(Z)-10-Tetracosenyl-2-O-tert.butyl-*sn*-glycero-3-phospho-N,N,N-trimethyl-propylammonium (n = 3)
C₃₇H₇₆NO₆P (661.99)

### 11. Wirkstoffe, die auf alkylierten (Ether)-Lysolecithinen aufgebaut sind - zweifach ungesättigte Verbindungen

### (A = III bzw. A = IV; n = 2 - 6; R₃, CH₃; m= 1. x = 1; z = 0)

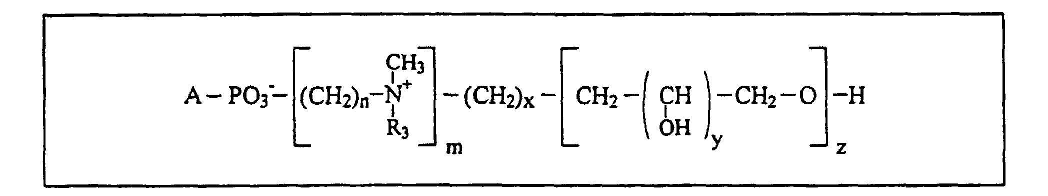

### 1-O-(Z,Z)-Alkadienyl-2-O-methyl-sn-glycero-3-phosphocholine

- 1178.): 1-O-(Z,Z)-6,12-Hexadecadienyl-2-O-methyl-sn-glycero-3-phosphocholin (n = 2)
C₂₅H₅₀NO₆P (491.65)
- 1179.): 1-O-(Z,Z)-6,12-Heptadecadienyl-2-O-methyl-sn-glycero-3-phosphocholin (n = 2)
C₂₆H₅₂NO₆P (505.68)
- 1180.): 1-O-(Z,Z)-6,12-Octadecadienyl-2-O-methyl-sn-glycero-3-phosphocholin (n = 2)
C₂₇H₅₄NO₆P (519.71)
- 1181.): 1-O-(Z,Z)-6,12-Nonadecadienyl-2-O-methyl-sn-glycero-3-phosphocholin (n = 2)
C₂₈H₅₆NO₆P (533.74)
- 1182.): 1-O-(Z,Z)-9,15-Eicosadienyl-2-O-methyl-sn-glycero-3-phosphocholin (n = 2)
C₂₉H₅₈NO₆P (547.77)
- 1183.): 1-O-(Z,Z)-9,15-Heneicosadienyl-2-O-methyl-*sn*-glycero-3-phosphocholin (n = 2)
C₃₀H₆₀NO₆P (561.8)
- 1184.): 1-O-(Z,Z)-5,17-Docosadienyl-2-O-methyl-sn-glycero-3-phosphocholin (n = 2)
C₃₁H₆₂NO₆P (575.83)
- 1185.): 1-O-(Z,Z)-6,18-Tricosadienyl-2-O-methyl-sn-glycero-3-phosphocholin (n = 2)
C₃₂H₆₄NO₆P (589.86)
- 1186.): 1-O-(Z,Z)-6,18-Tetracosadienyl-2-O-methyl-sn-glycero-3-phosphocholin (n = 2)
C₃₃H₆₆NO₆P (603.89)
- 1187.): 1-O-(Z,Z)-6,18-Pentacosadienyl-2-O-methyl-sn-glycero-3-phosphocholin (n = 2)
C₃₄H₆₈NO₆P (617.92)

### 1-O-(Z,Z)-Alkadienyl-2-O-methyl-sn-glycero-3-phospho-N,N,N-trimethyl-propylammonium -Verbindungen

- 1188.): 1-O-(Z,Z)-6,12-Hexadecadienyl-2-O-methyl-sn-glycero-3-phospho-N,N,N-trimethyl-propylammonium (n = 3)
C₂₆H₅₂NO₆P (505.68)
- 1189.): 1-O-(Z,Z)-6,12-Heptadecadienyl-2-O-methyl-sn-glycero-3-phospho-N,N,N-trimethyl-propylammonium (n = 3)
C₂₇H₅₄NO₆P (519.71)
- 1190.): 1-O-(Z,Z)-6,12-Octadecadienyl-2-O-methyl-sn-glycero-3-phospho-N,N,N-trimethyl-propylammonium (n = 3)
C₂₈H₅₆NO₆P (533.74)
- 1191.): 1-O-(Z,Z)-6,12-Nonadecadienyl-2-O-methyl-sn-glycero-3-phospho-N,N,N-trimethyl-propylammonium (n = 3)
C₂₉H₅₈NO₆P (547.77)
- 1192.): 1-O-(Z,Z)-9,15-Eicosadienyl-2-O-methyl-sn-glycero-3-phospho-N,N,N-trimethyl-propylammonium (n = 3)
C₃₀H₆₀NO₆P (561.8)
- 1193.): 1-O-(Z,Z)-9,15-Heneicosadienyl-2-O-methyl-sn-glycero-3-phospho-N,N,N-trimethyl-propylammonium (n = 3)
C₃₁H₆₂NO₆P (575.83)
- 1194.): 1-O-(Z,Z)-5,17-Docosadienyl-2-O-methyl-sn-glycero-3-phospho-N,N,N-trimethyl-propylammonium (n = 3)
C₃₂H₆₄NO₆P (589.86)
- 1195.): 1-O-(Z,Z)-6,18-Tricosadienyl-2-O-methyl-sn-glycero-3-phospho-N,N,N-trimethyl-propylammonium (n = 3)
C₃₃H₆₆NO₆P (603.89)
- 1196.): 1-O-(Z,Z)-6,18-Tetracosadienyl-2-O-methyl-sn-glycero-3-phospho-N,N,N-trimethyl-propylammonium (n = 3)
C₃₄H₆₈NO₆P (617.92)
- 1197.): 1-O-(Z,Z)-6,18-Pentacosadienyl-2-O-methyl-sn-glycero-3-phospho-N,N,N-trimethyl-propylammonium (n = 3)
C₃₅H₇₀NO₆P (631.95)

### 1-O-(Z,Z)-Alkadienyl-2-O-methyl-sn-glycero-3-phospho-N,N,N-trimethyl-butylammonium-Verbindungen

- 1198.): 1-O-(Z,Z)-6,12-Hexadecadienyl-2-O-methyl-sn-glycero-3-phospho-N,N,N-trimethyl-butylammonium (n = 4)
C₂₇H₅₄NO₆P (519.71)
- 1199.): 1-O-(Z,Z)-6,12-Heptadecadienyl-2-O-methyl-sn-glycero-3-phospho-N,N,N-trimethyl-butylammonium (n = 4)
C₂₈H₅₆NO₆P (533.74)
- 1200.): 1-O-(Z,Z)-6,12-Octadecadienyl-2-O-methyl-sn-glycero-3-phospho-N,N,N-trimethyl-butylammonium (n = 4)
C₂₉H₅₈NO₆P (547.77)
- 1201.): 1-O-(Z,Z)-6,12-Nonadecadienyl-2-O-methyl-sn-glycero-3-phospho-N,N,N-trimethyl-butylammonium (n = 4)
C₃₀H₆₀NO₆P (561.8)
- 1202.): 1-O-(Z,Z)-9,15-Eicosadienyl-2-O-methyl-sn-glycero-3-phospho-N,N,N-trimethyl-butylammonium (n = 4)
C₃₁H₆₂NO₆P (575.83)
- 1203.): 1-O-(Z,Z)-9,15-Heneicosadienyl-2-O-methyl-sn-glycero-3-phospho-N,N,N-trimethyl-butylammonium (n = 4)
C₃₂H₆₄NO₆P (589.86)
- 1204.): 1-O-(Z,Z)-5,17-Docosadienyl-2-O-methyl-sn-glycero-3-phospho-N,N,N-trimethyl-butylammonium (n = 4)
C₃₃H₆₆NO₆P (603.89)
- 1205.): 1-O-(Z,Z)-6,18-Tricosadienyl-2-O-methyl-sn-glycero-3-phospho-N,N,N-trimethyl-butylammonium (n = 4)
C₃₄H₆₈NO₆P (617.92)
- 1206.): 1-O-(Z,Z)-6,18-Tetracosadienyl-2-O-methyl-sn-glycero-3-phospho-N,N,N-trimethyl-butylammonium (n = 4)
C₃₅H₇₀NO₆P (631.95)
- 1207.): 1-O-(Z,Z)-6,18-Pentacosadienyl-2-O-methyl-sn-glycero-3-phospho-N,N,N-trimethyl-butylammonium (n = 4)
C₃₆H₇₂NO₆P (645.94)

### 1-O-(Z,Z)-Alkadienyl-3-O-methyl-sn-glycero-2-phosphocholin (n = 2)

- 1208.): 1-O-(Z,Z)-6,12-Hexadecadienyl-3-O-methyl-sn-glycero-2-phosphocholin (n = 2)
C₂₅H₅₀NO₆P (491.65)
- 1209.): 1-O-(Z,Z)-6,12-Heptadecadienyl-3-O-methyl-*sn*-glycero-2-phosphocholin (n = 2)
C₂₆H₅₂NO₆P (505.68)
- 1210.): 1-O-(Z,Z)-6,12-Octadecadienyl-3-O-methyl-sn-glycero-2-phosphocholin (n = 2)
C₂₇H₅₄NO₆P (519.71)
- 1211.): 1-O-(Z,Z)-6,12-Nonadecadienyl-3-O-methyl-sn-glycero-2-phosphocholin (n = 2)
C₂₈H₅₆NO₆P (533.74)
- 1212.): 1-O-(Z,Z)-9,15-Eicosadienyl-3-O-methyl-sn-glycero-2-phosphocholin (n = 2)
C₂₉H₅₈NO₆P (547.77)
- 1213.): 1-O-(Z,Z)-9,15-Heneicosadienyl-3-O-methyl-sn-glycero-2-phosphocholin (n = 2)
C₃₀H₆₀NO₆P (561.8)
- 1214.): 1-O-(Z,Z)-5,17-Docosadienyl-3-O-methyl-sn-glycero-2-phosphocholin (n = 2)
C₃₁H₆₂NO₆P (575.83)
- 1215.): 1-O-(Z,Z)-6,18-Tricosadienyl-3-O-methyl-sn-glycero-2-phosphocholin (n = 2)
C₃₂H₆₄NO₆P (589.86)
- 1216.): 1-O-(Z,Z)-6,18-Tetracosadienyl-3-O-methyl-sn-glycero-2-phosphocholin (n = 2)
C₂₉H₅₈NO₄P (515.76)
- 1217.): 1-O-(Z,Z)-6,18-Pentacosadienyl-3-O-methyl-sn-glycero-2-phosphocholin (n = 2)
C₃₄H₆₈NO₆P (617.92)

### 1-O-(Z,Z)-Alkadienyl-3-O-methyl-sn-glycero-2-phospho-N,N,N-trimethyl-propylammonium-Verbindungten

- 1218.): 1-O-(Z,Z)-6,12-Hexadecadienyl-3-O-methyl-sn-glycero-2-phospho-N,N,N-trimethyl-propylammonium (n = 3)
C₂₆H₅₂NO₆P (505.68)
- 1219.): 1-O-(Z,Z)-6,12-Heptadecadienyl-3-O-methyl-sn-glycero-2-phospho-N,N,N-trimethyl-propylammonium (n = 3)
C₂₇H₅₄NO₆P (519.71)
- 1220.): 1-O-(Z,Z)-6,12-Octadecadienyl-3-O-methyl-sn-glycero-2-phospho-N,N,N-trimethyl-propylammonium (n = 3)
C₂₈H₅₆NO₆P (533.74)
- 1221.): 1-O-(Z,Z)-6,12-Nonadecadienyl-3-O-methyl-sn-glycero-2-phospho-N,N,N-trimethyl-propylammonium (n = 3)
C₂₉H₅₈NO₆P (547.77)
- 1222.): 1-O-(Z,Z)-9,15-Eicosadienyl-3-O-methyl-sn-glycero-2-phospho-N,N,N-trimethyl-propylammonium (n = 3)
C₃₀H₆₀NO₆P (561.8)
- 1223.): 1-O-(Z,Z)-9,15-Heneicosadienyl-3-O-methyl-sn-glycero-2-phospho-N,N,N-trimethyl-propylammonium (n = 3)
C₃₁H₆₂NO₆P (575.83)
- 1224.): 1-O-(Z,Z)-5,17-Docosadienyl-3-O-methyl-sn-glycero-2-phospho-N,N,N-trimethyl-propylammonium (n = 3)
C₃₂H₆₄NO₆P (589.86)
- 1225.): 1-O-(Z,Z)-6,18-Tricosadienyl-3-O-methyl-sn-glycero-2-phospho-N,N,N-trimethyl-propylammonium (n = 3)
C₃₃H₆₆NO₆P (603.89)
- 1226.): 1-O-(Z,Z)-6,18-Tetracosadienyl-3-O-methyl-sn-glycero-2-phospho-N,N,N-trimethyl-propylammonium (n = 3)
C₃₄H₆₈NO₆P (617.92)
- 1227.): 1-O-(Z,Z)-6,18-Pentacosadienyl-3-O-methyl-sn-glycero-2-phospho-N,N,N-trimethyl-propylammonium (n = 3)
C₃₅H₇₀NO₆P (631.95)

### 1-O-(Z,Z)-Alkadienyl-2-O-tert.butyl-sn-glycero-3-phosphocholin (n = 2)

- 1228.): 1-O-(Z,Z)-6,12-Hexadecadienyl-2-O-tert.butyl-sn-glycero-3-phosphocholin (n = 2)
C₂₈H₅₆NO₆P (533.73)
- 1229.): 1-O-(Z,Z)-6,12-Heptadecadienyl-2-O-tert.butyl-sn-glycero-3-phosphocholin (n = 2)
C₂₉H₅₈NO₆P (547.76)
- 1230.): 1-O-(Z,Z)-6,12-Octadecadienyl-2-O-tert.butyl-sn-glycero-3-phosphocholin (n = 2)
C₃₀H₆₀NO₆P (561.78)
- 1231.): 1-O-(Z,Z)-6,12-Nonadecadienyl-2-O-tert.butyl-sn-glycero-3-phosphocholin (n = 2)
C₃₁H₆₂NO₆P (575.81)
- 1232.): 1-O-(Z,Z)-9,15-Eicosadienyl-2-O-tert.butyl-*sn*-glycero-3-phosphocholin (n = 2)
C₃₂H₆₄NO₆P (589.84)
- 1233.): 1-O-(Z,Z)-9,15-Heneicosadienyl-2-O-tert.butyl-sn-glycero-3-phosphocholin (n = 2)
C₃₃H₆₆NO₆P (603.87)
- 1234.): 1-O-(Z,Z)-5,17-Docosadienyl-2-O-tert.butyl-sn-glycero-3-phosphocholin (n = 2)
C₃₄H₆₈NO₆P (617.9)
- 1235.): 1-O-(Z,Z)-6,18-Tricosadienyl-2-O-tert.butyl-sn-glycero-3-phosphocholin (n = 2)
C₃₅H₇₀NO₆P (631.93)
- 1236.): 1-O-(Z,Z)-6,18-Tetracosadienyl-2-O-tert.butyl-sn-glycero-3-phosphocholin (n = 2)
C₃₆H₇₂NO₆P (645.96)
- 1237.): 1-O-(Z,Z)-6,18-Pentacosadienyl-2-O-tert.butyl-sn-glycero-3-phosphocholin (n = 2)
C₃₇H₇₄NO₆P (660.03)

### 1-O-(Z,Z)-Alkadienyl-2-O-tert.butyl-sn-glycero-3-phospho-N,N,N-trimethyl-propylammonium -Verbindungen

- 1238.): 1-O-(Z,Z)-6,12-Hexadecadienyl-2-O-tert.butyl-sn-glycero-3-phospho-N,N,N-trimethyl-propylammonium (n = 3)
C₂₉H₅₈NO₆P (547.76)
- 1239.): 1-O-(Z,Z)-6,12-Heptadecadienyl-2-O-tert.butyl-*sn*-glycero-3-phospho-N,N,N-trimethyl-propylammonium (n = 3) C₃₀H₆₀NO₆P (561.78)
- 1240.): 1-O-(Z,Z)-6,12-Octadecadienyl-2-O-tert.butyl-sn-glycero-3-phospho-N,N,N-trimethyl-propylammonium (n = 3)
C₃₁H₆₂NO₆P (575.81)
- 1241.): 1-O-(Z,Z)-6,12-Nonadecadienyl-2-O-tert.butyl-sn-glycero-3-phospho-N,N,N-trimethyl-propylammonium (n = 3)
C₃₂H₆₄NO₆P (589.84)
- 1242.): 1-O-(Z,Z)-9,15-Eicosadienyl-2-O-tert.butyl-sn-glycero-3-phospho-N,N,N-trimethyl-propylammonium (n = 3)
C₃₃H₆₆NO₆P (603.87)
- 1243.): 1-O-(Z,Z)-9,15-Heneicosadienyl-2-O-tert.butyl-sn-glycero-3-phospho-N,N,N-trimethyl-propylammonium (n = 3)
C₃₄H₆₈NO₆P (617.9)
- 1244.): 1-O-(Z,Z)-5,17-Docosadienyl-2-O-tert.butyl-sn-glycero-3-phospho-N,N,N-trimethyl-propylammonium (n = 3)
C₃₅H₇₀NO₆P (631.93)
- 1245.): 1-O-(Z,Z)-6,18-Tricosadienyl-2-O-tert.butyl-sn-glycero-3-phospho-N,N,N-trimethyl-propylammonium (n = 3)
C₃₆H₇₂NO₆P (645.96)
- 1246.): 1-O-(Z,Z)-6,18-Tetracosadienyl-2-O-tert.butyl-sn-glycero-3-phospho-N,N,N-trimethyl-propylammonium (n = 3)
C₃₇H₇₄NO₆P (660.03)
- 1247.): 1-O-(Z,Z)-6,18-Pentacosadienyl-2-O-tert.butyl-sn-glycero-3-phospho-N,N,N-trimethyl-propylammonium (n = 3)
C₃₈H₇₆NO₆P (674.03)

### 12. Wirkstoffe, die auf Alkandiolphospho-Verbindungen aufgebaut sind - - einfach ungesättigte Verbindungen

### (A = VI bzw. VII; n = 2 - 6; R₃, CH₃; m= 1, x = 1; z = 0)

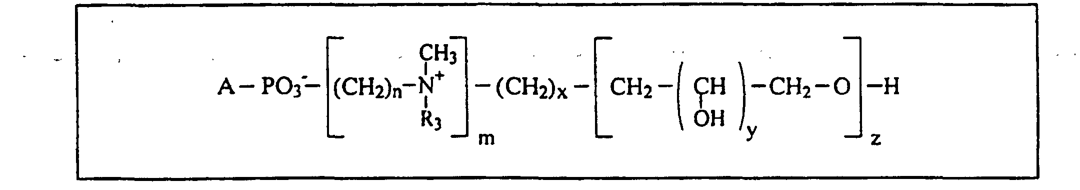

### 1-O-(Z)-Alkenyl-propandiol-(1,2)-phosphocholine

- 1248.): 1-O-(Z)-10-Octadecenyl-propandiol-(1,2)-phosphocholin
C₂₆H₅₄NO₅P (491.68)
- 1249.): 1-O-(Z)-6-Nonadecenyl-propandiol-(1,2)-phosphocholin
C₂₇H₅₆NO₅P (505.71)
- 1250.): 1-O-(Z)-12-Eicosenyl-propandiol-(1,2)-phosphocholin
C₂₈H₅₈NO₅P (519.74)
- 1251.): 1-O-(Z)-10-Heneicosenyl-propandiol-(1,2)-phosphocholin
C₂₉H₆₀NO₅P (533.77)
- 1252.): 1-O-(Z)-10-Docosenyl-propandiol-(1,2)-phosphocholin
C₃₀H₆₂NO₅P (547.80)
- 1253.): 1-O-(Z)-12-Docosenyl-propandiol-(1,2)-phosphocholin
C₃₀H₆₂NO₅P (547.80)
- 1254.): 1-O-(Z)-10-Tricosenyl-propandiol-(1,2)-phosphocholin
C₃₁H₆₄NO₅P (561.83)
- 1255.): 1-O-(Z)-10-Tetracosenyl-propandiol-(1,2)-phosphocholin
C₃₂H₆₆NO₅P (575.86)

### 1-O-(Z)-Alkenyl-propandiol-(1,2)-phospho-N,N,N-trimethylpropyl-ammonium-Verbindungen

- 1256.): 1-O-(Z)-10-Octadecenyl-propandiol-(1,2)-phospho-N,N,N-trimethylpropyl-ammonium
C₂₇H₅₆NO₅P (505.71)
- 1257.): 1-O-(Z)-6-Nonadecenyl-propandiol-(1,2)-phospho-N,N,N-trimethylpropyl-ammonium
C₂₈H₅₈NO₅P (519.74)
- 1258.): 1-O-(Z)-12-Eicosenyl-propandiol-(1,2)-phospho-N,N,N-trimethylpropyl-ammonium
C₂₉H₆₀NO₅P (533.77)
- 1259.): 1-O-(Z)-10-Heneicosenyl-propandiol-(1,2)-phospho-N,N,N-trimethylpropyl-ammonium
C₃₀H₆₂NO₅P (547.80)
- 1260.): 1-O-(Z)-10-Docosenyl-propandiol-(1,2)-phospho-N,N,N-trimethylpropyl-ammonium
C₃₁H₆₄NO₅P (561.83)
- 1261.): 1-O-(Z)-12-Docosenyl-propandiol-(1,2)-phospho-N,N,N-trimethylpropyl-ammonium
C₃₁H₆₄NO₅P (561.83)
- 1262.): 1-O-(Z)-10-Tricosenyl-propandiol-(1,2)-phospho-N,N,N-trimethylpropyl-ammonium
C₃₂H₆₆NO₅P (575.86)
- 1263.): 1-O-(Z)-10-Tetracosenyl-propandiol-(1,2)-phospho-N,N,N-trimethylpropyl-ammonium
C₃₃H₆₈NO₅P (589.89)

### 2-O-(Z)-Alkenyl-propandiol-(1,2)-phosphocholine

- 1264.): 2-O-(Z)-10-Octadecenyl-propandiol-(1,2)-phosphocholin
C₂₆H₅₄NO₅P (491.68)
- 1265.): 2-O-(Z)-6-Nonadecenyl-propandiol-(1,2)-phosphocholin
C₂₇H₅₆NO₅P (505.71)
- 1266.): 2-O-(Z)-12-Eicosenyl-propandiol-(1,2)-phosphocholin
C₂₈H₅₈NO₅P (519.74)
- 1267.): 2-O-(Z)-10-Heneicosenyl-propandiol-(1,2)-phosphocholin
C₂₉H₆₀NO₅P (533.77)
- 1268.): 2-O-(Z)-10-Docosenyl-propandiol-(1,2)-phosphocholin
C₃₀H₆₂NO₅P (547.80)
- 1269.): 2-O-(Z)-12-Docosenyl-propandiol-(1,2)-phosphocholin
C₃₀H₆₂NO₅P (547.80)
- 1270.): 2-O-(Z)-10-Tricosenyl-propandiol-(1,2)-phosphocholin
C₃₁H₆₄NO₅P (561.83)
- 1271.): 2-O-(Z)-10-Tetracosenyl-propandiol-(1,2)-phosphocholin
C₃₂H₆₆NO₅P (575.86)

### 2-O-(Z)-Alkenyl-propandiol-(1,2)-phospho-N,N,N-trimethylpropyl-ammonium-Verbindunaen

- 1272.): 2-O-(Z)-10-Octadecenyl-propandiol-(1,2)-phospho-N,N,N-trimethylpropyl-ammonium
C₂₇H₅₆NO₅P (505.71)
- 1273.): 2-O-(Z)-6-Nonadecenyl-propandiol-(1,2)-phospho-N,N,N-trimethylpropyl-ammonium
C₂₈H₅₈NO₅P (519.74)
- 1274.): 2-O-(Z)-12-Eicosenyl-propandiol-(1,2)-phospho-N,N,N-trimethylpropyl-ammonium
C₂₉H₆₀NO₅P (533.77)
- 1275.): 2-O-(Z)-10-Heneicosenyl-propandiol-(1,2)-phospho-N,N,N-trimethylpropyl-ammonium
C₃₀H₆₂NO₅P (547.80)
- 1276.): 2-O-(Z)-10-Docosenyl-propandiol-(1,2)-phospho-N,N,N-trimethylpropyl-ammonium
C₃₁H₆₄NO₅P (561.83)
- 1277.): 2-O-(Z)-12-Docosenyl-propandiol-(1,2)-phospho-N,N,N-trimethylpropyl-ammonium
C₃₁H₆₄NO₅P (561.83)
- 1278.): 2-O-(Z)-10-Tricosenyl-propandiol-(1,2)-phospho-N,N,N-trimethylpropyl-ammonium
C₃₂H₆₆NO₅P (575.86)
- 1279.): 2-O-(Z)-10-Tetracosenyl-propandiol-(1,2)-phospho-N,N,N-trimethylpropyl-ammonium
C₃₃H₆₈NO₅P (589.89)

### 13. Wirkstoffe, die auf Alkandiolphospho-Verbindungen aufgebaut sind - - zweifach ungesättigte Verbindungen

### (A = VI bzw. VII; n = 2 - 6; R₃, CH₃; m= 1, x = 1; z = 0)

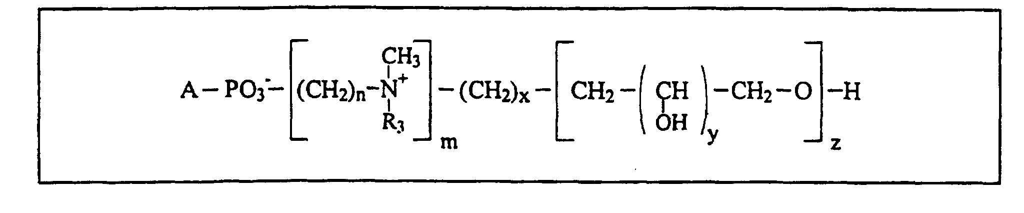

### 1-O-(Z,Z)-Alkadienyl-propandiol-(1,2)-phosphocholine

- 1280.): 1-O-(Z,Z)-6,12-Hexadecadienyl-propandiol-(1,2)-phosphocholin
C₂₄H₄₈NO₅P (461.62)
- 1281.): 1-O-(Z,Z)-6,12-Heptadecadienyl-propandiol-(1,2)-phosphocholin
C₂₅H₅₀NO₅P (475.65)
- 1282.): 1-O-(Z,Z)-6,12-Octadecadienyl-propandiol-(1,2)-phosphocholin
C₂₆H₅₂NO₅P (489.68)
- 1283.): 1-O-(Z,Z)-6,12-Nonadecadienyl-propandiol-(1,2)-phosphocholin
C₂₇H₅₄NO₅P (503.71)
- 1284.): 1-O-(Z,Z)-9,15-Eicosadienyl-propandiol-(1,2)-phosphocholin
C₂₈H₅₆NO₅P (517.74)
- 1285.): 1-O-(Z,Z)-9,15-Heneicosadienyl-propandiol-(1,2)-phosphocholin
C₂₉H₅₈NO₅P (531.77)
- 1286.): 1-O-(Z,Z)-5,17-Docosadienyl-propandiol-(1,2)-phosphocholin
C₃₀H₆₀NO₅P (545.8)
- 1287.): 1-O-(Z,Z)-6,18-Tricosadienyl-propandiol-(1,2)-phosphocholin
C₃₁H₆₂NO₅P (559.83)
- 1288.): 1-O-(Z,Z)-6,18-Tetracosadienyl-propandiol-(1,2)-phosphocholin
C₃₂H₆₄NO₅P (573.86)
- 1289.): 1-O-(Z,Z)-6,18-Pentacosadienyl-propandiol-(1,2)-phosphocholin
C₃₃H₆₆NO₅P (587.89)

### 1-O-(Z,Z)-Alkadienyl-propandiol-(1,2)-phospho-N,N,N-trimethylpropylammonium-Verbindungen

- 1290.): 1-O-(Z,Z)-6,12-Hexadecadienyl-propandiol-(1,2)-phospho-N,N,N-trimethylpropylammonium
C₂₅H₅₀NO₅P (475.65)
- 1291.): 1-O-(Z,Z)-6,12-Heptadecadienyl-propandiol-(1,2)-phospho-N,N,N-trimethylpropylammonium
C₂₆H₅₂NO₅P (489.68)
- 1292.): 1-O-(Z,Z)-6,12-Octadecadienyl-propandiol-(1,2)-phospho-N,N,N-trimethylpropylammonium
C₂₇H₅₄NO₅P (503.71)
- 1293.): 1-O-(Z,Z)-6,12-Nonadecadienyl-propandiol-(1,2)-phospho-N,N,N-trimethylpropylammonium
C₂₈H₅₆NO₅P (517.74)
- 1294.): 1-O-(Z,Z)-9,15-Eicosadienyl-propandiol-(1,2)-phospho-N,N,N-trimethylpropylammonium
C₂₉H₅₈NO₅P (531.77)
- 1295.): 1-O-(Z,Z)-9,15-Heneicosadienyl-propandiol-(1,2)-phospho-N,N,N-trimethylpropylammonium
C₃₀H₆₀NO₅P (545.8)
- 1296.): 1-O-(Z,Z)-5,17-Docosadienyl-propandiol-(1,2)-phospho-N,N,N-trimethylpropylammonium
C₃₁H₆₂NO₅P (559.83)
- 1297.): 1-O-(Z,Z)-6,18-Tricosadienyl-propandiol-(1,2)-phospho-N,N,N-trimethylpropylammonium
C₃₂H₆₄NO₅P (573.86)
- 1298.): 1-O-(Z,Z)-6,18-Tetracosadienyl-propandiol-(1,2)-phospho-N,N,N-trimethylpropylammonium
C₃₃H₆₆NO₅P (587.89)
- 1299.): 1-O-(Z,Z)-6,18-Pentacosadienyl-propandiol-(1,2)-phospho-N,N,N-trimethylpropylammonium
C₃₄H₆₈NO₅P (601.92)

### 2-O-(Z,Z)-Alkadienyl-propandiol-(1,2)-phosphocholine

- 1300.): 2-O-(Z,Z)-6,12-Hexadecadienyl-propandiol-(1,2)-phosphocholin
C₂₄H₄₈NO₅P (461.62)
- 1301.): 2-O-(Z,Z)-6,12-Heptadecadienyl-propandiol-(1,2)-phosphocholin
C₂₅H₅₀NO₅P (475.65)
- 1302.): 2-O-(Z,Z)-6,12-Octadecadienyl-propandiol-(1,2)-phosphocholin
C₂₆H₅₂NO₅P (489.68)
- 1303.): 2-O-(Z,Z)-6,12-Nonadecadienyl-propandiol-(1,2)-phosphocholin
C₂₇H₅₄NO₅P (503.71)
- 1304.): 2-O-(Z,Z)-9,15-Eicosadienyl-propandiol-(1,2)-phosphocholin
C₂₈H₅₆NO₅P (517.74)
- 1305.): 2-O-(Z,Z)-9,15-Heneicosadienyl-propandiol-(1,2)-phosphocholin
C₂₉H₅₈NO₅P (531.77)
- 1306.): 2-O-(Z,Z)-5,17-Docosadienyl-propandiol-(1,2)-phosphocholin
C₃₀H₆₀NO₅P (545.8)
- 1307.): 2-O-(Z,Z)-6,18-Tricosadienyl-propandiol-(1,2)-phosphocholin
C₃₁H₆₂NO₅P (559.83)
- 1308.): 2-O-(Z,Z)-6,18-Tetracosadienyl-propandiol-(1,2)-phosphocholin
C₃₂H₆₄NO₅P (573.86)
- 1309.): 2-O-(Z,Z)-6,18-Pentacosadienyl-propandiol-(1,2)-phosphocholin
C₃₃H₆₆NO₅P (587.89)

### 2-O-(Z,Z)-Alkadienyl-propandiol-(1,2)-phospho-N,N,N-trimethylpropylammonium-Verbindungen

- 1310.): 2-O-(Z,Z)-6,12-Hexadecadienyl-propandiol-(1,2)-phospho-N,N,N-trimethylpropylammonium
C₂₅H₅₀NO₅P (475.65)
- 1311.): 2-O-(Z,Z)-6,12-Heptadecadienyl-propandiol-(1,2)-phospho-N,N,N-trimethylpropylammonium
C₂₆H₅₂NO₅P (489.68)
- 1312.): 2-O-(Z,Z)-6,12-Octadecadienyl-propandiol-(1,2)-phospho-N,N,N-trimethylpropylammonium
C₂₇H₅₄NO₅P (503.71)
- 1313.): 2-O-(Z,Z)-6,12-Nonadecadienyl-propandiol-(1,2)-phospho-N,N,N-trimethylpropylammonium
C₂₈H₅₆NO₅P (517.74)
- 1314.): 2-O-(Z,Z)-9,15-Eicosadienyl-propandiol-(1,2)-phospho-N,N,N-trimethylpropylammonium
C₂₉H₅₈NO₅P (531.77)
- 1315.): 2-O-(Z,Z)-9,15-Heneicosadienyl-propandiol-(1,2)-phospho-N,N,N-trimethylpropylammonium
C₃₀H₆₀NO₅P (545.8)
- 1316.): 2-O-(Z,Z)-5,17-Docosadienyl-propandiol-(1,2)-phospho-N,N,N-trimethylpropylammonium
C₃₁H₆₂NO₅P (559.83)
- 1317.): 2-O-(Z,Z)-6,18-Tricosadienyl-propandiol-(1,2)-phospho-N,N,N-trimethylpropylammonium
C₃₂H₆₄NO₅P (573.86)
- 1318.): 2-O-(Z,Z)-6,18-Tetracosadienyl-propandiol-(1,2)-phospho-N,N,N-trimethylpropylammonium
C₃₃H₆₆NO₅P (587.89)
- 1319.): 2-O-(Z,Z)-6,18-Pentacosadienyl-propandiol-(1,2)-phospho-N,N,N-trimethylpropylammonium
C₃₄H₆₈NO₅P (601.92)

### Lösungsvermittler

### 1. Beispiele für einkettige Glycero-phospho-N,N-dimethyl-N-dihydroxypropylalkylammonium-Verbindungen

### (A = III bzw. IV; n = 2 - 6; R₃, CH₃; m= 1, x = 0; y = 1; z = 1)

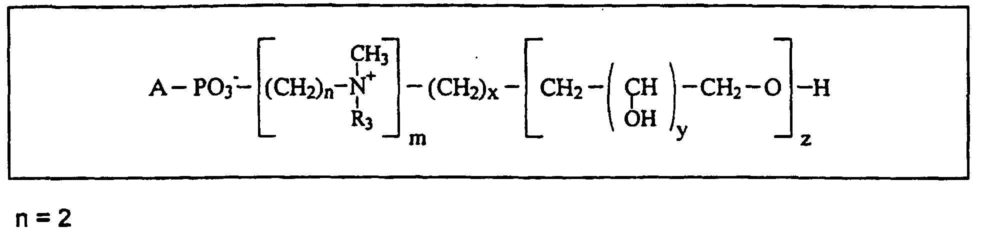

- 1320.): 1-(Z)-6-Hexadecenoyl-sn-glycero-3-phospho-N,N-dimethyl-N-dihydroxypropyl-ethylammonium (n = 2)
C₂₆H₅₂NO₉P (553.67)
- 1321.): 1-(Z)-10-Heptadecenoyl-*sn*-glycero-3-phospho-N,N-dimethyl-N-dihydroxypropyl-ethylammonium (n = 2)
C₂₇H₅₄NO₉P (567.70)
- 1322.): 1-(Z)-6-Octadecenoyl-*sn*-glycero-3-phospho-N,N-dimethyl-N-dihydroxypropyl-ethylammonium (n = 2)
C₂₈H₅₆NO₉P (581.73)
- 1323.): 1-(Z)-6-Nonadecenoyl-*sn*-glycero-3-phospho-N,N-dimethyl-N-dihydroxypropyl-ethylammonium (n = 2)
C₂₉H₅₈NO₉P (595.75)
- 1324.): 1-(Z)-12-Eicosenoyl-*sn*-glycero-3-phospho-N,N-dimethyl-N-dihydroxypropyl-ethylammonium (n = 2)
C₃₀H₆₀NO₉P (609.78)
- 1325.): 1-(Z)-10-Heneicosenoyl-sn-glycero-3-phospho-N,N-dimethyl-N-dihydroxypropyl-ethylammonium (n = 2)
C₃₁H₆₂NO₉P (623.81)
- 1326.): 1-(Z)-10-Docosenoyl-sn-glycero-3-phospho-N,N-dimethyl-N-dihydroxypropyl-ethylammonium (n = 2)
C₃₂H₆₄NO₉P (637.84)
- 1327.): 1-(Z)-12-Docosenoyl-*sn*-glycero-3-phospho-N,N-dimethyl-N-dihydroxypropyl-ethylammonium (n = 2)
C₃₂H₆₄NO₉P (637.84)
- 1328.): 1-(Z)-10-Tricosenoyl-*sn*-glycero-3-phospho-N,N-dimethyl-N-dihydroxypropyl-ethylammonium (n = 2)
C₃₃H₆₆NO₉P (651.86)
- 1329.): 1-(Z)-10-Tetracosenoyl-*sn*-glycero-3-phospho-N,N-dimethyl-N-dihydroxypropyl-ethylammonium (n = 2)
C₃₄H₆₈NO₉P (665.89)
- 1330.): 1-(Z)-15-Pentacosenoyl-sn-glycero-3-phospho-N,N-dimethyl-N-dihydroxypropyl-ethylammonium (n = 2)
C₃₅H₇₀NO₉P (679.92)
- 1331.): 1-(Z)-16-Hexacosenoyl-*sn*-glycero-3-phospho-N,N-dimethyl-N-dihydroxypropyl-ethylammonium (n = 2)
C₃₆H₇₂NO₉P (693.94)
- 1332.): 1-(Z,Z)-5,11-Hexadecadienoyl-*sn*-glycero-3-phospho-N,N-dimethyl-N-dihydroxypropyl-ethylammonium (n = 2)
C₂₆H₅₀NO₉P (551.66)
- 1333.): 1-(Z,Z)-5,11-Heptadecadienoyl-*sn*-glycero-3-phospho-N,N-dimethyl-N-dihydroxypropyl-ethylammonium (n = 2)
C₂₇H₅₂NO₉P (565.68)
- 1334.): 1-(Z,Z)-5,11-Octadecadienoyl-*sn*-glycero-3-phospho-N,N-dimethyl-N-dihydroxypropyl-ethylammonium (n = 2)
C₂₈H₅₄NO₉P (579.71)
- 1335.): 1-(Z,Z)-6,12-Nonadecadienoyl-*sn*-glycero-3-phospho-N,N-dimethyl-N-dihydroxypropyl-ethylammonium (n = 2)
C₂₉H₅₆NO₉P (593.74)
- 1336.): 1-(Z,Z)-10,16-Eicosadienoyl-*sn*-glycero-3-phospho-N,N-dimethyl-N-dihydroxypropyl-ethylammonium (n = 2)
C₃₀H₅₈NO₉P (607.77)
- 1337.): 1-(Z,Z)-10,16-Heneicosadienoyl-*sn*-glycero-3-phospho-N,N-dimethyl-N-dihydroxypropyl-ethylammonium (n = 2)
C₃₁H₆₀NO₉P (621.79)
- 1338.): 1-(Z,Z)-10,16-Docosadienoyl-*sn*-glycero-3-phospho-N,N-dimethyl-N-dihydroxypropyl-ethylammonium (n = 2)
C₃₂H₆₂NO₉P (635.82)
- 1339.): 1-(Z,Z)-10,16-Tricosadienoyl-*sn*-glycero-3-phospho-N,N-dimethyl-N-dihydroxypropyl-ethylammonium (n = 2)
C₃₃H₆₄NO₉P (649.85)
- 1340.): 1-(Z,Z)-6,18-Tetracosadienoyl-*sn*-glycero-3-phospho-N,N-dimethyl-N-dihydroxypropyl-ethylammonium (n = 2)
C₃₄H₆₆NO₉P (663.87)
- 1341.): 1-(Z,Z)-10,16-Pentacosadienoyl-*sn*-glycero-3-phospho-N,N-dimethyl-N-dihydroxypropyl-ethylammonium (n = 2)
C₃₅H₆₈NO₉P (677.90)
- 1342.): 1-(Z,Z)-6,18-Hexacosadienoyl-sn-glycero-3-phospho-N,N-dimethyl-N-dihydroxypropyl-ethylammonium (n = 2)
C₃₆H₇₀NO₉P (691.93)
Alkenyl
- 1343.): 1-O-(Z)-6-Hexadecenyl-*sn*-glycero-3-phospho-N,N-dimethyl-N-dihydroxypropyl-ethylammonium (n = 2)
C₂₆H₅₄NO₈P (539.69)
- 1344.): 1-O-(Z)-6-Octadecenyl-*sn*-glycero-3-phospho-N,N-dimethyl-N-dihydroxypropyl-ethylammonium (n = 2)
C₂₈H₅₈NO₈P (567.74)
- 1345.): 1-O-(Z)-12-Eicosenyl-*sn*-glycero-3-phospho-N,N-dimethyl-N-dihydroxypropyl-ethylammonium (n = 2)
C₃₀H₆₂NO₈P (595.80)
- 1346.): 1-O-(Z)-10-Docosenyl-*sn*-glycero-3-phospho-N,N-dimethyl-N-dihydroxypropyl-ethylammonium (n = 2)
C₃₂H₆₆NO₈P (623.85)
- 1347.): 1-O-(Z)-10-Tetracosenyl-*sn*-glycero-3-phospho-N,N-dimethyl-N-dihydroxypropyl-ethylammonium (n = 2)
C₃₄H₇₀NO₈P (651.91)
- 1348.): 1-O-(Z)-16-Hexacosenyl-*sn*-glycero-3-phospho-N,N-dimethyl-N-dihydroxypropyl-ethylammonium (n = 2)
C₃₆H₇₄NO₈P (679.96)
- 1349.): 1-O-(Z,Z)-5,11-Hexadecadienyl-*sn*-glycero-3-phospho-N,N-dimethyl-N-dihydroxypropyl-ethylammonium (n = 2)
C₂₆H₅₂NO₈P (537.67)
- 1350.): 1-O-(Z,Z)-5,11-Octadecadienyl-*sn*-glycero-3-phospho-N,N-dimethyl-N-dihydroxypropyl-ethylammonium (n = 2)
C₂₈H₅₆NO₈P (565.73)
- 1351.): 1-O-(Z,Z)-10,16-Eicosadienyl-*sn*-glycero-3-phospho-N,N-dimethyl-N-dihydroxypropyl-ethylammonium (n = 2)
C₃₀H₆₀NO₈P (593.78)
- 1352.): 1-O-(Z,Z)-10,16-Docosadienyl-*sn*-glycero-3-phospho-N,N-dimethyl-N-dihydroxypropyl-ethylammonium (n = 2)
C₃₂H₆₄NO₈P (621.84)
- 1353.): 1-O-(Z,Z)-6,18-Tetracosadienyl-*sn*-glycero-3-phospho-N,N-dimethyl-N-dihydroxypropyl-ethylammonium (n = 2)
C₃₄H₆₈NO₈P (649.89)
- 1354.): 1-O-(Z,Z)-6,18-Hexacosadienyl-*sn*-glycero-3-phospho-N,N-dimethyl-N-dihydroxypropyl-ethylammonium (n = 2)
C₃₆H₇₂NO₈P (677.94)
n=3
- 1355.): 1-(Z)-6-Hexadecenoyl-*sn*-glycero-3-phospho-N,N-dimethyl-N-dihydroxypropyl-propylammonium (n = 3)
C₂₇H₅₄NO₉P (567.70)
- 1356.): 1-(Z)-10-Heptadecenoyl-*sn*-glycero-3-phospho-N,N-dimethyl-N-dihydroxypropyl-propylammonium (n = 3)
C₂₈H₅₆NO₉P (581.73)
- 1357.): 1-(Z)-6-Octadecenoyl-*sn*-glycero-3-phospho-N,N-dimethyl-N-dihydroxypropyl-propylammonium (n = 3)
C₂₉H₅₈NO₉P (595.75)
- 1358.): 1-(Z)-12-Eicosenoyl-*sn*-glycero-3-phospho-N,N-dimethyl-N-dihydroxypropyl-propylammonium (n = 3)
C₃₁H₆₂NO₉P (623.81)
- 1359.): 1-(Z)-10-Docosenoyl-*sn*-glycero-3-phospho-N,N-dimethyl-N-dihydroxypropyl-propylammonium (n = 3)
C₃₃H₆₆NO₉P (651.86)
- 1360.): 1-(Z)-12-Docosenoyl-*sn*-glycero-3-phospho-N,N-dimethyl-N-dihydroxypropyl-propylammonium (n = 3)
C₃₃H₆₆NO₉P (651.86)
- 1361.): 1-(Z)-10-Tricosenoyl-*sn*-glycero-3-phospho-N,N-dimethyl-N-dihydroxypropyl-propylammonium (n = 3)
C₃₄H₆₈NO₉P (665.89)
- 1362.): 1-(Z)-10-Tetracosenoyl-*sn*-glycero-3-phospho-N,N-dimethyl-N-dihydroxypropyl-propylammonium (n = 3)
C₃₅H₇₀NO₉P (679.92)
- 1363.): 1-(Z,Z)-5,11-Hexadecadienoyl-*sn*-glycero-3-phospho-N,N-dimethyl-N-dihydroxypropyl-propylammonium (n = 3)
C₂₇H₅₂NO₉P (565.68)
- 1364.): 1-(Z,Z)-5,11-Octadecadienoyl-*sn*-glycero-3-phospho-N,N-dimethyl-N-dihydroxypropyl-propylammonium (n = 3)
C₂₉H₅₆NO₉P (593.74)
- 1365.): 1-(Z,Z)-10,16-Eicosadienoyl-*sn*-glycero-3-phospho-N,N-dimethyl-N-dihydroxypropyl-propylammonium (n = 3)
C₃₁H₆₀NO₉P (621.79)
- 1366.): 1-(Z,Z)-10,16-Heneicosadienoyl-*sn*-glycero-3-phospho-N,N-dimethyl-N-dihydroxypropyl-propylammonium (n = 3)
C₃₂H₆₂NO₉P (635.82)
- 1367.): 1-(Z,Z)-10,16-Docosadienoyl-*sn*-glycero-3-phospho-N,N-dimethyl-N-dihydroxypropyl-propylammonium (n = 3)
C₃₃H₆₄NO₉P (649.85)
- 1368.): 1-(Z,Z)-6,18-Tetracosadienoyl-*sn*-glycero-3-phospho-N,N-dimethyl-N-dihydroxypropyl-propylammonium (n = 3)
C₃₅H₆₈NO₉P (677.90)
- 1369.): 1-(Z,Z)-6,18-Hexacosadienoyl-*sn*-glycero-3-phospho-N,N-dimethyl-N-dihydroxypropyl-propylammonium (n = 3)
C₃₇H₇₂NO₉P (705.95)
- 1370.): 1-O-(Z)-6-Hexadecenyl-*sn*-glycero-3-phospho-N,N-dimethyl-N-dihydroxypropyl-propylammonium (n = 3)
C₂₇H₅₆NO₈P (553.72)
- 1371.): 1-O-(Z)-6-Octadecenyl-*sn*-glycero-3-phospho-N,N-dimethyl-N-dihydroxypropyl-propylammonium (n = 3)
C₂₉H₆₀NO₈P (581.77)
- 1372.): 1-O-(Z)-12-Eicosenyl-*sn*-glycero-3-phospho-N,N-dimethyl-N-dihydroxypropyl-propylammonium (n = 3)
C₃₁H₆₄NO₈P (609.83)
- 1373.): 1-O-(Z)-10-Docosenyl-*sn*-glycero-3-phospho-N,N-dimethyl-N-dihydroxypropyl-propylammonium (n = 3)
C₃₃H₆₈NO₈P (637.88)
- 1374.): 1-O-(Z)-10-Tetracosenyl-*sn*-glycero-3-phospho-N,N-dimethyl-N-dihydroxypropyl-propylammonium (n = 3)
C₃₅H₇₂NO₈P (665.94)
- 1375.): 1-O-(Z,Z)-5,11-Hexadecadienyl-*sn*-glycero-3-phospho-N,N-dimethyl-N-dihydroxypropyl-propylammonium (n = 3)
C₂₇H₅₄NO₈P (551.7)
- 1376.): 1-O-(Z,Z)-5,11-Octadecadienyl-*sn*-glycero-3-phospho-N,N-dimethyl-N-dihydroxypropyl-propylammonium (n = 3)
C₂₉H₅₈NO₈P (579.76)
- 1377.): 1-O-(Z,Z)-10,16-Eicosadienyl-*sn*-glycero-3-phospho-N,N-dimethyl-N-dihydroxypropyl-propylammonium (n = 3)
C₃₁H₆₂NO₈P (607.81)
- 1378.): 1-O-(Z,Z)-10,16-Docosadienyl-*sn*-glycero-3-phospho-N,N-dimethyl-N-dihydroxypropyl-propylammonium (n = 3)
C₃₃H₆₆NO₈P (635.87)
- 1379.): 1-O-(Z,Z)-6,18-Tetracosadienyl-*sn*-glycero-3-phospho-N,N-dimethyl-N-dihydroxypropyl-propylammonium (n = 3)
C₃₅H₇₀NO₈P (663.92)
- 1380.): 1-O-(Z,Z)-6,18-Hexacosadienyl-*sn*-glycero-3-phospho-N,N-dimethyl-N-dihydroxypropyl-Propylammonium (n = 3)
C₃₇H₇₄NO₆P (691.97)
n=4
- 1381.): 1-(Z)-6-Octadecenoyl-*sn*-glycero-3-phospho-N,N-dimethyl-N-dihydroxypropyl-butylammonium (n = 4)
C₃₀H₆₀NO₉P (609.78)
- 1382.): 1-(Z)-10-Docosenoyl-*sn*-glycero-3-phospho-N,'N-dimethyl-N-dihydroxypropyl-butylammonium (n = 4)
C₃₄H₆₈NO₉P (665.89)
- 1383.): 1-(Z,Z)-5,11-Hexadecadienoyl-*sn*-glycero-3-phospho-N,N-dimethyl-N-dihydroxypropyl-butylammonium (n = 4)
C₂₈H₅₄NO₉P (579.71)
- 1384.): 1-(Z,Z)-10,16-Docosadienoyl-*sn*-glycero-3-phospho-N,N-dimethyl-N-dihydroxypropyl-butylammonium (n = 4)
C₃₄H₆₆NO₉P (663.88)
- 1385.): 1-(Z,Z)-6,18-Hexacosadienoyl-*sn*-glycero-3-phospho-N,N-dimethyl-N-dihydroxypropyl-butylammonium (n = 4)
C₃₈H₇₄NO₉P (719.98)
- 1386.): 1-O-(Z)-6-Octadecenyl-*sn*-glycero-3-phospho-N,N-dimethyl-N-dihydroxypropyl-butylammonium (n = 4)
C₃₀H₆₂NO₈P (595.80)
- 1387.): 1-O-(Z)-10-Docosenyl-*sn*-glycero-3-phospho-N,N-dimethyl-N-dihydroxypropyl-butylammonium (n = 4)
C₃₄H₇₀NO₈P (651.91)
- 1388.): 1-O-(Z,Z)-5,11-Octadecadienyl-*sn*-glycero-3-phospho-N,N-dimethyl-N-dihydroxypropyl-butylammonium (n = 4)
C₃₀H₆₀NO₈P (593.78)
- 1389.): 1-O-(Z)-12-Eicosenyl-*sn*-glycero-3-phospho-N,N-dimethyl-N-dihydroxypropyl-butylammonium (n = 4)
C₃₂H₆₆NO₈P (623.85)
n=6
- 1390.): 1-(Z)-6-Octadecenoyl-*sn*-glycero-3-phospho-N,N-dimethyl-N-dihydroxypropyl-hexylammonium (n = 6)
C₃₂H₆₄NO₉P (637.84)
- 1391.): 1-(Z)-10-Docosenoyl-*sn*-glycero-3-phospho-N,N-dimethyl-N-dihydroxypropyl-hexylammonium (n = 6)
C₃₆H₇₂NO₉P (693.94)
- 1392.): 1-(Z,Z)-5,11-Hexadecadienoyl-*sn*-glycero-3-phospho-N,N-dimethyl-N-dihydroxypropyl-hexylammonium (n = 6)
C₃₀H₅₈NO₉P (607.77)
- 1393.): 1-(Z,Z)-10,16-Docosadienoyl-*sn*-glycero-3-phospho-N,N-dimethyl-N-dihydroxypropyl-hexylammonium (n = 6)
C₃₆H₇₀NO₉P (691.93)
- 1394.): 1-(Z,Z)-6,18-Hexacosadienoyl-*sn*-glycero-3-phospho-N,N-dimethyl-N-dihydroxypropyl-hexylammonium (n = 6)
C₄₀H₇₈NO₉P (748.03)
- 1395.): 1-O-(Z)-6-Octadecenyl-*sn*-glycero-3-phospho-N,N-dimethyl-N-dihydroxypropyl-hexylammonium (n = 6)
C₃₂H₆₆NO₈P (623.85)
- 1396.): 1-O-(Z)-10-Docosenyl-*sn*-glycero-3-phospho-N,N-dimethyl-N-dihydroxypropyl-hexylammonium (n = 6)
C₃₆H₇₄NO₈P (679.96)
- 1397.): 1-O-(Z,Z)-5,11-Octadecadienyl-*sn*-glycero-3-phospho-N,N-dimethyl-N-dihydroxypropyl-hexylammonium (n = 6)
C₃₂H₆₄NO₈P (621.84)
- 1398.): 1-O-(Z)-12-Eicosenyl-*sn*-glycero-3-phospho-N,N-dimethyl-N-dihydroxypropyl-hexylammonium (n = 6)
C₃₄H₇₀NO₈P (651.91)

### 2. Beispiele für einkettige Glycero-phospho-N,N-dimethyl-N-(2-hydroxypropyl-3,1-O,O-dihydroxypropyl)-alkylammonium-Verbindungen

### (A = III bzw. IV; n = 2 - 6; R₃, CH₃; m= 1, x = 0; y = 1; z = 2)

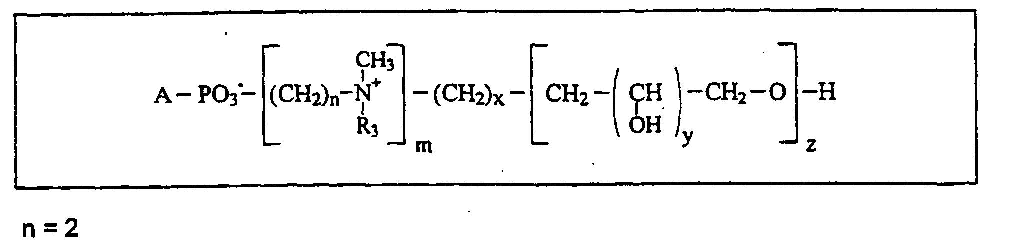

- 1399.): 1-(Z)-6-Hexadecenoyl-*sn*-glycero-3-phospho-N,N-dimethyl-N-(2-hydroxypropyl-3,1-O,O-dihydroxypropyl)-ethylammonium (n = 2)
C₂₉H₅₈NO₁₁P (627.75)
- 1400.): 1-(Z)-6-Nonadecenoyl-*sn*-glycero-3-phospho-N,N-dimethyl-N-(2-hydroxypropyl-3,1-O,O-dihydroxypropyl)-ethylammonium (n = 2)
C₃₂H₆₄NO₁₁P (669.83)
- 1401.): 1-(Z)-10-Docosenoyl-*sn*-glycero-3-phospho-N,N-dimethyl-N-(2-hydroxypropyl-3,1-O,O-dihydroxypropyl)-ethylammonium (n = 2)
C₃₅H₇₀NO₁₁P (711.91)
- 1402.): 1-(Z)-12-Docosenoyl-sn-glycero-3-phospho-N,N-dimethyl-N-(2-hydroxypropyl-3,1-O,O-dihydroxypropyl)-ethylammonium (n = 2)
C₃₅H₇₀NO₁₁P (711.91)
- 1403.): 1-(Z)-10-Tetracosenoyl-*sn*-glycero-3-phospho-N,N-dimethyl-N-(2-hydroxypropyl-3,1-O,O-dihydroxypropyl)-ethylammonium (n = 2)
C₃₇H₇₄NO₁₁P (739.97)
- 1404.): 1-(Z)-16-Hexacosenoyl-*sn*-glycero-3-phospho-N,N-dimethyl-N-(2-hydroxypropyl-3,1-O,O-dihydroxypropyl)-ethylammonium (n = 2)
C₃₉H₇₈NO₁₁P (768.02)
- 1405.): 1-(Z,Z)-5,11-Hexadecadienoyl-*sn*-glycero-3-phospho-N,N-dimethyl-N-(2-hydroxypropyl-3,1-O,O-dihydroxypropyl)-ethylammonium (n = 2)
C₂₉H₅₆NO₁₁P (625.74)
- 1406.): 1-(Z,Z)-5,11-Octadecadienoyl-*sn*-glycero-3-phospho-N,N-dimethyl-N-(2-hydroxypropyl-3,1-O,O-dihydroxypropyl)-ethylammonium (n = 2)
C₃₁H₆₀NO₁₁P (653.79)
- 1407.): 1-(Z,Z)-10,16-Heneicosadienoyl-*sn*-glycero-3-phospho-N,N-dimethyl-N-(2-hydroxypropyl-3,1-O,O-dihydroxypropyl)-ethylammonium (n = 2)
C₃₄H₆₆NO₁₁P (695.87)
- 1408.): 1-(Z,Z)-10,16-Docosadienoyl-*sn*-glycero-3-phospho-N,N-dimethyl-N-(2-hydroxypropyl-3,1-O,O-dihydroxypropyl)-ethylammonium (n = 2)
C₃₅H₆₈NO₁₁P (709.90)
- 1409.): 1-(Z,Z)-6,18-Hexacosadienoyl-*sn*-glycero-3-phospho-N,N-dimethyl-N-(2-hydroxypropyl-3,1-O,O-dihydroxypropyl)-ethylammonium (n = 2)
C₃₉H₇₆NO₁₁P (766.01)
Alkenyl
- 1410.): 1-O-(Z)-6-Octadecenyl-*sn*-glycero-3-phospho-N,N-dimethyl-N-(2-hydroxypropyl-3,1-O,O-dihydroxypropyl)-ethylammonium (n = 2)
C₃₁H₆₄NO₁₀P (641.82)
- 1411.): 1-O-(Z)-12-Eicosenyl-*sn*-glycero-3-phospho-N,N-dimethyl-N-(2-hydroxypropyl-3,1-O,O-dihydroxypropyl)-ethylammonium (n = 2)
C₃₃H₆₈NO₁₀P (669.88)
- 1412.): 1-O-(Z)-10-Docosenyl-*sn*-glycero-3-phospho-N,N-dimethyl-N-(2-hydroxypropyl-3,1-O,O-dihydroxypropyl)-ethylammonium (n = 2)
C₃₅H₇₂NO₁₀P (697.93)
- 1413.): 1-O-(Z)-10-Tetracosenyl-*sn*-glycero-3-phospho-N,N-dimethyl-N-(2-hydroxypropyl-3,1-O,O-dihydroxypropyl)-ethylammonium (n = 2)
C₃₇H₇₆NO₁₀P (725.98)
- 1414.): 1-O-(Z)-16-Hexacosenyl-*sn*-glycero-3-phospho-N,N-dimethyl-N-(2-hydroxypropyl-3,1-O,O-dihydroxypropyl)-ethylammonium (n = 2)
C₃₉H₈₀NO₁₀P (754.04)
- 1415.): 1-O-(Z,Z)-5,11-Octadecadienyl-*sn*-glycero-3-phospho-N,N-dimethyl-N-(2-hydroxypropyl-3,1-O,O-dihydroxypropyl)-ethylammonium (n = 2)
C₃₁H₆₂NO₁₀P (639.81)
- 1416.): 1-O-(Z,Z)-6,18-Tetracosadienyl-*sn*-glycero-3-phospho-N,N-dimethyl-N-(2-hydroxypropyl-3,1-O,O-dihydroxypropyl)-ethylammonium (n = 2)
C₃₇H₇₄NO₁₀P (723.97)
- 1417.): 1-O-(Z,Z)-6,18-Hexacosadienyl-*sn*-glycero-3-phospho-N,N-dimethyl-N-(2-hydroxypropyl-3,1-O,O-dihydroxypropyl)-ethylammonium (n = 2)
C₃₉H₇₈NO₁₀P (752.04)
n=3
- 1418.): 1-(Z)-6-Octadecenoyl-*sn*-glycero-3-phospho-N,N-dimethyl-N-(2-hydroxypropyl-3,1-O,O-dihydroxypropyl)-propylammonium (n = 3)
C₃₂H₆₄NO₁₁P (669.83)
- 1419.): 1-(Z)-12-Eicosenoyl-*sn*-glycero-3-phospho-N,N-dimethyl-N-(2-hydroxypropyl-3,1-O,O-dihydroxypropyl)-propylammonium (n = 3)
C₃₄H₆₈NO₁₁P (697.89)
- 1420.): 1-(Z)-10-Docosenoyl-*sn*-glycero-3-phospho-N,N-dimethyl-N-(2-hydroxypropyl-3,1-O,O-dihydroxypropyl)-propylammonium (n = 3)
C₃₆H₇₂NO₁₁P (725.94)
- 1421.): 1-(Z)-12-Docosenoyl-*sn*-glycero-3-phospho-N,N-dimethyl-N-(2-hydroxypropyl-3,1-O,O-dihydroxypropyl)-propylammonium (n = 3)
C₃₆H₇₂NO₁₁P (725.94)
- 1422.): 1-(Z)-10-Tetracosenoyl-*sn*-glycero-3-phospho-N,N-dimethyl-N-(2-hydroxypropyl-3,1-O,O-dihydroxypropyl)-propylammonium (n = 3)
C₃₈H₇₆NO₁₁P (754.0)
- 1423.): 1-(Z,Z)-5,11-Octadecadienoyl-*sn*-glycero-3-phospho-N,N-dimethyl-N-(2-hydroxypropyl-3,1-O,O-dihydroxypropyl)-propylammonium (n = 3)
C₃₂H₆₂NO₁₁P (667.83)
- 1424.): 1-(Z,Z)-10,16-Eicosadienoyl-*sn*-glycero-3-phospho-N,N-dimethyl-N-(2-hydroxypropyl-3,1-O,O-dihydroxypropyl)-propylammonium (n = 3)
C₃₄H₆₆NO₁₁P (695.89)
- 1425.): 1-(Z,Z)-10,16-Docosadienoyl-*sn*-glycero-3-phospho-N,N-dimethyl-N-(2-hydroxypropyl-3,1-O,O-dihydroxypropyl)-propylammonium (n = 3)
C₃₆H₇₀NO₁₁P (723.94)
- 1426.): 1-(Z,Z)-6,18-Tetracosadienoyl-*sn*-glycero-3-phospho-N,N-dimethyl-N-(2-hydroxypropyl-3,1-O,O-dihydroxypropyl)-propylammonium (n = 3)
C₃₆H₇₄NO₁₁P (751.98)
- 1427.): 1-(Z,Z)-6,18-Hexacosadienoyl-*sn*-glycero-3-phospho-N,N-dimethyl-N-(2-hydroxypropyl-3,1-O,O-dihydroxypropyl)-propylammonium (n = 3)
C₄₀H₇₈NO₁₁P (780.03)
- 1428.): 1-O-(Z)-6-Hexadecenyl-*sn*-glycero-3-phospho-N,N-dimethyl-N-(2-hydroxypropyl-3,1-O,O-dihydroxypropyl)-propylammonium (n = 3)
C₃₀H₆₂NO₁₀P (627.80)
- 1429.): 1-O-(Z)-10-Docosenyl-*sn*-glycero-3-phospho-N,N-dimethyl-N-(2-hydroxypropyl-3,1-O,O-dihydroxypropyl)-propylammonium (n = 3)
C₃₆H₇₄NO₁₀P (711.96)
- 1430.): 1-O-(Z)-10-Tetracosenyl-*sn*-glycero-3-phospho-N,N-dimethyl-N-(2-hydroxypropyl-3,1-O,O-dihydroxypropyl)-propylammonium (n = 3)
C₃₈H₇₈NO₁₀P (740.01)
- 1431.): 1-O-(Z,Z)-5,11-Hexadecadienyl-*sn*-glycero-3-phospho-N,N-dimethyl-N-(2-hydroxypropyl-3,1-O,O-dihydroxypropyl)-propylammonium (n = 3)
C₃₀H₆₀NO₁₀P (625.78)
- 1432.): 1-O-(Z,Z)-5,11-Octadecadienyl-*sn*-glycero-3-phospho-N,N-dimethyl-N-(2-hydroxypropyl-3,1-O,O-dihydroxypropyl)-propylammonium (n = 3)
C₃₂H₆₄NO₁₀P (653.83)
- 1433.): 1-O-(Z,Z)-10,16-Eicosadienyl-*sn*-glycero-3-phospho-N,N-dimethyl-N-(2-hydroxypropyl-3,1-O,O-dihydroxypropyl)-propylammonium (n = 3)
C₃₄H₆₈NO₁₀P (681.89)
- 1434.): 1-O-(Z,Z)-6,18-Tetracosadienyl-*sn*-glycero-3-phospho-N,N-dimethyl-N-(2-hydroxypropyl-3,1-O,O-dihydroxypropyl)-propylammonium (n = 3)
C₃₈H₇₆NO₁₀P (738.0)
- 1435.): 1-O-(Z,Z)-6,18-Hexacosadienyl-*sn*-glycero-3-phospho-N,N-dimethyl-N-(2-hydroxypropyl-3,1-O,O-dihydroxypropyl)-propylammonium (n = 3)
C₄₀H₈₀NO₁₀P (766.05)
n=4
- 1436.): 1-(Z)-6-Octadecenoyl-*sn*-glycero-3-phospho-N,N-dimethyl-N-(2-hydroxypropyl-3,1-O,O-dihydroxypropyl)-butylammonium (n = 4)
C₃₃H₈₆NO₁₁P (683.86)
- 1437.): 1-(Z)-10-Docosenoyl-*sn*-glycero-3-phospho-N,N-dimethyl-N-(2-hydroxypropyl-3,1-O,O-dihydroxypropyl)-butylammonium (n = 4)
C₃₇H₇₄NO₁₁P (739.97)
- 1438.): 1-(Z,Z)-5,1 1 -Hexadecadienoyl-sn-glycero-3-phospho-N,N-dimethyl-N-(2-hydroxypropyl-3,1-O,O-dihydroxypropyl)-butylammonium (n = 4)
C₃₁H₆₀NO₁₁P (653.79)
- 1439.): 1-(Z,Z)-10,16-Docosadienoyl-sn-glycero-3-phospho-N,N-dimethyl-N-(2-hydroxypropyl-3,1-O,O-dihydroxypropyl)-butylammonium (n = 4)
C₃₇H₇₂NO₁₁P (737.95)
- 1440.): 1-(Z,Z)-6,18-Hexacosadienoyl-*sn*-glycero-3-phospho-N,N-dimethyl-N-(2-hydroxypropyl-3,1-O,O-dihydroxypropyl)-butylammonium (n = 4)
C₄₁H₈₀NO₁₁P (794.06)
- 1441.): 1-O-(Z)-6-Octadecenyl-*sn*-glycero-3-phospho-N,N-dimethyl-N-(2-hydroxypropyl-3,1-O,O-dihydroxypropyl)-butylammonium (n = 4)
C₃₃H₆₈NO₁₀P (669.88)
- 1442.): 1-O-(Z)-10-Docosenyl-*sn*-glycero-3-phospho-N,N-dimethyl-N-(2-hydroxypropyl-3,1-O,O-dihydroxypropyl)-butylammonium (n = 4)
C₃₇H₇₆NO₁₀P (725.98)
- 1443.): 1-O-(Z,Z)-5,11-Octadecadienyl-*sn*-glycero-3-phospho-N,N-dimethyl-N-(2-hydroxypropyl-3,1-O,O-dihydroxypropyl)-butylammonium (n = 4)
C₃₃H₆₆NO₁₀P (667.86)
- 1444.): 1-O-(Z)-12-Eicosenyl-*sn*-glycero-3-phospho-N,N-dimethyl-N-(2-hydroxypropyl-3,1-O,O-dihydroxypropyl)-butylammonium (n = 4)
C₃₅H₇₂NO₁₀P (697.93)
n=6
- 1445.): 1-(Z)-6-Octadecenoyl-*sn*-glycero-3-phospho-N,N-dimethyl-N-(2-hydroxypropyl-3,1-O,O-dihydroxypropyl)-hexylammonium (n = 6)
C₃₅H₇₀NO₁₁P (711.91)
- 1446.): 1-(Z)-10-Docosenoyl-*sn*-glycero-3-phospho-N,N-dimethyl-N-(2-hydroxypropyl-3,1-O,O-dihydroxypropyl)-hexylammonium (n = 6)
C₃₉H₇₈NO₁₁P (768.02)
- 1447.): 1-(Z,Z)-5,11-Hexadecadienoyl-*sn*-glycero-3-phospho-N,N-dimethyl-N-(2-hydroxypropyl-3,1-O,O-dihydroxypropyl)-hexylammonium (n = 6)
C₃₃H₆₄NO₁₁P (681.85)
- 1448.): 1-(Z,Z)-10,16-Docosadienoyl-*sn*-glycero-3-phospho-N,N-dimethyl-N-(2-hydroxypropyl-3,1-O,O-dihydroxypropyl)-hexylammonium (n = 6)
C₃₉H₇₆NO₁₁P (766.01)
- 1449.): 1-(Z,Z)-6,18-Hexacosadienoyl-*sn*-glycero-3-phospho-N,N-dimethyl-N-(2-hydroxypropyl-3,1-O,O-dihydroxypropyl)-hexylammonium (n = 6)
C₄₃H₈₄NO₁₁P (822.11)
- 1450.): 1-O-(Z)-6-Octadecenyl-*sn*-glycero-3-phospho-N,N-dimethyl-N-(2-hydroxypropyl-3,1-0,0-dihydroxypropyl)-hexylammonium (n = 6)
C₃₅H₇₂NO₁₀P (697.93)
- 1451.): 1-O-(Z)-10-Docosenyl-*sn*-glycero-3-phospho-N,N-dimethyl-N-(2-hydroxypropyl-3,1-O,O-dihydroxypropyl)-hexylammonium (n = 6)
C₃₉H₈₀NO₁₀P (754.04)
- 1452.): 1-O-(Z,Z)-5,11-Octadecadienyl-*sn*-glycero-3-phospho-N,N-dimethyl-N-(2-hydroxypropyl-3,1-O,O-dihydroxypropyl)-hexylammonium (n = 6)
C₃₅H₇₀NO₁₀P (695.92)
- 1453.): 1-O-(Z)-12-Eicosenyl-*sn*-glycero-3-phospho-N,N-dimethyl-N-(2-hydroxypropyl-3,1-O,O-dihydroxypropyl)-hexylammonium (n = 6)
C₃₇H₇₆NO₁₀P (725.98)

### 3. Beispiele für einkettigre Glycero-phospho-N,N-dimethyl-N-(2-hydroxypropyl-3,1-O,O-2-hydroxypropyl-3,1-O,O-dihydroxypropyl)-alkylammonium-Verbindungen

### (A = III bzw. IV; n = 2 - 6; R₃, CH₃; m= 1, x = 0; y = 1; z = 3)

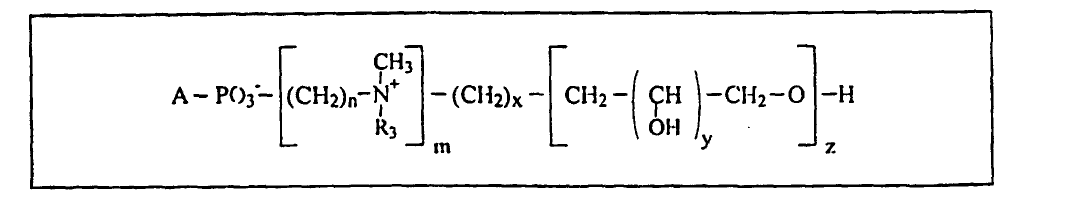

Im folgenden Text wird N-(2-hydroxypropyl-3,1-O,O-2-hydroxypropyl-3,1-O,Odihydroxypropyl) abgekürzt als N-(HP₁-HP₂-diHP₃)
n=2
- 1454.): 1-(Z)-6-Hexadecenoyl-*sn*-glycero-3-phospho-N,N-dimethyl-N-(HP₁-HP₂-diHP₃)-ethylammonium (n = 2)
C₃₂H₆₄NO₁₃P (701.83)
- 1455.): 1-(Z)-6-Nonadecenoyl-*sn*-glycero-3-phospho-N,N-dimethyl-N-(HP₁-HP₂-diHP₃)-ethylammonium (n = 2)
C₃₅H₇₀NO₁₃P (743.91)
- 1456.): 1-(Z)-10-Docosenoyl-*sn*-glycero-3-phospho-N,N-dimethyl-N-(HP₁-HP₂-diHP₃)-ethylammonium (n = 2)
C₃₈H₇₆NO₁₃P (785.99)
- 1457.): 1-(Z)-12-Docosenoyl-*sn*-glycero-3-phospho-N,N-dimethyl-N-(HP₁-HP₂-diHP₃)-ethylammonium (n = 2)
C₃₈H₇₆NO₁₃P (785.99)
- 1458.): 1-(Z)-16-Hexacosenoyl-*sn*-glycero-3-phospho-N,N-dimethyl-N-(HP₁-HP₂-diHP₃)-ethylammonium (n = 2)
C₄₂H₈₄NO₁₃P (842.10)
- 1459.): 1-(Z,Z)-5,11-Hexadecadienoyl-*sn*-glycero-3-phospho-N,N-dimethyl-N-(HP₁-HP₂-diHP₃)-ethylammonium (n = 2)
C₃₂H₆₂NO₁₃P (699.82)
- 1460.): 1-(Z,Z)-5,11-Octadecadienoyl-*sn*-glycero-3-phospho-N,N-dimethyl-N-(HP₁-HP₂-diHP₃)-ethylammonium (n = 2)
C₃₄H₆₆NO₁₃P (727.87)
- 1461.): 1-(Z,Z)-10,16-Docosadienoyl-*sn*-glycero-3-phospho-N,N-dimethyl-N-(HP₁-HP₂-diHP₃)-ethylammonium (n = 2)
C₃₈H₇₄NO₁₃P (783.98)
- 1462.): 1-(Z,Z)-6,18-Hexacosadienoyl-*sn*-glycero-3-phospho-N,N-dimethyl-N-(HP₁-HP₂-diHP₃)-ethylammonium (n = 2)
C₄₂H₈₂NO₁₃P (840.09)
Alkenyl
- 1463.): 1-O-(Z)-6-Octadecenyl-*sn*-glycero-3-phospho-N,N-dimethyl-N-(HP₁-HP₂-diHP₃)-ethylammonium (n = 2)
C₃₄H₇₀NO₁₂P (715.90)
- 1464.): 1-O-(Z)-12-Eicosenyl-*sn*-glycero-3-phospho-N,N-dimethyl-N-(HP₁-HP₂-diHP₃)-ethylammonium (n = 2)
C₃₆H₇₄NO₁₂P (743.96)
- 1465.): 1-O-(Z)-10-Docosenyl-*sn*-glycero-3-phospho-N,N-dimethyl-N-(HP₁-HP₂-diHP₃)-ethylammonium (n = 2)
C₃₈H₇₈NO₁₂P (772.01)
- 1466.): 1-O-(Z)-16-Hexacosenyl-*sn*-glycero-3-phospho-N,N-dimethyl-N-(HP₁-HP₂-diHP₃)-ethylammonium (n = 2)
C₄₂H₈₆NO₁₂P (828.12)
- 1467.): 1-O-(Z,Z)-5,11-Octadecadienyl-*sn*-glycero-3-phospho-N,N-dimethyl-N-(HP₁-HP₂-diHP₃)-ethylammonium (n = 2)
C₃₄H₆₈NO₁₂P (713.89)
- 1468.): 1-O-(Z,Z)-6,18-Hexacosadienyl-*sn*-glycero-3-phospho-N,N-dimethyl-N-(HP₁-HP₂-diHP₃)-ethylammonium (n = 2)
C₄₂H₈₄NO₁₂P (826.10)
n=3
- 1469.): 1-(Z)-6-Octadecenoyl-*sn*-glycero-3-phospho-N,N-dimethyl-N-(HP₁-HP₂-diHP₃)-propylammonium (n = 3)
C₃₅H₇₀NO₁₃P (743.91)
- 1470.): 1-(Z)-10-Docosenoyl-*sn*-glycero-3-phospho-N,N-dimethyl-N-(HP₁-HP₂-diHP₃)-propylammonium (n = 3)
C₃₉H₇₈NO₁₃P (800.02)
- 1471.): 1-(Z)-10-Tetracosenoyl-*sn*-glycero-3-phospho-N,N-dimethyl-N-(HP₁-HP₂-diHP₃)-propylammonium (n = 3)
C₄₁H₈₂NO₁₃P (828.07)
- 1472.): 1-(Z,Z)-5,11-Octadecadienoyl-*sn*-glycero-3-phospho-N,N-dimethyl-N-(HP₁-HP₂-diHP₃)-propylammonium (n = 3)
C₃₅H₆₈NO₁₃P (741.90)
- 1473.): 1-(Z,Z)-10,16-Eicosadienoyl-*sn*-glycero-3-phospho-N,N-dimethyl-N-(HP₁-HP₂-diHP₃)-propylammonium (n = 3)
C₃₇H₇₂NO₁₃P (769.95)
- 1474.): 1-(Z,Z)-10,16-Docosadienoyl-*sn*-glycero-3-phospho-N,N-dimethyl-N-(HP₁-HP₂-diHP₃)-propylammonium (n = 3)
C₃₉H₇₆NO₁₃P (798.01)
- 1475.): 1-(Z,Z)-6,18-Hexacosadienoyl-*sn*-glycero-3-phospho-N,N-dimethyl-N-(HP₁-HP₂-diHP₃)-propylammonium (n = 3)
C₄₃H₈₄NO₁₃P (854.11)
- 1476.): 1-O-(Z)-10-Docosenyl-*sn*-glycero-3-phospho-N,N-dimethyl-N-(HP₁-HP₂-diHP₃)-propylammonium (n = 3)
C₃₉H₈₀NO₁₂P (786.04)
- 1477.): 1-O-(Z)-10-Tetracosenyl-*sn*-glycero-3-phospho-N,N-dimethyl-N-(HP₁-HP₂-diHP₃)-propylammonium (n = 3)
C₄₁H₈₄NO₁₂P (814.09)
- 1478.): 1-O-(Z,Z)-10,16-Eicosadienyl-*sn*-glycero-3-phospho-N,N-dimethyl-N-(HP₁-HP₂-diHP₃)-propylammonium (n = 3)
C₃₇H₇₄NO₁₂P (812.08)
- 1479.): 1-O-(Z,Z)-6,18-Tetracosadienyl-*sn*-glycero-3-phospho-N,N-dimethyl-N-(HP₁-HP₂-diHP₃)-propylammonium (n = 3)
C₄₁H₈₂NO₁₂P (812.08)
- 1480.): 1-O-(Z,Z)-6,18-Hexacosadienyl-*sn*-glycero-3-phospho-N,N-dimethyl-N-(HP₁-HP₂-diHP₃)-propylammonium (n = 3)
C₄₃H₈₆NO₁₂P (840.13)
n=4
- 1481.): 1-(Z)-10-Docosenoyl-*sn*-glycero-3-phospho-N,N-dimethyl-N-(HP₁-HP₂-diHP₃)-butylammonium (n = 4)
C₄₀H₈₀NO₁₃P (814.05)
- 1482.): 1-(Z,Z)-10,16-Docosadienoyl-*sn*-glycero-3-phospho-N,N-dimethyl-N-(HP₁-HP₂-diHP₃)-butylammonium (n = 4)
C₄₀H₇₈NO₁₃P (812.03)
- 1483.): 1-(Z,Z)-6,18-Hexacosadienoyl-*sn*-glycero-3-phospho-N,N-dimethyl-N-(HP₁-HP₂-diHP₃)-butylammonium (n = 4)
C₄₄H₈₆NO₁₃P (868.14)
- 1484.): 1-O-(Z)-6-Octadecenyl-*sn*-glycero-3-phospho-N,N-dimethyl-N-(HP₁-HP₂-diHP₃)-butylammonium (n = 4)
C₃₆H₇₄NO₁₂P (743.96)
- 1485.): 1-O-(Z)-10-Docosenyl-*sn*-glycero-3-phospho-N,N-dimethyl-N-(HP₁-HP₂-diHP₃)-butylammonium (n = 4)
C₄₀H₈₂NO₁₂P (800.06)
- 1486.): 1-O-(Z,Z)-5,11-Octadecadienyl-*sn*-glycero-3-phospho-N,N-dimethyl-N-(HP₁-HP₂-diHP₃)-butylammonium (n = 4)
C₃₆H₇₂NO₁₂P (741.94)
- 1487.): 1-O-(Z)-12-Eicosenyl-*sn*-glycero-3-phospho-N,N-dimethyl-N-(HP₁-HP₂-diHP₃)-butylammonium (n = 4)
C₃₈H₇₈NO₁₂P (772.01)
n=6
- 1488.): 1-(Z)-6-Octadecenoyl-*sn*-glycero-3-phospho-N,N-dimethyl-N-(HP₁-HP₂-diHP₃)-hexylammonium (n = 6)
C₃₈H₇₆NO₁₃P (785.99)
- 1489.): 1-(Z)-10-Docosenoyl-*sn*-glycero-3-phospho-N,N-dimethyl-N-(HP₁-HP₂-diHP₃)-hexylammonium (n = 6)
C₄₂H₈₄NO₁₃P (842.10)
- 1490.): 1-(Z,Z)-5,11-Hexadecadienoyl-*sn*-glycero-3-phospho-N,N-dimethyl-N-(HP₁-HP₂-diHP₃)-hexylammonium (n = 6)
C₃₆H₇₀NO₁₃P (755.92)
- 1491.): 1-(Z,Z)-10,16-Docosadienoyl-*sn*-glycero-3-phospho-N,N-dimethyl-N-(HP₁-HP₂-diHP₃)-hexylammonium (n = 6)
C₄₂H₈₂NO₁₃P (840.09)
- 1492.): 1-(Z,Z)-6,18-Hexacosadienoyl-*sn*-glycero-3-phospho-N,N-dimethyl-N-(HP₁-HP₂-diHP₃)-hexylammonium (n = 6)
C₄₆H₉₀NO₁₃P (896.19)
- 1493.): 1-O-(Z)-6-Octadecenyl-*sn*-glycero-3-phospho-N,N-dimethyl-N-(HP₁-HP₂-diHP₃)-hexylammonium (n = 6)
C₃₈H₇₈NO₁₂P (772.01)
- 1494.): 1-O-(Z)-10-Docosenyl-*sn*-glycero-3-phospho-N,N-dimethyl-N-(HP₁-HP₂-diHP₃)-hexylammonium (n = 6)
C₄₂H₈₆NO₁₂P (828.12)
- 1495.): 1-O-(Z,Z)-5,11-Octadecadienyl-*sn*-glycero-3-phospho-N,N-dimethyl-N-(HP₁-HP₂-diHP₃)-hexylammonium (n = 6)
C₃₈H₇₆NO₁₂P (769.99)
- 1496.): 1-O-(Z)-12-Eicosenyl-*sn*-glycero-3-phospho-N,N-dimethyl-N-(HP₁-HP₂-diHP₃)-hexylammonium (n = 6)
C₄₀H₈₂NO₁₂P (800.06)

### 4. Beispiele für einkettige Glycero-phospho-Verbindungen, die nicht am Stickstoff hydroxyliert sind

### (A = III; n = 2 - 6; R₃, CH₃; m= 1, x = 1; z = 0)

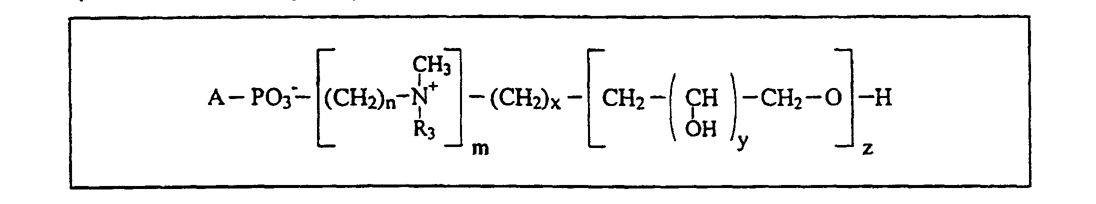

- 1497.): 1-(Z)-6-Octadecenoyl-sn-glycero-3-phospho-N,N,N-trimethyl-propylammonium (n = 3)
C₂₇H₅₄NO₇P (535.70)
- 1498.): 1-(Z)-10-Docosenoyl-*sn*-glycero-3-phospho-N,N,N-trimethyl-propylammonium (n = 3)
C₃₁H₆₂NO₇P (591.81)
- 1499.): 1-(Z)-10-Tetracosenoyl-*sn*-glycero-3-phospho-N,N,N-trimethyl-propylammonium (n = 3)
C₃₃H₆₆NO₇P (619.86)
- 1500.): 1-(Z,Z)-5,11-Octadecadienoyl-*sn*-glycero-3-phospho-N,N,N-trimethyl-- propylammonium (n = 3)
C₂₇H₅₂NO₇P (533.69)
- 1501.): 1-(Z,Z)-10,16-Eicosadienoyl-*sn*-glycero-3-phospho-N,N,N-trimethyl-propylammonium (n = 3)
C₂₉H₅₆NO₇P (561.74)
- 1502.): 1-(Z,Z)-10,16-Docosadienoyl-*sn*-glycero-3-phospho-N,N,N-trimethyl-propylammonium (n = 3)
C₃₁H₆₀NO₇P (589.79)
- 1503.): 1-(Z,Z)-6,18-Hexacosadienoyl-*sn*-glycero-3-phospho-N,N,N-trimethyl-propylammonium (n = 3)
C₃₅H₆₈NO₇P (645.90)
- 1504.): 1-O-(Z)-10-Docosenyl-*sn*-glycero-3-phospho-N,N,N-trimethyl-propylammonium (n = 3)
C₃₁H₆₄NO₆P (577.83)
- 1505.): 1-O-(Z)-10-Tetracosenyl-*sn*-glycero-3-phospho-N,N,N-trimethyl-propylammonium (n = 3)
C₃₃H₆₈NO₆P (605.88)
- 1506.): 1-O-(Z,Z)-10,16-Eicosadienyl-*sn*-glycero-3-phospho-N,N,N-trimethyl-propylammonium (n = 3)
C₂₉H₅₈NO₆P (547.76)
- 1507.): 1-O-(Z,Z)-6,18-Tetracosadienyl-*sn*-glycero-3-phospho-N,N,N-trimethyl-propylammonium (n = 3)
C₃₃H₆₆NO₆P (603.86)
- 1508.): 1-O-(Z,Z)-6,18-Hexacosadienyl-*sn*-glycero-3-phospho-N,N,N-trimethyl-propylammonium (n = 3)
C₃₅H₇₀NO₆P (631.92)

### 5. Beispiele für ω,ω -Alkandiol-phospho-N,N-dimethyl-N-dihydroxypropylalkylammonium-Verbindungen

### (A = V: n = 2 - 6; R₃, CH₃; m= 1. x = 0; y = 1; z = 1)

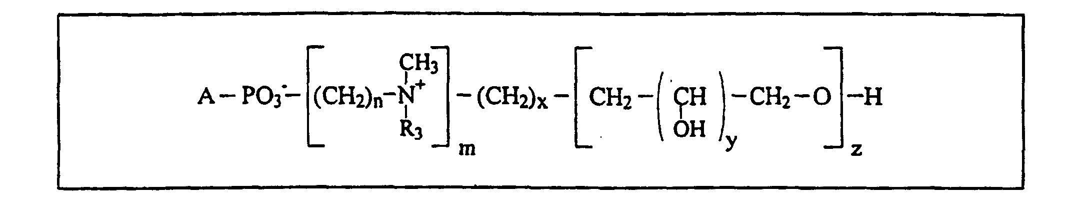

- 1509.): 1-(Z)-10-Docosenoyl-ethylenglykol-phospho-N,N-dimethyl-N-dihydroxypropyl-ethylammonium (n = 2)
C₃₁H₆₂NO₈P (607.81)
- 1510.): 1-(Z)-6-Octadecenoyl-propandiol-(1,3)-phospho-N,N-dimethyl-N-dihydroxypropyl-ethylammonium (n = 2)
C₂₈H₅₆NO₈P (565.73)
- 1511.): 1-(Z)-10-Docosenoyl-propandiol-(1,3)-phospho-N,N-dimethyl-N-dihydroxypropyl-ethylammonium (n = 2)
C₃₂H₆₄NO₈P (621.84)
- 1512.): 1-(Z)-10-Tetracosenoyl-propandiol-(1,3)-phospho-N,N-dimethyl-N-dihydroxypropyl-ethylammonium (n = 2)
C₃₄H₆₈NO₈P (649.89)
- 1513.): 1-(Z,Z)-5,11-Octadecadienoyl-propandiol-(1,3)-phospho-N,N-dimethyl-N-dihydroxypropyl-ethylammonium (n = 2)
C₂₈H₅₄NO₈P (563.71)
- 1514.): 1-(Z,Z)-10,16-Eicosadienoyl-propandiol-(1,3)-phospho-N,N-dimethyl-N-dihydroxypropyl-ethylammonium (n = 2)
C₃₀H₅₈NO₈P (591.77)
- 1515.): 1-(Z,Z)-10,16-Docosadienoyl-propandiol-(1,3)-phospho-N,N-dimethyl-N-dihydroxypropyl-ethylammonium (n = 2)
C₃₂H₆₂NO₈P (619.82)
- 1516.): 1-(Z,Z)-6,18-Hexacosadienoyl-propandiol-(1,3)-phospho-N,N-dimethyl-N-dihydroxypropyl-ethylammonium (n = 2)
C₃₆H₇₀NO₈P (675.93)
- 1517.): 1-(Z)-10-Docosenoyl-propandiol-(1,3)-phospho-N,N-dimethyl-N-dihydroxypropyl-propylammonium (n = 3)
C₃₃H₆₆NO₈P (635.86)
- 1518.): 1-(Z)-10-Docosenoyl-propandiol-(1,3)-phospho-N,N-dimethyl-N-dihydroxypropyl-butylammonium (n = 4)
C₃₄H₆₈NO₈P (649.89)

### 6. Beispiele für Alkandiol-(1,2)-phospho-N,N-dimethyl-N-dihydroxypropylalkylammonium-Verbindungen

### (A = VII; n = 2 - 6: R₃, CH₃; m= 1, x = 0; y = 1; z = 1)

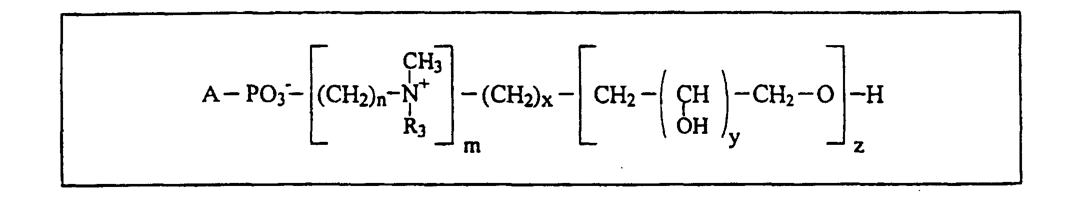

- 1519.): 2-(Z)-10-Docosenoyl-propandiol-(1,2)-phospho-N,N-dimethyl-N-dihydroxypropyl-ethylammonium (n = 2)
C₃₂H₆₄NO₈P (621.84)
- 1520.): 1-(Z)-10-Docosenoyl-propandiol-(1,2)-phospho-N,N-dimethyl-N-dihydroxypropyl-ethylammonium (n = 2)
C₃₂H₆₄NO₈P (621.84)
- 1521.): 2-(Z)-10-Docosenoyl-propandiol-(1,2)-phospho-N,N-dimethyl-N-dihydroxypropyl-propylammonium (n = 3)
C₃₃H₆₆NO₈P (635.86)
- 1522.): 1-(Z)-10-Docosenoyl-propandiol-(1,2)-phospho-N,N-dimethyl-N-dihydroxypropyl-butylammonium (n = 4)
C₃₄H₆₈NO₈P (649.89)

### 7. Beispiele für ω,ω -Alkandiol-phospho-N,N-dimethyl-N-(2-hydroxypropyl-3,1-O,O-dihydroxypropyl)-alkylammonium-Verbindungen

### (A = V; n = 2 - 6; R₃, CH₃; m= 1, x = 0; y = 1; z = 2)

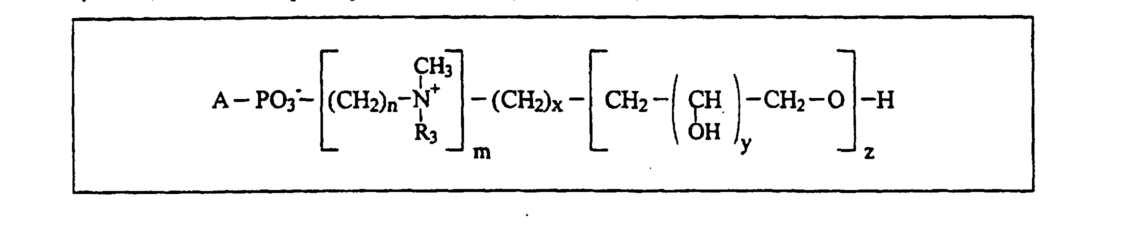

- 1523.): 1-(Z)-10-Docosenoyl-ethylenglykol-phospho-N,N-dimethyl-N-(2-hydroxypropyl-3,1-O,O-dihydroxypropyl)-ethylammonium (n = 2)
C₃₄H₆₈NO₁₀P (681.89)
- 1524.): 1-(Z)-6-Octadecenoyl-propandiol-(1,3)-phospho-N,N-dimethyl-N-(2-hydroxypropyl-3,1-O,O-dihydroxypropyl)-ethylammonium (n = 2)
C₃₁H₆₂NO₁₀P (639.81)
- 1525.): 1-(Z)-10-Docosenoyl-propandiol-(1,3)-phospho-N,N-dimethyl-N-(2-hydroxypropyl-3,1-O,O-dihydroxypropyl)-ethylammonium (n = 2)
C₃₅H₇₀NO₁₀P (695.92)
- 1526.): 1-(Z)-10-Tetracosenoyl-propandiol-(1,3)-phospho-N,N-dimethyl-N-(2-hydroxypropyl-3,1-O,O-dihydroxypropyl)-ethylammonium (n = 2)
C₃₇H₇₄NO₁₀P (723.97)
- 1527.): 1-(Z,Z)-5,11-Octadecadienoyl-propandiol-(1,3)-phospho-N,N-dimethyl-N-(2-hydroxypropyl-3,1-O,O-dihydroxypropyl)-ethylammonium (n = 2)
C₃₁H₆₀NO₁₀P (637.79)
- 1528.): 1-(Z,Z)-10,16-Eicosadienoyl-propandiol-(1,3)-phospho-N,N-dimethyl-N-(2-hydroxypropyl-3,1-O,O-dihydroxypropyl)-ethylammonium (n = 2)
C₃₃H₆₄NO₁₀P (665.85)
- 1529.): 1-(Z,Z)-10,16-Docosadienoyl-propandiol-(1,3)-phospho-N,N-dimethyl-N-(2-hydroxypropyl-3,1-O,O-dihydroxypropyl)-ethylammonium (n = 2)
C₃₅H₆₈NO₁₀P (693.90)
- 1530.): 1-(Z,Z)-6,18-Hexacosadienoyl-propandiol-(1,3)-phospho-N,N-dimethyl-N-(2-hydroxypropyl-3,1-O,O-dihydroxypropyl)-ethylammonium (n = 2)
C₃₉H₇₆NO₁₀P (750.01)
- 1531.): 1-(Z)-10-Docosenoyl-propandiol-(1,3)-phospho-N,N-dimethyl-N-(2-hydroxypropyl-3,1-O,O-dihydroxypropyl)-propylammonium (n = 3)
C₃₆H₇₂NO₁₀P (709.94)
- 1532.): 1-(Z)-10-Docosenoyl-propandiol-(1,3)-phospho-N,N-dimethyl-N-(2-hydroxypropyl-3,1-O,O-dihydroxypropyl)-butylammonium (n = 4)
C₃₇H₇₄NO₁₀P (723.96)
- 1533.): 1-(Z)-10-Docosenoyl-butandiol-(1,4)-phospho-N,N-dimethyl-N-(2-hydroxypropyl-3,1-O,O-dihydroxypropyl)-propylammonium (n = 3)
C₃₇H₇₄NO₁₀P (723.96)
- 1534.): 1-(Z)-10-Docosenoyl-hexandiol-(1,6)-phospho-N,N-dimethyl-N-(2-hydroxypropyl-3,1-O,O-dihydroxypropyl)-propylammonium (n = 3)
C₃₉H₇₈NO₁₀P (752.02)
- 1535.): 1-(Z)-10-Docosenoyl-octandiol-(1,8)-phospho-N,N-dimethyl-N-(2-hydroxypropyl-3,1-O,O-dihydroxypropyl)-propylammonium (n = 3)
C₄₁H₈₂NO₁₀P (780.07)

### 8. Beispiele für Alkandiol-(1,2)-phospho-N,N-dimethyl-N-(2-hydroxypropyl-3,1-O,O-dihydroxypropyl)-alkylammonium-Verbindungen

### (A = VII; n = 2 - 6; R₃, CH₃; m= 1, x = 0; y = 1; z = 2)

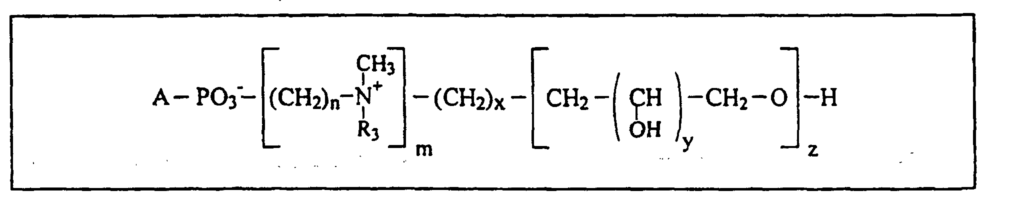

- 1536.): 2-(Z)-10-Docosenoyl-propandiol-(1,2)-phospho-N,N-dimethyl-N-(2-hydroxypropyl-3,1-O,O-dihydroxypropyl)-ethylammonium (n = 2)
C₃₅H₇₀NO₁₀P (695.91)
- 1537.): 1-(Z)-10-Docosenoyl-propandiol-(1,2)-phospho-N,N-dimethyl-N-(2-hydroxypropyl-3,1-O,O-dihydroxypropyl)-ethylammonium (n = 2)
C₃₅H₇₀NO₁₀P (695.91)
- 1538.): 2-(Z)-10-Docosenoyl-propandiol-(1,2)-phospho-N,N-dimethyl-N-(2-hydroxypropyl-3,1-O,O-dihydroxypropyl)-propylammonium (n = 3)
C₃₆H₇₂NO₁₀P (709.94)
- 1539.): 1-(Z)-10-Docosenoyl-propandiol-(1,2)-phospho-N,N-dimethyl-N-(2-hydroxypropyl-3,1-O,O-dihydroxypropyl)-butylammonium (n = 4)
C₃₇H₇₄NO₁₀P (723.97)
- 1540.): 1-(Z)-10-Docosenoyl-butandiol-(1,2)-phospho-N,N-dimethyl-N-(2-hydroxypropyl-3,1-O,O-dihydroxypropyl)-propylammonium (n = 3)
C₃₇H₇₄NO₁₀P (723.97)
- 1541.): 1-(Z)-10-Docosenoyl-hexandiol-(1,2)-phospho-N,N-dimethyl-N-(2-hydroxypropyl-3,1-O,O-dihydroxypropyl)-propylammonium (n = 3)
C₃₉H₇₈NO₁₀P (752.02)
- 1542.): 1-(Z)-10-Docosenoyl-octandiol-(1,2)-phospho-N,N-dimethyl-N-(2-hydroxypropyl-3,1-O,O-dihydroxypropyl)-propylammonium (n = 3)
C₄₁H₈₂NO₁₀P (780.07)

### 9. Beispiele für ω,ω'-Alkandiol-phospho-N,N-dimethyl-N-(2-hydroxypropyl-3,1-O,O-2-hydroxypropyl-3,1-O,O-dihydroxypropyl)-alkylammonium-Verbindungen

### (A = V; n = 2 - 6; R₃, CH₃; m= 1, x = 0; y = 1; z = 3)

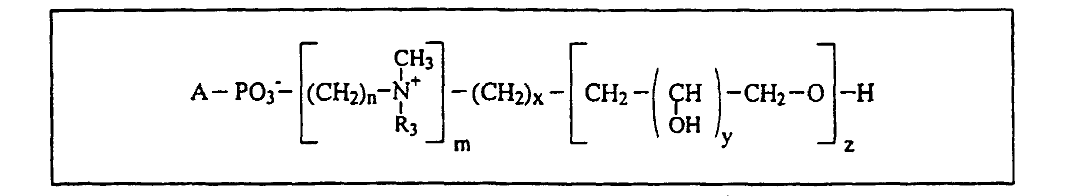

- 1543.): 1-(Z)-10-Docosenoyl-ethylenglykol-phospho-N,N-dimethyl-N-(HP₁-HP₂-diHP₃)-ethylammonium (n = 2)
C₃₇H₇₄NO₁₂P (755.97)
- 1544.): 1-(Z)-6-Octadecenoyl-propandiol-(1,3)-phospho-N,N-dimethyl-N-(HP₁-HP₂-diHP₃)-ethylammonium (n = 2)
C₃₄H₆₈NO₁₂P (713.89)
- 1545.): 1-(Z)-10-Docosenoyl-propandiol-(1,3)-phospho-N,N-dimethyl-N-(HP₁-HP₂-diHP₃)-ethylammonium (n = 2)
C₃₈H₇₆NO₁₂P (769.99)
- 1546.): 1-(Z)-10-Tetracosenoyl-propandiol-(1,3)-phospho-N,N-dimethyl-N-(HP₁-HP₂-diHP₃)-ethylammonium (n = 2)
C₄₀H₈₀NO₁₂P (798.05)
- 1547.): 1-(Z,Z)-5,11-Octadecadienoyl-propandiol-(1,3)-phospho-N,N-dimethyl-N-(HP₁-HP₂-diHP₃)-ethylammonium (n = 2)
C₃₄H₆₆NO₁₂P (711.89)
- 1548.): 1-(Z,Z)-10,16-Eicosadienoyl-propandiol-(1,3)-phospho-N,N-dimethyl-N-(HP₁-HP₂-diHP₃)-ethylammonium (n = 2)
C₃₆H₇₀NO₁₂P (739.93)
- 1549.): 1-(Z,Z)-10,16-Docosadienoyl-propandiol-(1,3)-phospho-N,N-dimethyl-N-(HP₁-HP₂-diHP₃)-ethylammonium (n = 2)
C₃₈H₇₄NO₁₂P (767.98)
- 1550.): 1-(Z,Z)-6,18-Hexacosadienoyl-propandiol-(1,3)-phospho-N,N-dimethyl-N-(HP₁-HP₂-diHP₃)-ethylammonium (n = 2)
C₄₂H₈₂NO₁₂P (824.09)
- 1551.): 1-(Z)-10-Docosenoyl-propandiol-(1,3)-phospho-N,N-dimethyl-N-(HP₁-HP₂-diHP₃)-propylammonium (n = 3)
C₃₉H₇₈NO₁₂P (784.01)
- 1552.): 1-(Z)-10-Docosenoyl-propandiol-(1,3)-phospho-N,N-dimethyl-N-(HP₁-HP₂-diHP₃)-butylammonium (n = 4)
C₄₀H₈₀NO₁₂P (798.04)
- 1553.): 1-(Z)-10-Docosenoyl-butandiol-(1,4)-phospho-N,N-dimethyl-N-(HP₁-HP₂-diHP₃)-propylammonium (n = 3)
C₄₀H₈₀NO₁₂P (798.04)
- 1554.): 1-(Z)-10-Docosenoyl-hexandiol-(1,6)-phospho-N,N-dimethyl-N-(HP₁-HP₂-diHP₃)-propylammonium (n = 3)
C₄₂H₈₄NO₁₂P (826.10)
- 1555.): 1-(Z)-10-Docosenoyl-octandiol-(1,8)-phospho-N,N-dimethyl-N-(HP₁-HP₂-diHP₃)-propylammonium (n = 3)
C₄₄H₈₈NO₁₂P (854.16)

### 10. Beispiele für Alkandiol-phospho-Verbindungen, die nicht am Stickstoff hydroxyliert sind

### (A = V; n = 2 - 6; R₃, CH₃; m= 1, x = 1; z = 0)

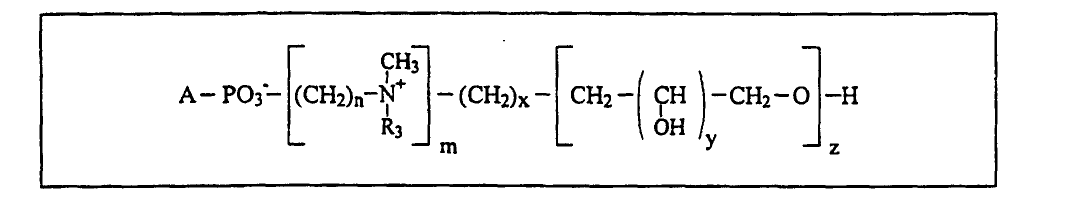

- 1556.): 1-(Z)-10-Docosenoyl-ethylenglykol-phospho-N,N,N-trimethyl-propylammonium (n = 3)
C₃₀H₆₀NO₆P (561.78)
- 1557.): 1-(Z)-6-Octadecenoyl-propandiol-(1,3)-phospho-N,N,N-trimethyl-ethylammonium (n = 2)
C₂₆H₅₂NO₆P (505.68)
- 1558.): 1-(Z)-10-Docosenoyl-propandial-(1,3)-phospho-N,N,N-trimethyl-ethylammonium (n = 2)
C₃₀H₆₀NO₆P (561.78)
- 1559.): 1-(Z)-10-Tetracosenoyl-propandiol-(1,3)-phospho-N,N,N-trimethyl-propylammonium (n = 3)
C₃₃H₆₆NO₆P (603.86)
- 1560.): 1-(Z,Z)-5,11-Octadecadienoyl-propandiol-(1,3)-phospho-N,N,N-trimethyl-propylammonium (n = 3)
C₂₇H₅₂NO₆P (517.69)
- 1561.): 1-(Z,Z)-10,16-Eicosadienoyl-propandiol-(1,3)-phospho-N,N,N-trimethyl-propylammonium (n = 3)
C₂₉H₅₆NO₆P (545.74)
- 1562.): 1-(Z,Z)-10,16-Docosadienoyl-propandiol-(1,3)-phospho-N,N,N-trimethyl-propylammonium (n = 3)
C₃₁H₆₀NO₆P (573.79)
- 1563.): 1-(Z,Z)-6,18-Hexacosadienoyl-propandiol-(1,3)-phospho-N,N,N-trimethyl-propylammonium (n = 3)
C₃₅H₆₈NO₆P (629.90)
- 1564.): 1-(Z)-10-Docosenoyl-propandiol-(1,3)-phospho-N,N,N-trimethyl-propylammonium (n = 3)
C₃₁H₆₂NO₆P (575.81)
- 1565.): 1-(Z)-10-Docosenoyl-propandiol-(1,3)-phospho-N,N,N-trimethyl-butylammonium (n = 4)
C₃₂H₆₄NO₆P (589.84)
- 1566.): 1-(Z)-10-Docosenoyl-butandiol-(1,4)-phospho-N,N,N-trimethyl-propylammonium (n = 3)
C₃₂H₆₄NO₆P (589.84)
- 1567.): 1-(Z)-10-Docosenoyl-hexandiol-(1,6)-phospho-N,N,N-trimethyl-propylammonium (n = 3)
C₃₄H₆₈NO₆P (617.89)
- 1568.): 1-(Z)-10-Docosenoyl-octandiol-(1,8)-phospho-N,N,N-trimethyl-propylammonium (n = 3)
C₃₆H₇₂NO₆P (645.94)

### Liposomenbestandteile

### Neutrale Phospholipide

### 1. Beispiele für zweikettige Glycero-phospho-N,N-dimethyl-N-dihydroxypropylalkylammonium-Verbindungen

### (A = III; n = 2 - 6; R₃, CH₃; m= 1, x = 0; y = 1; z = 1)

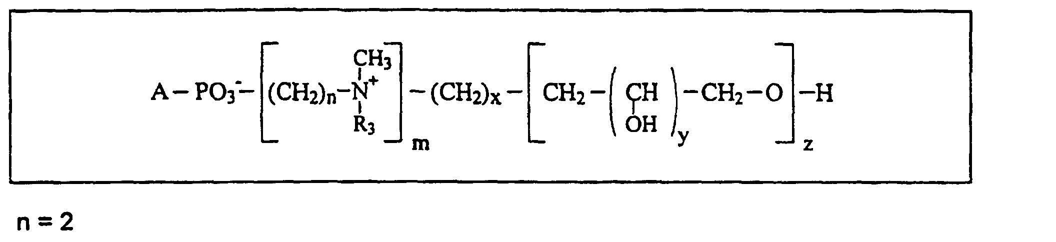

- 1569.): 1,2-Di-(Z)-6-Hexadecenoyl-*sn*-glycero-3-phospho-N,N-dimethyl-N-dihydroxypropyl-ethylammonium (n = 2)
C₄₂H₈₀NO₁₀P (790.07)
- 1570.): 1,2-Di-(Z)-10-Heptadecenoyl-*sn*-glycero-3-phospho-N,N-dimethyl-N-dihydroxypropyl-ethylammonium (n = 2)
C₄₄H₈₄NO₁₀P (818.13)
- 1571.): 1,2-Di-(Z)-6-Octadecenoyl-*sn*-glycero-3-phospho-N,N-dimethyl-N-dihydroxypropyl-ethylammonium (n = 2)
C₄₆H₈₈NO₁₀P (846.18)
- 1572.): 1,2-Di-(Z)-6-Nonadecenoyl-*sn*-glycero-3-phospho-N,N-dimethyl-N-dihydroxypropyl-ethylammonium (n = 2)
C₄₈H₉₂NO₁₀P (874.23)
- 1573.): 1,2-Di-(Z)-12-Eicosenoyl-*sn*-glycero-3-phospho-N,N-dimethyl-N-dihydroxypropyl-ethylammonium (n = 2)
C₅₀H₉₆NO₁₀P (902.29)
- 1574.): 1,2-Di-(Z)-10-Heneicosenoyl-*sn*-glycero-3-phospho-N,N-dimethyl-N-dihydroxypropyl-ethylammonium (n = 2)
C₅₂H₁₀₀NO₁₀P (930.34)
- 1575.): 1,2-Di-(Z)-10-Docosenoyl-*sn*-glycero-3-phospho-N,N-dimethyl-N-dihydroxypropyl-ethylammonium (n = 2)
C₅₄H₁₀₄NO₁₀P (958.39)
- 1576.): 1,2-Di-(Z)-12-Docosenoyl-sn-glycero-3-phospho-N,N-dimethyl-N-dihydroxypropyl-ethylammonium (n = 2)
C₅₄H₁₀₄NO₁₀P (958.39)
- 1577.): 1,2-Di-(Z)-10-Tricosenoyl-sn-glycero-3-phospho-N,N-dimethyl-N-dihydroxypropyl-ethylammonium (n = 2)
C₅₆H₁₀₈NO₁₀P (986.45)
- 1578.): 1,2-Di-(Z)-10-Tetracosenoyl-*sn*-glycero-3-phospho-N,N-dimethyl-N-dihydroxypropyl-ethylammonium (n = 2)
C₅₈H₁₁₂NO₁₀P (1014.50)
- 1579.): 1,2-Di-(Z)-15-Pentacosenoyl-*sn*-glycero-3-phospho-N,N-dimethyl-N-dihydroxypropyl-ethylammonium (n = 2)
C₆₀H₁₁₆NO₁₀P (1042.56)
- 1580.): 1,2-Di-(Z)-16-Hexacosenoyl-sn-glycero-3-phospho-N,N-dimethyl-N-dihydroxypropyl-ethylammonium (n = 2)
C₆₂H₁₂₀NO₁₀P (1070.61)
- 1581.): 1,2-Di-(Z,Z)-5,11-Hexadecadienoyl-*sn*-glycero-3-phospho-N,N-dimethyl-N-dihydroxypropyl-ethylammonium (n = 2)
C₄₂H₇₆NO₁₀P (786.04)
- 1582.): 1,2-Di-(Z,Z)-5,11-Heptadecadienoyl-*sn*-glycero-3-phospho-N,N-dimethyl-N-dihydroxypropyl-ethylammonium (n = 2)
C₄₄H₈₀NO₁₀P (814.09)
- 1583.): 1,2-Di-(Z,Z)-5,11-Octadecadienoyl-sn-glycero-3-phospho-N,N-dimethyl-N-dihydroxypropyl-ethylammonium (n = 2)
C₄₆H₈₄NO₁₀P (842.15)
- 1584.): 1,2-Di-(Z,Z)-6,12-Nonadecadienoyl-*sn*-glycero-3-phospho-N,N-dimethyl-N-dihydroxypropyl-ethylammonium (n = 2)
C₄₈H₈₈NO₁₀P (870.20)
- 1585.): 1,2-Di-(Z,Z)-10,16-Eicosadienoyl-sn-glycero-3-phospho-N,N-dimethyl-N-dihydroxypropyl-ethylammonium (n = 2)
C₅₀H₉₂NO₁₀P (898.25)
- 1586.): 1,2-Di-(Z,Z)-10,16-Heneicosadienoyl-sn-glycero-3-phospho-N,N-dimethyl-N-dihydroxypropyl-ethylammonium (n = 2)
C₅₂H₉₆NO₁₀P (926.31)
- 1587.): 1,2-Di-(Z,Z)-10,16-Docosadienoyl-*sn*-glycero-3-phospho-N,N-dimethyl-N-dihydroxypropyl-ethylammonium (n = 2)
C₅₄H₁₀₀NO₁₀P (955.36)
- 1588.): 1,2-Di-(Z,Z)-10,16-Tricosadienoyl-*sn*-glycero-3-phospho-N,N-dimethyl-N-dihydroxypropyl-ethylammonium (n = 2)
C₅₆H₁₀₄NO₁₀P (982.42)
- 1589.): 1,2-Di-(Z,Z)-6,18-Tetracosadienoyl-*sn*-glycero-3-phospho-N,N-dimethyl-N-dihydroxypropyl-ethylammonium (n = 2)
C₅₈H₁₀₈NO₁₀P (1010.47)
- 1590.): 1,2-Di-(Z,Z)-10,16-Pentacosadienoyl-*sn*-glycero-3-phospho-N,N-dimethyl-N-dihydroxypropyl-ethylammonium (n = 2)
C₆₀H₁₁₂NO₁₀P (1038.52)
- 1591.): 1,2-Di-(Z,Z)-6,18-Hexacosadienoyl-*sn*-glycero-3-phospho-N,N-dimethyl-N-dihydroxypropyl-ethylammonium (n = 2)
C₆₂H₁₁₆NO₁₀P (1066.58)
- 1592.): 2-(Z)-6-Hexadecenoyl-1-stearoyl-*sn*-glycero-3-phospho-N,N-dimethyl-N-dihydroxypropyl-ethylammonium (n = 2)
C₄₄H₈₆NO₁₀P (820.14)
- 1593.): 2-(Z)-10-Octadecenoyl-1-stearoyl-*sn*-glycero-3-phospho-N,N-dimethyl-N-dihydroxypropyl-ethylammonium (n = 2)
C₄₆H₉₀NO₁₀P (848.20)
- 1594.): 2-(Z)-10-Eicosenoyl-1-stearoyl-*sn*-glycero-3-phospho-N,N-dimethyl-N-dihydroxypropyl-ethylammonium (n = 2)
C₄₈H₉₄NO₁₀P (876.25)
- 1595.): 1-Behenyl-2-(Z)-10-docosenoyl-sn-glycero-3-phospho-N,N-dimethyl-N-dihydroxypropyl-ethylammonium (n = 2)
C₅₂H₁₀₂NO₁₀P (932.36)
- 1596.): 2-(Z,Z)-6,12-Hexadecadienoyl-1-stearoyl-*sn*-glycero-3-phospho-N,N-dimethyl-N-dihydroxypropyl-ethylammonium (n = 2)
C₄₄H₈₄O₁₀P (818.13)
- 1597.): 2-(Z,Z)-10,16-Docosadienoyl-1-stearoyl-*sn*-glycero-3-phospho-N,N-dimethyl-N-dihydroxypropyl-ethylammonium (n = 2)
C₅₀H₉₆NO₁₀P (902.29)
- 1598.): 1-Stearoyl-2-(Z,Z)-6,18-tetracosadienoyl-sn-glycero-3-phospho-N,N-dimethyl-N-dihydroxypropyl-ethylammonium (n = 2)
C₅₂H₁₀₀NO₁₀P (930.34)
- 1599.): 1-(Z)-10-Octadecenoyl-2-stearoyl-*sn*-glycero-3-phospho-N,N-dimethyl-N-dihydroxypropyl-ethylammonium (n = 2)
C₄₆H₉₀NO₁₀P (848.20)
- 1600.): 1-(Z,Z)-6,18-Hexacosadienoyl-2-stearoyl-sn-glycero-3-phospho-N,N-dimethyl-N-dihydroxypropyl-ethylammonium (n = 2)
C₅₄H₁₀₄NO₁₀P (958.39)
- 1601.): 1-(Z,Z)-6,18-Hexacosadienoyl-2-(Z)-6-Hexadecenoyl-*sn*-glycero-3-phospho-N,N-dimethyl-N-dihydroxypropyl-ethylammonium (n = 2)
C₅₂H₉₈NO₁₀P (928.32)
- 1602.): 2-(Z,Z)-6,18-Hexacosadienoyl-1-(Z)-6-Hexadecenoyl-*sn*-glycero-3-phospho-N,N-dimethyl-N-dihydroxypropyl-ethylammonium (n = 2)
C₅₂H₉₈NO₁₀P (928.32)
n=3
- 1603.): 1,2-Di-(Z)-6-Hexadecenoyl-*sn*-glycero-3-phospho-N,N-dimethyl-N-dihydroxypropyl-propylammonium (n = 3)
C₄₃H₈₂NO₁₀P (804.10)
- 1604.): 1,2-Di-(Z)-10-Heptadecenoyl-*sn*-glycero-3-phospho-N,N-dimethyl-N-dihydroxypropyl-propylammonium (n = 3)
C₄₅H₈₆NO₁₀P (832.15)
- 1605.): 1,2-Di-(Z)-6-Octadecenoyl-*sn*-glycero-3-phospho-N,N-dimethyl-N-dihydroxypropyl-propylammonium (n = 3)
C₄₇H₉₀NO₁₀P (860.21)
- 1606.): 1,2-Di-(Z)-12-Eicosenoyl-*sn*-glycero-3-phospho-N,N-dimethyl-N-dihydroxypropyl-propylammonium (n = 3)
C₅₁H₉₈NO₁₀P (916.31)
- 1607.): 1,2-Di-(2)-10-Docosenoyl-*sn*-glycero-3-phospho-N,N-dimethyl-N-dihydroxypropyl-propylammonium (n = 3)
C₅₅H₁₀₆NO₁₀P (972.42)
- 1608.): 1,2-Di-(Z)-12-Docosenoyl-*sn*-glycero-3-phospho-N,N-dimethyl-N-dihydroxypropyl-propylammonium (n = 3)
C₅₅H₁₀₆NO₁₀P (972.42)
- 1609.): 1,2-Di-(Z)-10-Tricosenoyl-*sn*-glycero-3-phospho-N,N-dimethyl-N-dihydroxypropyl-propylammonium (n = 3)
C₅₇H₁₁₀NO₁₀P (1000.47)
- 1610.): 1,2-Di-(Z)-10-Tetracosenoyl-*sn*-glycero-3-phospho-N,N-dimethyl-N-dihydroxypropyl-propylammonium (n = 3)
C₅₉H₁₁₄NO₁₀P (1028.53)
- 1611.): 1,2-Di-(Z,Z)-5,11-Octadecadienoyl-*sn*-glycero-3-phospho-N,N-dimethyl-N-dihydroxypropyl-propylammonium (n = 3)
C₄₇H₈₆NO₁₀P (856.17)
- 1612.): 1,2-Di-(Z,Z)-10,16-Eicosadienoyl-*sn*-glycero-3-phospho-N,N-dimethyl-N-dihydroxypropyl-propylammonium (n = 3)
C₅₁H₉₄NO₁₀P (912.28)
- 1613.): 1,2-Di-(Z,Z)-10,16-Docosadienoyl-*sn*-glycero-3-phospho-N,N-dimethyl-N-dihydroxypropyl-propylammonium (n = 3)
C₅₅H₁₀₂NO₁₀P (968.39)
- 1614.): 1,2-Di-(Z,Z)-6,18-Hexacosadienoyl-*sn*-glycero-3-phospho-N,N-dimethyl-N-dihydroxypropyl-propylammonium (n = 3)
C₆₃H₁₁₈NO₁₀P (1080.60)
- 1615.): 2-(Z)-6-Hexadecenoyl-1-stearoyl-*sn*-glycero-3-phospho-N,N-dimethyl-N-dihydroxypropyl-propylammonium (n = 3)
C₄₅H₈₈NO₁₀P (834.17)
- 1616.): 2-(Z)-10-Octadecenoyl-1-stearoyl-*sn*-glycero-3-phospho-N,N-dimethyl-N-dihydroxypropyl-propylammonium (n = 3)
C₄₇H₉₂NO₁₀P (862.22)
- 1617.): 2-(Z)-10-Docosenoyl-1-behenyl-*sn*-glycero-3-phospho-N,N-dimethyl-N-dihydroxypropyl-propylammonium (n = 3)
C₅₃H₁₀₄NO₁₀P (946.38)
- 1618.): 2-(Z,Z)-6,12-Hexadecadienoyl-1-stearoyl-sn-glycero-3-phospho-N,N-dimethyl-N-dihydroxypropyl-propylammonium (n = 3)
C₄₅H₈₆NO₁₀P (832.15)
- 1619.): 1-(Z)-10-Octadecenoyl-2-stearoyl-*sn*-glycero-3-phospho-N,N-dimethyl-N-dihydroxypropyl-propylammonium (n = 3)
C₄₇H₉₂NO₁₀P (862.22)
- 1620.): 1-(Z,Z)-6,18-Hexacosadienoyl-2-stearoyl-*sn*-glycero-3-phospho-N,N-dimethyl-N-dihydroxypropyl-propylammonium (n = 3)
C₅₅H₁₀₆NO₁₀P (972.42)
n=4
- 1621.): 1,2-Di-(Z)-6-Octadecenoyl-*sn*-glycero-3-phospho-N,N-dimethyl-N-dihydroxypropyl-butylammonium (n = 4)
C₄₈H₉₂NO₁₀P (874.23)
- 1622.): 1,2-Di-(Z)-10-Docosenoyl-*sn*-glycero-3-phospho-N,N-dimethyl-N-dihydroxypropyl-butylammonium (n = 4)
C₅₆H₁₀₈NO₁₀P (986.45)
- 1623.): 1,2-Di-(Z,Z)-5,11-Hexadecadienoyl-*sn*-glycero-3-phospho-N,N-dimethyl-N-dihydroxypropyl-butylammonium (n = 4)
C₄₄H₈₀NO₁₀P (814.09)
- 1624.): 1,2-Di-(Z,Z)-10,16-Docosadienoyl-*sn*-glycero-3-phospho-N,N-dimethyl-N-dihydroxypropyl-butylammonium (n = 4)
C₅₆H₁₀₄NO₁₀P (982.42)
- 1625.): 1,2-Di-(Z,Z)-6,18-Hexacosadienoyl-sn-glycero-3-phospho-N,N-dimethyl-N-dihydroxypropyl-butylammonium (n = 4)
C₆₄H₁₂₀NO₁₀P (1094.63)
n=6
- 1626.): 1,2-Di-(Z)-6-Octadecenoyl-sn-glycero-3-phospho-N,N-dimethyl-N-dihydroxypropyl-hexylammonium (n = 6)
C₅₀H₉₆NO₁₀P (902.29)
- 1627.): 1,2-Di-(Z)-10-Docosenoyl-*sn*-glycero-3-phospho-N,N-dimethyl-N-dihydroxypropyl-hexylammonium (n = 6)
C₅₈H₁₁₂NO₁₀P (1014.50)
- 1628.): 1,2-Di-(Z,Z)-10,16-Docosadienoyl-*sn*-glycero-3-phospho-N,N-dimethyl-N-dihydroxypropyl-hexylammonium (n = 6)
C₅₈H₁₀₈NO₁₀P (1010.47)
- 1629.): 1,2-Di-(Z,Z)-6,18-Hexacosadienoyl-*sn*-glycero-3-phospho-N,N-dimethyl-N-dihydroxypropyl-hexylammonium (n = 6)
C₆₆H₁₂₄NO₁₀P (1122.69)

### 2. Beispiele für zweikettige Glycero-phospho-N,N-dimethyl-N-(2-hydroxypropyl-3,1-O,O-dihydroxypropyl)-alkylammonium-Verbindungen

### (A = III; n = 2 - 6; R₃, CH₃; m= 1, x = 0; y = 1; z = 2)

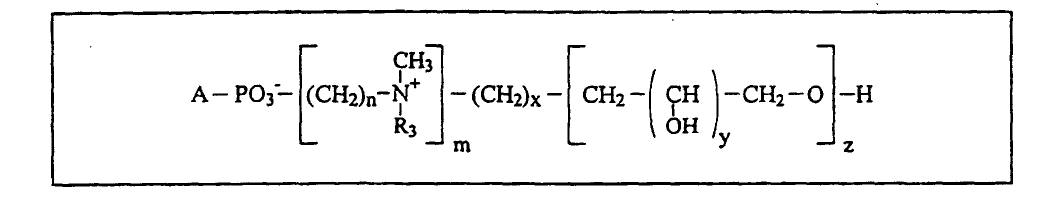

- 1630.): 1,2-Di-(Z)-6-Hexadecenoyl-*sn*-glycero-3-phospho-N,N-dimethyl-N-(2-hydroxypropyl-3,1-O,O-dihydroxypropyl)-ethylammonium (n = 2)
C₄₅H₈₆NO₁₂P (864.15)
- 1631.): 1,2-Di-(Z)-10-Heptadecenoyl-*sn*-glycero-3-phospho-N,N-dimethyl-N-(2-hydroxypropyl-3,1-O,O-dihydroxypropyl)-ethylammonium (n = 2)
C₄₇H₉₀NO₁₂P (892.20)
- 1632.): 1,2-Di-(Z)-6-Octadecenoyl-*sn*-glycero-3-phospho-N,N-dimethyl-N-(2-hydroxypropyl-3,1-O,O-dihydroxypropyl)-ethylammonium (n = 2)
C₄₉H₉₄NO₁₂P (920.26)
- 1633.): 1,2-Di-(Z)-6-Nonadecenoyl-sn-glycero-3-phospho-N,N-dimethyl-N-(2-hydroxypropyl-3,1-O,O-dihydroxypropyl)-ethylammonium (n = 2)
C₅₁H₉₈NO₁₂P (948.31)
- 1634.): 1,2-Di-(Z)-12-Eicosenoyl-sn-glycero-3-phospho-N,N-dimethyl-N-(2-hydroxypropyl-3,1-O,O-dihydroxypropyl)-ethylammonium (n = 2)
C₅₃H₁₀₂NO₁₂P (976.37)
- 1635.): 1,2-Di-(Z)-10-Heneicosenoyl-*sn*-glycero-3-phospho-N,N-dimethyl-N-(2-hydroxypropyl-3,1-O,O-dihydroxypropyl)-ethylammonium (n = 2)
C₅₅H₁₀₆NO₁₂P (1004.42)
- 1636.): 1,2-Di-(Z)-10-Docosenoyl-*sn*-glycero-3-phospho-N,N-dimethyl-N-(2-hydroxypropyl-3,1-O,O-dihydroxypropyl)-ethylammonium (n = 2)
C₅₇H₁₁₀NO₁₂P (1032.47)
- 1637.): 1,2-Di-(Z)-12-Docosenoyl-*sn*-glycero-3-phospho-N,N-dimethyl-N-(2-hydroxypropyl-3,1-O,O-dihydroxypropyl)-ethylammonium (n = 2)
C₅₇H₁₁₀NO₁₂P (1032.47)
- 1638.): 1,2-Di-(Z)-10-Tricosenoyl-*sn*-glycero-3-phospho-N,N-dimethyl-N-(2-hydroxypropyl-3,1-O,O-dihydroxypropyl)-ethylammonium (n = 2)
C₅₉H₁₁₄NO₁₂P (1060.53)
- 1639.): 1,2-Di-(Z)-10-Tetracosenoyl-*sn*-glycero-3-phospho-N,N-dimethyl-N-(2-hydroxypropyl-3,1-O,O-dihydroxypropyl)-ethylammonium (n = 2)
C₆₁H₁₁₈NO₁₂P (1088.58)
- 1640.): 1,2-Di-(Z)-15-Pentacosenoyl-*sn*-glycero-3-phospho-N,N-dimethyl-N-(2-hydroxypropyl-3,1-O,O-dihydroxypropyl)-ethylammonium (n = 2)
C₆₃H₁₂₂NO₁₂P (1116.63)
- 1641.): 1,2-Di-(Z)-16-Hexacosenoyl-*sn*-glycero-3-phospho-N,N-dimethyl-N-(2-hydroxypropyl-3,1-O,O-dihydroxypropyl)-ethylammonium (n = 2)
C₆₅H₁₂₆NO₁₂P (1144.69)
- 1642.): 1,2-Di-(Z,Z)-5,11-Hexadecadienoyl-*sn*-glycero-3-phospho-N,N-dimethyl-N-(2-hydroxypropyl-3,1-O,O-dihydroxypropyl)-ethylammonium (n = 2)
C₄₅H₈₂NO₁₂P (860.12)
- 1643.): 1,2-Di-(Z,Z)-5,11-Heptadecadienoyl-*sn*-glycero-3-phospho-N,N-dimethyl-N-(2-hydroxypropyl-3,1-O,O-dihydroxypropyl)-ethylammonium (n = 2)
C₄₇H₈₆NO₁₂P (888.17)
- 1644.): 1,2-Di-(Z,Z)-5,11-Octadecadienoyl-*sn*-glycero-3-phospho-N,N-dimethyl-N-(2-hydroxypropyl-3,1-O,O-dihydroxypropyl)-ethylammonium (n = 2)
C₄₉H₉₀NO₁₂P (916.23)
- 1645.): 1,2-Di-(Z,Z)-6,12-Nonadecadienoyl-*sn*-glycero-3-phospho-N,N-dimethyl-N-(2-hydroxypropyl-3,1-O,O-dihydroxypropyl)-ethylammonium (n = 2)
C₅₁H₉₄NO₁₂P (944.28)
- 1646.): 1,2-Di-(Z,Z)-10,16-Eicosadienoyl-*sn*-glycero-3-phospho-N,N-dimethyl-N-(2-hydroxypropyl-3,1-O,O-dihydroxypropyl)-ethylammonium (n = 2)
C₅₃H₉₈NO₁₂P (972.33)
- 1647.): 1,2-Di-(Z,Z)-10,16-Heneicosadienoyl-*sn*-glycero-3-phospho-N,N-dimethyl-N-(2-hydroxypropyl-3,1-O,O-dihydroxypropyl)-ethylammonium (n = 2)
C₅₅H₁₀₂NO₁₂P (1000.39)
- 1648.): 1,2-Di-(Z,Z)-10,16-Docosadienoyl-*sn*-glycero-3-phospho-N,N-dimethyl-N-(2-hydroxypropyl-3,1-O,O-dihydroxypropyl)-ethylammonium (n = 2)
C₅₇H₁₀₆NO₁₂P (1028.44)
- 1649.): 1,2-Di-(Z,Z)-10,16-Tricosadienoyl-*sn*-glycero-3-phospho-N,N-dimethyl-N-(2-hydroxypropyl-3,1-O,O-dihydroxypropyl)-ethylammonium (n = 2)
C₅₉H₁₁₀NO₁₂P (1056.50)
- 1650.): 1,2-Di-(Z,Z)-6,18-Tetracosadienoyl-*sn*-glycero-3-phospho-N,N-dimethyl-N-(2-hydroxypropyl-3,1-O,O-dihydroxypropyl)-ethylammonium (n = 2)
C₆₁H₁₁₄NO₁₂P (1084.55)
- 1651.): 1,2-Di-(Z,Z)-10,16-Pentacosadienoyl-*sn*-glycero-3-phospho-N,N-dimethyl-N-(2-hydroxypropyl-3,1-O,O-dihydroxypropyl)-ethylammonium (n = 2)
C₆₃H₁₁₈NO₁₂P (1112.60)
- 1652.): 1,2-Di-(Z,Z)-6,18-Hexacosadienoyl-*sn*-glycero-3-phospho-N,N-dimethyl-N-(2-hydroxypropyl-3,1-O,O-dihydroxypropyl)-ethylammonium (n = 2)
C₆₅H₁₂₂NO₁₂P (1140.66)
- 1653.): 2-(Z)-6-Hexadecenoyl-1-stearoyl-*sn*-glycero-3-phospho-N,N-dimethyl-N-(2-hydroxypropyl-3,1-O,O-dihydroxypropyl)-ethylammonium (n = 2)
C₄₇H₉₂NO₁₂P (894.22)
- 1654.): 2-(Z)-10-Octadecenoyl-1-stearoyl-*sn*-glycero-3-phospho-N,N-dimethyl-N-(2-hydroxypropyl-3,1-O,O-dihydroxypropyl)-ethylammonium (n = 2)
C₄₉H₉₆NO₁₂P (922.27)
- 1655.): 2-(Z)-10-Eicosenoyl-1-stearoyl-*sn*-glycero-3-phospho-N,N-dimethyl-N-(2-hydroxypropyl-3,1-O,O-dihydroxypropyl)-ethylammonium (n = 2)
C₅₁H₁₀₀NO₁₂P (950.33)
- 1656.): 1-Behenyl-2-(Z)-10-docosenoyl-*sn*-glycero-3-phospho-N,N-dimethyl-N-(2-hydroxypropyl-3,1-O,O-dihydroxypropyl)-ethylammonium (n = 2)
C₅₅H₁₀₈NO₁₂P (1006.44)
- 1657.): 2-(Z,Z)-6,12-Hexadecadienoyl-1-stearoyl-*sn*-glycero-3-phospho-N,N-dimethyl-N-(2-hydroxypropyl-3,1-O,O-dihydroxypropyl)-ethylammonium (n = 2)
C₄₇H₉₀NO₁₂P (892.20)
- 1658.): 2-(Z,Z)-10,16-Docosadienoyl-1-stearoyl-*sn*-glycero-3-phospho-N,N-dimethyl-N-(2-hydroxypropyl-3,1-O,O-dihydroxypropyl)-ethylammonium (n = 2)
C₅₃H₁₀₂NO₁₂P (976.37)
- 1659.): 1-Stearoyl-2-(Z,Z)-6,18-tetracosadienoyl-*sn*-glycero-3-phospho-N,N-dimethyl-N-(2-hydroxypropyl-3,1-O,O-dihydroxypropyl)-ethylammonium (n = 2)
C₅₅H₁₀₆NO₁₂P (1004.42)
- 1660.): 1-(Z)-10-Octadecenoyl-2-stearoyl-*sn*-glycero-3-phospho-N,N-dimethyl-N-(2-hydroxypropyl-3,1-O,O-dihydroxypropyl)-ethylammonium (n = 2)
C₄₉H₉₆NO₁₂P (922.27)
- 1661.): 1-(Z,Z)-6,18-Hexacosadienoyl-2-stearoyl-sn-glycero-3-phospho-N,N-dimethyl-N-(2-hydroxypropyl-3,1-O,O-dihydroxypropyl)-ethylammonium (n = 2)
C₅₇H₁₁₀NO₁₂P (1032.47)
- 1662.): 1-(Z,Z)-6,18-Hexacosadienoyl-2-(Z)-6-Hexadecenoyl-sn-glycero-3-phospho-N,N-dimethyl-N-(2-hydroxypropyl-3,1-O,O-dihydroxypropyl)-ethylammonium (n = 2)
C₅₅H₁₀₄NO₁₂P (1002.40)
- 1663.): 2-(Z,Z)-6,18-Hexacosadienoyl-1-(Z)-6-Hexadecenoyl-*sn*-glycero-3-phospho-N,N-dimethyl-N-(2-hydroxypropyl-3,1-O,O-dihydroxypropyl)-ethylammonium (n = 2)
C₅₅H₁₀₄NO₁₂P (1002.40)
n=3
- 1664.): 1,2-Di-(Z)-6-Hexadecenoyl-*sn*-glycero-3-phospho-N,N-dimethyl-N-(2-hydroxypropyl-3,1-O,O-dihydroxypropyl)-propylammonium (n = 3)
C₄₆H₈₈NO₁₂P (878.18)
- 1665.): 1,2-Di-(Z)-10-Heptadecenoyl-*sn*-glycero-3-phospho-N,N-dimethyl-N-(2-hydroxypropyl-3,1-O,O-dihydroxypropyl)-propylammonium (n = 3)
C₄₈H₉₂NO₁₂P (906.23)
- 1666.): 1,2-Di-(Z)-6-Octadecenoyl-*sn*-glycero-3-phospho-N,N-dimethyl-N-(2-hydroxypropyl-3,1-O,O-dihydroxypropyl)-propylammonium (n = 3)
C₅₀H₉₆NO₁₂P (934.29)
- 1667.): 1,2-Di-(Z)-12-Eicosenoyl-*sn*-glycero-3-phospho-N,N-dimethyl-N-(2-hydroxypropyl-3,1-O,O-dihydroxypropyl)-propylammonium (n = 3)
C₅₄H₁₀₄NO₁₂P (990.39)
- 1668.): 1,2-Di-(Z)-10-Docosenoyl-*sn*-glycero-3-phospho-N,N-dimethyl-N-(2-hydroxypropyl-3,1-O,O-dihydroxypropyl)-propylammonium (n = 3)
C₅₈H₁₁₂NO₁₂P (1046.50)
- 1669.): 1,2-Di-(Z)-12-Docosenoyl-*sn*-glycero-3-phospho-N,N-dimethyl-N-(2-hydroxypropyl-3,1-O,O-dihydroxypropyl)-propylammonium (n = 3)
C₅₈H₁₁₂NO₁₂P (1046.50)
- 1670.): 1,2-Di-(Z)-10-Tricosenoyl-*sn*-glycero-3-phospho-N,N-dimethyl-N-(2-hydroxypropyl-3,1-O,O-dihydroxypropyl)-propylammonium (n = 3)
C₆₀H₁₁₆NO₁₂P (1074.55)
- 1671.): 1,2-Di-(2)-10-Tetracosenoyl-*sn*-glycero-3-phospho-N,N-dimethyl-N-(2-hydroxypropyl-3,1-O,O-dihydroxypropyl)-propylammonium (n = 3)
C₆₂H₁₂₀NO₁₂P (1102.61)
- 1672.): 1,2-Di-(Z,Z)-5,11-Octadecadienoyl-*sn*-glycero-3-phospho-N,N-dimethyl-N-(2-hydroxypropyl-3,1-O,O-dihydroxypropyl)-propylammonium (n = 3)
C₅₀H₉₂NO₁₂P (930.25)
- 1673.): 1,2-Di-(Z,Z)-10,16-Eicosadienoyl-*sn*-glycero-3-phospho-N,N-dimethyl-N-(2-hydroxypropyl-3,1-O,O-dihydroxypropyl)-propylammonium (n = 3)
C₅₄H₁₀₀NO₁₂P (986.36)
- 1674.): 1,2-Di-(Z,Z)-10,16-Docosadienoyl-*sn*-glycero-3-phospho-N,N-dimethyl-N-(2-hydroxypropyl-3,1-O,O-dihydroxypropyl)-propylammonium (n = 3)
C₅₈H₁₀₈NO₁₂P (1042.47)
- 1675.): 1,2-Di-(Z,Z)-6,18-Hexacosadienoyl-*sn*-glycero-3-phospho-N,N-dimethyl-N-(2-hydroxypropyl-3,1-O,O-dihydroxypropyl)-propylammonium (n = 3)
C₆₆H₁₂₄NO₁₂P (1154.68)
- 1676.): 2-(Z)-6-Hexadecenoyl-1-stearoyl-*sn*-glycero-3-phospho-N,N-dimethyl-N-(2-hydroxypropyl-3,1-O,O-dihydroxypropyl)-propylammonium (n = 3)
C₄₈H₉₄NO₁₂P (908.25)
- 1677.): 2-(Z)-10-Octadecenoyl-1-stearoyl-*sn*-glycero-3-phospho-N,N-dimethyl-N-(2-hydroxypropyl-3,1-O,O-dihydroxypropyl)-propylammonium (n = 3)
C₅₀H₉₈NO₁₂P (936.30)
- 1678.): 2-(Z)-10-Docosenoyl-1-behenyl-*sn*-glycero-3-phospho-N,N-dimethyl-N-(2-hydroxypropyl-3,1-O,O-dihydroxypropyl)-propylammonium (n = 3)
C₅₆H₁₁₀NO₁₂P (1020.46)
- 1679.): 2-(Z,Z)-6,12-Hexadecadienoyl-1-stearoyl-sn-glycero-3-phospho-N,N-dimethyl-N-(2-hydroxypropyl-3,1-O,O-dihydroxypropyl)-propylammonium (n = 3)
C₄₈H₉₂NO₁₂P (906.23)
- 1680.): 1-(Z)-10-Octadecenoyl-2-stearoyl-*sn*-glycero-3-phospho-N,N-dimethyl-N-(2-hydroxypropyl-3,1-O,O-dihydroxypropyl)-propylammonium (n = 3)
C₅₀H₉₈NO₁₂P (936.30)
- 1681.): 1-(Z,Z)-6,18-Hexacosadienoyl-2-stearoyl-sn-glycero-3-phospho-N,N-dimethyl-N-(2-hydroxypropyl-3,1-O,O-dihydroxypropyl)-propylammonium (n = 3)
C₅₈H₁₁₂NO₁₂P (1046.50)
n=4
- 1682.): 1,2-Di-(Z)-6-Octadecenoyl-*sn*-glycero-3-phospho-N,N-dimethyl-N-(2-hydroxypropyl-3,1-O,O-dihydroxypropyl)-butylammonium (n = 4)
C₅₁H₉₈NO₁₂P (948.31)
- 1683.): 1,2-Di-(Z)-10-Docosenoyl-*sn*-glycero-3-phospho-N,N-dimethyl-N-(2-hydroxypropyl-3,1-O,O-dihydroxypropyl)-butylammonium (n = 4)
C₅₉H₁₁₄NO₁₂P (1060.53)
- 1684.): 1,2-Di-(Z,Z)-5,11-Hexadecadienoyl-*sn*-glycero-3-phospho-N,N-dimethyl-N-(2-hydroxypropyl-3,1-O,O-dihydroxypropyl)-butylammonium (n = 4)
C₄₇H₈₆NO₁₂P (888.17)
- 1685.): 1,2-Di-(Z,Z)-10,16-Docosadienoyl-*sn*-glycero-3-phospho-N,N-dimethyl-N-(2-hydroxypropyl-3,1-O,O-dihydroxypropyl)-butylammonium (n = 4)
C₅₉H₁₁₀NO₁₂P (1056.50)
- 1686.): 1,2-Di-(Z,Z)-6,18-Hexacosadienoyl-*sn*-glycero-3-phospho-N,N-dimethyl-N-(2-hydroxypropyl-3,1-O,O-dihydroxypropyl)-butylammonium (n = 4)
C₆₇H₁₂₆NO₁₂P (1168.71)
n=6
- 1687.): 1,2-Di-(Z)-6-Octadecenoyl-*sn*-glycero-3-phospho-N,N-dimethyl-N-(2-hydroxypropyl-3,1-O,O-dihydroxypropyl)-hexylammonium (n = 6)
C₅₃H₁₀₂NO₁₂P (976.37)
- 1688.): 1,2-Di-(Z)-10-Docosenoyl-*sn*-glycero-3-phospho-N,N-dimethyl-N-(2-hydroxypropyl-3,1-O,O-dihydroxypropyl)-hexylammonium (n = 6)
C₆₁H₁₁₈NO₁₂P (1088.58)
- 1689.): 1,2-Di-(Z,Z)-10,16-Docosadienoyl-*sn*-glycero-3-phospho-N,N-dimethyl-N-(2-hydroxypropyl-3,1-O,O-dihydroxypropyl)-hexylammonium (n = 6)
C₆₁H₁₁₄NO₁₂P (1084.55)
- 1690.): 1,2-Di-(Z,Z)-6,18-Hexacosadienoyl-*sn*-glycero-3-phospho-N,N-dimethyl-N-(2-hydroxypropyl-3,1-O,O-dihydroxypropyl)-hexylammonium (n = 6)
C₆₉H₁₃₀NO₁₂P (1196.76)

### 3. Beispiele für zweikettige Glycero-phospho-N,N-dimethyl-N-(2-hydroxypropyl-3,1-O,O-2-hydroxypropyl-3,1-O,O-dihydroxypropyl)-alkylammonium-Verbindungen

### (A = III; n = 2 - 6; R₃, CH₃; m= 1, x = 0; y = 1; z = 3)

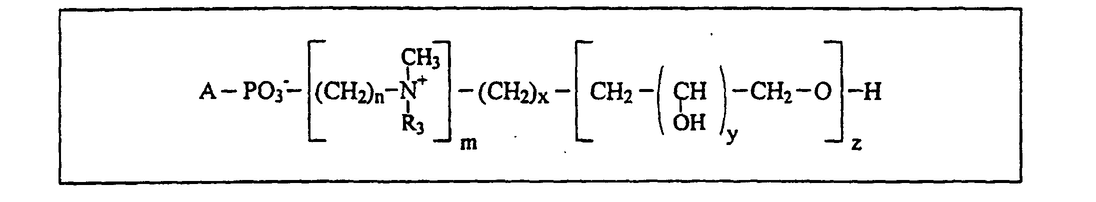

- 1691.): 1,2-Di-(Z)-6-Hexadecenoyl-*sn*-glycero-3-phospho-N,N-dimethyl-N-(HP₁-HP₂-diHP₃)-ethylammonium (n = 2)
C₄₈H₉₂NO₁₄P (938.23)
- 1692.): 1,2-Di-(Z)-10-Heptadecenoyl-*sn*-glycero-3-phospho-N,N-dimethyl-N-(HP₁-HP₂-diHP₃)-ethylammonium (n = 2)
C₅₀H₉₆NO₁₄P (966.28)
- 1693.): 1,2-Di-(Z)-6-Octadecenoyl-*sn*-glycero-3-phospho-N,N-dimethyl-N-(HP₁-HP₂-diHP₃)-ethylammonium (n = 2)
C₅₂H₁₀₀NO₁₄P (994.34)
- 1694.): 1,2-Di-(Z)-6-Nonadecenoyl-*sn*-glycero-3-phospho-N,N-dimethyl-N-(HP₁-HP₂-diHP₃)-ethylammonium (n = 2)
C₅₄H₁₀₄NO₁₄P (1022.39)
- 1695.): 1,2-Di-(Z)-12-Eicosenoyl-*sn*-glycero-3-phospho-N,N-dimethyl-N-(HP₁-HP₂-diHP₃)-ethylammonium (n = 2)
C₅₆H₁₀₈NO₁₄P (1050.45)
- 1696.): 1,2-Di-(Z)-10-Heneicosenoyl-*sn*-glycero-3-phospho-N,N-dimethyl-N-(HP₁-HP₂-diHP₃)-ethylammonium (n = 2)
C₅₈H₁₁₂NO₁₄P (1078.50)
- 1697.): 1,2-Di-(Z)-10-Docosenoyl-*sn*-glycero-3-phospho-N,N-dimethyl-N-(HP₁-HP₂-diHP₃)-ethylammonium (n = 2)
C₆₀H₁₁₆NO₁₄P (1106.55)
- 1698.): 1,2-Di-(Z)-12-Docosenoyl-*sn*-glycero-3-phospho-N,N-dimethyl-N-(HP₁-HP₂-diHP₃)-ethylammonium (n = 2)
C₆₀H₁₁₆NO₁₄P (1106.55)
- 1699.): 1,2-Di-(Z)-10-Tricosenoyl-*sn*-glycero-3-phospho-N,N-dimethyl-N-(HP₁-HP₂-diHP₃)-ethylammonium (n = 2)
C₆₂H₁₂₀NO₁₄P (1134.61)
- 1700.): 1,2-Di-(Z)-10-Tetracosenoyl-*sn*-glycero-3-phospho-N,N-dimethyl-N-(HP₁-HP₂-diHP₃)-ethylammonium (n = 2)
C₆₄H₁₂₄NO₁₄P (1134.61)
- 1701.): 1,2-Di-(Z)-15-Pentacosenoyl-*sn*-glycero-3-phospho-N,N-dimethyl-N-(HP₁-HP₂-diHP₃)-ethylammonium (n = 2)
C₆₆H₁₂₈NO₁₄P (1190.71)
- 1702.): 1,2-Di-(Z)-16-Hexacosenoyl-*sn*-glycero-3-phospho-N,N-dimethyl-N-(HP₁-HP₂-diHP₃)-ethylammonium (n = 2)
C₆₈H₁₃₂NO₁₄P (1218.77)
- 1703.): 1,2-Di-(Z,Z)-5,11-Hexadecadienoyl-*sn*-glycero-3-phospho-N,N-dimethyl-N-(HP₁-HP₂-diHP₃)-ethylammonium (n = 2)
C₄₈H₈₈NO₁₄P (934.20)
- 1704.): 1,2-Di-(Z,Z)-5,11-Heptadecadienoyl-*sn*-glycero-3-phospho-N,N-dimethyl-N-(HP₁-HP₂-diHP₃)-ethylammonium (n = 2)
C₅₀H₉₂NO₁₄P (962.25)
- 1705.): 1,2-Di-(Z,Z)-5,11-Octadecadienoyl-*sn*-glycero-3-phospho-N,N-dimethyl-N-(HP₁-HP₂-diHP₃)-ethylammonium (n = 2)
C₅₂H₉₆NO₁₄P (990.31)
- 1706.): 1,2-Di-(Z,Z)-6,12-Nonadecadienoyl-*sn*-glycero-3-phospho-N,N-dimethyl-N-(HP₁-HP₂-diHP₃)-ethylammonium (n = 2)
C₅₄H₁₀₀NO₁₄P (1018.36)
- 1707.): 1,2-Di-(Z,Z)-10,16-Eicosadienoyl-*sn*-glycero-3-phospho-N,N-dimethyl-N-(HP₁-HP₂-diHP₃)-ethylammonium (n = 2)
C₅₆H₁₀₄NO₁₄P (1046.41)
- 1708.): 1,2-Di-(Z,Z)-10,16-Heneicosadienoyl-*sn*-glycero-3-phospho-N,N-dimethyl-N-(HP₁-HP₂-diHP₃)-ethylammonium (n = 2)
C₅₈H₁₀₈NO₁₄P (1074.47)
- 1709.): 1,2-Di-(Z,Z)-10,16-Docosadienoyl-*sn*-glycero-3-phospho-N,N-dimethyl-N-(HP₁-HP₂-diHP₃)-ethylammonium (n = 2)
C₆₀H₁₁₂NO₁₄P (1102.52)
- 1710.): 1,2-Di-(Z,Z)-10,16-Tricosadienoyl-sn-glycero-3-phospho-N,N-dimethyl-N-(HP₁-HP₂-diHP₃)-ethylammonium (n = 2)
C₆₂H₁₁₆NO₁₄P (1130.58)
- 1711.): 1,2-Di-(Z,Z)-6,18-Tetracosadienoyl-sn-glycero-3-phospho-N,N-dimethyl-N-(HP₁-HP₂-diHP₃)-ethylammonium (n = 2)
C₆₄H₁₂₀NO₁₄P (1158.63)
- 1712.): 1,2-Di-(Z,Z)-10,16-Pentacosadienoyl-*sn*-glycero-3-phospho-N,N-dimethyl-N-(HP₁-HP₂-diHP₃)-ethylammonium (n = 2)
C₆₆H₁₂₄NO₁₄P (1186.68)
- 1713.): 1,2-Di-(Z,Z)-6,18-Hexacosadienoyl-*sn*-glycero-3-phospho-N,N-dimethyl-N-(HP₁-HP₂-diHP₃)-ethylammonium (n = 2)
C₆₈H₁₂₈NO₁₄P (1214.74)
- 1714.): 2-(Z)-6-Hexadecenoyl-1-stearoyl-*sn*-glycero-3-phospho-N,N-dimethyl-N-(HP₁-HP₂-diHP₃)-ethylammonium (n = 2)
C₅₀H₉₈NO₁₄P (968.30)
- 1715.): 2-(Z)-10-Octadecenoyl-1-stearoyl-*sn*-glycero-3-phospho-N,N-dimethyl-N-(HP₁-HP₂-diHP₃)-ethylammonium (n = 2)
C₅₂H₁₀₂NO₁₄P (996.35)
- 1716.): 2-(Z)-10-Eicosenoyl-1-stearoyl-*sn*-glycero-3-phospho-N,N-dimethyl-N-(HP₁-HP₂-diHP₃)-ethylammonium (n = 2)
C₅₄H₁₀₆NO₁₄P (1024.41)
- 1717.): 1-Behenyl-2-(Z)-10-docosenoyl-*sn*-glycero-3-phospho-N,N-dimethyl-N-(HP₁-HP₂-diHP₃)-ethylammonium (n = 2)
C₅₈H₁₁₄NO₁₄P (1080.52)
- 1718.): 2-(Z,Z)-6,12-Hexadecadienoyl-1-stearoyl-sn-glycero-3-phospho-N,N-dimethyl-N-(HP₁-HP₂-diHP₃)-ethylammonium (n = 2)
C₅₀H₉₆NO₁₄P (966.28)
- 1719.): 2-(Z,Z)-10,16-Docosadienoyl-1-stearoyl-*sn*-glycero-3-phospho-N,N-dimethyl-N-(HP₁-HP₂-diHP₃)-ethylammonium (n = 2)
C₅₆H₁₀₈NO₁₄P (1050.45)
- 1720.): 1-Stearoyl-2-(Z,Z)-6,18-tetracosadienoyl-*sn*-glycero-3-phospho-N,N-dimethyl-N-(HP₁-HP₂-diHP₃)-ethylammonium (n = 2)
C₅₈H₁₁₂NO₁₄P (1078.50)
- 1721.): 1-(Z)-10-Octadecenoyl-2-stearoyl-*sn*-glycera-3-phospho-N,N-dimethyl-N-(HP₁-HP₂-diHP₃)-ethylammonium (n = 2)
C₅₂H₁₀₂NO₁₄P (996.35)
- 1722.): 1-(Z,Z)-6,18-Hexacosadienoyl-2-stearoyl-sn-glycero-3-phospho-N,N-dimethyl-N-(HP₁-HP₂-diHP₃)-ethylammonium (n = 2)
C₆₀H₁₁₆NO₁₄P (1106.55)
- 1723.): 1-(Z,Z)-6,18-Hexacosadienoyl-2-(Z)-6-Hexadecenoyl-*sn*-glycero-3-phospho-N,N-dimethyl-N-(HP₁-HP₂-diHP₃)-ethylammonium (n = 2)
C₅₈H₁₁₀NO₁₄P (1076.48)
- 1724.): 2-(Z,Z)-6,18-Hexacosadienoyl-1-(Z)-6-Hexadecenoyl-*sn*-glycero-3-phospho-N,N-dimethyl-N-(HP₁-HP₂-diHP₃)-ethylammonium (n = 2)
C₅₈H₁₁₀NO₁₄P (1076.48)
n=3
- 1725.): 1,2-Di-(Z)-6-Hexadecenoyl-*sn*-glycero-3-phospho-N,N-dimethyl-N-(HP₁-HP₂-diHP₃)-propylammonium (n = 3)
C₄₉H₉₄NO₁₄P (952.26)
- 1726.): 1,2-Di-(Z)-10-Heptadecenoyl-*sn*-glycero-3-phospho-N,N-dimethyl-N-(HP₁-HP₂-diHP₃)-propylammonium (n = 3)
C₅₁H₉₈NO₁₄P (980.31)
- 1727.): 1,2-Di-(Z)-6-Octadecenoyl-*sn*-glycero-3-phospho-N,N-dimethyl-N-(HP₁-HP₂-diHP₃)-propylammonium (n = 3)
C₅₃H₁₀₂NO₁₄P (1008.36)
- 1728.): 1,2-Di-(Z)-12-Eicosenoyl-*sn*-glycero-3-phospho-N,N-dimethyl-N-(HP₁-HP₂-diHP₃)-propylammonium (n = 3)
C₅₇H₁₁₀NO₁₄P (1064.47)
- 1729.): 1,2-Di-(Z)-10-Docosenoyl-*sn*-glycero-3-phospho-N,N-dimethyl-N-(HP₁-HP₂-diHP₃)-propylammonium (n = 3)
C₆₁H₁₁₈NO₁₄P (1120.58)
- 1730.): 1,2-Di-(Z)-12-Docosenoyl-*sn*-glycero-3-phospho-N,N-dimethyl-N-(HP₁-HP₂-diHP₃)-propylammonium (n = 3)
C₆₁H₁₁₈NO₁₄P (1120.58)
- 1731.): 1,2-Di-(Z)-10-Tricosenoyl-*sn*-glycero-3-phospho-N,N-dimethyl-N-(HP₁-HP₂-diHP₃)-propylammonium (n = 3)
C₆₃H₁₂₂NO₁₄P (1148.63)
- 1732.): 1,2-Di-(Z)-10-Tetracosenoyl-*sn*-glycero-3-phospho-N,N-dimethyl-N-(HP₁-HP₂-diHP₃)-propylammonium (n = 3)
C₆₅H₁₂₆NO₁₄P (1176.69)
- 1733.): 1,2-Di-(Z,Z)-5,11-Octadecadienoyl-*sn*-glycero-3-phospho-N,N-dimethyl-N-(HP₁-HP₂-diHP₃)-propylammonium (n = 3)
C₅₃H₉₈NO₁₄P (1004.33)
- 1734.): 1,2-Di-(Z,Z)-10,16-Eicosadienoyl-*sn*-glycero-3-phospho-N,N-dimethyl-N-(HP₁-HP₂-diHP₃)-propylammonium (n = 3)
C₅₇H₁₀₆NO₁₄P (1060.44)
- 1735.): 1,2-Di-(Z,Z)-10,16-Docosadienoyl-*sn*-glycero-3-phospho-N,N-dimethyl-N-(HP₁-HP₂-diHP₃)-propylammonium (n = 3)
C₆₁H₁₁₄NO₁₄P (1116.55)
- 1736.): 1,2-Di-(Z,Z)-6,18-Hexacosadienoyl-*sn*-glycero-3-phospho-N,N-dimethyl-N-(HP₁-HP₂-diHP₃)-propylammonium (n = 3)
C₆₉H₁₃₀NO₁₄P (1228.76)
- 1737.): 2-(Z)-6-Hexadecenoyl-1-stearoyl-*sn*-glycero-3-phospho-N,N-dimethyl-N-(HP₁-HP₂-diHP₃)-propylammonium (n = 3)
C₅₁H₁₀₀NO₁₄P (982.33)
- 1738.): 2-(Z)-10-Octadecenoyl-1-stearoyl-*sn*-glycero-3-phospho-N,N-dimethyl-N-(HP₁-HP₂-diHP₃)-propylammonium (n = 3)
C₅₃H₁₀₄NO₁₄P (1010.38)
- 1739.): 2-(Z)-10-Docosenoyl-1-behenyl-*sn*-glycero-3-phospho-N,N-dimethyl-N-(HP₁-HP₂-diHP₃)-propylammonium (n = 3)
C₅₉H₁₁₆NO₁₄P (1094.54)
- 1740.): 2-(Z,Z)-6,12-Hexadecadienoyl-1-stearoyl-*sn*-glycero-3-phospho-N,N-dimethyl-N-(HP₁-HP₂-diHP₃)-propylammonium (n = 3)
C₅₁H₉₈NO₁₄P (980.31)
- 1741.): 1-(Z)-10-Octadecenoyl-2-stearoyl-*sn*-glycero-3-phospho-N,N-dimethyl-N-(HP₁-HP₂-diHP₃)-propylammonium (n = 3)
C₅₃H₁₀₄NO₁₄P (1010.38)
- 1742.): 1-(Z,Z)-6,18-Hexacosadienoyl-2-stearoyl-*sn*-glycero-3-phospho-N,N-dimethyl-N-(HP₁-HP₂-diHP₃)-propylammonium (n = 3)
C₆₁H₁₁₈NO₁₄P (1120.58)
n=4
- 1743.): 1,2-Di-(Z)-6-Octadecenoyl-*sn*-glycero-3-phospho-N,N-dimethyl-N-(HP₁-HP₂-diHP₃)-butylammonium (n = 4)
C₅₄H₁₀₄NO₁₄P (1022.39)
- 1744.): 1,2-Di-(Z)-10-Docosenoyl-*sn*-glycero-3-phospho-N,N-dimethyl-N-'(HP₁-HP₂-diHP₃)-butylammonium (n = 4)
C₆₂H₁₂₀NO₁₄P (1134.61)
- 1745.): 1,2-Di-(Z,Z)-5,11-Hexadecadienoyl-*sn*-glycero-3-phospho-N,N-dimethyl-N-(HP₁-HP₂-diHP₃)-butylammonium (n = 4)
C₅₀H₉₂NO₁₄P (962.25)
- 1746.): 1,2-Di-(Z,Z)-10,16-Docosadienoyl-*sn*-glycero-3-phospho-N,N-dimethyl-N-(HP₁-HP₂-diHP₃)-butylammonium (n = 4)
C₆₂H₁₁₆NO₁₄P (1130.58)
- 1747.): 1,2-Di-(Z,Z)-6,18-Hexacosadienoyl-*sn*-glycero-3-phospho-N,N-dimethyl-N-(HP₁-HP₂-diHP₃)-butylammonium (n = 4)
C₇₀H₁₃₂NO₁₄P (1242.79)
n=6
- 1748.): 1,2-Di-(Z)-6-Octadecenoyl-*sn*-glycero-3-phospho-N,N-dimethyl-N-(HP₁-HP₂-diHP₃)-hexylammonium (n = 6)
C₅₆H₁₀₈NO₁₄P (1050.45)
- 1749.): 1,2-Di-(Z)-10-Docosenoyl-*sn*-glycero-3-phospho-N,N-dimethyl-N-(HP₁-HP₂-diHP₃)-hexylammonium (n = 6)
C₆₄H₁₂₄NO₁₄P (1162.66)
- 1750.): 1,2-Di-(Z,Z)-10,16-Docosadienoyl-*sn*-glycero-3-phospho-N,N-dimethyl-N-(HP₁-HP₂-diHP₃)-hexylammonium (n = 6)
C₆₄H₁₂₀NO₁₄P (1158.63)
- 1751.): 1,2-Di-(Z,Z)-6,18-Hexacosadienoyl-*sn*-glycero-3-phospho-N,N-dimethyl-N-(HP₁-HP₂-diHP₃)-hexylammonium (n = 6)
C₇₂H₁₃₆NO₁₄P (1270.84)

### 4. Beispiele für zweikettige Glycero-phospho-N,N-dimethyl-N-(2-hydroxypropyl-3,1-O,O-2-hydroxypropyl-3,1-O,O-2-hydroxypropyl 3,1-O,Odihydroxypropyl)-alkylammonium-Verbindungen

### (A = III; n = 2 - 6; R₃, CH₃; m= 1, x = 0; y = 1; z = 4)

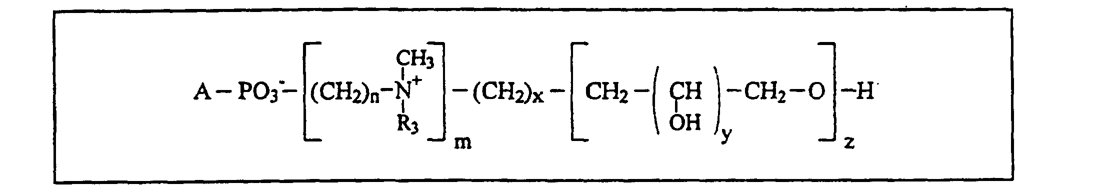

Im folgenden Text wird N-(2-hydroxypropyl-3,1-O,O-2-hydroxypropyl-3,1-O,O-2-hydroxypropyl 3,1-O,O-dihydroxypropyl) abgekürzt als N-(HP₁-HP₂-HP₃-diHP₄).
- 1752.): 1,2-Di-(Z)-6-Hexadecenoyl-*sn*-glycero-3-phospho-N,N-dimethyl-N-(HP₁-HP₂-HP₃-diHP₄)-ethylammonium (n = 2)
C₅₁H₉₈NO₁₆P (1012.31)
- 1753.): 1,2-Di-(Z)-10-Heptadecenoyl-*sn*-glycero-3-phospho-N,N-dimethyl-N-(HP₁-HP₂-HP₃-diHP₄)-ethylammonium (n = 2)
C₅₃H₁₀₂NO₁₆P (1040.36)
- 1754.): 1,2-Di-(Z)-6-Octadecenoyl-*sn*-glycero-3-phospho-N,N-dimethyl-N-(HP₁-HP₂-HP₃-diHP₄)-ethylammonium (n = 2)
C₅₅H₁₀₆NO₁₆P (1068.42)
- 1755.): 1,2-Di-(Z)-6-Nonadecenoyl-*sn*-glycero-3-phospho-N,N-dimethyl-N-(HP₁-HP₂-HP₃-diHP₄)-ethylammonium (n = 2)
C₅₇H₁₁₀NO₁₆P (1096.47)
- 1756.): 1,2-Di-(Z)-12-Eicosenoyl-*sn*-glycero-3-phospho-N,N-dimethyl-N-(HP₁-HP₂-HP₃-diHP₄)-ethylammonium (n = 2)
C₅₉H₁₁₄NO₁₆P (1124.53)
- 1757.): 1,2-Di-(Z)-10-Heneicosenoyl-*sn*-glycero-3-phospho-N,N-dimethyl-N-(HP₁-HP₂-HP₃-diHP₄)-ethylammonium (n = 2)
C₆₁H₁₁₈NO₁₆P (1152.58)
- 1758.): 1,2-Di-(Z)-10-Docosenoyl-*sn*-glycero-3-phospho-N,N-dimethyl-N-(HP₁-HP₂-HP₃-diHP₄)-ethylammonium (n = 2)
C₆₃H₁₂₂NO₁₆P (1180.63)
- 1759.): 1,2-Di-(Z)-12-Docosenoyl-*sn*-glycero-3-phospho-N,N-dimethyl-N-(HP₁-HP₂-HP₃-diHP₄)-ethylammonium (n = 2)
C₆₃H₁₂₂NO₁₆P (1180.63)
- 1760.): 1,2-Di-(Z)-10-Tricosenoyl-*sn*-glycero-3-phospho-N,N-dimethyl-N-(HP₁-HP₂-HP₃-diHP₄)-ethylammonium (n = 2)
C₆₅H₁₂₆NO₁₆P (1208.69)
- 1761.): 1,2-Di-(Z)-10-Tetracosenoyl-*sn*-glycero-3-phospho-N,N-dimethyl-N-(HP₁-HP₂-HP₃-diHP₄)-ethylammonium (n = 2)
C₆₇H₁₃₀NO₁₆P (1236.74)
- 1762.): 1,2-Di-(Z)-15-Pentacosenoyl-*sn*-glycero-3-phospho-N,N-dimethyl-N-(HP₁-HP₂-HP₃-diHP₄)-ethylammonium (n = 2)
C₆₉H₁₃₄NO₁₆P (1264.79)
- 1763.): 1,2-Di-(Z)-16-Hexacosenoyl-*sn*-glycero-3-phospho-N,N-dimethyl-N-(HP₁-HP₂-HP₃-diHP₄)-ethylammonium (n = 2)
C₇₁H₁₃₈NO₁₆P (1292.85)
- 1764.): 1,2-Di-(Z,Z)-5,11-Hexadecadienoyl-*sn*-glycero-3-phospho-N,N-dimethyl-N-(HP₁-HP₂-HP₃-diHP₄)-ethylammonium (n = 2)
C₅₁H₉₄NO₁₆P (1008.28)
- 1765.): 1,2-Di-(Z,Z)-5,11-Heptadecadienoyl-*sn*-glycero-3-phospho-N,N-dimethyl-N-(HP₁-HP₂-HP₃-diHP₄)-ethylammonium (n = 2)
C₅₃H₉₈NO₁₆P (1036.33)
- 1766.): 1,2-Di-(Z,Z)-5,11-Octadecadienoyl-sn-glycero-3-phospho-N,N-dimethyl-N-(HP₁-HP₂-HP₃-diHP₄)-ethylammonium (n = 2)
C₅₅H₁₀₂NO₁₆P (1064.39)
- 1767.): 1,2-Di-(Z,Z)-6,12-Nonadecadienoyl-*sn*-glycero-3-phospho-N,N-dimethyl-N-(HP₁-HP₂-HP₃-diHP₄)-ethylammonium (n = 2)
C₅₇H₁₀₆NO₁₆P (1092.44)
- 1768.): 1,2-Di-(Z,Z)-10,16-Eicosadienoyl-*sn*-glycero-3-phospho-N,N-dimethyl-N-(HP₁-HP₂-HP₃-diHP₄)-ethylammonium (n = 2)
C₅₉H₁₁₀NO₁₆P (1120.49)
- 1769.): 1,2-Di-(Z,Z)-10,16-Heneicosadienoyl-*sn*-glycero-3-phospho-N,N-dimethyl-N-(HP₁-HP₂-HP₃-diHP₄)-ethylammonium (n = 2)
C₆₁H₁₁₄NO₁₆P (1148.55)
- 1770.): 1,2-Di-(Z,Z)-10,16-Docosadienoyl-*sn*-glycero-3-phospho-N,N-dimethyl-N-(HP₁-HP₂-HP₃-diHP₄)-ethylammonium (n = 2)
C₆₃H₁₁₈NO₁₆P (1176.60)
- 1771.): 1,2-Di-(Z,Z)-10,16-Tricosadienoyl-sn-glycero-3-phospho-N,N-dimethyl-N-(HP₁-HP₂-HP₃-diHP₄)-ethylammonium (n = 2)
C₆₅H₁₂₂NO₁₆P (1204.65)
- 1772.): 1,2-Di-(Z,Z)-6,18-Tetracosadienoyl-*sn*-glycero-3-phospho-N,N-dimethyl-N-(HP₁-HP₂-HP₃-diHP₄)-ethylammonium (n = 2)
C₆₇H₁₂₆NO₁₆P (1232.71)
- 1773.): 1,2-Di-(Z,Z)-10,16-Pentacosadienoyl-sn-glycero-3-phospho-N,N-dimethyl-N-(HP₁-HP₂-HP₃-diHP₄)-ethylammonium (n = 2)
C₆₉H₁₃₀NO₁₆P (1260.76)
- 1774.): 1,2-Di-(Z,Z)-6,18-Hexacosadienoyl-*sn*-glycero-3-phospho-N,N-dimethyl-N-(HP₁-HP₂-HP₃-diHP₄)-ethylammonium (n = 2)
C₇₁H₁₃₄NO₁₆P (1288.82)
- 1775.): 2-(Z)-6-Hexadecenoyl-1-stearoyl-*sn*-glycero-3-phospho-N,N-dimethyl-N-(HP₁-HP₂-HP₃-diHP₄)-ethylammonium (n = 2)
C₅₃H₁₀₄NO₁₆P (1042.38)
- 1776.): 2-(Z)-10-Octadecenoyl-1-stearoyl-*sn*-glycero-3-phospho-N,N-dimethyl-N-(HP₁-HP₂-HP₃-diHP₄)-ethylammonium (n = 2)
C₅₅H₁₀₈NO₁₆P (1070.43)
- 1777.): 2-(Z)-10-Eicosenoyl-1-stearoyl-*sn*-glycero-3-phospho-N,N-dimethyl-N-(HP₁-HP₂-HP₃-diHP₄)-ethylammonium (n = 2)
C₅₇H₁₁₂NO₁₆P (1098.49)
- 1778.): 1-Behenyl-2-(Z)-10-docosenoyl-*sn*-glycero-3-phospho-N,N-dimethyl-N-(HP₁-HP₂-HP₃-diHP₄)-ethylammonium (n = 2)
C₆₁H₁₂₀NO₁₆P (1154.59)
- 1779.): 2-(Z,Z)-6,12-Hexadecadienoyl-1-stearoyl-*sn*-glycero-3-phospho-N,N-dimethyl-N-(HP₁-HP₂-HP₃-diHP₄)-ethylammonium (n = 2)
C₅₃H₁₀₂NO₁₆P (1040.36)
- 1780.): 2-(Z,Z)-10,16-Docosadienoyl-1-stearoyl-*sn*-glycero-3-phospho-N,N-dimethyl-N-(HP₁-HP₂-HP₃-diHP₄)-ethylammonium (n = 2)
C₅₉H₁₁₄NO₁₆P (1124.53)
- 1781.): 1-Stearoyl-2-(Z,Z)-6,18-tetracosadienoyl-*sn*-glycero-3-phospho-N,N-dimethyl-N-(HP₁-HP₂-HP₃-diHP₄)-ethylammonium (n = 2)
C₆₁H₁₁₈NO₁₆P (1152.58)
- 1782.): 1-(Z)-10-Octadecenoyl-2-stearoyl-*sn*-glycero-3-phospho-N,N-dimethyl-N-(HP₁-HP₂-HP₃-diHP₄)-ethylammonium (n = 2)
C₅₅H₁₀₈NO₁₆P (1070.43)
- 1783.): 1-(Z,Z)-6,18-Hexacosadienoyl-2-stearoyl-*sn*-glycero-3-phospho-N,N-dimethyl-N-(HP₁-HP₂-HP₃-diHP₄)-ethylammonium (n = 2)
C₆₃H₁₂₂NO₁₆P (1180.63)
- 1784.): 1-(Z,Z)-6,18-Hexacosadienoyl-2-(Z)-6-Hexadecenoyl-*sn*-glycero-3-phospho-N,N-dimethyl-N-(HP₁-HP₂-HP₃-diHP₄)-ethylammonium (n = 2)
C₆₁H₁₁₆NO₁₆P (1150.56)
- 1785.): 2-(Z,Z)-6,18-Hexacosadienoyl-1-(Z)-6-Hexadecenoyl-*sn*-glycero-3-phospho-N,N-dimethyl-N-(HP₁-HP₂-HP₃-diHP₄)-ethylammonium (n = 2)
C₆₁H₁₁₆NO₁₆P (1150.56)
n=3
- 1786.): 1,2-Di-(Z)-6-Hexadecenoyl-*sn*-glycero-3-phospho-N,N-dimethyl-N-(HP₁-HP₂-HP₃-diHP₄)-propylammonium (n = 3)
C₅₂H₁₀₀NO₁₆P (1026.34)
- 1787.): 1,2-Di-(Z)-10-Heptadecenoyl-*sn*-glycero-3-phospho-N,N-dimethyl-N-(HP₁-HP₂-HP₃-diHP₄)-propylammonium (n = 3)
C₅₄H₁₀₄NO₁₆P (1054.39)
- 1788.): 1,2-Di-(Z)-6-Octadecenoyl-*sn*-glycero-3-phospho-N,N-dimethyl-N-(HP₁-HP₂-HP₃-diHP₄)-propylammonium (n = 3)
C₅₆H₁₀₈NO₁₆P (1082.44)
- 1789.): 1,2-Di-(Z)-12-Eicosenoyl-*sn*-glycero-3-phospho-N,N-dimethyl-N-(HP₁-HP₂-HP₃-diHP₄)-propylammonium (n = 3)
C₆₀H₁₁₆NO₁₆P (1138.55)
- 1790.): 1,2-Di-(Z)-10-Docosenoyl-*sn*-glycero-3-phospho-N,N-dimethyl-N-(HP₁-HP₂-HP₃-diHP₄)-propylammonium (n = 3)
C₆₄H₁₂₄NO₁₆P (1194.66)
- 1791.): 1,2-Di-(Z)-12-Docosenoyl-*sn*-glycero-3-phospho-N,N-dimethyl-N-(HP₁-HP₂-HP₃-diHP₄)-propylammonium (n = 3)
C₆₄H₁₂₄NO₁₆P (1194.66)
- 1792.): 1,2-Di-(Z)-10-Tricosenoyl-*sn*-glycero-3-phospho-N,N-dimethyl-N-(HP₁-HP₂-HP₃-diHP₄)-propylammonium (n = 3)
C₆₆H₁₂₈NO₁₆P (1222.71)
- 1793.): 1,2-Di-(Z)-10-Tetracosenoyl-*sn*-glycero-3-phospho-N,N-dimethyl-N-(HP₁-HP₂-HP₃-diHP₄)-propylammonium (n = 3)
C₆₈H₁₃₂NO₁₆P (1250.77)
- 1794.): 1,2-Di-(Z,Z)-5,11-Octadecadienoyl-*sn*-glycero-3-phospho-N,N-dimethyl-N-(HP₁-HP₂-HP₃-diHP₄)-propylammonium (n = 3)
C₅₆H₁₀₄NO₁₆P (1078.41)
- 1795.): 1,2-Di-(Z,Z)-10,16-Eicosadienoyl-*sn*-glycero-3-phospho-N,N-dimethyl-N-(HP₁-HP₂-HP₃-diHP₄)-propylammonium (n = 3)
C₆₀H₁₁₂NO₁₆P (1134.52)
- 1796.): 1,2-Di-(Z,Z)-10,16-Docosadienoyl-*sn*-glycero-3-phospho-N,N-dimethyl-N-(HP₁-HP₂-HP₃-diHP₄)-propylammonium (n = 3)
C₆₄H₁₂₀NO₁₆P (1190.63)
- 1797.): 1,2-Di-(Z,Z)-6,18-Hexacosadienoyl-sn-glycero-3-phospho-N,N-dimethyl-N-(HP₁-HP₂-HP₃-diHP₄)-propylammonium (n = 3)
C₇₂H₁₃₆NO₁₆P (1302.84)
- 1798.): 2-(Z)-6-Hexadecenoyl-1-stearoyl-*sn*-glycero-3-phospho-N,N-dimethyl-N-(HP₁-HP₂-HP₃-diHP₄)-propylammonium (n = 3)
C₅₄H₁₀₆NO₁₆P (1056.41)
- 1799.): 2-(Z)-10-Octadecenoyl-1-stearoyl-*sn*-glycero-3-phospho-N,N-dimethyl-N-(HP₁-HP₂-HP₃-diHP₄)-propylammonium (n = 3)
C₅₆H₁₁₀NO₁₆P (1084.46)
- 1800.): 2-(Z)-10-Docosenoyl-1-behenyl-*sn*-glycero-3-phospho-N,N-dimethyl-N-(HP₁-HP₂-HP₃-diHP₄)-propylammonium (n = 3)
C₆₂H₁₂₂NO₁₆P (1168.62)
- 1801.): 2-(Z,Z)-6,12-Hexadecadienoyl-1-stearoyl-*sn*-glycero-3-phospho-N,N-dimethyl-N-(HP₁-HP₂-HP₃-diHP₄)-propylammonium (n = 3)
C₅₄H₁₀₄NO₁₆P (1054.39)
- 1802.): 1-(Z)-10-Octadecenoyl-2-stearoyl-*sn*-glycero-3-phospho-N,N-dimethyl-N-(HP₁-HP₂-HP₃-diHP₄)-propylammonium (n = 3)
C₅₆H₁₁₀NO₁₆P (1084.46)
- 1803.): 1-(Z,Z)-6,18-Hexacosadienoyl-2-stearoyl-*sn*-glycero-3-phospho-N,N-dimethyl-N-(HP₁-HP₂-HP₃-diHP₄)-propylammonium (n = 3)
C₆₄H₁₂₄NO₁₆P (1194.66)
n=4
- 1804.): 1,2-Di-(Z)-6-Octadecenoyl-*sn*-glycero-3-phospho-N,N-dimethyl-N-(HP₁-HP₂-HP₃-diHP₄)-butylammonium (n = 4)
C₅₇H₁₁₀NO₁₆P (1096.47)
- 1805.): 1,2-Di-(Z)-10-Docosenoyl-*sn*-glycero-3-phospho-N,N-dimethyl-N-(HP₁-HP₂-HP₃-diHP₄)-butylammonium (n = 4)
C₆₅H₁₂₆NO₁₆P (1208.69)
- 1806.): 1,2-Di-(Z,Z)-5,11-Hexadecadienoyl-*sn*-glycero-3-phospho-N,N-dimethyl-N-(HP₁-HP₂-HP₃-diHP₄)-butylammonium (n = 4)
C₅₃H₉₈NO₁₆P (1036.33)
- 1807.): 1,2-Di-(Z,Z)-10,16-Docosadienoyl-*sn*-glycero-3-phospho-N,N-dimethyl-N-(HP₁-HP₂-HP₃-diHP₄)-butylammonium (n = 4)
C₆₅H₁₂₂NO₁₆P (1204.65)
- 1808.): 1,2-Di-(Z,Z)-6,18-Hexacosadienoyl-*sn*-glycero-3-phospho-N,N-dimethyl-N-(HP₁-HP₂-HP₃-diHP₄)-butylammonium (n = 4)
C₇₃H₁₃₈NO₁₆P (1316.87)
n=6
- 1809.): 1,2-Di-(Z)-6-Octadecenoyl-*sn*-glycero-3-phospho-N,N-dimethyl-N-(HP₁-HP₂-HP₃-diHP₄)-hexylammonium (n = 6)
C₅₉H₁₁₄NO₁₆P (1124.53)
- 1810.): 1,2-Di-(Z)-10-Docosenoyl-*sn*-glycero-3-phospho-N,N-dimethyl-N-(HP₁-HP₂-HP₃-diHP₄)-hexylammonium (n = 6)
C₆₇H₁₃₀NO₁₆P (1236.74)
- 1811.): 1,2-Di-(Z,Z)-10,16-Docosadienoyl-*sn*-glycero-3-phospho-N,N-dimethyl-N-(HP₁-HP₂-HP₃-diHP₄)-hexylammonium (n = 6)
C₆₇H₁₂₆NO₁₆P (1232.71)
- 1812.): 1,2-Di-(Z,Z)-6,18-Hexacosadienoyl-*sn*-glycero-3-phospho-N,N-dimethyl-N-(HP₁-HP₂-HP₃-diHP₄)-hexylammonium (n = 6)
C₇₅H₁₄₂NO₁₆P (1344.92)

### 5. Beispiele für zweikettige Glycero-phospho-Verbindungen, die nicht am Stickstoff hydroxyliert sind

### (A = III; n = 2 - 6; R₃, CH₃; m= 1, x = 1; z = 0)

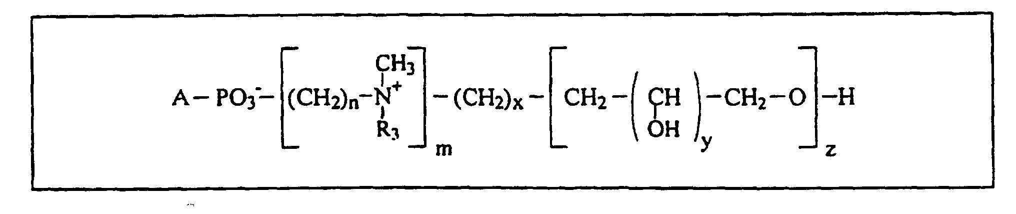

- 1813.): 1,2-Di-(Z)-6-Hexadecenoyl-*sn*-glycero-3-phospho-N,N,N-trimethyl-propylammonium (n = 3)
C₄₁H₇₈NO₈P (744.05)
- 1814.): 1,2-Di-(Z)-10-Heptadecenoyl-*sn*-glycero-3-phospho-N,N,N-trimethyl-propylammonium (n = 3)
C₄₃H₈₂NO₈P (772.10)
- 1815.): 1,2-Di-(Z)-6-Octadecenoyl-*sn*-glycero-3-phospho-N,N,N-trimethyl-propylammonium (n = 3)
C₄₅H₈₆NO₈P (800.15)
- 1816.): 1,2-Di-(Z)-12-Eicosenoyl-*sn*-glycero-3-phospho-N,N,N-trimethyl-propylammonium (n = 3)
C₄₉H₉₄NO₈P (856.26)
- 1817.): 1,2-Di-(Z)-10-Docosenoyl-*sn*-glycero-3-phospho-N,N,N-trimethyl-propylammonium (n = 3)
C₅₃H₁₀₂NO₈P (912.37)
- 1818.): 1,2-Di-(Z)-12-Docosenoyl-*sn*-glycero-3-phospho-N,N,N-trimethyl-propylammonium (n = 3)
C₅₃H₁₀₂NO₈P (912.37)
- 1819.): 1,2-Di-(Z)-10-Tricosenoyl-*sn*-glycero-3-phospho-N,N,N-trimethyl-propylammonium (n = 3)
C₅₅H₁₀₆NO₈P (940.42)
- 1820.): 1,2-Di-(Z)-10-Tetracosenoyl-*sn*-glycero-3-phospho-N,N,N-trimethyl-propylammonium (n = 3)
C₅₇H₁₁₀NO₈P (968.48)
- 1821.): 1,2-Di-(Z,Z)-5,11-Octadecadienoyl-*sn*-glycero-3-phospho-N,N,N-trimethyl-propylammonium (n = 3)
C₄₅H₈₂NO₈P (796.12)
- 1822.): 1,2-Di-(Z,Z)-10,16-Eicosadienoyl-*sn*-glycero-3-phospho-N,N,N-trimethyl-propylammonium (n = 3)
C₄₉H₉₀NO₈P (852.23)
- 1823.): 1,2-Di-(Z,Z)-10,16-Docosadienoyl-*sn*-glycero-3-phospho-N,N,N-trimethyl-propylammonium (n = 3)
C₅₃H₉₈NO₈P (908.34)
- 1824.): 1,2-Di-(Z,Z)-6,18-Hexacosadienoyl-*sn*-glycero-3-phospho-N,N,N-trimethyl-propylammonium (n = 3)
C₆₁H₁₁₄NO₈P (1020.55)
- 1825.): 2-(Z)-6-Hexadecenoyl-1-stearoyl-*sn*-glycero-3-phospho-N,N,N-trimethyl-propylammonium (n = 3)
C₄₃H₈₄NO₈P (774.12)
- 1826.): 2-(Z)-10-Octadecenoyl-1-stearoyl-*sn*-glycero-3-phospho-N,N,N-trimethyl-propylammonium (n = 3)
C₄₅H₈₈NO₈P (802.17)
- 1827.): 2-(Z)-10-Docosenoyl-1-behenyl-*sn*-glycero-3-phospho-N,N,N-trimethyl-propylammonium (n = 3)
C₅₁H₁₀₀NO₈P (886.33)
- 1828.): 2-(Z,Z)-6,12-Hexadecadienoyl-1-stearoyl-*sn*-glycero-3-phospho-N,N,N-trimethyl-propylammonium (n = 3)
C₄₃H₈₂NO₈P (772.10)
- 1829.): 1-(Z)-10-Octadecenoyl-2-stearoyl-*sn*-glycero-3-phospho-N,N,N-trimethyl-propylammonium (n = 3)
C₄₅H₈₈NO₈P (802.17)
- 1830.): 1-(Z,Z)-6,18-Hexacosadienoyl-2-stearoyl-*sn*-glycero-3-phospho-N,N,N-trimethyl-propylammonium (n = 3)
C₅₃H₁₀₂NO₈P (912.37)
n=4
- 1831.): 1,2-Di-(Z)-6-Octadecenoyl-*sn*-glycero-3-phospho-N,N,N-trimethyl-butylammonium (n = 4)
C₄₆H₈₈NO₈P (814.18)
- 1832.): 1,2-Di-(Z)-10-Docosenoyl-sn-glycero-3-phospho-N,N,N-trimethyl-butylammonium (n = 4)
C₅₄H₁₀₄NO₈P (926.40)
- 1833.): 1,2-Di-(Z,Z)-5,11-Hexadecadienoyl-*sn*-glycero-3-phospho-N,N,N-trimethyl-butylammonium (n = 4)
C₄₂H₇₆NO₈P (796.12)
- 1834.): 1,2-Di-(Z,Z)-10,16-Docosadienoyl-*sn*-glycero-3-phospho-N,N,N-trimethyl-butylammonium (n = 4)
C₅₄H₁₀₀NO₈P (922.36)
- 1835.): 1,2-Di-(Z,Z)-6,18-Hexacosadienoyl-sn-glycero-3-phospho-N,N,N-trimethyl-butylammonium (n = 4)
C₈₂H₁₁₆NO₈P (1034.58)
n=6
- 1836.): 1,2-Di-(Z)-6-Octadecenoyl-*sn*-glycero-3-phospho-N,N,N-trimethyl-hexylammonium (n = 6)
C₄₈H₉₂NO₈P (842.23)
- 1837.): 1,2-Di-(Z)-10-Docosenoyl-*sn*-glycero-3-phospho-N,N,N-trimethyl-hexylammonium (n = 6)
C₅₆H₁₀₈NO₈P (954.45)
- 1838.): 1,2-Di-(Z,Z)-10,16-Docosadienoyl-*sn*-glycero-3-phospho-N,N,N-trimethyl-hexylammonium (n = 6)
C₅₆H₁₀₄NO₈P (950.42)
- 1839.): 1,2-Di-(Z,Z)-6,18-Hexacosadienoyl-*sn*-glycero-3-phospho-N,N,N-trimethyl-hexylammonium (n = 6)
C₆₄H₁₂₀NO₈P (1062.63)

### Negativ geladene Phospholipide: Phosphatidyloligoglycerine

### 6. Beispiele für Glycero-glycerine (Na-Salze der Phospho-G₁-G₂-Verbindungen)

### (A = III; m= 0, x = 0; y = 1; z = 2)

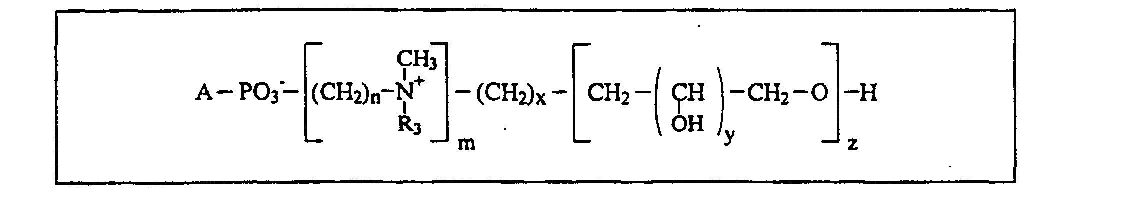

- 1840.): 1,2-Di-(Z)-6-Hexadecenoyl-*sn*-glycero-3-phospho-glycero-glycerin; Na-Salz
C₄₁H₇₆NaO₁₂P (815.01)
- 1841.): 1,2-Di-(Z)-10-Heptadecenoyl-*sn*-glycero-3-phospho-glycero-glycerin; Na-Salz
C₄₃H₈₀NaO₁₂P (843.06)
- 1842.): 1,2-Di-(Z)-6-Octadecenoyl-*sn*-glycero-3-phospho-glycero-glycerin; Na-Salz
C₄₅H₈₄NaO₁₂P (871.12)
- 1843.): 1,2-Di-(Z)-6-Nonadecenoyl-*sn*-glycero-3-phospho-glycero-glycerin; Na-Salz
C₄₇H₈₈NaO₁₂P (899.17)
- 1844.): 1,2-Di-(Z)-12-Eicosenoyl-*sn*-glycero-3-phospho-glycero-glycerin; Na-Salz
C₄₉H₉₂NaO₁₂P (927.23)
- 1845.): 1,2-Di-(Z)-10-Heneicosenoyl-*sn*-glycero-3-phospho-glycero-glycerin; Na-Salz
C₅₁H₉₆NaO₁₂P (955.28)
- 1846.): 1,2-Di-(Z)-10-Docosenoyl-*sn*-glycero-3-phospho-glycero-glycerin; Na-Salz
C₅₃H₁₀₀NaO₁₂P (983.33)
- 1847.): 1,2-Di-(Z)-12-Docosenoyl-*sn*-glycero-3-phospho-glycero-glycerin; Na-Salz
C₅₃H₁₀₀NaO₁₂P (983.33)
- 1848.): 1,2-Di-(Z)-10-Tricosenoyl-*sn*-glycero-3-phospho-glycero-glycerin; Na-Salz
C₅₅H₁₀₄NaO₁₂P (1011.39)
- 1849.): 1,2-Di-(Z)-10-Tetracosenoyl-sn-glycero-3-phospho-glycero-glycerin; Na-Salz
C₅₇H₁₀₈NaO₁₂P (1039.44)
- 1850.): 1,2-Di-(Z)-15-Pentacosenoyl-*sn*-glycero-3-phospho-glycero-glycerin; Na-Salz
C₅₉H₁₁₂NaO₁₂P (1067.49)
- 1851.): 1,2-Di-(Z)-16-Hexacosenoyl-*sn*-glycero-3-phospho-glycero-glycerin; Na-Salz
C₆₁H₁₁₆NaO₁₂P (1095.55)
- 1852.): 1,2-Di-(Z,Z)-5,11-Hexadecadienoyl-*sn*-glycero-3-phospho-glycero-glycerin; Na-Salz
C₄₁H₇₂NaO₁₂P (810.98)
- 1853.): 1,2-Di-(Z,Z)-5,11-Octadecadienoyl-*sn*-glycero-3-phospho-glycero-glycerin; Na-Salz
C₄₅H₈₀NaO₁₂P (867.09)
- 1854.): 1,2-Di-(Z,Z)-6,12-Nonadecadienoyl-*sn*-glycero-3-phospho-glycero-glycerin; Na-Salz
C₄₇H₈₄NaO₁₂P (895.14)
- 1855.): 1,2-Di-(Z,Z)-10,16-Eicosadienoyl-*sn*-glycero-3-phospho-glycero-glycerin; Na-Salz
C₄₉H₈₈NaO₁₂P (923.19)
- 1856.): 1,2-Di-(Z,Z)-10,16-Docosadienoyl-*sn*-glycero-3-phospho-glycero-glycerin; Na-Salz
C₅₃H₉₆NaO₁₂P (979.30)
- 1857.): 1,2-Di-(Z,Z)-6,18-Tetracosadienoyl-*sn*-glycero-3-phospho-glycero-glycerin; Na-Salz
C₅₇H₁₀₄NaO₁₂P (1035.41)
- 1858.): 1,2-Di-(Z,Z)-10,16-Pentacosadienoyl-*sn*-glycero-3-phospho-glycero-glycerin; Na-Salz
C₅₉H₁₀₈NaO₁₂P (1063.46)
- 1859.): 1,2-Di-(Z,Z)-6,18-Hexacosadienoyl-*sn*-glycero-3-phospho-glycero-glycerin; Na-Salz
C₆₁H₁₁₂NaO₁₂P (1091.52)
- 1860.): 2-(Z)-6-Hexadecenoyl-1-stearoyl-*sn*-glycero-3-phospho-glycero-glycerin; Na-Salz
C₄₃H₈₂NaO₁₂P (845.08)
- 1861.): 2-(Z)-10-Octadecenoyl-1-stearoyl-*sn*-glycero-3-phospho-glycero-glycerin; Na-Salz
C₄₅H₈₆NaO₁₂P (873.13)
- 1862.): 2-(Z)-10-Eicosenoyl-1-stearoyl-*sn*-glycero-3-phospho-glycero-glycerin; Na-Salz
C₄₇H₉₀NaO₁₂P (901.19)
- 1863.): 2-(Z,Z)-6,12-Hexadecadienoyl-1-stearoyl-*sn*-glycero-3-phospho-glycero-glycerin; Na-Salz
C₄₃H₈₀NaO₁₂P (843.06)
- 1864.): 2-(Z,Z)-10,16-Docosadienoyl-1-stearoyl-*sn*-glycero-3-phospho-glycero-glycerin; Na-Salz
C₄₉H₉₂NaO₁₂P (927.23)
- 1865.): 1-Stearoyl-2-(Z,Z)-6,18-tetracosadienoyl-*sn*-glycero-3-phospho-glycero-glycerin; Na-Salz
C₅₁H₉₆NaO₁₂P (955.28)
- 1866.): 1-(Z)-10-Octadecenoyl-2-stearoyl-*sn*-glycero-3-phospho-glycero-glycerin; Na-Salz
C₄₅H₈₆NaO₁₂P (873.13)
- 1867.): 1-(Z,Z)-6,18-Hexacosadienoyl-2-stearoyl-*sn*-glycero-3-phospho-glycero-glycerin; Na-Salz
C₅₃H₁₀₀NaO₁₂P (983.33)
- 1868.): 1-(Z,Z)-6,18-Hexacosadienoyl-2-(Z)-6-Hexadecenoyl-*sn*-glycero-3-phospho-glycero-glycerin; Na-Salz
C₅₁H₉₄NaO₁₂P (953.26)
- 1869.): 2-(Z,Z)-6,18-Hexacosadienoyl-1-(Z)-6-Hexadecenoyl-*sn*-glycero-3-phospho-glycero-glycerin; Na-Salz
C₅₁H₉₄NaO₁₂P (953.26)

### 7. Beispiele für Phosphatidyl-glycero-glycero-glycerine (Na-Salze der Phospho-G₁-G₂-G₃-Verbindungen)

### (A = III; m= 0, x = 0; y = 1; z = 3)

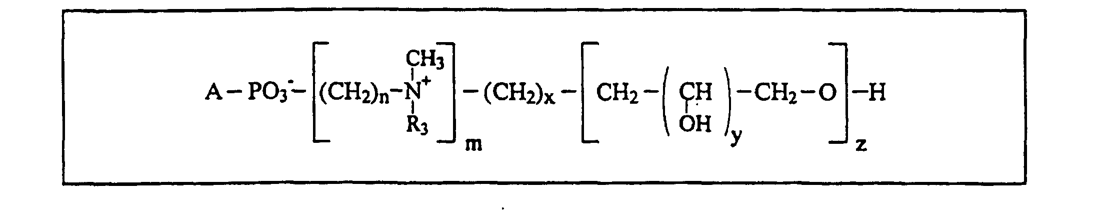

- 1870.): 1,2-Di-(Z)-6-Hexadecenoyl-*sn*-glycero-3-phospho-glycero-glycero-glycerin; Na-Salz
C₄₄H₈₂NaO₁₄P (889.09)
- 1871.): 1,2-Di-(Z)-10-Heptadecenoyl-*sn*-glycero-3-phospho-glycero-glycero-glycerin; Na-Salz
C₄₆H₈₆NaO₁₄P (917.14)
- 1872.): 1,2-Di-(Z)-6-Octadecenoyl-*sn*-glycero-3-phospho-glycero-glycero-glycerin; Na-Salz
C₄₈H₉₀NaO₁₄P (945.20)
- 1873.): 1,2-Di-(Z)-6-Nonadecenoyl-*sn*-glycero-3-phospho-glycero-glycero-glycerin; Na-Salz
C₅₀H₉₄NaO₁₄P (973.25)
- 1874.): 1,2-Di-(Z)-12-Eicosenoyl-*sn*-glycero-3-phospho-glycero-glycero-glycerin; Na-Salz
C₅₂H₉₈NaO₁₄P (1001.31)
- 1875.): 1,2-Di-(Z)-10-Heneicosenoyl-*sn*-glycero-3-phospho-glycero-glycero-glycerin; Na-Salz
C₅₄H₁₀₂NaO₁₄P (1029.36)
- 1876.): 1,2-Di-(Z)-10-Docosenoyl-*sn*-glycero-3-phospho-glycero-glycero-glycerin; Na-Salz
C₅₆H₁₀₆NaO₁₄P (1057.41)
- 1877.): 1,2-Di-(Z)-12-Docosenoyl-*sn*-glycero-3-phospho-glycero-glycero-glycerin; Na-Salz
C₅₆H₁₀₆NaO₁₄P (1057.41)
- 1878.): 1,2-Di-(Z)-10-Tricosenoyl-*sn*-glycero-3-phospho-glycero-glycero-glycerin; Na-Salz
C₅₈H₁₁₀NaO₁₄P (1085.47)
- 1879.): 1,2-Di-(Z)-10-Tetracosenoyl-*sn*-glycero-3-phospho-glycero-glycero-glycerin; Na-Salz
C₆₀H₁₁₄NaO₁₄P (1113.52)
- 1880.): 1,2-Di-(Z)-15-Pentacosenoyl-*sn*-glycero-3-phospho-glycero-glycero-glycerin; Na-Salz
C₆₂H₁₁₈NaO₁₄P (1141.57)
- 1881.): 1,2-Di-(Z)-16-Hexacosenoyl-*sn*-glycero-3-phospho-glycero-glycero-glycerin; Na-Salz
C₆₄H₁₂₂NaO₁₄P (1169.63)
- 1882.): 1,2-Di-(Z,Z)-5,11-Hexadecadienoyl-*sn*-glycero-3-phospho-glycero-glycero-glycerin; Na-Salz
C₄₄H₇₈NaO₁₄P (885.06)
- 1883.): 1,2-Di-(Z,Z)-5,11-Octadecadienoyl-*sn*-glycero-3-phospho-glycero-glycero-glycerin; Na-Salz
C₄₈H₈₆NaO₁₄P (941.17)
- 1884.): 1,2-Di-(Z,Z)-6,12-Nonadecadienoyl-*sn*-glycero-3-phospho-glycero-glycero-glycerin; Na-Salz
C₅₀H₉₀NaO₁₄P (969.22)
- 1885.): 1,2-Di-(Z,Z)-10,16-Eicosadienoyl-*sn*-glycero-3-phospho-glycero-glycero-glycerin; Na-Salz
C₅₂H₉₄NaO₁₄P (997.27)
- 1886.): 1,2-Di-(Z,Z)-10,16-Docosadienoyl-*sn*-glycero-3-phospho-glycero-glycero-glycerin; Na-Salz
C₅₆H₁₀₂NaO₁₄P (1053.38)
- 1887.): 1,2-Di-(Z,Z)-6,18-Tetracosadienoyl-*sn*-glycero-3-phospho-glycero-glycero-glycerin; Na-Salz
C₆₀H₁₁₀NaO₁₄P (1109.49)
- 1888.): 1,2-Di-(Z,Z)-10,16-Pentacosadienoyl-*sn*-glycero-3-phospho-glycero-glycero-glycerin; Na-Salz
C₆₂H₁₁₄NaO₁₄P (1137.54)
- 1889.): 1,2-Di-(Z,Z)-6,18-Hexacosadienoyl-*sn*-glycero-3-phospho-glycero-glycero-glycerin; Na-Salz
C₆₄H₁₁₈NaO₁₄P (1165.60)
- 1890.): 2-(Z)-6-Hexadecenoyl-1-stearoyl-*sn*-glycero-3-phospho-glycero-glycero-glycerin; Na-Salz
C₄₆H₈₈NaO₁₄P (919.16)
- 1891.): 2-(Z)-10-Octadecenoyl-1-stearoyl-*sn*-glycero-3-phospho-glycero-glycero-glycerin; Na-Salz
C₄₈H₉₂NaO₁₄P (947.21)
- 1892.): 2-(Z)-10-Eicosenoyl-1-stearoyl-*sn*-glycero-3-phospho-glycero-glycero-glycerin; Na-Salz
C₅₀H₉₆NaO₁₄P (975.27)
- 1893.): 2-(Z,Z)-6,12-Hexadecadienoyl-1-stearoyl-*sn*-glycero-3-phospho-glycero-glycero-glycerin; Na-Salz
C₄₆H₈₆NaO₁₄P (917.14)
- 1894.): 2-(Z,Z)-10,16-Docosadienoyl-1-stearoyl-*sn*-glycero-3-phospho-glycero-glycero-glycerin; Na-Salz
C₅₂H₉₈NaO₁₄P (1001.31)
- 1895.): 1-Stearoyl-2-(Z,Z)-6,18-tetracosadienoyl-*sn*-glycero-3-phospho-glycero-glycero-glycerin; Na-Salz
C₅₄H₁₀₂NaO₁₄P (1029.36)
- 1896.): 1-(Z)-10-Octadecenoyl-2-stearoyl-*sn*-glycero-3-phospho-glycero-glycero-glycerin; Na-Salz
C₄₈H₉₂NaO₁₄P (947.21)
- 1897.): 1-(Z,Z)-6,18-Hexacosadienoyl-2-stearoyl-*sn*-glycero-3-phospho-glycero-glycero-glycerin; Na-Salz
C₅₆H₁₀₆NaO₁₄P (1057.41)
- 1898.): 1-(Z,Z)-6,18-Hexacosadienoyl-2-(Z)-6-Hexadecenoyl-*sn*-glycero-3-phospho-glycero-glycero-glycerin; Na-Salz
C₅₄H₁₀₀NaO₁₄P (1027.34)
- 1899.): 2-(Z,Z)-6,18-Hexacosadienoyl-1-(Z)-6-Hexadecenoyl-*sn*-glycero-3-phospho-glycero-glycero-glycerin; Na-Salz
C₅₄H₁₀₀NaO₁₄P (1027.34)

### 8. Beispiele für Phosphatidyl-glycero-glycero-glycero-glycerine (Na-Salze der Phospho-G₁-G₂-G₃-G₄-Verbindungen)

### (A = III; m= 0, x = 0; y = 1; z = 4)

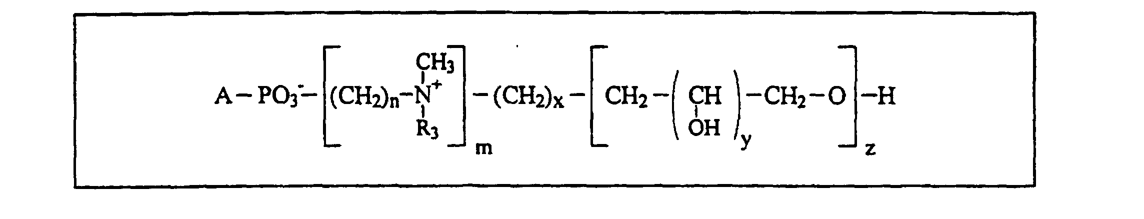

- 1900.): 1,2-Di-(Z)-6-Hexadecenoyl-*sn*-glycero-3-phospho-glycero-glycero-glycero-glycerin; Na-Salz
C₄₇H₈₈NaO₁₆P (963.17)
- 1901.): 1,2-Di-(Z)-10-Heptadecenoyl-*sn*-glycero-3-phospho-glycero-glycero-glycero-glycerin; Na-Salz
C₄₉H₉₂NaO₁₆P (991.22)
- 1902.): 1,2-Di-(Z)-6-Octadecenoyl-*sn*-glycero-3-phospho-glycero-glycero-glycero-glycerin; Na-Salz
C₅₁H₉₆NaO₁₆P (1019.28)
- 1903.): 1,2-Di-(Z)-6-Nonadecenoyl-*sn*-glycero-3-phospho-glycero-glycero-glycero-glycerin; Na-Salz
C₅₃H₁₀₀NaO₁₆P (1047.33)
- 1904.): 1,2-Di-(Z)-12-Eicosenoyl-*sn*-glycero-3-phospho-glycero-glycero-glycero-glycerin; Na-Salz
C₅₅H₁₀₄NaO₁₆P (1075.38)
- 1905.): 1,2-Di-(Z)-10-Heneicosenoyl-*sn*-glycero-3-phospho-glycero-glycero-glycero-glycerin; Na-Salz
C₅₇H₁₀₈NaO₁₆P (1103.44)
- 1906.): 1,2-Di-(Z)-10-Docosenoyl-*sn*-glycero-3-phospho-glycero-glycero-glycero-glycerin; Na-Salz
C₅₉H₁₁₂NaO₁₆P (1131.49)
- 1907.): 1,2-Di-(Z)-12-Docosenoyl-sn-glycero-3-phospho-glycero-glycero-glycero-glycerin; Na-Salz
C₅₉H₁₁₂NaO₁₆P (1131.49)
- 1908.): 1,2-Di-(Z)-10-Tricosenoyl-*sn*-glycero-3-phospho-glycero-glycero-glycero-glycerin; Na-Salz
C₆₁H₁₁₆NaO₁₆P (1159.55)
- 1909.): 1,2-Di-(Z)-10-Tetracosenoyl-sn-glycero-3-phospho-glycero-glycero-glycero-glycerin; Na-Salz
C₆₃H₁₂₀NaO₁₆P (1187.60)
- 1910.): 1,2-Di-(Z)-15-Pentacosenoyl-*sn*-glycero-3-phospho-glycero-glycero-glycero-glycerin; Na-Salz
C₆₅H₁₂₄NaO₁₆P (1215.65)
- 1911.): 1,2-Di-(Z)-16-Hexacosenoyl-*sn*-glycero-3-phospho-glycero-glycero-glycero-glycerin; Na-Salz
C₆₇H₁₂₈NaO₁₆P (1243.71)
- 1912.): 1,2-Di-(Z,Z)-5,11-Hexadecadienoyl-*sn*-glycero-3-phospho-glycero-glycero-glycero-glycerin; Na-Salz
C₄₇H₈₄NaO₁₆P (959.14)
- 1913.): 1,2-Di-(Z,Z)-5,11-Octadecadienoyl-*sn*-glycero-3-phospho-glycero-glycero-glycero-glycerin; Na-Salz
C₅₁H₉₂NaO₁₆P (1015.25)
- 1914.): 1,2-Di-(Z,Z)-6,12-Nonadecadienoyl-sn-glycero-3-phospho-glycero-glycero-glycero-glycerin; Na-Salz
C₅₃H₉₆NaO₁₆P (1043.30)
- 1915.): 1,2-Di-(Z,Z)-10,16-Eicosadienoyl-sn-glycero-3-phospho-glycero-glycero-glycero-glycerin; Na-Salz
C₅₅H₁₀₀NaO₁₆P (1071.35)
- 1916.): 1,2-Di-(Z,Z)-10,16-Docosadienoyl-*sn*-glycero-3-phospho-glycero-glycero-glycero-glycerin; Na-Salz
C₅₉H₁₀₈NaO₁₆P (1127.46)
- 1917.): 1,2-Di-(Z,Z)-6,18-Tetracosadienoyl-*sn*-glycero-3-phospho-glycero-glycero-glycero-glycerin; Na-Salz
C₆₃H₁₁₆NaO₁₆P (1183.57)
- 1918.): 1,2-Di-(Z,Z)-10,16-Pentacosadienoyl-*sn*-glycero-3-phospho-glycero-glycero-glycero-glycerin; Na-Salz
C₆₅H₁₂₀NaO₁₆P (1211.62)
- 1919.): 1,2-Di-(Z,Z)-6,18-Hexacosadienoyl-*sn*-glycero-3-phospho-glycero-glycero-glycero-glycerin; Na-Salz
C₆₇H₁₂₄NaO₁₆P (1239.68)
- 1920.): 2-(Z)-6-Hexadecenoyl-1-stearoyl-*sn*-glycero-3-phospho-glycero-glycero-glycero-glycerin; Na-Salz
C₄₉H₉₄NaO₁₆P (993.24)
- 1921.): 2-(Z)-10-Octadecenoyl-1-stearoyl-*sn*-glycero-3-phospho-glycero-glycero-glycero-glycerin; Na-Salz
C₅₁H₉₈NaO₁₆P (1021.29)
- 1922.): 2-(Z)-10-Eicosenoyl-1-stearoyl-*sn*-glycero-3-phospho-glycero-glycero-glycero-glycerin; Na-Salz
C₅₃H₁₀₂NaO₁₆P (1049.35)
- 1923.): 2-(Z,Z)-6,12-Hexadecadienoyl-1-stearoyl-*sn*-glycero-3-phospho-glycero-glycero-glycero-glycerin; Na-Salz
C₄₉H₉₂NaO₁₆P (991.22)
- 1924.): 2-(Z,Z)-10,16-Docosadienoyl-1-stearoyl-*sn*-glycero-3-phospho-glycero-glycero-glycero-glycerin; Na-Salz
C₅₅H₁₀₄NaO₁₆P (1075.38)
- 1925.): 1-Stearoyl-2-(Z,Z)-6,18-tetracosadienoyl-*sn*-glycero-3-phospho-glycero-glycero-glycero-glycerin; Na-Salz
C₅₇H₁₀₈NaO₁₆P (1103.44)
- 1926.): 1-(Z)-10-Octadecenoyl-2-stearoyl-*sn*-glycero-3-phospho-glycero-glycero-glycero-glycerin; Na-Salz
C₅₁H₉₈NaO₁₆P (1021.29)
- 1927.): 1-(Z,Z)-6,18-Hexacosadienoyl-2-stearoyl-*sn*-glycero-3-phospho-glycero-glycero-glycero-glycerin; Na-Salz
C₅₉H₁₁₂NaO₁₆P (1131.49)
- 1928.): 1-(Z,Z)-6,18-Hexacosadienoyl-2-(Z)-6-Hexadecenoyl-*sn*-glycero-3-phospho-glycero-glycero-glycero-glycerin; Na-Salz
C₅₇H₁₀₆NaO₁₆P (1101.42)
- 1929.): 2-(Z,Z)-6,18-Hexacosadienoyl-1-(Z)-6-Hexadecenoyl-*sn*-glycero-3-phospho-glycero-glycero-glycero-glycerin; Na-Salz
C₅₇H₁₀₆NaO₁₆P (1101.42)

### 9. Beispiele für Phospho-sn-G₁-Verknüpfungen

### sn-1-G₁-G₂-Verbindungen

- 1930.): 1,2-Di-(Z)-6-Octadecenoyl-*sn*-glycero-3-phospho-*sn*-1-glycero-glycerin; Na-Salz
C₄₅H₈₄NaO₁₂P (871.12)
- 1931.): 1,2-Di-(Z)-6-Nonadecenoyl-*sn*-glycero-3-phospho-*sn*-1-glycero-glycerin; Na-Salz
C₄₇H₈₈NaO₁₂P (899.17)
- 1932.): 1,2-Di-(Z)-10-Docosenoyl-*sn*-glycero-3-phospho-*sn*-1-glycero-glycerin; Na-Salz
C₅₃H₁₀₀NaO₁₂P (983.33)
- 1933.): 1,2-Di-(Z)-12-Docosenoyl-*sn*-glycero-3-phospho-*sn*-1-glycero-glycerin; Na-Salz
C₅₃H₁₀₀NaO₁₂P (983.33)
- 1934.): 1,2-Di-(Z)-10-Tetracosenoyl-*sn*-glycero-3-phospho-*sn*-1-glycero-glycerin; Na-Salz
C₅₇H₁₀₈NaO₁₂P (1039.44)
- 1935.): 1,2-Di-(Z)-16-Hexacosenoyl-*sn*-glycero-3-phospho-*sn*-1-glycero-glycerin; Na-Salz
C₆₁H₁₁₆NaO₁₂P (1095.55)
- 1936.): 1,2-Di-(Z,Z)-5,11-Octadecadienoyl-*sn*-glycero-3-phospho-*sn*-1-glycero-glycerin; Na-Salz
C₄₅H₈₀NaO₁₂P (867.09)
- 1937.): 1,2-Di-(Z,Z)-10,16-Docosadienoyl-*sn*-glycero-3-phospho-*sn*-1-glycero-glycerin; Na-Salz
C₅₃H₉₆NaO₁₂P (979.30)
- 1938.): 1,2-Di-(Z,Z)-6,18-Tetracosadienoyl-*sn*-glycero-3-phospho-*s*n-1-glycero-glycerin; Na-Salz
C₅₇H₁₀₄NaO₁₂P (1035.41)
- 1939.): 1,2-Di-(Z,Z)-6,18-Hexacosadienoyl-*sn*-glycero-3-phospho-sn-1-glycero-glycerin; Na-Salz
C₆₁H₁₁₂NaO₁₂P (1091.52)
- 1940.): 2-(Z)-10-Octadecenoyl-1-stearoyl-*sn*-glycero-3-phospho-*sn*-1-glycero-glycerin; Na-Salz
C₄₅H₈₆NaO₁₂P (873.13)
- 1941.): 2-(Z)-10-Eicosenoyl-1-stearoyl-*sn*-glycero-3-phospho-*sn*-1-glycero-glycerin; Na-Salz
C₄₇H₉₀NaO₁₂P (901.19)
- 1942.): 2-(Z,Z)-6,12-Hexadecadienoyl-1-stearoyl-*sn*-glycero-3-phospho-*sn*-1-glycero-glycerin; Na-Salz
C₄₃H₈₀NaO₁₂P (843.06)
- 1943.): 2-(Z,Z)-10,16-Docosadienoyl-1-stearoyl-*sn*-glycero-3-phospho-*sn*-1-glycero-glycerin; Na-Salz
C₄₉H₉₂NaO₁₂P (927.23)
- 1944.): 1-(Z,Z)-6,18-Hexacosadienoyl-2-stearoyl-*sn*-glycero-3-phospho-*sn*-1-glycero-glycerin; Na-Salz
C₅₃H₁₀₀NaO₁₂P (983.33)

### sn-1-G₁-G₂-G₃-Verbindungen

- 1945.): 1,2-Di-(Z)-6-Octadecenoyl-*sn*-glycero-3-phospho-*sn*-1-glycero-glycero-glycerin; Na-Salz
C₄₈H₉₀NaO₁₄P (945.20)
- 1946.): 1,2-Di-(Z)-6-Nonadecenoyl-*sn*-glycero-3-phospho-*sn*-1-glycero-glycero-glycerin; Na-Salz
C₅₀H₉₄NaO₁₄P (973.25)
- 1947.): 1,2-Di-(Z)-10-Docosenoyl-*sn*-glycero-3-phospho-*sn*-1-glycero-glycero-glycerin; Na-Salz
C₅₆H₁₀₆NaO₁₄P (1057.41)
- 1948.): 1,2-Di-(Z)-12-Docosenoyl-*sn*-glycero-3-phospho-*sn*-1-glycero-glycero-glycerin; Na-Salz
C₅₆H₁₀₆NaO₁₄P (1057.41)
- 1949.): 1,2-Di-(Z)-10-Tetracosenoyl-*sn*-glycero-3-phospho-*sn*-1-glycero-glycero-glycerin; Na-Salz
C₆₀H₁₁₄NaO₁₄P (1113.52)
- 1950.): 1,2-Di-(Z)-16-Hexacosenoyl-*sn*-glycero-3-phospho-*sn*-1-glycero-glycero-glycerin; Na-Salz
C₆₄H₁₂₂NaO₁₄P (1169.63)
- 1951.): 1,2-Di-(Z,Z)-5,11-Octadecadienoyl-*sn*-glycero-3-phospho-*sn*-1-glycero-glycero-glycerin; Na-Salz
C₄₈H₈₆NaO₁₄P (941.17)
- 1952.): 1,2-Di-(Z,Z)-10,16-Docosadienoyl-*sn*-glycero-3-phospho-*sn*-1-glycero-glycero-glycerin; Na-Salz
C₅₆H₁₀₂NaO₁₄P (1053.38)
- 1953.): 1,2-Di-(Z,Z)-6,18-Tetracosadienoyl-*sn*-glycero-3-phospho-*sn*-1-glycero-glycero-glycerin; Na-Salz
C₆₀H₁₁₀NaO₁₄P (1109.49)
- 1954.): 1,2-Di-(Z,Z)-6,18-Hexacosadienoyl-*sn*-glycero-3-phospho-*sn*-1-glycero-glycero-glycerin; Na-Salz
C₆₄H₁₁₈NaO₁₄P (1165.60)
- 1955.): 2-(Z)-10-Octadecenoyl-1-stearoyl-*sn*-glycero-3-phospho-*sn*-1-glycero-glycero-glycerin; Na-Salz
C₄₈H₉₂NaO₁₄P (947.21)
- 1956.): 2-(Z)-10-Eicosenoyl-1-stearoyl-*sn*-glycero-3-phospho-*sn*-1-glycero-glycero-glycerin; Na-Salz
C₅₀H₉₆NaO₁₄P (975.27)
- 1957.): 2-(Z,Z)-6,12-Hexadecadienoyl-1-stearoyl-*sn*-glycero-3-phospho-*sn*-1-glycero-glycero-glycerin; Na-Salz
C₄₆H₈₆NaCl₁₄P (917.14)
- 1958.): 2-(Z,Z)-10,16-Docosadienoyl-1-stearoyl-*sn*-glycero-3-phospho-*sn*-1-glycero-glycero-glycerin; Na-Salz
C₅₂H₉₈NaO₁₄P (1001.31)
- 1959.): 1-(Z,Z)-6,18-Hexacosadienoyl-2-stearoyl-*sn*-glycero-3-phospho-*sn*-1-glycero-glycero-glycerin; Na-Salz
C₅₆H₁₀₆NaO₁₄P (1057.41)

### sn-1-G₁-G₂-G₃-G₄-Verbindungen

- 1960.): 1,2-Di-(Z)-6-Octadecenoyl-*sn*-glycero-3-phospho-*sn*-1-glycero-glycero-glycero-glycerin; Na-Salz
C₅₁H₉₆NaO₁₆P (1019.28)
- 1961.): 1,2-Di-(Z)-6-Nonadecenoyl-*sn*-glycero-3-phospho-*sn*-1-glycero-glycero-glycero-glycerin; Na-Salz
C₅₃H₁₀₀NaO₁₆P (1047.33)
- 1962.): 1,2-Di-(Z)-10-Docosenoyl-*sn*-glycero-3-phospho-*sn*-1-glycero-glycero-glycero-glycerin; Na-Salz
C₅₉H₁₁₂NaO₁₆P (1131.49)
- 1963.): 1,2-Di-(Z)-12-Docosenoyl-*sn*-glycero-3-phospho-*sn*-1-glycero-glycero-glycero-glycerin; Na-Salz
C₅₉H₁₁₂NaO₁₆P (1131.49)
- 1964.): 1,2-Di-(Z)-10-Tetracosenoyl-*sn*-glycero-3-phospho-*sn*-1-glycero-glycero-glycero-glycerin; Na-Salz
C₆₃H₁₂₀NaO₁₆P (1187.60)
- 1965.): 1,2-Di-(2)-16-Hexacosenoyl-*sn*-glycero-3-phospho-*sn*-1-glycero-glycero-glycero-glycerin; Na-Salz
C₆₇H₁₂₈NaO₁₆P (1243.71)
- 1966.): 1,2-Di-(Z,Z)-5,11-Octadecadienoyl-*sn*-glycero-3-phospho-*sn*-1-glycero-glycero-glycero-glycerin; Na-Salz
C₅₁H₉₂NaO₁₆P (1015.25)
- 1967.): 1,2-Di-(Z,Z)-10,16-Docosadienoyl-*sn*-glycero-3-phospho-*sn*-1-glycero-glycero-glycero-glycerin; Na-Salz
C₅₉H₁₀₈NaO₁₆P (1127.46)
- 1968.): 1,2-Di-(Z,Z)-6,18-Tetracosadienoyl-*sn*-glycero-3-phospho-*sn*-1-glycero-glycero-glycero-glycerin; Na-Salz
C₆₃H₁₁₆NaO₁₆P (1183.57)
- 1969.): 1,2-Di-(Z,Z)-6,18-Hexacosadienoyl-*sn*-glycero-3-phospho-*sn*-1-glycero-glycero-glycero-glycerin; Na-Salz
C₆₇H₁₂₄NaO₁₆P (1239.68)
- 1970.): 2-(Z)-10-Octadecenoyl-1-stearoyl-*sn*-glycero-3-phospho-*sn*-1-glycero-glycero-glycero-glycerin; Na-Salz
C₅₁H₉₈NaO₁₆P (1021.29)
- 1971.): 2-(Z)-10-Eicosenoyl-1-stearoyl-*sn*-glycero-3-phospho-*sn*-1-glycero-glycero-glycero-glycerin; Na-Salz
C₅₃H₁₀₂NaO₁₆P (1049.35)
- 1972.): 2-(Z,Z)-6,12-Hexadecadienoyl-1-stearoyl-*sn*-glycero-3-phospho-*sn*-1-glycero-glycero-glycero-glycerin; Na-Salz
C₄₉H₉₂NaO₁₆P (991.22)
- 1973.): 2-(Z,Z)-10,16-Docosadienoyl-1-stearoyl-*sn*-glycero-3-phospho-*sn*-1-glycero-glycero-glycero-glycerin; Na-Salz
C₅₅H₁₀₄NaO₁₆P (1075.38)
- 1974.): 1-(Z,Z)-6,18-Hexacosadienoyl-2-stearoyl-*sn*-glycero-3-phospho-*sn*-1-glycero-glycerö-glycero-glycerin; Na-Salz
C₅₉H₁₁₂NaO₁₆P (1131.49)

### Verknüpfungen mit Zuckeralkoholen

### 10. Phospho-D-mannit-Verbindungen

### (A = III; m= 0, x = 0; y = 4; z = 1)

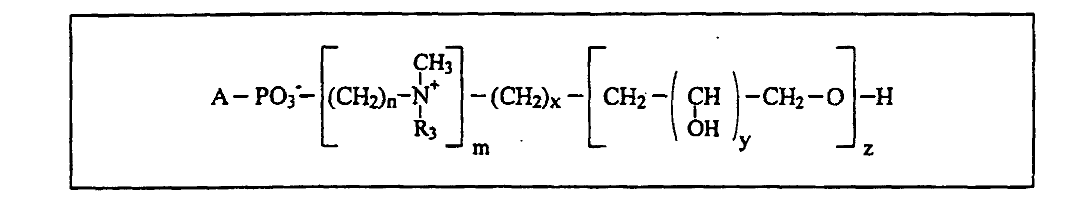

- 1975.): 1,2-Di-(Z)-6-Hexadecenoyl-*sn*-glycero-3-phospho-D-mannit; Na-Salz
C₄₁H₇₆NaO₁₃P (831.01)
- 1976.): 1,2-Di-(Z)-6-Nonadecenoyl-*sn*-glycero-3-phospho-D-mannit; Na-Salz
C₄₇H₈₈NaO₁₃P (915.17)
- 1977.): 1,2-Di-(Z)-12-Eicosenoyl-*sn*-glycero-3-phospho-D-mannit; Na-Salz
C₄₉H₉₂NaO₁₃P (943.23)
- 1978.): 1,2-Di-(Z)-10-Docosenoyl-*sn*-glycero-3-phospho-D-mannit; Na-Salz
C₅₃H₁₀₀NaO₁₃P (999.33)
- 1979.): 1,2-Di-(Z)-12-Docosenoyl-*sn*-glycero-3-phospho-D-mannit; Na-Salz
C₅₃H₁₀₀NaO₁₃P (999.33)
- 1980.): 1,2-Di-(Z)-10-Tetracosenoyl-sn-glycero-3-phospho-D-mannit; Na-Salz
C₅₇H₁₀₈NaO₁₃P (1055.44)
- 1981.): 1,2-Di-(Z)-16-Hexacosenoyl-*sn*-glycero-3-phospho-D-mannit; Na-Salz
C₆₁H₁₁₆NaO₁₃P (1111.55)
- 1982.): 1,2-Di-(Z,Z)-5,11-Hexadecadienoyl-*sn*-glycero-3-phospho-D-mannit; Na-Salz
C₄₁H₇₂NaO₁₃P (826.98)
- 1983.): 1,2-Di-(Z,Z)-5,11-Octadecadienoyl-*sn*-glycero-3-phospho-D-mannit; Na-Salz
C₄₅H₈₀NaO₁₃P (883.09)
- 1984.): 1,2-Di-(Z,Z)-6,12-Nonadecadienoyl-*sn*-glycero-3-phospho-D-mannit; Na-Salz
C₄₇H₈₄NaO₁₃P (911.14)
- 1985.): 1,2-Di-(Z,Z)-10,16-Docosadienoyl-*sn*-glycero-3-phospho-D-mannit; Na-Salz
C₅₃H₉₆NaO₁₃P (995.30)
- 1986.): 1,2-Di-(Z,Z)-6,18-Hexacosadienoyl-*sn*-glycero-3-phospho-D-mannit; Na-Salz
C₆₁H₁₁₂NaO₁₃P (1107.52)
- 1987.): 2-(Z)-6-Hexadecenoyl-1-stearoyl-*sn*-glycero-3-phospho-D-mannit; Na-Salz
C₄₃H₈₂NaO₁₃P (861.08)
- 1988.): 2-(Z)-10-Octadecenoyl-1-stearoyl-*sn*-glycero-3-phospho-D-mannit; Na-Salz
C₄₅H₈₆NaO₁₃P (889.13)
- 1989.): 2-(Z,Z)-6,12-Hexadecadienoyl-1-stearoyl-*sn*-glycero-3-phospho-D-mannit; Na-Salz
C₄₃H₈₀NaO₁₃P (859.06)
- 1990.): 2-(Z,Z)-10,16-Docosadienoyl-1-stearoyl-*sn*-glycero-3-phospho-D-mannit; Na-Salz
C₄₉H₉₂NaO₁₃P (943.23)
- 1991.): 1-Stearoyl-2-(Z,Z)-6,18-tetracosadienoyl-*sn*-glycero-3-phospho-D-mannit; Na-Salz
C₅₁H₉₆NaO₁₃P (971.28)
- 1992.): 1-(Z)-10-Octadecenoyl-2-stearoyl-*sn*-glycero-3-phaspho-D-mannit; Na-Salz
C₄₅H₈₆NaO₁₃P (889.13)
- 1993.): 1-(Z,Z)-6,18-Hexacosadienoyl-2-stearoyl-*sn*-glycero-3-phospho-D-mannit; Na-Salz
C₅₃H₁₀₀NaO₁₃P (999.33)
- 1994.): 1-(Z,Z)-6,18-Hexacosadienoyl-2-(Z)-6-Hexadecenoyl-*sn*-glycero-3-phospho-D-mannit; Na-Salz
C₅₁H₉₄NaO₁₃P (969.26)
- 1995.): 2-(Z,Z)-6,18-Hexacosadienoyl-1-(Z)-6-Hexadecenoyl-*sn*-glycero-3-phospho-D-mannit; Na-Salz
C₅₁H₉₄NaO₁₃P (969.26)
- 1996.): 1-(Z)-12-Docosenoyl-*sn*-glycero-3-phospho-D-mannit; Na-Salz
C₃₁H₆₀NaO₁₂P (678.77)
- 1997.): 1-(Z,Z)-10,16-Docosadienoyl-*sn*-glycero-3-phospho-D-mannit; Na-Salz
C₃₁H₅₈NaO₁₂P (676.76)
- 1998.): 1-(Z)-12-Docosenyl-phospho-D-mannit; Na-Salz
C₂₈H₅₆NaO₉P (590.71)
- 1999.): 1-(Z,Z)-10,16-Docosadienyl-phospho-D-mannit; Na-Salz
C₂₈H₅₄NaO₉P (588.69)
- 2000.): 1-O-(Z)-10-Docosenyl-2-O-methyl-sn-glycero-3-phospho-D-mannit; Na-Salz
C₃₂H₆₄NaO₁₁P (678.82)
- 2001.): 1-O-(Z,Z)-10,16-Docosadienyl-2-O-methyl-sn-glycero-3-phospho-D-mannit; Na-Salz
C₃₂H₆₂NaO₁₁P (676.80)

### 11. Phospho-D-lyxit-Verbindungen

### (A = III; m= 0, x = 0; y = 3; z = 1)

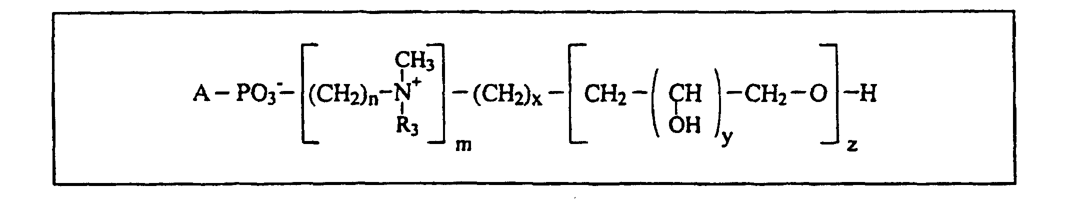

- 2002.): 1,2-Di-(Z)-6-Hexadecenoyl-*sn*-glycero-3-phospho-D-lyxit; Na-Salz
C₄₀H₇₄NaO₁₂P (800.98)
- 2003.): 1,2-Di-(Z)-6-Nonadecenoyl-*sn*-glycero-3-phospho-D-lyxit; Na-Salz
C₄₆H₈₆NaO₁₂P (885.15)
- 2004.): 1,2-Di-(Z)-10-Docosenoyl-*sn*-glycero-3-phospho-D-lyxit; Na-Salz
C₅₂H₉₈NaO₁₂P (969.31)
- 2005.): 1,2-Di-(Z)-10-Tetracosenoyl-*sn*-glycero-3-phospho-D-lyxit; Na-Salz
C₅₆H₁₀₆NaO₁₂P (1025.41)
- 2006.): 1,2-Di-(Z)-16-Hexacosenoyl-*sn*-glycero-3-phospho-D-lyxit; Na-Salz
C₆₀H₁₁₄NaO₁₂P (1081.52)
- 2007.): 1,2-Di-(Z,Z)-5,11-Hexadecadienoyl-*sn*-glycero-3-phospho-D-lyxit; Na-Salz
C₄₀H₇₀NaO₁₂P (796.95)
- 2008.): 1,2-Di-(Z,Z)-5,11-Octadecadienoyl-*sn*-glycero-3-phospho-D-lyxit; Na-Salz
C₄₄H₇₈NaO₁₂P (853.06)
- 2009.): 1,2-Di-(Z,Z)-10,16-Docosadienoyl-*sn*-glycero-3-phospho-D-lyxit; Na-Salz
C₅₂H₉₄NaO₁₂P (965.27)
- 2010.): 1,2-Di-(Z,Z)-6,18-Hexacosadienoyl-*sn*-glycero-3-phospho-D-lyxit; Na-Salz
C₆₀H₁₁₀NaO₁₂P (1077.49)
- 2011.): 2-(Z)-6-Hexadecenoyl-1-stearoyl-*sn*-glycero-3-phospho-D-lyxit; Na-Salz
C₄₂H₈₀NaO₁₂P (831.05)
- 2012.): 2-(Z)-10-Octadecenoyl-1-stearoyl-*sn*-glycero-3-phospho-D-lyxit; Na-Salz
C₄₄H₈₄NaO₁₂P (859.11)
- 2013.): 2-(Z,Z)-6,12-Hexadecadienoyl-1-stearoyl-*sn*-glycero-3-phospho-D-lyxit; Na-Salz
C₄₂H₇₈NaO₁₂P (829.04)
- 2014.): 2-(Z,Z)-10,16-Docosadienoyl-1-stearoyl-*sn*-glycero-3-phospho-D-lyxit; Na-Salz
C₄₈H₉₀NaO₁₂P (913.20)
- 2015.): 1-Stearoyl-2-(Z,Z)-6,18-tetracosadienoyl-*sn*-glycero-3-phospho-D-lyxit; Na-Salz
C₅₀H₉₄NaO₁₂P (941.25)
- 2016.): 1-(Z)-10-Octadecenoyl-2-stearoyl-*sn*-glycero-3-phospho-D-lyxit; Na-Salz
C₄₄H₈₄NaO₁₂P (859.11)
- 2017.): 1-(Z,Z)-6,18-Hexacosadienoyl-2-stearoyl-*sn*-glycero-3-phospho-D-lyxit; Na-Salz
C₅₂H₉₈NaO₁₂P (969.31)
- 2018.): 1-(Z,Z)-6,18-Hexacosadienoyl-2-(Z)-6-Hexadecenoyl-sn-glycero-3-phospho-D-lyxit; Na-Salz
C₅₀H₉₂NaO₁₂P (939.24)
- 2019.): 2-(Z,Z)-6,18-Hexacosadienoyl-1-(Z)-6-Hexadecenoyl-sn-glycero-3-phospho-D-lyxit; Na-Salz
C₅₀H₉₂NaO₁₂P (939.24)

### 12. Phospho-D-threit-Verbindungen

### (A = III; m= 0, x = 0; y = 2; z = 1)

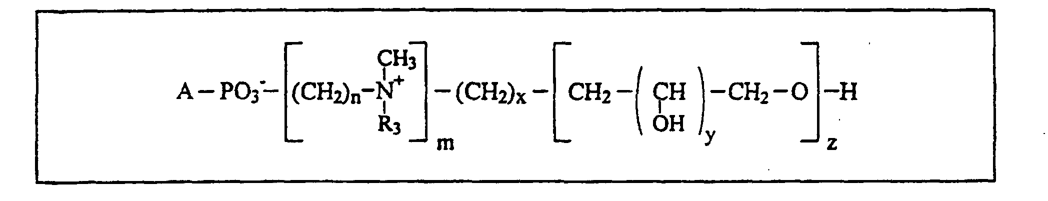

- 2020.): 1,2-Di-(Z)-6-Hexadecenoyl-*sn*-glycero-3-phospho-D-threit; Na-Salz
C₃₉H₇₂NaO₁₁P (770.96)
- 2021.): 1,2-Di-(Z)-6-Nonadecenoyl-*sn*-glycero-3-phospho-D-threit; Na-Salz
C₄₅H₈₄NaO₁₁P (855.12)
- 2022.): 1,2-Di-(Z)-10-Docosenoyl-*sn*-glycero-3-phospho-D-threit; Na-Salz
C₅₁H₉₆NaO₁₁P (939.28)
- 2023.): 1,2-Di-(Z)-10-Tetracosenoyl-*sn*-glycero-3-phospho-D-threit; Na-Salz
C₅₅H₁₀₄NaO₁₁P (995.39)
- 2024.): 1,2-Di-(Z)-16-Hexacosenoyl-*sn*-glycero-3-phospho-D-threit; Na-Salz
C₅₉H₁₁₂NaO₁₁P (1051.50)
- 2025.): 1,2-Di-(Z,Z)-5,11-Hexadecadienoyl-*sn*-glycero-3-phospho-D-threit; Na-Salz
C₃₉H₆₈NaO₁₁P (766.93)
- 2026.): 1,2-Di-(Z,Z)-5,11-Octadecadienoyl-*sn*-glycero-3-phospho-D-threit; Na-Salz
C₄₃H₇₆NaO₁₁P (823.03)
- 2027.): 1,2-Di-(Z,Z)-10,16-Docosadienoyl-*sn*-glycero-3-phospho-D-threit; Na-Salz
C₅₁H₉₂NaO₁₁P (935.25)
- 2028.): 1,2-Di-(Z,Z)-6,18-Hexacosadienoyl-*sn*-glycero-3-phospho-D-threit; Na-Salz
C₅₉H₁₀₈NaO₁₁P (1047.46)
- 2029.): 2-(Z)-6-Hexadecenoyl-1-stearoyl-*sn*-glycero-3-phospho-D-threit; Na-Salz
C₄₁H₇₈NaO₁₁P (801.03)
- 2030.): 2-(Z)-10-Octadecenoyl-1-stearoyl-*sn*-glycero-3-phospho-D-threit; Na-Salz
C₄₃H₈₂NaO₁₁P (829.08)
- 2031.): 2-(Z,Z)-6,12-Hexadecadienoyl-1-stearoyl-*sn*-glycero-3-phospho-D-threit; Na-Salz
C₄₁H₇₆NaO₁₁P (799.01)
- 2032.): 2-(Z,Z)-10,16-Docosadienoyl-1-stearoyl-*sn*-glycero-3-phospho-D-threit; Na-Salz
C₄₇H₈₈NaO₁₁P (883.17)
- 2033.): 1-Stearoyl-2-(Z,Z)-6,18-tetracosadienoyl-*sn*-glycero-3-phospho-D-threit; Na-Salz
C₄₉H₉₂NaO₁₁P (911.23)
- 2034.): 1-(Z)-10-Octadecenoyl-2-stearoyl-*sn*-glycero-3-phospho-D-threit; Na-Salz
C₄₃H₈₂NaO₁₁P (829.08)
- 2035.): 1-(Z,Z)-6,18-Hexacosadienoyl-2-stearoyl-*sn*-glycero-3-phospho-D-threit; Na-Salz
C₅₁H₉₆NaO₁₁P (939.28)
- 2036.): 1-(Z,Z)-6,18-Hexacosadienoyl-2-(Z)-6-Hexadecenoyl-*sn*-glycero-3-phospho-D-threit; Na-Salz
C₄₉H₉₀NaO₁₁P (909.21)
- 2037.): 2-(Z,Z)-6,18-Hexacosadienoyl-1-(Z)-6-Hexadecenoyl-*sn*-glycero-3-phospho-D-threit; Na-Salz
C₄₉H₉₀NaO₁₁P (909.21)

### Quellenangaben:

### [1] Kaufmann-Kolle, P., Berger M.R., Unger, C. und H.Eibl

Systemicadministrationofalkylphosphocholines: Erucylphosphocholineand liposomal hexadecylphosphocholine
*Adv. Exp. Med. Biol. 416, 165-168 (1996)*

## Patentansprüche

1. Verbindung der allgemeinen Formel (I)
(I) A - PO₃⁻ - B
worin B einen Rest der allgemeinen Formel (II) darstellt 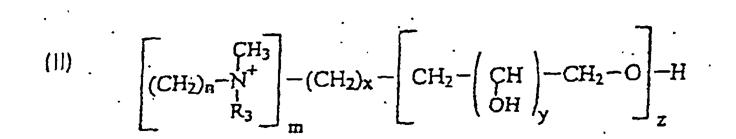 worin
n eine ganze Zahl *von* 2 bis 8 ist;
m 0, 1 oder 2 ist;
x eine ganze Zahl von 0 bis 8 ist;
y eine ganze Zahl von 1 bis 4 ist;
z eine ganze Zahl von 0 bis 5 ist;
R₃ einen Alkylrest mit 1 bis 3 C-Atomen darstellt, der mit einer oder mehreren Hydroxylgruppen substituiert sein kann;
und worin A einen Rest, ausgewählt aus einer der Formeln (III) bis (VII) darstellt: 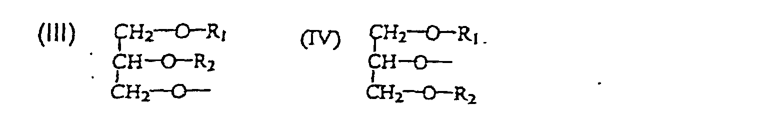 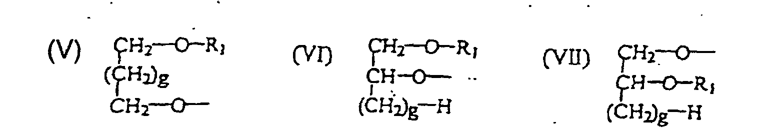 worin
g eine ganze Zahl von 0 bis 8 ist;
p, q, r, s ≥ 0;
12 ≤ p + q ≤ 30 und
8 ≤ s + t + r ≤ 26 ist;
wobei R₁ und R₂ jeweils unabhängig Wasserstoff, einen gesättigten oder ungesättigten Acyl- oder Alkylrest oder einen Rest, ausgewählt aus einer der Formeln (X), (XI), (XII) und (XIII), darstellen und mindestens einer von R₁ und R₂ einen Rest, ausgewählt aus einer der. Formeln (X), (XI), (XII) und (XIII), darstellt: 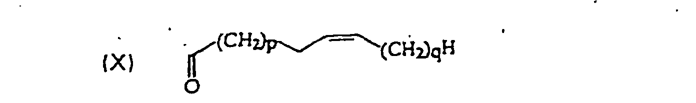 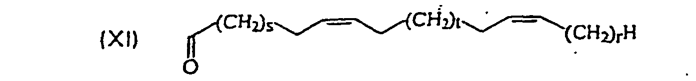 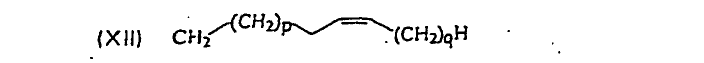 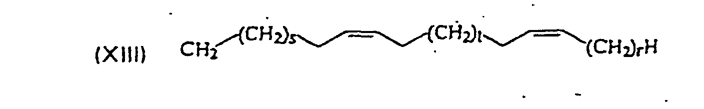 wobei q ≠ 8 für p + q = 14, 16, 18 oder 20 ist, wenn keiner der Reste R₁ und R₂ einen Rest der Formel (XI) oder (XIII) darstellt,
t ≥ 0 ist und p verschieden ist von der Bedeutung von p in den natürlich vorkommenden entsprechenden Resten.

2. Verbindung nach Anspruch 1, worin
für B gilt:
m = 1.

3. Verbindung nach Anspruch 2, worin
für B gilt:
m = 1;
x = 1 bis 3;
z = 0.

4. Verbindung nach Anspruch 3, worin
für B gilt
m = 1;
x = 1;
z = 0.

5. Verbindung nach Anspruch 1, 'worin
für B gilt
m = 1;
x = 0;
y = 1;
z = 1 bis 5.

6. Verbindung nach Anspruch 5, worin
für B gilt:
m = 1;
x = 0;
y = 1;
z = 1 bis 3.

7. Verbindung nach Anspruch 1, worin
für B gilt:
m = 1;
x = 0;
y = 2 bis 4;
z = 1.

8. Verbindung nach Anspruch 1, worin
für B gilt:
m = 0;
x = 0;
y = 1;
z = 1 bis 5.

9. Verbindung nach Anspruch 1, worin
für B gilt:
m = 0;
x = 0;
y = 2 bis 4;
z = 1.

10. Verbindung nach einem der vorhergehenden Ansprüche, worin
für B gilt:
R₃ = CH₃.

11. Verbindung nach einem der Ansprüche 1 bis 9, worin
für B gilt:
R₃ = 1,2-Dihydroxypropyl.

12. Verbindung nach einem der vorhergehenden Ansprüche, worin
für B gilt:
n = 2 bis 6.

13. Verbindung nach einem der vorhergehenden Ansprüche, worin
für B gilt:
n = 3.

14. Verbindung nach einem der Ansprüche 1 bis 13, worin
A einen Rest, ausgewählt aus einer der Formeln (III) bis (VII), darstellt und R₁ und R₂ jeweils unabhängig einen Rest, ausgewählt aus einer der Formeln (X) bis (XIII), darstellen.

15. Verbindung nach Anspruch 14, worin
für 8 gilt:
x = 1 und z = 0.

16. Verbindung nach Anspruch 14 oder 15, worin
A einen Rest der Formel (III) oder (IV) darstellt und R₁ und R₂ jeweils unabhängig einen Rest, ausgewählt aus einer der Formeln (X) bis (XIII), darstellen, wobei einer von R₁ und R₂ 16 bis 32 Kohlenstoffatome aufweist und einer von R₁ und R₂ 16 bis 26 Kohlenstoffatome aufweist.

17. Verbindung nach Anspruch 14 oder 15, worin
A einen Rest der Formel (III) oder (IV) darstellt und R₁ und R₂ beide einen Rest, ausgewählt aus einer der Formeln (X) bis (XIII), darstellen und 16 bis 26 Kohlenstoffatome aufweisen.

18. Verbindung nach Anspruch 14 oder 15, worin
A einen Rest der formel (III) oder (IV) darstellt und R₁ und R₂ jeweils unabhängig einen Rest der Formeln (X) bis (XIII) darstellen und 16 bis 24 Kohlenstoffatome aufweisen.

19. Verbindung nach einem der Ansprüche 14 bis 18, worin
R₁ und R₂ jeweils unabhängig einen Rest der Formel (X) 'oder (XI) darstellen.

20. Verbindung nach einem der Ansprüche 14 bis 18, worin
R₁ und R₂ jeweils unabhängig einen Rest der Formel (XII) oder (XIII) darstellen.

21. Verbindung nach Anspruch 14, 15, 17 oder 19, worin
R₁ und R₂ beide einen Rest der Formel (XI) darstellen.

22. Verbindung nach Anspruch 14, 15, 17 oder 20, worin
R₁ und R₂ beide einen Rest der Formel (XIII) darstellen.

23. Verbindung nach Anspruch 14 oder 15, worin
A einen Rest der Formel (III) oder (IV) darstellt und einer von R, und R₂ einen Alkylrest mit 1 bis 4 Kohlenstoffatomen darstellt.

24. Verbindung nach Anspruch 14 oder 15, worin
A einen Rest, ausgewählt aus einer der Formeln (III) oder (IV), darstellt und einer von R₁ und R₂ einen Wasserstoffrest darstellt.

25. Liposomen,
**dadurch gekennzeichnet daß**
sie als Liposomenhüllbestandteile Phospholipide und/oder Alkylphospholipide, gegebenenfalls Cholesterin und 1 bis 50 Mol-% einer Verbindung nach einem der Ansprüche 1, 14 bis 22 oder deren Salz umfassen, wobei das Cholesterin,.die Phospholipide, die Alkylphospholipide und die Verbindung zusammen 100 Mol-% der Liposomenhüllbestandteile ergeben.

26. Liposomen nach Anspruch 25,
**dadurch gekennzeichnet,daß**
sie zusätzlich einen Wirkstoff gegebenenfalls zusammen mit pharmazeutisch annehmbaren Verdünnungs-, Hilfs-, Träger-, und Füllstoffen enthalten.

27. Liposomen nach Anspruch 26,
**dadurch gekennzeichnet, daß**
der Wirkstoff eine Verbindung nach einem der Ansprüche 1 und 23 bis 24 ist.

28. Liposomen nach einem der Ansprüche 25 bis 27,
**dadurch gekennzeichnet, daß**
sie zusätzlich eine Nucleinsäure umfassen.

29. Pharmazeutische Zusammensetzung,
**dadurch gekennzeichnet, daß**
sie einen. Wirkstoff nach einem der Ansprüche 1 und 23 bis 25 gegebenenfalls zusammen mit pharmazeutisch annehmbaren Verdünnungs-, Hilfs-, Träger-, und Füllstoffen enthält.

30. Verfahren zur Herstellung von ungesättigten (Z)-Fettsäuren oder (Z)-Alkenolen entsprechend einem Rest nach einer der Formeln (X) und (XI) mit 16 bis 34 Kohlenstoffatomen,
**dadurch gekennzeichnet, daß**
man als Ausgangsprodukt ein Lacton der Formel (XIV) verwendet: 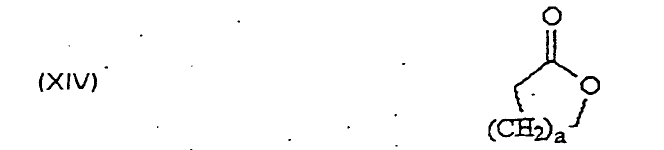 wobei a = 10 bis 16,
und daß es die Schritte umfaßt:
1) Spalten des Lactonringes mit einem Trimethylsilylhalogenid zu dem entsprechenden Halogen-Carbonsäuretrimethylsilylester,
2) gleichzeitige oder anschließende Alkoholyse des Halogen-Carbonsäuretrimethylsilylesters zudementsprechenden Halogen-Carbonsäureester,
3) Umsetzung des Halogen-Carbonsäureesters mit Triphenylphosphan zu dem entsprechenden Phosphoniumsalz,
4) Umsetzung des Phosphoniumsalzes mit einem Aldehyd unter Verwendung einer Base und anschließender Verseifung zu einem entsprechenden (Z)-Fettsäuresalz,
5) Freisetzung der (Z)-Fettsäure aus dem (Z)-Fettsäuresalz,
6) gegebenenfalls Umsetzung der (Z)-Fettsäure in das entsprechende (Z)-Alkenol mittels Lithiumaluminiumhydrid.

31. Verfahren nach Anspruch 30,
**dadurch gekennzeichnet, daß**
die (Z)-Fettsäure 15-(Z)-Tetracosensäure ist, wobei Cyclopentadecanolid als Ausgangslacton verwendet wird und in Schritt 4 Pelargonaldehyd als das Aldehyd verwendet wird.

32. Verbindung der allgemeinen Formel (I) nach einem der Ansprüche 1 bis 13, 23 und 24 zur Verwendung als cytostatischer Wirkstoff.

33. Verbindung der allgemeinen Formel (I) nach einem der Ansprüche 1 bis 13, 23 und 24 zur Verwendung als Wirkstoff gegen Protozoenerkrankungen wie etwa Leishmaniose und Trypanosomiasis.

34. Verbindung der allgemeinen Formel (I) nach einem der Ansprüche 1 bis 13 und 14 bis 22 zur Verwendung als Liposomenhüllbestandteil.

35. Verwendung einer Verbindung der allgemeinen Formel (I) nach einem der Ansprüche 1 bis 13 und 18 bis 22 als Lösungsvermittler für wasserunlösliche Wirkstoffe.

36. Liposomen nach Anspruch 28 zur Verwendung als Gentransportvehikel.

37. Liposomen nach Anspruch 26 zur Verwendung als Antitumormittel, wobei der Wirkstoff Doxorubicin ist.

38. Liposomen nach Anspruch 26 zur Verwendung als Mittel zur Beeinflussung der Zellproliferation, wobei der Wirkstoff ein Cytokin ist.

## Claims

1. Compound of the general formula (I)
(I) A - PO₃⁻ - B
in which B is a radical of the general formula (II) 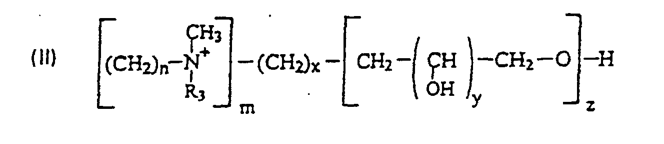 in which
n is an integer from 2 to 8;
m is 0, 1 or 2;
x is an integer from 0 to 8;
y is an integer from 1 to 4;
z is an integer from 0 to 5;
R₃ is an alkyl radical having 1 to 3 C atoms, which may be substituted by one or more hydroxyl groups;
and in which A is a radical selected from one of the formulae (III) to (VII): 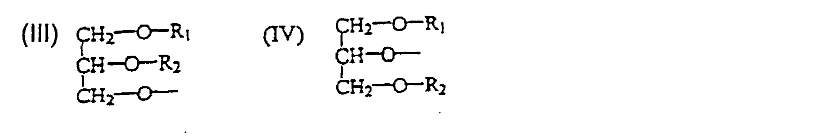 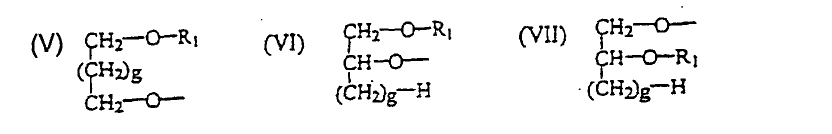 in which
g is an integer from 0 to 8;
p, q, r, s ≥ 0;
12 ≤ p + q ≤ 30 and
8 ≤ s + t + r ≤ 26;
where R₁ and R₂ are each independently hydrogen, a saturated or unsaturated acyl or alkyl radical or a radical selected from one of the formulae (X), (XI) , (XII) and (XIII), and at least one of R₁ and R₂ is a radical selected from one of the formulae (X), (XI), (XII) and (XIII): 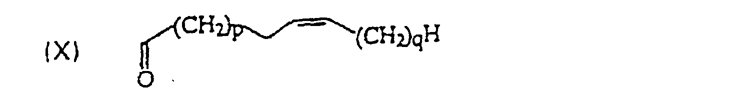 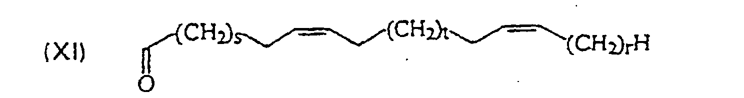 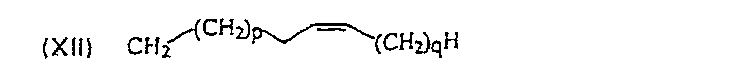 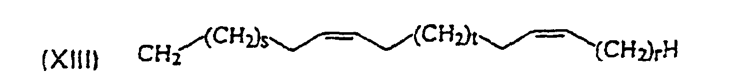 where q ≠ 8 for p + q = 14, 16, 18 or 20, if neither of the radicals R₁ and R₂ is a radical of the formula (XI) or (XIII), t is ≥ 0, and p is different from the meaning of p in the naturally occurring corresponding radicals.

2. Compound according to Claim 1, in which the following applies to B:
m = 1.

3. Compound according to Claim 2, in which the following applies to B:
m = 1;
x = 1 to 3;
z = 0.

4. Compound according to Claim 3, in which the following applies to B:
m = 1;
x = 1;
z = 0.

5. Compound according to Claim 1, in which the following applies to B:
m = 1;
x = 0;
y = 1;
z = 1 to 5.

6. Compound according to Claim 5, in which the following applies to B:
m = 1;
x = 0;
y = 1;
z = 1 to 3.

7. Compound according to Claim 1, in which the following applies to B:
m = 1;
x = 0;
y = 2 to 4;
z = 1.

8. Compound according to Claim 1, in which the following applies to B:
m = 0;
x = 0;
y = 1;
z = 1 to 5.

9. Compound according to Claim 1, in which the following applies to B:
m = 0;
x = 0;
y = 2 to 4;
z = 1.

10. Compound according to any of the preceding claims, in which the following applies to B:
R₃ = CH₃.

11. Compound according to any of Claims 1 to 9, in which the following applies to B:
R₃ = 1,2-dihydroxypropyl.

12. Compound according to any of the preceding claims, in which the following applies to B:
n = 2 to 6.

13. Compound according to any of the preceding claims, in which the following applies to B:
n = 3.

14. Compound according to any of Claims 1 to 13, in which A is a radical selected from one of the formulae (III) to (VII), and R₁ and R₂ are each independently a radical selected from one of the formulae (X) to (XIII).

15. Compound according to Claim 14, in which the following applies to B:
x = 1 and z = 0.

16. Compound according to Claim 14 or 15, in which A is a radical of the formula (III) or (IV), and R₁ and R₂ are each independently a radical selected from one of the formulae (X) to (XIII), where one of R₁ and R₂ has 16 to 32 carbon atoms and one of R₁ and R₂ has 16 to 26 carbon atoms.

17. Compound according to Claim 14 or 15, in which A is a radical of the formula (III) or (IV), and R₁ and R₂ are both a radical selected from one of the formulae (X) to (XIII) and have 16 to 26 carbon atoms.

18. Compound according to Claim 14 or 15, in which A is a radical of the formula (III) or (IV), and R₁ and R₂ are each independently a radical of the formulae (X) to (XIII) and have 16 to 24 carbon atoms.

19. Compound according to any of Claims 14 to 18, in which R₁ and R₂ are each independently a radical of the formula (X) or (XI).

20. Compound according to any of Claims 14 to 18, in which R₁ and R₂ are each independently a radical of the formula (XII) or (XIII).

21. Compound according to Claim 14, 15, 17 or' 19, in which R₁ and R₂ are both a radical of the formula (XI).

22. Compound according to Claim 14, 15, 17 or 20, in which R₁ and R₂ are both a radical of the formula (XIII).

23. Compound according to Claim 14 or 15, in which A is a radical of the formula (III) or (IV), and one of R₁ and R₂ is an alkyl radical having 1 to 4 carbon atoms.

24. Compound according to Claim 14 or 15, in which A is a radical selected from one of the formulae (III) or (IV), and one of R₁ and R₂ is a hydrogen radical.

25. Liposomes **characterized in that** they comprise as liposome shell constituents phospholipids and/or alkylphospholipids, where appropriate cholesterol and 1 to 50 mol% of a compound according to any of Claims 1, 14 to 22 or salt thereof, where the cholesterol, the phospholipids, the alkylphospholipids and the compound together result in 100 mol% of the liposome shell constituents.

26. Liposomes according to Claim 25, **characterized in that** additionally comprise an active ingredient, where appropriate together with pharmaceutically acceptable diluents, excipients, carriers and fillers.

27. Liposomes according to Claim 26, **characterized in that** the active ingredient is a compound according to any of Claims 1 and 23 to 24.

28. Liposomes according to any of Claims 25 to 27, **characterized in that** they additionally comprise a nucleic acid.

29. Pharmaceutical composition, **characterized in that** it comprises an active ingredient according to any of Claims 1 and 23 to 25, where appropriate together with pharmaceutically acceptable diluents, excipients, carriers and fillers.

30. A process for preparing unsaturated (Z)-fatty acids or (Z)-alkenols corresponding to a radical as set forth in either of the formulae (X) and (XI) having 16 to 34 carbon atoms, **characterized in that** the starting material used is a lactone of the formula (XIV): 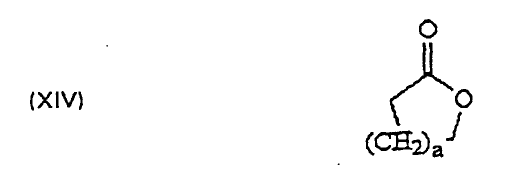 where a = 10 to 16,
and which comprises the steps:
1) cleavage of the lactone ring with a trimethylsilyl halide to give the corresponding trimethylsilyl halo-carboxylate,
2) simultaneous or subsequent alcoholysis of the trimethylsilyl halo-carboxylate to give the corresponding halo-carboxylic ester,
3) reaction of the halo-carboxylic ester with triphenylphosphane to give the corresponding phosphonium salt,
4) reaction of the phosphonium salt with an aldehyde using a base and subsequent hydrolysis to give a corresponding (Z)-fatty acid salt,
5) liberation of the (Z)-fatty acid from the (Z)-fatty acid salt, and
6) where appropriate conversion of the (Z)-fatty acid into the corresponding (Z)-alkenol using lithium aluminium hydride.

31. The process according to Claim 30, **characterized in that** the (Z)-fatty acid is 15-(Z)-tetracosenoic acid, in which case cyclopentadecanolide is used as starting lactone, and pelargonaldehyde is used as the aldehyde in step 4.

32. Compound of the general formula (I) according to any of Claims 1 to 13, 23 and 24 for use as cytostatic active ingredient.

33. Compound of the general formula (I) according to any of Claims 1 to 13, 23 and 24 for use as active ingredient against protozoal infections such as, for example, leishmaniosis and trypanosomiasis.

34. Compound of the general formula (I) according to any of Claims 1 to 13 and 14 to 22 for use as liposome shell constituent.

35. The use of a compound of the general formula (I) according to any of Claims 1 to 13 and 18 to 22 as solubilizer for active ingredients insoluble in water.

36. Liposomes according to Claim 28 for use as gene transport vehicles.

37. Liposomes according to Claim 26 for use as antitumour compositions, where the active ingredient is doxorubicin.

38. Liposomes according to Claim 26 for use as compositions for influencing the proliferation of cells, where the active ingredient is a cytokine.

## Revendications

1. Composé de formule générale (I)
(I) A - PO₃- B
où B représente un groupement de formule générale (II) 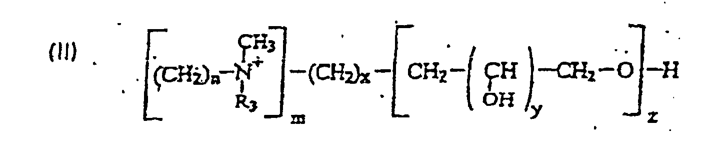 où
n est un nombre entier de 2 à 8 ;
m est 0, 1 ou 2 ;
x est un nombre entier de 0 à 8 ;
y est un nombre entier de 1 à 4 ;
z est un nombre entier de 0 à 5 ;
R₃ représente un groupement alkyle ayant 1 à 3 atomes de carbone, qui peut être substitué avec un ou plusieurs groupes hydroxyle ;
et où A représente un groupement choisi panni l'une des formules (III) à (VII) : 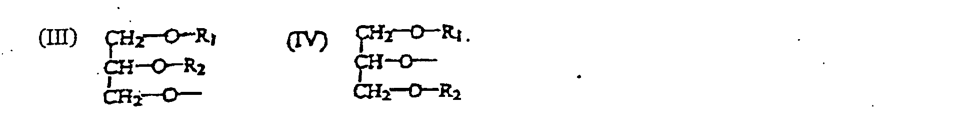 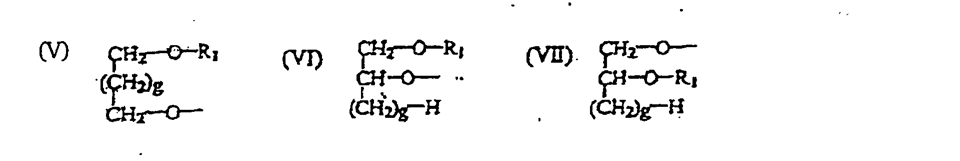 où
g est un nombre entier de 0 à 8 ;
p, q, r, s ≥ 0;
12 ≤ p + q ≤ 30 et
8 ≤ s + t + r ≤ 26;
on R₁ et R₂ représentent chacun indépendamment l'hydrogène, un groupement acyle ou alkyle saturé ou insaturé ou un groupement choisi parmi l'une des formules (X), (XI), (XII) et (XIII) et l'un au moins de R₁ et R₂ représente un groupement choisi parmi l'une des formules (X), (XI), (XII) et (XIII) : 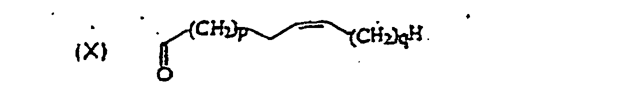 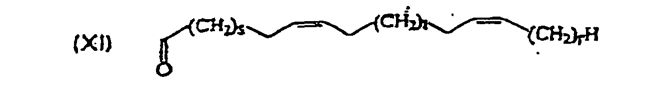 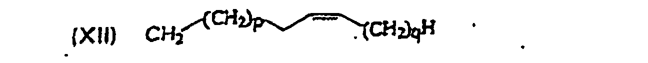 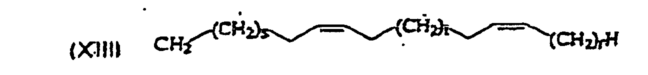 où q ≠8 pour p + q = 14, 16, 18 ou 20, quand aucun des groupements R₁ et R₂ ne représente un groupement de formule (XI) ou (XIII),
t ≥0 et p est différent de la signification de p dans les groupements naturels correspondants.

2. Composé selon la revendication 1 où
pour B :
m = 1.

3. Composé selon la revendication 2 où
pour B :
m = 1 ;
x = 1 à 3 ;
z = 0.

4. Composé selon la revendication 3 où
pour B :
m = 1 ;
x = 1 ;
Z = 0.

5. Composé selon la revendication 1 où
pour B :
m = 1 ;
x = 0 ;
y = 1 ;
z = 1 à 5.

6. Composé selon la revendication 5 où
pour B :
m = 1 ;
x = 0 ;
y = 1 ;
z = 1 à 3.

7. Composé selon la revendication 1 où
pour B :
m = 1 ;
x = 0 ;
y = 2 à 4 ;
z = 1.

8. Composé selon la revendication 1 où
pour B :
m = 0 ;
x = 0 ;
y = 1 ;
z = 1 à 5.

9. Composé selon la revendication 1 où
pour B :
m = 0 ;
x = 0 ;
y = 2 à 4 ;
z = 1.

10. Composé selon l'une des revendications précédentes où
pour B :
R₃ = CH₃.

11. Composé selon l'une des revendications 1 à 9 où
pour B :
R₃ = 1,2-dihydroxypropyle.

12. Composé selon l'une des revendications précédentes où
pour B :
n = 2 à 6.

13. Composé selon l'une des revendications précédentes où
pour B :
n = 3.

14. Composé selon l'une des revendications 1 à 13 où A représente un groupement choisi parmi l'une des formules (III) à (VII) et R₁ et R₂ représentent chacun indépendamment un groupement choisi parmi l'une des formules (X) à (XIII).

15. Composé selon la revendication 14 où
pour B :
x = 1 et z = 0.

16. Composé selon la revendication 14 ou 15 où A représente un groupement de formule (III) ou (IV) et R₁ et R₂ représentent chacun indépendamment un groupement choisi parmi l'une des formules (X) à (XIII), où l'un de R₁ et R₂ comporte 16 à 32 atomes de carbone et l'un de R₁ et R₂ comporte 16 à 26 atomes de carbone.

17. Composé selon la revendication 14 ou 15 où A représente un groupement de formule (III) ou (IV) et R₁ et R₂ représentent l'un et l'autre un groupement choisi parmi l'une des formules (X) à (XIII) et comportent 16 à 26 atomes de carbone.

18. Composé selon la revendication 14 ou 15 où A représente un groupement de formule (III) ou (IV) et R₁ et R₂ représentent chacun indépendamment un groupement des formules (X) à (XIII) et comportent 16 à 24 atomes de carbone.

19. Composé selon l'une des revendications 14 à 18 où R₁ et R₂ représentent chacun indépendamment un groupement de formule (X) ou (XI).

20. Composé selon l'une des revendications 14 à 18 où R₁ et R₂ représentent chacun indépendamment un groupement de formule (XII) ou (XIII).

21. Composé selon la revendication 14, 15, 17 ou 19 où R₁ et R₂ représentent l'un et l'autre un groupement de formule (XI).

22. Composé selon la revendication 14, 15, 17 ou 20 où R₁ et R₂ représentent l'un et l'autre un groupement de formule (XIII).

23. Composé selon la revendication 14 ou 15 où A représente un groupement de formule (III) ou (IV) et l'un de R₁ et R₂ représente un groupement alkyle ayant 1 à 4 atomes de carbone.

24. Composé selon la revendication 14 ou 15 où A représente un groupement choisi parmi l'une des formules (III) ou (IV) et l'un de R₁ et R₂ représente un groupement hydrogène.

25. Liposomes **caractérisés en ce qu'**ils comprennent comme constituants de l'enveloppe des liposomes des phospholipides et/ou des alkylphospholipides, éventuellement du cholestérol et 1 à 50 mol% d'un composé selon l'une des revendications 1, 14 à 22 ou un sel de celui-ci, où le cholestérol, les phospholipides, les alkylphospholipides et le composé représentent ensemble 100 mol% des constituants de l'enveloppe des liposomes.

26. Liposomes selon la revendication 25 **caractérisés en ce qu'**ils contiennent en outre un principe actif éventuellement en même temps que des diluants, adjuvants, supports et charges pharmaceutiquement acceptables.

27. Liposomes selon la revendication 26 **caractérisés en ce que** le principe actif est un composé selon l'une des revendications 1 et 23 à 24.

28. Liposomes selon l'une des revendications 25 à 27 **caractérisés en ce qu'**ils comprennent en outre un acide nucléique.

29. Composition pharmaceutique **caractérisée en ce qu'**elle contient un principe actif selon l'une des revendications 1 et 23 à 25 éventuellement en même temps que des diluants, adjuvants, supports et charges phannaceutiquement acceptables.

30. Procédé de production d'acides gras insaturés (Z) ou d'alcénols (Z) correspondant à un groupement selon l'une des formules (X) et (XI) ayant 16 à 34 atomes de carbone, **caractérisé en ce que** l'on utilise comme produit de départ une lactone de formule (XIV): 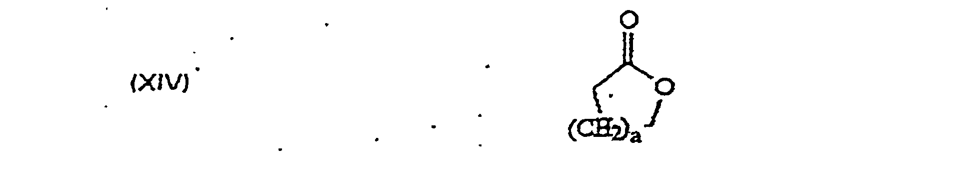 où a = 10 à 16,
et **en ce qu'**il comprend les étapes :
1) clivage du cycle lactone avec un halogénure de triméthylsilyle en le triméthylsilylester d'acide halogénocarboxylique correspondant,
2) alcoolyse simultanée ou successive du triméthylsilylester d'acide. halogénocarboxylique en l'ester d'acide halogénocarboxylique correspondant,
3) conversion de l'ester d'acide halogénocarboxylique avec le triphénylphosphane en le sel de phosphonium correspondant,
4) réaction du sel de phosphonium avec un aldéhyde avec utilisation d'une base puis saponification en un sel d'acide gras (Z) correspondant,
5) libération de l'acide gras (Z) à partir du sel d'acide gras (Z),
6) éventuellement conversion de l'acide gras (Z) en l'alcénol (Z) correspondant au moyen de l'hydrure de lithium et d'aluminium.

31. Procédé selon la revendication 30 **caractérisé en ce que** l'acide gras (Z) est l'acide 15-(Z)-tétracosénoïque, où le cyclopentadécanolide est utilisé comme lactone de départ et l'aldéhyde pélargonique est utilisé comme aldéhyde dans l'étape 4.

32. Composé de formule générale (I) selon l'une des revendications 1 à 13, 23 et 24 destiné à être utilisé comme principe actif cytostatique.

33. Composé de formule générale (I) selon l'une des revendications 1 à 13, 23 et 24 destiné à être utilisé comme principe actif contre les protozooses comme la leishmaniose et la trypanosomiase par exemple.

34. Composé de formule générale (I) selon l'une des revendications 1 à 13 et 14 à 22 destiné à être utilisé comme constituant d'enveloppe de liposomes.

35. Utilisation d'un composé de formule générale (I) selon l'une des revendications 1 à 13 et 18 à 22 comme promoteur de dissolution pour des principes actifs insolubles dans l'eau.

36. Liposomes selon la revendication 28 destinés à être utilisés comme véhicules de transport de gènes.

37. Liposomes selon la revendication 26 destinés à être utilisés comme agents antitumoraux, où le principe actif est la doxorubicine.

38. Liposomes selon la revendication 26 destinés à être utilisés comme agents pour influencer la prolifération cellulaire, où le principe actif est une cytokine.
